# EUROPEAN PATENT APPLICATION

(11) **EP 2 192 181 A1**
(43) Date of publication of application: **02.06.2010**
(21) Application number: 08792114.4
(22) Date of filing: 01.08.2008
(51) Int. Cl.: C12N 15/09, C12Q 1/02, G01N 33/15, G01N 33/50

(54) **SCREENING METHOD UTILIZING NOVEL SUBSTRATE C-RET FOR GAMMA-SECRETASE**

(30) Priority: 01.08.2007 US 953230 P
(71) Applicant: Eisai R&D Management Co., Ltd., Tokyo 112-8088 (JP)
(72) Inventor: INOUE, Eiji, Kobe-shi Hyogo 650-0047 (JP)
(74) Representative: Woods, Geoffrey Corlett
(86) International application number: PCT/JP2008/063901
(87) International publication number: WO 2009/017234

(57) **Abstract**

The present invention provides a method of screening for compounds which affect the processing of c-Ret by γ-secretase, comprising the following steps:
(i) contacting a first biological composition containing γ-secretase or a biologically active fragment thereof with a second biological composition containing c-Ret in the presence and absence of a candidate compound;
(ii) measuring the cleavage of the c-Ret in the presence and absence of the candidate compound;
(iii) selecting those candidate compounds which affect the cleavage of the c-Ret by γ-secretase; and
(iv) identifying the candidate compounds selected in step (iii) as compounds which affect the processing of c-Ret by γ-secretase.

SEQUENCE LISTING

<110> Eisai R&D Management Co., Ltd.

<120> A method of screening using a novel substrate c-Ret of
      gamma-secretase

<130> G09-0095EP

<150> US 60/953,230
<151> 2007-08-01

<160> 34

<170> PatentIn version 3.4

<210> 1
<211> 3345
<212> DNA
<213> Homo sapiens

<220>
<221> CDS
<222> (1)..(3342)

<400> 1
atg gcg aag gcg acg tcc ggt gcc gcg ggg ctg cgt ctg ctg ttg ctg      48
ctg ctg ctg ccg ctg cta ggc aaa gtg gca ttg ggc ctc tac ttc tcg      96
agg gat gct tac tgg gag aag ctg tat gtg gac cag gcg gcc ggc acg      144
ccc ttg ctg tac gtc cat gcc ctg cgg gac gcc cct gag gag gtg ccc      192
agc ttc cgc ctg ggc cag cat ctc tac ggc acg tac cgc aca cgg ctg      240
cat gag aac aac tgg atc tgc atc cag gag gac acc ggc ctc ctc tac      288
ctt aac cgg agc ctg gac cat agc tcc tgg gag aag ctc agt gtc cgc 336
aac cgc ggc ttt ccc ctg ctc acc gtc tac ctc aag gtc ttc ctg tca 384
ccc aca tcc ctt cgt gag ggc gag tgc cag tgg cca ggc tgt gcc cgc 432
gta tac ttc tcc ttc ttc aac acc tcc ttt cca gcc tgc agc tcc ctc 480
aag ccc cgg gag ctc tgc ttc cca gag aca agg ccc tcc ttc cgc att 528
cgg gag aac cga ccc cca ggc acc ttc cac cag ttc cgc ctg ctg cct 576
gtg cag ttc ttg tgc ccc aac atc agc gtg gcc tac agg ctc ctg gag 624
ggt gag ggt ctg ccc ttc cgc tgc gcc ccg gac agc ctg gag gtg agc 672
acg cgc tgg gcc ctg gac cgc gag cag cgg gag aag tac gag ctg gtg 720
gcc gtg tgc acc gtg cac gcc ggc gcg cgc gag gag gtg gtg atg gtg 768
ccc ttc ccg gtg acc gtg tac gac gag gac gac tcg gcg ccc acc ttc 816
ccc gcg ggc gtc gac acc gcc agc gcc gtg gtg gag ttc aag cgg aag 864
gag gac acc gtg gtg gcc acg ctg cgt gtc ttc gat gca gac gtg gta 912
cct gca tca ggg gag ctg gtg agg cgg tac aca agc acg ctg ctc ccc 960
ggg gac acc tgg gcc cag cag acc ttc cgg gtg gaa cac tgg ccc aac 1008
gag acc tcg gtc cag gcc aac ggc agc ttc gtg cgg gcg acc gta cat 1056
gac tat agg ctg gtt ctc aac cgg aac ctc tcc atc tcg gag aac cgc 1104
acc atg cag ctg gcg gtg ctg gtc aat gac tca gac ttc cag ggc cca 1152
gga gcg ggc gtc ctc ttg ctc cac ttc aac gtg tcg gtg ctg ccg gtc 1200
agc ctg cac ctg ccc agt acc tac tcc ctc tcc gtg agc agg agg gct 1248
cgc cga ttt gcc cag atc ggg aaa gtc tgt gtg gaa aac tgc cag gca 1296
ttc agt ggc atc aac gtc cag tac aag ctg cat tcc tct ggt gcc aac 1344
tgc agc acg cta ggg gtg gtc acc tca gcc gag gac acc tcg ggg atc 1392
ctg ttt gtg aat gac acc aag gcc ctg cgg cgg ccc aag tgt gcc gaa 1440
ctt cac tac atg gtg gtg gcc acc gac cag cag acc tct agg cag gcc 1488
cag gcc cag ctg ctt gta aca gtg gag ggg tca tat gtg gcc gag gag 1536
gcg ggc tgc ccc ctg tcc tgt gca gtc agc aag aga cgg ctg gag tgt      1584
gag gag tgt ggc ggc ctg ggc tcc cca aca ggc agg tgt gag tgg agg      1632
caa gga gat ggc aaa ggg atc acc agg aac ttc tcc acc tgc tct ccc      1680
agc acc aag acc tgc ccc gac ggc cac tgc gat gtt gtg gag acc caa      1728
gac atc aac att tgc cct cag gac tgc ctc cgg ggc agc att gtt ggg      1776
gga cac gag cct ggg gag ccc cgg ggg att aaa gct ggc tat ggc acc      1824
tgc aac tgc ttc cct gag gag gag aag tgc ttc tgc gag ccc gaa gac      1872
atc cag gat cca ctg tgc gac gag ctg tgc cgc acg gtg atc gca gcc      1920
gct gtc ctc ttc tcc ttc atc gtc tcg gtg ctg ctg tct gcc ttc tgc      1968
atc cac tgc tac cac aag ttt gcc cac aag cca ccc atc tcc tca gct      2016
gag atg acc ttc cgg agg ccc gcc cag gcc ttc ccg gtc agc tac tcc      2064
tct tcc ggt gcc cgc cgg ccc tcg ctg gac tcc atg gag aac cag gtc      2112
tcc gtg gat gcc ttc aag atc ctg gag gat cca aag tgg gaa ttc cct      2160
cgg aag aac ttg gtt ctt gga aaa act cta gga gaa ggc gaa ttt gga     2208
aaa gtg gtc aag gca acg gcc ttc cat ctg aaa ggc aga gca ggg tac     2256
acc acg gtg gcc gtg aag atg ctg aaa gag aac gcc tcc ccg agt gag     2304
ctt cga gac ctg ctg tca gag ttc aac gtc ctg aag cag gtc aac cac     2352
cca cat gtc atc aaa ttg tat ggg gcc tgc agc cag gat ggc ccg ctc     2400
ctc ctc atc gtg gag tac gcc aaa tac ggc tcc ctg cgg ggc ttc ctc     2448
cgc gag agc cgc aaa gtg ggg cct ggc tac ctg ggc agt gga ggc agc     2496
cgc aac tcc agc tcc ctg gac cac ccg gat gag cgg gcc ctc acc atg     2544
ggc gac ctc atc tca ttt gcc tgg cag atc tca cag ggg atg cag tat     2592
ctg gcc gag atg aag ctc gtt cat cgg gac ttg gca gcc aga aac atc     2640
ctg gta gct gag ggg cgg aag atg aag att tcg gat ttc ggc ttg tcc     2688
cga gat gtt tat gaa gag gat tcc tac gtg aag agg agc cag ggt cgg     2736
att cca gtt aaa tgg atg gca att gaa tcc ctt ttt gat cat atc tac     2784
acc acg caa agt gat gta tgg tct ttt ggt gtc ctg ctg tgg gag atc     2832
gtg acc cta ggg gga aac ccc tat cct ggg att cct cct gag cgg ctc     2880
ttc aac ctt ctg aag acc ggc cac cgg atg gag agg cca gac aac tgc     2928
agc gag gag atg tac cgc ctg atg ctg caa tgc tgg aag cag gag ccg     2976
gac aaa agg ccg gtg ttt gcg gac  atc agc aaa gac ctg  gag aag atg     3024
atg gtt  aag agg aga gac tac  ttg gac ctt gcg gcg  tcc act cca     3069
tct gac  tcc ctg att tat gac  gac ggc ctc tca gag  gag gag aca     3114
ccg ctg  gtg gac tgt aat aat  gcc ccc ctc cct cga  gcc ctc cct     3159
tcc aca  tgg att gaa aac aaa  ctc tat ggc atg tca  gac ccg aac     3204
tgg cct  gga gag agt cct gta  cca ctc acg aga gct  gat ggc act     3249
aac act  ggg ttt cca aga tat  cca aat gat agt gta  tat gct aac     3294
tgg atg  ctt tca ccc tca gcg  gca aaa tta atg gac  acg ttt gat     3339
agt taa                                                    3345

<210>  2
<211>  1114
<212>  PRT
<213>  Homo sapiens

<400>  2

<210> 3
<211> 2583
<212> DNA
<213> Homo sapiens

<220>
<221> CDS
<222> (1)..(2580)

<400> 3
atg gtg ccc ttc ccg gtg acc gtg tac gac gag gac gac tcg gcg ccc     48
Met Val Pro Phe Pro Val Thr Val Tyr Asp Glu Asp Asp Ser Ala Pro
1               5                   10                  15

acc ttc ccc gcg ggc gtc gac acc gcc agc gcc gtg gtg gag ttc aag     96
Thr Phe Pro Ala Gly Val Asp Thr Ala Ser Ala Val Val Glu Phe Lys
            20                  25                  30

cgg aag gag gac acc gtg gtg gcc acg ctg cgt gtc ttc gat gca gac     144
Arg Lys Glu Asp Thr Val Val Ala Thr Leu Arg Val Phe Asp Ala Asp
        35                  40                  45

gtg gta cct gca tca ggg gag ctg gtg agg cgg tac aca agc acg ctg     192
Val Val Pro Ala Ser Gly Glu Leu Val Arg Arg Tyr Thr Ser Thr Leu
    50                  55                  60

ctc ccc ggg gac acc tgg gcc cag cag acc ttc cgg gtg gaa cac tgg     240
Leu Pro Gly Asp Thr Trp Ala Gln Gln Thr Phe Arg Val Glu His Trp
65                  70                  75                  80

ccc aac gag acc tcg gtc cag gcc aac ggc agc ttc gtg cgg gcg acc     288
Pro Asn Glu Thr Ser Val Gln Ala Asn Gly Ser Phe Val Arg Ala Thr
                85                  90                  95

gta cat gac tat agg ctg gtt ctc aac cgg aac ctc tcc atc tcg gag     336
Val His Asp Tyr Arg Leu Val Leu Asn Arg Asn Leu Ser Ile Ser Glu
            100                 105                 110

aac cgc acc atg cag ctg gcg gtg ctg gtc aat gac tca gac ttc cag     384
Asn Arg Thr Met Gln Leu Ala Val Leu Val Asn Asp Ser Asp Phe Gln
        115                 120                 125

ggc cca gga gcg ggc gtc ctc ttg ctc cac ttc aac gtg tcg gtg ctg     432
Gly Pro Gly Ala Gly Val Leu Leu Leu His Phe Asn Val Ser Val Leu
    130                 135                 140

ccg gtc agc ctg cac ctg ccc agt acc tac tcc ctc tcc gtg agc agg     480
Pro Val Ser Leu His Leu Pro Ser Thr Tyr Ser Leu Ser Val Ser Arg
145                 150                 155                 160

agg gct cgc cga ttt gcc cag atc ggg aaa gtc tgt gtg gaa aac tgc     528
Arg Ala Arg Arg Phe Ala Gln Ile Gly Lys Val Cys Val Glu Asn Cys
                165                 170                 175

cag gcg ttc agt ggc atc aac gtc cag tac aag ctg cat tcc tct ggt     576
Gln Ala Phe Ser Gly Ile Asn Val Gln Tyr Lys Leu His Ser Ser Gly
            180                 185                 190

gcc aac tgc agc acg cta ggg gtg gtc acc tca gcc gag gac acc tcg     624

Ala Asn Cys Ser Thr Leu Gly Val Val Thr Ser Ala Glu Asp Thr Ser
        195                 200                 205

ggg atc ctg ttt gtg aat gac acc aag gcc ctg cgg cgg ccc aag tgt        672
Gly Ile Leu Phe Val Asn Asp Thr Lys Ala Leu Arg Arg Pro Lys Cys
    210                 215                 220

gcc gaa ctt cac tac atg gtg gtg gcc acc gac cag cag acc tct agg        720
Ala Glu Leu His Tyr Met Val Val Ala Thr Asp Gln Gln Thr Ser Arg
225                 230                 235                 240

cag gcc cag gcc cag ctg ctt gta aca gtg gag ggg tca tat gtg gcc        768
Gln Ala Gln Ala Gln Leu Leu Val Thr Val Glu Gly Ser Tyr Val Ala
                245                 250                 255

gag gag gcg ggc tgc ccc ctg tcc tgt gca gtc agc aag aga cgg ctg        816
Glu Glu Ala Gly Cys Pro Leu Ser Cys Ala Val Ser Lys Arg Arg Leu
            260                 265                 270

gag tgt gag gag tgt ggc ggc ctg ggc tcc cca aca ggc agg tgt gag        864
Glu Cys Glu Glu Cys Gly Gly Leu Gly Ser Pro Thr Gly Arg Cys Glu
        275                 280                 285

tgg agg caa gga gat ggc aaa ggg atc acc agg aac ttc tcc acc tgc        912
Trp Arg Gln Gly Asp Gly Lys Gly Ile Thr Arg Asn Phe Ser Thr Cys
    290                 295                 300

tct ccc agc acc aag acc tgc ccc gac ggc cac tgc gat gtt gtg gag        960
Ser Pro Ser Thr Lys Thr Cys Pro Asp Gly His Cys Asp Val Val Glu
305                 310                 315                 320

acc caa gac atc aac att tgc cct cag gac tgc ctc cgg ggc agc att       1008
Thr Gln Asp Ile Asn Ile Cys Pro Gln Asp Cys Leu Arg Gly Ser Ile
                325                 330                 335

gtt ggg gga cac gag cct ggg gag ccc cgg ggg att aaa gct ggc tat       1056
Val Gly Gly His Glu Pro Gly Glu Pro Arg Gly Ile Lys Ala Gly Tyr
            340                 345                 350

ggc acc tgc aac tgc ttc cct gag gag gag aag tgc ttc tgc gag ccc       1104
Gly Thr Cys Asn Cys Phe Pro Glu Glu Glu Lys Cys Phe Cys Glu Pro
        355                 360                 365

gaa gac atc cag gat cca ctg tgc gac gag ctg tgc cgc acg gtg atc       1152
Glu Asp Ile Gln Asp Pro Leu Cys Asp Glu Leu Cys Arg Thr Val Ile
    370                 375                 380

gca gcc gct gtc ctc ttc tcc ttc atc gtc tcg gtg ctg ctg tct gcc       1200
Ala Ala Ala Val Leu Phe Ser Phe Ile Val Ser Val Leu Leu Ser Ala
385                 390                 395                 400

ttc tgc atc cac tgc tac cac aag ttt gcc cac aag cca ccc atc tcc 1248
Phe Cys Ile His Cys Tyr His Lys Phe Ala His Lys Pro Pro Ile Ser
                405                 410                 415

tca gct gag atg acc ttc cgg agg ccc gcc cag gcc ttc ccg gtc agc 1296
Ser Ala Glu Met Thr Phe Arg Arg Pro Ala Gln Ala Phe Pro Val Ser
            420                 425                 430

tac tcc tct tcc ggt gcc cgc cgg ccc tcg ctg gac tcc atg gag aac 1344
Tyr Ser Ser Ser Gly Ala Arg Arg Pro Ser Leu Asp Ser Met Glu Asn
        435                 440                 445

cag gtc tcc gtg gat gcc ttc aag atc ctg gag gat cca aag tgg gaa 1392
Gln Val Ser Val Asp Ala Phe Lys Ile Leu Glu Asp Pro Lys Trp Glu
    450                 455                 460

ttc cct cgg aag aac ttg gtt ctt gga aaa act cta gga gaa ggc gaa 1440
Phe Pro Arg Lys Asn Leu Val Leu Gly Lys Thr Leu Gly Glu Gly Glu
465                 470                 475                 480

ttt gga aaa gtg gtc aag gca acg gcc ttc cat ctg aaa ggc aga gca 1488
Phe Gly Lys Val Val Lys Ala Thr Ala Phe His Leu Lys Gly Arg Ala
                485                 490                 495

ggg tac acc acg gtg gcc gtg aag atg ctg aaa gag aac gcc tcc ccg 1536
Gly Tyr Thr Thr Val Ala Val Lys Met Leu Lys Glu Asn Ala Ser Pro
            500                 505                 510

agt gag ctt cga gac ctg ctg tca gag ttc aac gtc ctg aag cag gtc 1584
Ser Glu Leu Arg Asp Leu Leu Ser Glu Phe Asn Val Leu Lys Gln Val
        515                 520                 525

aac cac cca cat gtc atc aaa ttg tat ggg gcc tgc agc cag gat ggc 1632
Asn His Pro His Val Ile Lys Leu Tyr Gly Ala Cys Ser Gln Asp Gly
    530                 535                 540

ccg ctc ctc ctc atc gtg gag tac gcc aaa tac ggc tcc ctg cgg ggc 1680
Pro Leu Leu Leu Ile Val Glu Tyr Ala Lys Tyr Gly Ser Leu Arg Gly
545                 550                 555                 560

ttc ctc cgc gag agc cgc aaa gtg ggg cct ggc tac ctg ggc agt gga 1728
Phe Leu Arg Glu Ser Arg Lys Val Gly Pro Gly Tyr Leu Gly Ser Gly
                565                 570                 575

ggc agc cgc aac tcc agc tcc ctg gac cac ccg gat gag cgg gcc ctc 1776
Gly Ser Arg Asn Ser Ser Ser Leu Asp His Pro Asp Glu Arg Ala Leu
            580                 585                 590

acc atg ggc gac ctc atc tca ttt gcc tgg cag atc tca cag ggg atg 1824
Thr Met Gly Asp Leu Ile Ser Phe Ala Trp Gln Ile Ser Gln Gly Met
        595                 600                 605

cag tat ctg gcc gag atg aag ctc gtt cat cgg gac ttg gca gcc aga 1872
Gln Tyr Leu Ala Glu Met Lys Leu Val His Arg Asp Leu Ala Ala Arg
    610                 615                 620

aac atc ctg gta gct gag ggg cgg aag atg aag att tcg gat ttc ggc 1920
Asn Ile Leu Val Ala Glu Gly Arg Lys Met Lys Ile Ser Asp Phe Gly
625                 630                 635                 640

ttg tcc cga gat gtt tat gaa gag gat tcc tac gtg aag agg agc cag 1968
Leu Ser Arg Asp Val Tyr Glu Glu Asp Ser Tyr Val Lys Arg Ser Gln
                645                 650                 655

ggt cgg att cca gtt aaa tgg atg gca att gaa tcc ctt ttt gat cat 2016
Gly Arg Ile Pro Val Lys Trp Met Ala Ile Glu Ser Leu Phe Asp His
            660                 665                 670

atc tac acc acg caa agt gat gta tgg tct ttt ggt gtc ctg ctg tgg 2064
Ile Tyr Thr Thr Gln Ser Asp Val Trp Ser Phe Gly Val Leu Leu Trp
        675                 680                 685

gag atc gtg acc cta ggg gga aac ccc tat cct ggg att cct cct gag 2112
Glu Ile Val Thr Leu Gly Gly Asn Pro Tyr Pro Gly Ile Pro Pro Glu
    690                 695                 700

cgg ctc ttc aac ctt ctg aag acc ggc cac cgg atg gag agg cca gac 2160
Arg Leu Phe Asn Leu Leu Lys Thr Gly His Arg Met Glu Arg Pro Asp
705                 710                 715                 720

aac tgc agc gag gag atg tac cgc ctg atg ctg caa tgc tgg aag cag 2208
Asn Cys Ser Glu Glu Met Tyr Arg Leu Met Leu Gln Cys Trp Lys Gln
                725                 730                 735

gag ccg gac aaa agg ccg gtg ttt gcg gac atc agc aaa gac ctg gag 2256
Glu Pro Asp Lys Arg Pro Val Phe Ala Asp Ile Ser Lys Asp Leu Glu
            740                 745                 750

aag atg atg gtt aag agg aga gac tac ttg gac ctt gcg gcg tcc act 2304
Lys Met Met Val Lys Arg Arg Asp Tyr Leu Asp Leu Ala Ala Ser Thr
        755                 760                 765

cca tct gac tcc ctg att tat gac gac ggc ctc tca gag gag gag aca 2352
Pro Ser Asp Ser Leu Ile Tyr Asp Asp Gly Leu Ser Glu Glu Glu Thr
    770                 775                 780

ccg ctg gtg gac tgt aat aat gcc ccc ctc cct cga gcc ctc cct tcc 2400
Pro Leu Val Asp Cys Asn Asn Ala Pro Leu Pro Arg Ala Leu Pro Ser
785                 790                 795                 800

aca tgg att gaa aac aaa ctc tat ggc atg tca gac ccg aac tgg cct 2448
Thr Trp Ile Glu Asn Lys Leu Tyr Gly Met Ser Asp Pro Asn Trp Pro

805                 810                 815

gga gag agt cct gta cca ctc acg aga gct gat ggc act aac act ggg        2496
Gly Glu Ser Pro Val Pro Leu Thr Arg Ala Asp Gly Thr Asn Thr Gly
            820                 825                 830

ttt cca aga tat cca aat gat agt gta tat gct aac tgg atg ctt tca        2544
Phe Pro Arg Tyr Pro Asn Asp Ser Val Tyr Ala Asn Trp Met Leu Ser
        835                 840                 845

ccc tca gcg gca aaa tta atg gac acg ttt gat agt taa                    2583
Pro Ser Ala Ala Lys Leu Met Asp Thr Phe Asp Ser
    850                 855                 860

<210>  4
<211>  860
<212>  PRT
<213>  Homo sapiens

<400>  4
   Met Val Pro Phe Pro Val Thr Val Tyr Asp Glu Asp Asp Ser Ala Pro
   1               5                   10                  15
   Thr Phe Pro Ala Gly Val Asp Thr Ala Ser Ala Val Val Glu Phe Lys
               20                  25                  30
   Arg Lys Glu Asp Thr Val Val Ala Thr Leu Arg Val Phe Asp Ala Asp
           35                  40                  45
   Val Val Pro Ala Ser Gly Glu Leu Val Arg Arg Tyr Thr Ser Thr Leu
       50                  55                  60
   Leu Pro Gly Asp Thr Trp Ala Gln Gln Thr Phe Arg Val Glu His Trp
   65                  70                  75                  80
   Pro Asn Glu Thr Ser Val Gln Ala Asn Gly Ser Phe Val Arg Ala Thr
                   85                  90                  95
   Val His Asp Tyr Arg Leu Val Leu Asn Arg Asn Leu Ser Ile Ser Glu
               100                 105                 110
   Asn Arg Thr Met Gln Leu Ala Val Leu Val Asn Asp Ser Asp Phe Gln
   115                 120                 125
   Gly Pro Gly Ala Gly Val Leu Leu Leu His Phe Asn Val Ser Val Leu
       130                 135                 140
   Pro Val Ser Leu His Leu Pro Ser Thr Tyr Ser Leu Ser Val Ser Arg
   145                 150                 155                 160
   Arg Ala Arg Arg Phe Ala Gln Ile Gly Lys Val Cys Val Glu Asn Cys
                   165                 170                 175
   Gln Ala Phe Ser Gly Ile Asn Val Gln Tyr Lys Leu His Ser Ser Gly
               180                 185                 190
   Ala Asn Cys Ser Thr Leu Gly Val Val Thr Ser Ala Glu Asp Thr Ser
           195                 200                 205
   Gly Ile Leu Phe Val Asn Asp Thr Lys Ala Leu Arg Arg Pro Lys Cys
       210                 215                 220
   Ala Glu Leu His Tyr Met Val Val Ala Thr Asp Gln Gln Thr Ser Arg
   225                 230                 235                 240
   Gln Ala Gln Ala Gln Leu Leu Val Thr Val Glu Gly Ser Tyr Val Ala
                   245                 250                 255
   Glu Glu Ala Gly Cys Pro Leu Ser Cys Ala Val Ser Lys Arg Arg Leu
               260                 265                 270
   Glu Cys Glu Glu Cys Gly Gly Leu Gly Ser Pro Thr Gly Arg Cys Glu
           275                 280                 285
   Trp Arg Gln Gly Asp Gly Lys Gly Ile Thr Arg Asn Phe Ser Thr Cys
       290                 295                 300
   Ser Pro Ser Thr Lys Thr Cys Pro Asp Gly His Cys Asp Val Val Glu
   305                 310                 315                 320
   Thr Gln Asp Ile Asn Ile Cys Pro Gln Asp Cys Leu Arg Gly Ser Ile
                   325                 330                 335
   Val Gly Gly His Glu Pro Gly Glu Pro Arg Gly Ile Lys Ala Gly Tyr
               340                 345                 350
   Gly Thr Cys Asn Cys Phe Pro Glu Glu Glu Lys Cys Phe Cys Glu Pro
           355                 360                 365
   Glu Asp Ile Gln Asp Pro Leu Cys Asp Glu Leu Cys Arg Thr Val Ile
       370                 375                 380
   Ala Ala Ala Val Leu Phe Ser Phe Ile Val Ser Val Leu Leu Ser Ala
   385                 390                 395                 400
   Phe Cys Ile His Cys Tyr His Lys Phe Ala His Lys Pro Pro Ile Ser
                   405                 410                 415
   Ser Ala Glu Met Thr Phe Arg Arg Pro Ala Gln Ala Phe Pro Val Ser
               420                 425                 430
   Tyr Ser Ser Ser Gly Ala Arg Arg Pro Ser Leu Asp Ser Met Glu Asn
           435                 440                 445
   Gln Val Ser Val Asp Ala Phe Lys Ile Leu Glu Asp Pro Lys Trp Glu
       450                 455                 460
   Phe Pro Arg Lys Asn Leu Val Leu Gly Lys Thr Leu Gly Glu Gly Glu
   465                 470                 475                 480
   Phe Gly Lys Val Val Lys Ala Thr Ala Phe His Leu Lys Gly Arg Ala
                   485                 490                 495
   Gly Tyr Thr Thr Val Ala Val Lys Met Leu Lys Glu Asn Ala Ser Pro
               500                 505                 510
   Ser Glu Leu Arg Asp Leu Leu Ser Glu Phe Asn Val Leu Lys Gln Val
           515                 520                 525
   Asn His Pro His Val Ile Lys Leu Tyr Gly Ala Cys Ser Gln Asp Gly
       530                 535                 540
   Pro Leu Leu Leu Ile Val Glu Tyr Ala Lys Tyr Gly Ser Leu Arg Gly
   545                 550                 555                 560
   Phe Leu Arg Glu Ser Arg Lys Val Gly Pro Gly Tyr Leu Gly Ser Gly
                   565                 570                 575
   Gly Ser Arg Asn Ser Ser Ser Leu Asp His Pro Asp Glu Arg Ala Leu
               580                 585                 590
   Thr Met Gly Asp Leu Ile Ser Phe Ala Trp Gln Ile Ser Gln Gly Met
           595                 600                 605
   Gln Tyr Leu Ala Glu Met Lys Leu Val His Arg Asp Leu Ala Ala Arg
       610                 615                 620
   Asn Ile Leu Val Ala Glu Gly Arg Lys Met Lys Ile Ser Asp Phe Gly
   625                 630                 635                 640
   Leu Ser Arg Asp Val Tyr Glu Glu Asp Ser Tyr Val Lys Arg Ser Gln
                   645                 650                 655
   Gly Arg Ile Pro Val Lys Trp Met Ala Ile Glu Ser Leu Phe Asp His
               660                 665                 670
   Ile Tyr Thr Thr Gln Ser Asp Val Trp Ser Phe Gly Val Leu Leu Trp
           675                 680                 685
   Glu Ile Val Thr Leu Gly Gly Asn Pro Tyr Pro Gly Ile Pro Pro Glu
       690                 695                 700
   Arg Leu Phe Asn Leu Leu Lys Thr Gly His Arg Met Glu Arg Pro Asp
   705                 710                 715                 720
   Asn Cys Ser Glu Glu Met Tyr Arg Leu Met Leu Gln Cys Trp Lys Gln
                   725                 730                 735
   Glu Pro Asp Lys Arg Pro Val Phe Ala Asp Ile Ser Lys Asp Leu Glu
               740                 745                 750
   Lys Met Met Val Lys Arg Arg Asp Tyr Leu Asp Leu Ala Ala Ser Thr
           755                 760                 765
   Pro Ser Asp Ser Leu Ile Tyr Asp Asp Gly Leu Ser Glu Glu Glu Thr
       770                 775                 780
   Pro Leu Val Asp Cys Asn Asn Ala Pro Leu Pro Arg Ala Leu Pro Ser
   785                 790                 795                 800
   Thr Trp Ile Glu Asn Lys Leu Tyr Gly Met Ser Asp Pro Asn Trp Pro
                   805                 810                 815
   Gly Glu Ser Pro Val Pro Leu Thr Arg Ala Asp Gly Thr Asn Thr Gly
               820                 825                 830
   Phe Pro Arg Tyr Pro Asn Asp Ser Val Tyr Ala Asn Trp Met Leu Ser
           835                 840                 845
   Pro Ser Ala Ala Lys Leu Met Asp Thr Phe Asp Ser
       850                 855                 860

<210>  5
<211>  3219
<212>  DNA
<213>  Homo sapiens

<220>
<221>  CDS
<222>  (1)..(3216)

<400>  5
atg gcg aag gcg acg tcc ggt gcc gcg ggg ctg cgt ctg ctg ttg ctg        48
Met Ala Lys Ala Thr Ser Gly Ala Ala Gly Leu Arg Leu Leu Leu Leu
1               5                   10                  15

ctg ctg ctg ccg ctg cta ggc aaa gtg gca ttg ggc ctc tac ttc tcg        96
Leu Leu Leu Pro Leu Leu Gly Lys Val Ala Leu Gly Leu Tyr Phe Ser

            20                  25                  30

agg gat gct tac tgg gag aag ctg tat gtg gac cag gcg gcc ggc acg 144
Arg Asp Ala Tyr Trp Glu Lys Leu Tyr Val Asp Gln Ala Ala Gly Thr
        35                  40                  45

ccc ttg ctg tac gtc cat gcc ctg cgg gac gcc cct gag gag gtg ccc 192
Pro Leu Leu Tyr Val His Ala Leu Arg Asp Ala Pro Glu Glu Val Pro
    50                  55                  60

agc ttc cgc ctg ggc cag cat ctc tac ggc acg tac cgc aca cgg ctg 240
Ser Phe Arg Leu Gly Gln His Leu Tyr Gly Thr Tyr Arg Thr Arg Leu
65                  70                  75                  80

cat gag aac aac tgg atc tgc atc cag gag gac acc ggc ctc ctc tac 288
His Glu Asn Asn Trp Ile Cys Ile Gln Glu Asp Thr Gly Leu Leu Tyr
                85                  90                  95

ctt aac cgg agc ctg gac cat agc tcc tgg gag aag ctc agt gtc cgc 336
Leu Asn Arg Ser Leu Asp His Ser Ser Trp Glu Lys Leu Ser Val Arg
            100                 105                 110

aac cgc ggc ttt ccc ctg ctc acc gtc tac ctc aag gtc ttc ctg tca 384
Asn Arg Gly Phe Pro Leu Leu Thr Val Tyr Leu Lys Val Phe Leu Ser
        115                 120                 125

ccc aca tcc ctt cgt gag ggc gag tgc cag tgg cca ggc tgt gcc cgc 432
Pro Thr Ser Leu Arg Glu Gly Glu Cys Gln Trp Pro Gly Cys Ala Arg
    130                 135                 140

gta tac ttc tcc ttc ttc aac acc tcc ttt cca gcc tgc agc tcc ctc 480
Val Tyr Phe Ser Phe Phe Asn Thr Ser Phe Pro Ala Cys Ser Ser Leu
145                 150                 155                 160

aag ccc cgg gag ctc tgc ttc cca gag aca agg ccc tcc ttc cgc att 528
Lys Pro Arg Glu Leu Cys Phe Pro Glu Thr Arg Pro Ser Phe Arg Ile
                165                 170                 175

cgg gag aac cga ccc cca ggc acc ttc cac cag ttc cgc ctg ctg cct 576
Arg Glu Asn Arg Pro Pro Gly Thr Phe His Gln Phe Arg Leu Leu Pro
            180                 185                 190

gtg cag ttc ttg tgc ccc aac atc agc gtg gcc tac agg ctc ctg gag 624
Val Gln Phe Leu Cys Pro Asn Ile Ser Val Ala Tyr Arg Leu Leu Glu
        195                 200                 205

ggt gag ggt ctg ccc ttc cgc tgc gcc ccg gac agc ctg gag gtg agc 672
Gly Glu Gly Leu Pro Phe Arg Cys Ala Pro Asp Ser Leu Glu Val Ser
    210                 215                 220

acg cgc tgg gcc ctg gac cgc gag cag cgg gag aag tac gag ctg gtg 720

Thr Arg Trp Ala Leu Asp Arg Glu Gln Arg Glu Lys Tyr Glu Leu Val
225                 230                 235                 240

gcc gtg tgc acc gtg cac gcc ggc gcg cgc gag gag gtg gtg atg gtg      768
Ala Val Cys Thr Val His Ala Gly Ala Arg Glu Glu Val Val Met Val
                245                 250                 255

ccc ttc ccg gtg acc gtg tac gac gag gac gac tcg gcg ccc acc ttc      816
Pro Phe Pro Val Thr Val Tyr Asp Glu Asp Asp Ser Ala Pro Thr Phe
            260                 265                 270

ccc gcg ggc gtc gac acc gcc agc gcc gtg gtg gag ttc aag cgg aag      864
Pro Ala Gly Val Asp Thr Ala Ser Ala Val Val Glu Phe Lys Arg Lys
        275                 280                 285

gag gac acc gtg gtg gcc acg ctg cgt gtc ttc gat gca gac gtg gta      912
Glu Asp Thr Val Val Ala Thr Leu Arg Val Phe Asp Ala Asp Val Val
    290                 295                 300

cct gca tca ggg gag ctg gtg agg cgg tac aca agc acg ctg ctc ccc      960
Pro Ala Ser Gly Glu Leu Val Arg Arg Tyr Thr Ser Thr Leu Leu Pro
305                 310                 315                 320

ggg gac acc tgg gcc cag cag acc ttc cgg gtg gaa cac tgg ccc aac      1008
Gly Asp Thr Trp Ala Gln Gln Thr Phe Arg Val Glu His Trp Pro Asn
                325                 330                 335

gag acc tcg gtc cag gcc aac ggc agc ttc gtg cgg gcg acc gta cat      1056
Glu Thr Ser Val Gln Ala Asn Gly Ser Phe Val Arg Ala Thr Val His
            340                 345                 350

gac tat agg ctg gtt ctc aac cgg aac ctc tcc atc tcg gag aac cgc      1104
Asp Tyr Arg Leu Val Leu Asn Arg Asn Leu Ser Ile Ser Glu Asn Arg
        355                 360                 365

acc atg cag ctg gcg gtg ctg gtc aat gac tca gac ttc cag ggc cca      1152
Thr Met Gln Leu Ala Val Leu Val Asn Asp Ser Asp Phe Gln Gly Pro
    370                 375                 380

gga gcg ggc gtc ctc ttg ctc cac ttc aac gtg tcg gtg ctg ccg gtc      1200
Gly Ala Gly Val Leu Leu Leu His Phe Asn Val Ser Val Leu Pro Val
385                 390                 395                 400

agc ctg cac ctg ccc agt acc tac tcc ctc tcc gtg agc agg agg gct      1248
Ser Leu His Leu Pro Ser Thr Tyr Ser Leu Ser Val Ser Arg Arg Ala
                405                 410                 415

cgc cga ttt gcc cag atc ggg aaa gtc tgt gtg gaa aac tgc cag gca      1296
Arg Arg Phe Ala Gln Ile Gly Lys Val Cys Val Glu Asn Cys Gln Ala
            420                 425                 430

ttc agt ggc atc aac gtc cag tac aag ctg cat tcc tct ggt gcc aac 1344
Phe Ser Gly Ile Asn Val Gln Tyr Lys Leu His Ser Ser Gly Ala Asn
        435                 440                 445

tgc agc acg cta ggg gtg gtc acc tca gcc gag gac acc tcg ggg atc 1392
Cys Ser Thr Leu Gly Val Val Thr Ser Ala Glu Asp Thr Ser Gly Ile
    450                 455                 460

ctg ttt gtg aat gac acc aag gcc ctg cgg cgg ccc aag tgt gcc gaa 1440
Leu Phe Val Asn Asp Thr Lys Ala Leu Arg Arg Pro Lys Cys Ala Glu
465                 470                 475                 480

ctt cac tac atg gtg gtg gcc acc gac cag cag acc tct agg cag gcc 1488
Leu His Tyr Met Val Val Ala Thr Asp Gln Gln Thr Ser Arg Gln Ala
                485                 490                 495

cag gcc cag ctg ctt gta aca gtg gag ggg tca tat gtg gcc gag gag 1536
Gln Ala Gln Leu Leu Val Thr Val Glu Gly Ser Tyr Val Ala Glu Glu
            500                 505                 510

gcg ggc tgc ccc ctg tcc tgt gca gtc agc aag aga cgg ctg gag tgt 1584
Ala Gly Cys Pro Leu Ser Cys Ala Val Ser Lys Arg Arg Leu Glu Cys
        515                 520                 525

gag gag tgt ggc ggc ctg ggc tcc cca aca ggc agg tgt gag tgg agg 1632
Glu Glu Cys Gly Gly Leu Gly Ser Pro Thr Gly Arg Cys Glu Trp Arg
    530                 535                 540

caa gga gat ggc aaa ggg atc acc agg aac ttc tcc acc tgc tct ccc 1680
Gln Gly Asp Gly Lys Gly Ile Thr Arg Asn Phe Ser Thr Cys Ser Pro
545                 550                 555                 560

agc acc aag acc tgc ccc gac ggc cac tgc gat gtt gtg gag acc caa 1728
Ser Thr Lys Thr Cys Pro Asp Gly His Cys Asp Val Val Glu Thr Gln
                565                 570                 575

gac atc aac att tgc cct cag gac tgc ctc cgg ggc agc att gtt ggg 1776
Asp Ile Asn Ile Cys Pro Gln Asp Cys Leu Arg Gly Ser Ile Val Gly
            580                 585                 590

gga cac gag cct ggg gag ccc cgg ggg att aaa gct ggc tat ggc acc 1824
Gly His Glu Pro Gly Glu Pro Arg Gly Ile Lys Ala Gly Tyr Gly Thr
        595                 600                 605

tgc aac tgc ttc cct gag gag gag aag tgc ttc tgc gag ccc gaa gac 1872
Cys Asn Cys Phe Pro Glu Glu Glu Lys Cys Phe Cys Glu Pro Glu Asp
    610                 615                 620

atc cag gat cca ctg tgc gac gag ctg tgc cgc acg gtg atc gca gcc 1920
Ile Gln Asp Pro Leu Cys Asp Glu Leu Cys Arg Thr Val Ile Ala Ala
625                 630                 635                 640

gct gtc ctc ttc tcc ttc atc gtc tcg gtg ctg ctg tct gcc ttc tgc 1968
Ala Val Leu Phe Ser Phe Ile Val Ser Val Leu Leu Ser Ala Phe Cys
                645                 650                 655

atc cac tgc tac cac aag ttt gcc cac aag cca ccc atc tcc tca gct 2016
Ile His Cys Tyr His Lys Phe Ala His Lys Pro Pro Ile Ser Ser Ala
            660                 665                 670

gag atg acc ttc cgg agg ccc gcc cag gcc ttc ccg gtc agc tac tcc 2064
Glu Met Thr Phe Arg Arg Pro Ala Gln Ala Phe Pro Val Ser Tyr Ser
        675                 680                 685

tct tcc ggt gcc cgc cgg ccc tcg ctg gac tcc atg gag aac cag gtc 2112
Ser Ser Gly Ala Arg Arg Pro Ser Leu Asp Ser Met Glu Asn Gln Val
    690                 695                 700

tcc gtg gat gcc ttc aag atc ctg gag gat cca aag tgg gaa ttc cct 2160
Ser Val Asp Ala Phe Lys Ile Leu Glu Asp Pro Lys Trp Glu Phe Pro
705                 710                 715                 720

cgg aag aac ttg gtt ctt gga aaa act cta gga gaa ggc gaa ttt gga 2208
Arg Lys Asn Leu Val Leu Gly Lys Thr Leu Gly Glu Gly Glu Phe Gly
                725                 730                 735

aaa gtg gtc aag gca acg gcc ttc cat ctg aaa ggc aga gca ggg tac 2256
Lys Val Val Lys Ala Thr Ala Phe His Leu Lys Gly Arg Ala Gly Tyr
            740                 745                 750

acc acg gtg gcc gtg aag atg ctg aaa gag aac gcc tcc ccg agt gag 2304
Thr Thr Val Ala Val Lys Met Leu Lys Glu Asn Ala Ser Pro Ser Glu
        755                 760                 765

ctt cga gac ctg ctg tca gag ttc aac gtc ctg aag cag gtc aac cac 2352
Leu Arg Asp Leu Leu Ser Glu Phe Asn Val Leu Lys Gln Val Asn His
    770                 775                 780

cca cat gtc atc aaa ttg tat ggg gcc tgc agc cag gat ggc ccg ctc 2400
Pro His Val Ile Lys Leu Tyr Gly Ala Cys Ser Gln Asp Gly Pro Leu
785                 790                 795                 800

ctc ctc atc gtg gag tac gcc aaa tac ggc tcc ctg cgg ggc ttc ctc 2448
Leu Leu Ile Val Glu Tyr Ala Lys Tyr Gly Ser Leu Arg Gly Phe Leu
                805                 810                 815

cgc gag agc cgc aaa gtg ggg cct ggc tac ctg ggc agt gga ggc agc 2496
Arg Glu Ser Arg Lys Val Gly Pro Gly Tyr Leu Gly Ser Gly Gly Ser
            820                 825                 830

cgc aac tcc agc tcc ctg gac cac ccg gat gag cgg gcc ctc acc atg 2544
Arg Asn Ser Ser Ser Leu Asp His Pro Asp Glu Arg Ala Leu Thr Met

835                 840                 845

ggc gac ctc atc tca ttt gcc tgg cag atc tca cag ggg atg cag tat      2592
Gly Asp Leu Ile Ser Phe Ala Trp Gln Ile Ser Gln Gly Met Gln Tyr
    850                 855                 860

ctg gcc gag atg aag ctc gtt cat cgg gac ttg gca gcc aga aac atc      2640
Leu Ala Glu Met Lys Leu Val His Arg Asp Leu Ala Ala Arg Asn Ile
865                 870                 875                 880

ctg gta gct gag ggg cgg aag atg aag att tcg gat ttc ggc ttg tcc      2688
Leu Val Ala Glu Gly Arg Lys Met Lys Ile Ser Asp Phe Gly Leu Ser
                885                 890                 895

cga gat gtt tat gaa gag gat tcc tac gtg aag agg agc cag ggt cgg      2736
Arg Asp Val Tyr Glu Glu Asp Ser Tyr Val Lys Arg Ser Gln Gly Arg
            900                 905                 910

att cca gtt aaa tgg atg gca att gaa tcc ctt ttt gat cat atc tac      2784
Ile Pro Val Lys Trp Met Ala Ile Glu Ser Leu Phe Asp His Ile Tyr
        915                 920                 925

acc acg caa agt gat gta tgg tct ttt ggt gtc ctg ctg tgg gag atc      2832
Thr Thr Gln Ser Asp Val Trp Ser Phe Gly Val Leu Leu Trp Glu Ile
    930                 935                 940

gtg acc cta ggg gga aac ccc tat cct ggg att cct cct gag cgg ctc      2880
Val Thr Leu Gly Gly Asn Pro Tyr Pro Gly Ile Pro Pro Glu Arg Leu
945                 950                 955                 960

ttc aac ctt ctg aag acc ggc cac cgg atg gag agg cca gac aac tgc      2928
Phe Asn Leu Leu Lys Thr Gly His Arg Met Glu Arg Pro Asp Asn Cys
                965                 970                 975

agc gag gag atg tac cgc ctg atg ctg caa tgc tgg aag cag gag ccg      2976
Ser Glu Glu Met Tyr Arg Leu Met Leu Gln Cys Trp Lys Gln Glu Pro
            980                 985                 990

gac aaa agg ccg gtg ttt gcg gac  atc agc aaa gac ctg  gag aag atg      3024
Asp Lys Arg Pro Val Phe Ala Asp  Ile Ser Lys Asp Leu  Glu Lys Met
        995                 1000                1005

atg gtt  aag agg aga gac tac  ttg gac ctt gcg gcg  tcc act cca      3069
Met Val  Lys Arg Arg Asp Tyr  Leu Asp Leu Ala Ala  Ser Thr Pro
    1010                1015                1020

tct gac  tcc ctg att tat gac  gac ggc ctc tca gag  gag gag aca      3114
Ser Asp  Ser Leu Ile Tyr Asp  Asp Gly Leu Ser Glu  Glu Glu Thr
    1025                1030                1035

ccg ctg  gtg gac tgt aat aat  gcc ccc ctc cct cga  gcc ctc cct      3159

Pro Leu Val Asp Cys Asn Asn Ala Pro Leu Pro Arg Ala Leu Pro
    1040                1045                1050

tcc aca tgg att gaa aac aaa ctc tat ggt aga att tcc cat gca 3204
Ser Thr Trp Ile Glu Asn Lys Leu Tyr Gly Arg Ile Ser His Ala
    1055                1060                1065

ttt act aga ttc tag 3219
Phe Thr Arg Phe
    1070

<210> 6
<211> 1072
<212> PRT
<213> Homo sapiens

<400> 6

Met Ala Lys Ala Thr Ser Gly Ala Ala Gly Leu Arg Leu Leu Leu Leu
1               5                   10                  15

Leu Leu Leu Pro Leu Leu Gly Lys Val Ala Leu Gly Leu Tyr Phe Ser
            20                  25                  30

Arg Asp Ala Tyr Trp Glu Lys Leu Tyr Val Asp Gln Ala Ala Gly Thr
        35                  40                  45

Pro Leu Leu Tyr Val His Ala Leu Arg Asp Ala Pro Glu Glu Val Pro
    50                  55                  60

Ser Phe Arg Leu Gly Gln His Leu Tyr Gly Thr Tyr Arg Thr Arg Leu
65                  70                  75                  80

His Glu Asn Asn Trp Ile Cys Ile Gln Glu Asp Thr Gly Leu Leu Tyr
                85                  90                  95

Leu Asn Arg Ser Leu Asp His Ser Ser Trp Glu Lys Leu Ser Val Arg
            100                 105                 110

Asn Arg Gly Phe Pro Leu Leu Thr Val Tyr Leu Lys Val Phe Leu Ser
        115                 120                 125

Pro Thr Ser Leu Arg Glu Gly Glu Cys Gln Trp Pro Gly Cys Ala Arg
    130                 135                 140

Val Tyr Phe Ser Phe Phe Asn Thr Ser Phe Pro Ala Cys Ser Ser Leu
145                 150                 155                 160

Lys Pro Arg Glu Leu Cys Phe Pro Glu Thr Arg Pro Ser Phe Arg Ile
                165                 170                 175

Arg Glu Asn Arg Pro Pro Gly Thr Phe His Gln Phe Arg Leu Leu Pro
            180                 185                 190

Val Gln Phe Leu Cys Pro Asn Ile Ser Val Ala Tyr Arg Leu Leu Glu
        195                 200                 205

Gly Glu Gly Leu Pro Phe Arg Cys Ala Pro Asp Ser Leu Glu Val Ser
    210                 215                 220

Thr Arg Trp Ala Leu Asp Arg Glu Gln Arg Glu Lys Tyr Glu Leu Val
225                 230                 235                 240

Ala Val Cys Thr Val His Ala Gly Ala Arg Glu Glu Val Val Met Val
                245                 250                 255

Pro Phe Pro Val Thr Val Tyr Asp Glu Asp Asp Ser Ala Pro Thr Phe
            260                 265                 270

Pro Ala Gly Val Asp Thr Ala Ser Ala Val Val Glu Phe Lys Arg Lys
        275                 280                 285

Glu Asp Thr Val Val Ala Thr Leu Arg Val Phe Asp Ala Asp Val Val
    290                 295                 300

Pro Ala Ser Gly Glu Leu Val Arg Arg Tyr Thr Ser Thr Leu Leu Pro
305                 310                 315                 320

Gly Asp Thr Trp Ala Gln Gln Thr Phe Arg Val Glu His Trp Pro Asn
                325                 330                 335

Glu Thr Ser Val Gln Ala Asn Gly Ser Phe Val Arg Ala Thr Val His
            340                 345                 350

Asp Tyr Arg Leu Val Leu Asn Arg Asn Leu Ser Ile Ser Glu Asn Arg
        355                 360                 365

Thr Met Gln Leu Ala Val Leu Val Asn Asp Ser Asp Phe Gln Gly Pro
    370                 375                 380

Gly Ala Gly Val Leu Leu Leu His Phe Asn Val Ser Val Leu Pro Val
385                 390                 395                 400

Ser Leu His Leu Pro Ser Thr Tyr Ser Leu Ser Val Ser Arg Arg Ala
                405                 410                 415

Arg Arg Phe Ala Gln Ile Gly Lys Val Cys Val Glu Asn Cys Gln Ala
            420                 425                 430

Phe Ser Gly Ile Asn Val Gln Tyr Lys Leu His Ser Ser Gly Ala Asn
        435                 440                 445

Cys Ser Thr Leu Gly Val Val Thr Ser Ala Glu Asp Thr Ser Gly Ile
    450                 455                 460

Leu Phe Val Asn Asp Thr Lys Ala Leu Arg Arg Pro Lys Cys Ala Glu
465                 470                 475                 480

Leu His Tyr Met Val Val Ala Thr Asp Gln Gln Thr Ser Arg Gln Ala
                485                 490                 495

Gln Ala Gln Leu Leu Val Thr Val Glu Gly Ser Tyr Val Ala Glu Glu
            500                 505                 510

Ala Gly Cys Pro Leu Ser Cys Ala Val Ser Lys Arg Arg Leu Glu Cys
        515                 520                 525

Glu Glu Cys Gly Gly Leu Gly Ser Pro Thr Gly Arg Cys Glu Trp Arg
    530                 535                 540

Gln Gly Asp Gly Lys Gly Ile Thr Arg Asn Phe Ser Thr Cys Ser Pro
545                 550                 555                 560

Ser Thr Lys Thr Cys Pro Asp Gly His Cys Asp Val Val Glu Thr Gln
                565                 570                 575

Asp Ile Asn Ile Cys Pro Gln Asp Cys Leu Arg Gly Ser Ile Val Gly
            580                 585                 590

Gly His Glu Pro Gly Glu Pro Arg Gly Ile Lys Ala Gly Tyr Gly Thr
        595                 600                 605

Cys Asn Cys Phe Pro Glu Glu Glu Lys Cys Phe Cys Glu Pro Glu Asp
    610                 615                 620

Ile Gln Asp Pro Leu Cys Asp Glu Leu Cys Arg Thr Val Ile Ala Ala
625                 630                 635                 640

Ala Val Leu Phe Ser Phe Ile Val Ser Val Leu Leu Ser Ala Phe Cys
                645                 650                 655

Ile His Cys Tyr His Lys Phe Ala His Lys Pro Pro Ile Ser Ser Ala
            660                 665                 670

Glu Met Thr Phe Arg Arg Pro Ala Gln Ala Phe Pro Val Ser Tyr Ser
        675                 680                 685

Ser Ser Gly Ala Arg Arg Pro Ser Leu Asp Ser Met Glu Asn Gln Val
    690                 695                 700

Ser Val Asp Ala Phe Lys Ile Leu Glu Asp Pro Lys Trp Glu Phe Pro
705                 710                 715                 720

Arg Lys Asn Leu Val Leu Gly Lys Thr Leu Gly Glu Gly Glu Phe Gly
                725                 730                 735

Lys Val Val Lys Ala Thr Ala Phe His Leu Lys Gly Arg Ala Gly Tyr

            740                 745                 750

Thr Thr Val Ala Val Lys Met Leu Lys Glu Asn Ala Ser Pro Ser Glu
        755                 760                 765

Leu Arg Asp Leu Leu Ser Glu Phe Asn Val Leu Lys Gln Val Asn His
    770                 775                 780

Pro His Val Ile Lys Leu Tyr Gly Ala Cys Ser Gln Asp Gly Pro Leu
785                 790                 795                 800

Leu Leu Ile Val Glu Tyr Ala Lys Tyr Gly Ser Leu Arg Gly Phe Leu
                805                 810                 815

Arg Glu Ser Arg Lys Val Gly Pro Gly Tyr Leu Gly Ser Gly Gly Ser
            820                 825                 830

Arg Asn Ser Ser Ser Leu Asp His Pro Asp Glu Arg Ala Leu Thr Met
        835                 840                 845

Gly Asp Leu Ile Ser Phe Ala Trp Gln Ile Ser Gln Gly Met Gln Tyr
    850                 855                 860

Leu Ala Glu Met Lys Leu Val His Arg Asp Leu Ala Ala Arg Asn Ile
865                 870                 875                 880

Leu Val Ala Glu Gly Arg Lys Met Lys Ile Ser Asp Phe Gly Leu Ser
                885                 890                 895

Arg Asp Val Tyr Glu Glu Asp Ser Tyr Val Lys Arg Ser Gln Gly Arg
            900                 905                 910

Ile Pro Val Lys Trp Met Ala Ile Glu Ser Leu Phe Asp His Ile Tyr
        915                 920                 925

Thr Thr Gln Ser Asp Val Trp Ser Phe Gly Val Leu Leu Trp Glu Ile
    930                 935                 940

Val Thr Leu Gly Gly Asn Pro Tyr Pro Gly Ile Pro Pro Glu Arg Leu
945                 950                 955                 960

Phe Asn Leu Leu Lys Thr Gly His Arg Met Glu Arg Pro Asp Asn Cys
                965                 970                 975

Ser Glu Glu Met Tyr Arg Leu Met Leu Gln Cys Trp Lys Gln Glu Pro
            980                 985                 990

Asp Lys Arg Pro Val Phe Ala Asp  Ile Ser Lys Asp Leu  Glu Lys Met
        995                 1000                 1005

Met Val  Lys Arg Arg Asp Tyr  Leu Asp Leu Ala Ala  Ser Thr Pro
    1010                 1015                 1020

Ser Asp  Ser Leu Ile Tyr Asp  Asp Gly Leu Ser Glu  Glu Glu Thr
    1025                 1030                 1035

Pro Leu  Val Asp Cys Asn Asn  Ala Pro Leu Pro Arg  Ala Leu Pro
    1040                 1045                 1050

Ser Thr  Trp Ile Glu Asn Lys  Leu Tyr Gly Arg Ile  Ser His Ala
    1055                 1060                 1065

Phe Thr  Arg Phe
    1070

<210>  7
<211>  3219
<212>  DNA
<213>  Homo sapiens

<220>
<221>  CDS
<222>  (1)..(3216)

<400>  7
atg gcg aag gcg acg tcc ggt gcc gcg ggg ctg cgt ctg ctg ttg ctg        48
Met Ala Lys Ala Thr Ser Gly Ala Ala Gly Leu Arg Leu Leu Leu Leu
1               5                   10                  15

ctg ctg ctg ccg ctg cta ggc aaa gtg gca ttg ggc ctc tac ttc tcg 96
Leu Leu Leu Pro Leu Leu Gly Lys Val Ala Leu Gly Leu Tyr Phe Ser
            20                  25                  30

agg gat gct tac tgg gag aag ctg tat gtg gac cag gcg gcc ggc acg 144
Arg Asp Ala Tyr Trp Glu Lys Leu Tyr Val Asp Gln Ala Ala Gly Thr
        35                  40                  45

ccc ttg ctg tac gtc cat gcc ctg cgg gac gcc cct gag gag gtg ccc 192
Pro Leu Leu Tyr Val His Ala Leu Arg Asp Ala Pro Glu Glu Val Pro
    50                  55                  60

agc ttc cgc ctg ggc cag cat ctc tac ggc acg tac cgc aca cgg ctg 240
Ser Phe Arg Leu Gly Gln His Leu Tyr Gly Thr Tyr Arg Thr Arg Leu
65                  70                  75                  80

cat gag aac aac tgg atc tgc atc cag gag gac acc ggc ctc ctc tac 288
His Glu Asn Asn Trp Ile Cys Ile Gln Glu Asp Thr Gly Leu Leu Tyr
                85                  90                  95

ctt aac cgg agc ctg gac cat agc tcc tgg gag aag ctc agt gtc cgc 336
Leu Asn Arg Ser Leu Asp His Ser Ser Trp Glu Lys Leu Ser Val Arg
            100                 105                 110

aac cgc ggc ttt ccc ctg ctc acc gtc tac ctc aag gtc ttc ctg tca 384
Asn Arg Gly Phe Pro Leu Leu Thr Val Tyr Leu Lys Val Phe Leu Ser
        115                 120                 125

ccc aca tcc ctt cgt gag ggc gag tgc cag tgg cca ggc tgt gcc cgc 432
Pro Thr Ser Leu Arg Glu Gly Glu Cys Gln Trp Pro Gly Cys Ala Arg
    130                 135                 140

gta tac ttc tcc ttc ttc aac acc tcc ttt cca gcc tgc agc tcc ctc 480
Val Tyr Phe Ser Phe Phe Asn Thr Ser Phe Pro Ala Cys Ser Ser Leu
145                 150                 155                 160

aag ccc cgg gag ctc tgc ttc cca gag aca agg ccc tcc ttc cgc att 528
Lys Pro Arg Glu Leu Cys Phe Pro Glu Thr Arg Pro Ser Phe Arg Ile
                165                 170                 175

cgg gag aac cga ccc cca ggc acc ttc cac cag ttc cgc ctg ctg cct 576
Arg Glu Asn Arg Pro Pro Gly Thr Phe His Gln Phe Arg Leu Leu Pro
            180                 185                 190

gtg cag ttc ttg tgc ccc aac atc agc gtg gcc tac agg ctc ctg gag 624
Val Gln Phe Leu Cys Pro Asn Ile Ser Val Ala Tyr Arg Leu Leu Glu
        195                 200                 205

ggt gag ggt ctg ccc ttc cgc tgc gcc ccg gac agc ctg gag gtg agc 672
Gly Glu Gly Leu Pro Phe Arg Cys Ala Pro Asp Ser Leu Glu Val Ser
    210                 215                 220

acg cgc tgg gcc ctg gac cgc gag cag cgg gag aag tac gag ctg gtg 720
Thr Arg Trp Ala Leu Asp Arg Glu Gln Arg Glu Lys Tyr Glu Leu Val
225                 230                 235                 240

gcc gtg tgc acc gtg cac gcc ggc gcg cgc gag gag gtg gtg atg gtg 768
Ala Val Cys Thr Val His Ala Gly Ala Arg Glu Glu Val Val Met Val
                245                 250                 255

ccc ttc ccg gtg acc gtg tac gac gag gac gac tcg gcg ccc acc ttc 816
Pro Phe Pro Val Thr Val Tyr Asp Glu Asp Asp Ser Ala Pro Thr Phe
            260                 265                 270

ccc gcg ggc gtc gac acc gcc agc gcc gtg gtg gag ttc aag cgg aag 864
Pro Ala Gly Val Asp Thr Ala Ser Ala Val Val Glu Phe Lys Arg Lys
        275                 280                 285

gag gac acc gtg gtg gcc acg ctg cgt gtc ttc gat gca gac gtg gta 912
Glu Asp Thr Val Val Ala Thr Leu Arg Val Phe Asp Ala Asp Val Val
    290                 295                 300

cct gca tca ggg gag ctg gtg agg cgg tac aca agc acg ctg ctc ccc 960
Pro Ala Ser Gly Glu Leu Val Arg Arg Tyr Thr Ser Thr Leu Leu Pro
305                 310                 315                 320

ggg gac acc tgg gcc cag cag acc ttc cgg gtg gaa cac tgg ccc aac 1008
Gly Asp Thr Trp Ala Gln Gln Thr Phe Arg Val Glu His Trp Pro Asn
                325                 330                 335

gag acc tcg gtc cag gcc aac ggc agc ttc gtg cgg gcg acc gta cat 1056
Glu Thr Ser Val Gln Ala Asn Gly Ser Phe Val Arg Ala Thr Val His
            340                 345                 350

gac tat agg ctg gtt ctc aac cgg aac ctc tcc atc tcg gag aac cgc 1104
Asp Tyr Arg Leu Val Leu Asn Arg Asn Leu Ser Ile Ser Glu Asn Arg
        355                 360                 365

acc atg cag ctg gcg gtg ctg gtc aat gac tca gac ttc cag ggc cca 1152
Thr Met Gln Leu Ala Val Leu Val Asn Asp Ser Asp Phe Gln Gly Pro
    370                 375                 380

gga gcg ggc gtc ctc ttg ctc cac ttc aac gtg tcg gtg ctg ccg gtc 1200
Gly Ala Gly Val Leu Leu Leu His Phe Asn Val Ser Val Leu Pro Val
385                 390                 395                 400

agc ctg cac ctg ccc agt acc tac tcc ctc tcc gtg agc agg agg gct 1248
Ser Leu His Leu Pro Ser Thr Tyr Ser Leu Ser Val Ser Arg Arg Ala
                405                 410                 415

cgc cga ttt gcc cag atc ggg aaa gtc tgt gtg gaa aac tgc cag gca 1296
Arg Arg Phe Ala Gln Ile Gly Lys Val Cys Val Glu Asn Cys Gln Ala

420                 425                 430

ttc agt ggc atc aac gtc cag tac aag ctg cat tcc tct ggt gcc aac     1344
Phe Ser Gly Ile Asn Val Gln Tyr Lys Leu His Ser Ser Gly Ala Asn
        435                 440                 445

tgc agc acg cta ggg gtg gtc acc tca gcc gag gac acc tcg ggg atc     1392
Cys Ser Thr Leu Gly Val Val Thr Ser Ala Glu Asp Thr Ser Gly Ile
    450                 455                 460

ctg ttt gtg aat gac acc aag gcc ctg cgg cgg ccc aag tgt gcc gaa     1440
Leu Phe Val Asn Asp Thr Lys Ala Leu Arg Arg Pro Lys Cys Ala Glu
465                 470                 475                 480

ctt cac tac atg gtg gtg gcc acc gac cag cag acc tct agg cag gcc     1488
Leu His Tyr Met Val Val Ala Thr Asp Gln Gln Thr Ser Arg Gln Ala
                485                 490                 495

cag gcc cag ctg ctt gta aca gtg gag ggg tca tat gtg gcc gag gag     1536
Gln Ala Gln Leu Leu Val Thr Val Glu Gly Ser Tyr Val Ala Glu Glu
            500                 505                 510

gcg ggc tgc ccc ctg tcc tgt gca gtc agc aag aga cgg ctg gag tgt     1584
Ala Gly Cys Pro Leu Ser Cys Ala Val Ser Lys Arg Arg Leu Glu Cys
        515                 520                 525

gag gag tgt ggc ggc ctg ggc tcc cca aca ggc agg tgt gag tgg agg     1632
Glu Glu Cys Gly Gly Leu Gly Ser Pro Thr Gly Arg Cys Glu Trp Arg
    530                 535                 540

caa gga gat ggc aaa ggg atc acc agg aac ttc tcc acc tgc tct ccc     1680
Gln Gly Asp Gly Lys Gly Ile Thr Arg Asn Phe Ser Thr Cys Ser Pro
545                 550                 555                 560

agc acc aag acc tgc ccc gac ggc cac tgc gat gtt gtg gag acc caa     1728
Ser Thr Lys Thr Cys Pro Asp Gly His Cys Asp Val Val Glu Thr Gln
                565                 570                 575

gac atc aac att tgc cct cag gac tgc ctc cgg ggc agc att gtt ggg     1776
Asp Ile Asn Ile Cys Pro Gln Asp Cys Leu Arg Gly Ser Ile Val Gly
            580                 585                 590

gga cac gag cct ggg gag ccc cgg ggg att aaa gct ggc tat ggc acc     1824
Gly His Glu Pro Gly Glu Pro Arg Gly Ile Lys Ala Gly Tyr Gly Thr
        595                 600                 605

tgc aac tgc ttc cct gag gag gag aag tgc ttc tgc gag ccc gaa gac     1872
Cys Asn Cys Phe Pro Glu Glu Glu Lys Cys Phe Cys Glu Pro Glu Asp
    610                 615                 620

atc cag gat cca ctg tgc gac gag ctg tgc cgc acg gtg atc gca gcc     1920

Ile Gln Asp Pro Leu Cys Asp Glu Leu Cys Arg Thr Val Ile Ala Ala
625                 630                 635                 640

gct gtc ctc ttc tcc ttc atc gtc tcg gtg ctg ctg tct gcc ttc tgc      1968
Ala Val Leu Phe Ser Phe Ile Val Ser Val Leu Leu Ser Ala Phe Cys
                645                 650                 655

atc cac tgc tac cac aag ttt gcc cac aag cca ccc atc tcc tca gct      2016
Ile His Cys Tyr His Lys Phe Ala His Lys Pro Pro Ile Ser Ser Ala
            660                 665                 670

gag atg acc ttc cgg agg ccc gcc cag gcc ttc ccg gtc agc tac tcc      2064
Glu Met Thr Phe Arg Arg Pro Ala Gln Ala Phe Pro Val Ser Tyr Ser
        675                 680                 685

tct tcc agt gcc cgc cgg ccc tcg ctg gac tcc atg gag aac cag gtc      2112
Ser Ser Ser Ala Arg Arg Pro Ser Leu Asp Ser Met Glu Asn Gln Val
    690                 695                 700

tcc gtg gat gcc ttc aag atc ctg gag gat cca aag tgg gaa ttc cct      2160
Ser Val Asp Ala Phe Lys Ile Leu Glu Asp Pro Lys Trp Glu Phe Pro
705                 710                 715                 720

cgg aag aac ttg gtt ctt gga aaa act cta gga gaa ggc gaa ttt gga      2208
Arg Lys Asn Leu Val Leu Gly Lys Thr Leu Gly Glu Gly Glu Phe Gly
                725                 730                 735

aaa gtg gtc aag gca acg gcc ttc cat ctg aaa ggc aga gca ggg tac      2256
Lys Val Val Lys Ala Thr Ala Phe His Leu Lys Gly Arg Ala Gly Tyr
            740                 745                 750

acc acg gtg gcc gtg aag atg ctg aaa gag aac gcc tcc ccg agt gag      2304
Thr Thr Val Ala Val Lys Met Leu Lys Glu Asn Ala Ser Pro Ser Glu
        755                 760                 765

ctt cga gac ctg ctg tca gag ttc aac gtc ctg aag cag gtc aac cac      2352
Leu Arg Asp Leu Leu Ser Glu Phe Asn Val Leu Lys Gln Val Asn His
    770                 775                 780

cca cat gtc atc aaa ttg tat ggg gcc tgc agc cag gat ggc ccg ctc      2400
Pro His Val Ile Lys Leu Tyr Gly Ala Cys Ser Gln Asp Gly Pro Leu
785                 790                 795                 800

ctc ctc atc gtg gag tac gcc aaa tac ggc tcc ctg cgg ggc ttc ctc      2448
Leu Leu Ile Val Glu Tyr Ala Lys Tyr Gly Ser Leu Arg Gly Phe Leu
                805                 810                 815

cgc gag agc cgc aaa gtg ggg cct ggc tac ctg ggc agt gga ggc agc      2496
Arg Glu Ser Arg Lys Val Gly Pro Gly Tyr Leu Gly Ser Gly Gly Ser
            820                 825                 830

cgc aac tcc agc tcc ctg gac cac ccg gat gag cgg gcc ctc acc atg 2544
Arg Asn Ser Ser Ser Leu Asp His Pro Asp Glu Arg Ala Leu Thr Met
        835                 840                 845

ggc gac ctc atc tca ttt gcc tgg cag atc tca cag ggg atg cag tat 2592
Gly Asp Leu Ile Ser Phe Ala Trp Gln Ile Ser Gln Gly Met Gln Tyr
    850                 855                 860

ctg gcc gag atg aag ctc gtt cat cgg gac ttg gca gcc aga aac atc 2640
Leu Ala Glu Met Lys Leu Val His Arg Asp Leu Ala Ala Arg Asn Ile
865                 870                 875                 880

ctg gta gct gag ggg cgg aag atg aag att tcg gat ttc ggc ttg tcc 2688
Leu Val Ala Glu Gly Arg Lys Met Lys Ile Ser Asp Phe Gly Leu Ser
                885                 890                 895

cga gat gtt tat gaa gag gat tcg tac gtg aag agg agc cag ggt cgg 2736
Arg Asp Val Tyr Glu Glu Asp Ser Tyr Val Lys Arg Ser Gln Gly Arg
            900                 905                 910

att cca gtt aaa tgg atg gca att gaa tcc ctt ttt gat cat atc tac 2784
Ile Pro Val Lys Trp Met Ala Ile Glu Ser Leu Phe Asp His Ile Tyr
        915                 920                 925

acc acg caa agt gat gta tgg tct ttt ggt gtc ctg ctg tgg gag atc 2832
Thr Thr Gln Ser Asp Val Trp Ser Phe Gly Val Leu Leu Trp Glu Ile
    930                 935                 940

gtg acc cta ggg gga aac ccc tat cct ggg att cct cct gag cgg ctc 2880
Val Thr Leu Gly Gly Asn Pro Tyr Pro Gly Ile Pro Pro Glu Arg Leu
945                 950                 955                 960

ttc aac ctt ctg aag acc ggc cac cgg atg gag agg cca gac aac tgc 2928
Phe Asn Leu Leu Lys Thr Gly His Arg Met Glu Arg Pro Asp Asn Cys
                965                 970                 975

agc gag gag atg tac tgc ctg atg ctg caa tgc tgg aag cag gag ccg 2976
Ser Glu Glu Met Tyr Cys Leu Met Leu Gln Cys Trp Lys Gln Glu Pro
            980                 985                 990

gac aaa agg ccg gtg ttt gcg gac atc agc aaa gac ctg gag aag atg 3024
Asp Lys Arg Pro Val Phe Ala Asp Ile Ser Lys Asp Leu Glu Lys Met
        995                 1000                1005

atg gtt aag agg aga gac tac ttg gac ctt gcg gcg tcc act cca 3069
Met Val Lys Arg Arg Asp Tyr Leu Asp Leu Ala Ala Ser Thr Pro
    1010                1015                1020

tct gac tcc ctg att tat gac gac ggc ctc tca gag gag gag aca 3114
Ser Asp Ser Leu Ile Tyr Asp Asp Gly Leu Ser Glu Glu Glu Thr
    1025                1030                1035

ccg ctg gtg gac tgt aat aat gcc ccc ctc cct cga gcc ctc cct 3159
Pro Leu Val Asp Cys Asn Asn Ala Pro Leu Pro Arg Ala Leu Pro
    1040                1045                1050

tcc aca tgg att gaa aac aaa ctc tat ggt aga att tcc cat gca 3204
Ser Thr Trp Ile Glu Asn Lys Leu Tyr Gly Arg Ile Ser His Ala
    1055                1060                1065

ttt act aga ttc tag 3219
Phe Thr Arg Phe
    1070

<210> 8
<211> 1072
<212> PRT
<213> Homo sapiens

<400> 8

Met Ala Lys Ala Thr Ser Gly Ala Ala Gly Leu Arg Leu Leu Leu Leu
1               5                   10                  15

Leu Leu Leu Pro Leu Leu Gly Lys Val Ala Leu Gly Leu Tyr Phe Ser
            20                  25                  30

Arg Asp Ala Tyr Trp Glu Lys Leu Tyr Val Asp Gln Ala Ala Gly Thr
        35                  40                  45

Pro Leu Leu Tyr Val His Ala Leu Arg Asp Ala Pro Glu Glu Val Pro
    50                  55                  60

Ser Phe Arg Leu Gly Gln His Leu Tyr Gly Thr Tyr Arg Thr Arg Leu
65                  70                  75                  80

His Glu Asn Asn Trp Ile Cys Ile Gln Glu Asp Thr Gly Leu Leu Tyr
                85                  90                  95

Leu Asn Arg Ser Leu Asp His Ser Ser Trp Glu Lys Leu Ser Val Arg
            100                 105                 110

Asn Arg Gly Phe Pro Leu Leu Thr Val Tyr Leu Lys Val Phe Leu Ser
        115                 120                 125

Pro Thr Ser Leu Arg Glu Gly Glu Cys Gln Trp Pro Gly Cys Ala Arg
    130                 135                 140

Val Tyr Phe Ser Phe Phe Asn Thr Ser Phe Pro Ala Cys Ser Ser Leu
145                 150                 155                 160

Lys Pro Arg Glu Leu Cys Phe Pro Glu Thr Arg Pro Ser Phe Arg Ile
                165                 170                 175

Arg Glu Asn Arg Pro Pro Gly Thr Phe His Gln Phe Arg Leu Leu Pro
            180                 185                 190

Val Gln Phe Leu Cys Pro Asn Ile Ser Val Ala Tyr Arg Leu Leu Glu
        195                 200                 205

Gly Glu Gly Leu Pro Phe Arg Cys Ala Pro Asp Ser Leu Glu Val Ser
    210                 215                 220

Thr Arg Trp Ala Leu Asp Arg Glu Gln Arg Glu Lys Tyr Glu Leu Val
225                 230                 235                 240

Ala Val Cys Thr Val His Ala Gly Ala Arg Glu Glu Val Val Met Val
                245                 250                 255

Pro Phe Pro Val Thr Val Tyr Asp Glu Asp Asp Ser Ala Pro Thr Phe
            260                 265                 270

Pro Ala Gly Val Asp Thr Ala Ser Ala Val Val Glu Phe Lys Arg Lys
        275                 280                 285

Glu Asp Thr Val Val Ala Thr Leu Arg Val Phe Asp Ala Asp Val Val
    290                 295                 300

Pro Ala Ser Gly Glu Leu Val Arg Arg Tyr Thr Ser Thr Leu Leu Pro
305                 310                 315                 320

Gly Asp Thr Trp Ala Gln Gln Thr Phe Arg Val Glu His Trp Pro Asn

                325                 330                 335

Glu Thr Ser Val Gln Ala Asn Gly Ser Phe Val Arg Ala Thr Val His
            340                 345                 350

Asp Tyr Arg Leu Val Leu Asn Arg Asn Leu Ser Ile Ser Glu Asn Arg
        355                 360                 365

Thr Met Gln Leu Ala Val Leu Val Asn Asp Ser Asp Phe Gln Gly Pro
    370                 375                 380

Gly Ala Gly Val Leu Leu Leu His Phe Asn Val Ser Val Leu Pro Val
385                 390                 395                 400

Ser Leu His Leu Pro Ser Thr Tyr Ser Leu Ser Val Ser Arg Arg Ala
                405                 410                 415

Arg Arg Phe Ala Gln Ile Gly Lys Val Cys Val Glu Asn Cys Gln Ala
            420                 425                 430

Phe Ser Gly Ile Asn Val Gln Tyr Lys Leu His Ser Ser Gly Ala Asn
        435                 440                 445

Cys Ser Thr Leu Gly Val Val Thr Ser Ala Glu Asp Thr Ser Gly Ile
    450                 455                 460

Leu Phe Val Asn Asp Thr Lys Ala Leu Arg Arg Pro Lys Cys Ala Glu
465                 470                 475                 480

Leu His Tyr Met Val Val Ala Thr Asp Gln Gln Thr Ser Arg Gln Ala
                485                 490                 495

Gln Ala Gln Leu Leu Val Thr Val Glu Gly Ser Tyr Val Ala Glu Glu
            500                 505                 510

Ala Gly Cys Pro Leu Ser Cys Ala Val Ser Lys Arg Arg Leu Glu Cys
        515                 520                 525

Glu Glu Cys Gly Gly Leu Gly Ser Pro Thr Gly Arg Cys Glu Trp Arg
    530                 535                 540

Gln Gly Asp Gly Lys Gly Ile Thr Arg Asn Phe Ser Thr Cys Ser Pro
545                 550                 555                 560

Ser Thr Lys Thr Cys Pro Asp Gly His Cys Asp Val Val Glu Thr Gln
                565                 570                 575

Asp Ile Asn Ile Cys Pro Gln Asp Cys Leu Arg Gly Ser Ile Val Gly
            580                 585                 590

Gly His Glu Pro Gly Glu Pro Arg Gly Ile Lys Ala Gly Tyr Gly Thr
        595                 600                 605

Cys Asn Cys Phe Pro Glu Glu Glu Lys Cys Phe Cys Glu Pro Glu Asp
    610                 615                 620

Ile Gln Asp Pro Leu Cys Asp Glu Leu Cys Arg Thr Val Ile Ala Ala
625                 630                 635                 640

Ala Val Leu Phe Ser Phe Ile Val Ser Val Leu Leu Ser Ala Phe Cys
                645                 650                 655

Ile His Cys Tyr His Lys Phe Ala His Lys Pro Pro Ile Ser Ser Ala
            660                 665                 670

Glu Met Thr Phe Arg Arg Pro Ala Gln Ala Phe Pro Val Ser Tyr Ser
        675                 680                 685

Ser Ser Ser Ala Arg Arg Pro Ser Leu Asp Ser Met Glu Asn Gln Val
    690                 695                 700

Ser Val Asp Ala Phe Lys Ile Leu Glu Asp Pro Lys Trp Glu Phe Pro
705                 710                 715                 720

Arg Lys Asn Leu Val Leu Gly Lys Thr Leu Gly Glu Gly Glu Phe Gly
                725                 730                 735

Lys Val Val Lys Ala Thr Ala Phe His Leu Lys Gly Arg Ala Gly Tyr
            740                 745                 750

Thr Thr Val Ala Val Lys Met Leu Lys Glu Asn Ala Ser Pro Ser Glu
        755                 760                 765

Leu Arg Asp Leu Leu Ser Glu Phe Asn Val Leu Lys Gln Val Asn His
    770                 775                 780

Pro His Val Ile Lys Leu Tyr Gly Ala Cys Ser Gln Asp Gly Pro Leu
785                 790                 795                 800

Leu Leu Ile Val Glu Tyr Ala Lys Tyr Gly Ser Leu Arg Gly Phe Leu
                805                 810                 815

Arg Glu Ser Arg Lys Val Gly Pro Gly Tyr Leu Gly Ser Gly Gly Ser
            820                 825                 830

Arg Asn Ser Ser Ser Leu Asp His Pro Asp Glu Arg Ala Leu Thr Met
        835                 840                 845

Gly Asp Leu Ile Ser Phe Ala Trp Gln Ile Ser Gln Gly Met Gln Tyr
    850                 855                 860

Leu Ala Glu Met Lys Leu Val His Arg Asp Leu Ala Ala Arg Asn Ile
865                 870                 875                 880

Leu Val Ala Glu Gly Arg Lys Met Lys Ile Ser Asp Phe Gly Leu Ser
                885                 890                 895

Arg Asp Val Tyr Glu Glu Asp Ser Tyr Val Lys Arg Ser Gln Gly Arg
            900                 905                 910

Ile Pro Val Lys Trp Met Ala Ile Glu Ser Leu Phe Asp His Ile Tyr
        915                 920                 925

Thr Thr Gln Ser Asp Val Trp Ser Phe Gly Val Leu Leu Trp Glu Ile
    930                 935                 940

Val Thr Leu Gly Gly Asn Pro Tyr Pro Gly Ile Pro Pro Glu Arg Leu
945                 950                 955                 960

Phe Asn Leu Leu Lys Thr Gly His Arg Met Glu Arg Pro Asp Asn Cys
                965                 970                 975

Ser Glu Glu Met Tyr Cys Leu Met Leu Gln Cys Trp Lys Gln Glu Pro
            980                 985                 990

Asp Lys Arg Pro Val Phe Ala Asp  Ile Ser Lys Asp Leu  Glu Lys Met
        995                 1000                 1005

Met Val  Lys Arg Arg Asp Tyr  Leu Asp Leu Ala Ala  Ser Thr Pro
    1010                 1015                 1020

Ser Asp  Ser Leu Ile Tyr Asp  Asp Gly Leu Ser Glu  Glu Glu Thr
    1025                 1030                 1035

Pro Leu  Val Asp Cys Asn Asn  Ala Pro Leu Pro Arg  Ala Leu Pro
    1040                 1045                 1050

Ser Thr  Trp Ile Glu Asn Lys  Leu Tyr Gly Arg Ile  Ser His Ala
    1055                 1060                 1065

Phe Thr  Arg Phe
    1070

<210>  9
<211>  3351
<212>  DNA
<213>  Rattus norvegicus

<220>
<221>  CDS
<222>  (1)..(3348)

<220>
<221>  misc_feature
<222>  (1485)..(1485)

<223>  n is a, c, g, or t

<220>
<221>  misc_feature
<222>  (1997)..(1997)
<223>  n is a, c, g, or t

<220>
<221>  misc_feature
<222>  (2000)..(2001)
<223>  n is a, c, g, or t

<220>
<221>  misc_feature
<222>  (2015)..(2015)
<223>  n is a, c, g, or t

<220>
<221>  misc_feature
<222>  (2022)..(2022)
<223>  n is a, c, g, or t

<220>
<221>  misc_feature
<222>  (2043)..(2043)
<223>  n is a, c, g, or t

<220>
<221>  misc_feature
<222>  (2052)..(2052)
<223>  n is a, c, g, or t

<220>
<221>  misc_feature
<222>  (2080)..(2080)
<223>  n is a, c, g, or t

<220>
<221>  misc_feature
<222>  (2735)..(2735)
<223>  n is a, c, g, or t

<400>  9
atg gcg aaa gcg agg tcc ggc gcc gca ggg ctg ggg ctg aag ctg ttt        48
Met Ala Lys Ala Arg Ser Gly Ala Ala Gly Leu Gly Leu Lys Leu Phe
1               5                   10                  15

ttg ctg ctg ccg cta ctg gga gaa gcc ccg ctg ggt ctc tgc ttc tca        96
Leu Leu Leu Pro Leu Leu Gly Glu Ala Pro Leu Gly Leu Cys Phe Ser
            20                  25                  30

agg gat gct tac tgg gag agg ctg tat gtg gac cag cca gct ggc aca 144
Arg Asp Ala Tyr Trp Glu Arg Leu Tyr Val Asp Gln Pro Ala Gly Thr
        35                  40                  45

cct ctg ctc tat gtc cat gcc cta cgg gat gcc cct gga gaa gtg ccc 192
Pro Leu Leu Tyr Val His Ala Leu Arg Asp Ala Pro Gly Glu Val Pro
    50                  55                  60

agc ttc cgc ctg ggc cag tat ctc tat ggc gtc tac cgc acg cgt ctg 240
Ser Phe Arg Leu Gly Gln Tyr Leu Tyr Gly Val Tyr Arg Thr Arg Leu
65                  70                  75                  80

cat gag aat gac tgg atc cac atc gat gcg ggc act ggc ctc ctc tac 288
His Glu Asn Asp Trp Ile His Ile Asp Ala Gly Thr Gly Leu Leu Tyr
                85                  90                  95

ctc aat cag agc ctg gac cat agt tcc tgg gag cag ctc agc atc cga 336
Leu Asn Gln Ser Leu Asp His Ser Ser Trp Glu Gln Leu Ser Ile Arg
            100                 105                 110

aat ggc ggc ttc ccc ttg ctc acc gtc ttc ctc cag gtc ttc ctg ggg 384
Asn Gly Gly Phe Pro Leu Leu Thr Val Phe Leu Gln Val Phe Leu Gly
        115                 120                 125

tcc aca gcc cag aga gag gga gag tgt cat tgg cca ggc tgt gcc cgt 432
Ser Thr Ala Gln Arg Glu Gly Glu Cys His Trp Pro Gly Cys Ala Arg
    130                 135                 140

gtc tac ttc tcc ttc atc aac gac acc ttc cca aat tgt agc tcc ttc 480
Val Tyr Phe Ser Phe Ile Asn Asp Thr Phe Pro Asn Cys Ser Ser Phe
145                 150                 155                 160

aaa gcc cgg gat ctc tgc acc cca gag acg ggt gtg tcc ttc cgc atc 528
Lys Ala Arg Asp Leu Cys Thr Pro Glu Thr Gly Val Ser Phe Arg Ile
                165                 170                 175

agg gag aac agg ccc cct ggc acc ttc tac cag ttc cgc atg cta cct 576
Arg Glu Asn Arg Pro Pro Gly Thr Phe Tyr Gln Phe Arg Met Leu Pro
            180                 185                 190

gtg cag ttc ctt tgt cct aac atc agt gtg aag tac aaa ctc tta gaa 624
Val Gln Phe Leu Cys Pro Asn Ile Ser Val Lys Tyr Lys Leu Leu Glu
        195                 200                 205

ggg gac ggt ctg ccc ttc cgt tgt gac ccc gac tgt ctg gag gtg agc 672
Gly Asp Gly Leu Pro Phe Arg Cys Asp Pro Asp Cys Leu Glu Val Ser
    210                 215                 220

act cgg tgg gca ctg gat cgt gag ctt cag gag aag tat gtg ctg gag 720
Thr Arg Trp Ala Leu Asp Arg Glu Leu Gln Glu Lys Tyr Val Leu Glu
225                 230                 235                 240

gct gag tgc gca gtg gca ggc cct gga gcc aac aag gag aag gtg gcc 768
Ala Glu Cys Ala Val Ala Gly Pro Gly Ala Asn Lys Glu Lys Val Ala
                245                 250                 255

gtg tcc ttc ccg gtg acg gtg tat gat gat gaa gac gac tcc ccg ccc 816
Val Ser Phe Pro Val Thr Val Tyr Asp Asp Glu Asp Asp Ser Pro Pro
            260                 265                 270

acc ttc tcc gga ggt gtg ggc acc gcc agt gct gtg gtg gag ttt aag 864
Thr Phe Ser Gly Gly Val Gly Thr Ala Ser Ala Val Val Glu Phe Lys
        275                 280                 285

cgg aag gag ggc act gtg gta gcc act ctg cag gtg ttt gat gca gat 912
Arg Lys Glu Gly Thr Val Val Ala Thr Leu Gln Val Phe Asp Ala Asp
    290                 295                 300

gtg gtg cca gca tct ggg gag ctg gtg agg cgg tac aca agc aca cta 960
Val Val Pro Ala Ser Gly Glu Leu Val Arg Arg Tyr Thr Ser Thr Leu
305                 310                 315                 320

ctc tca ggg gat tcc tgg gcc cag cag acc ttc cgg gtg gag cac aca 1008
Leu Ser Gly Asp Ser Trp Ala Gln Gln Thr Phe Arg Val Glu His Thr
                325                 330                 335

ccc aac gag acc ttg gtc cag tcc aac aac aac tcc gtg cgg gca acc 1056
Pro Asn Glu Thr Leu Val Gln Ser Asn Asn Asn Ser Val Arg Ala Thr
            340                 345                 350

atg cac aat tac agg ctg gtt ctc aac agg agc ctg tcg atc tca gag 1104
Met His Asn Tyr Arg Leu Val Leu Asn Arg Ser Leu Ser Ile Ser Glu
        355                 360                 365

agc cga gtc ctg cag cta gta gtc ctg gtc aat gac tca gac ttc cag 1152
Ser Arg Val Leu Gln Leu Val Val Leu Val Asn Asp Ser Asp Phe Gln
    370                 375                 380

ggg cct ggg tca ggt ttt ctc ttc ctc cat ttc aac gtg tct gtg ctg 1200
Gly Pro Gly Ser Gly Phe Leu Phe Leu His Phe Asn Val Ser Val Leu
385                 390                 395                 400

cct gtc acc ctg aac cta ccc atg gcc tac tcc ttc cca gtg aat agg 1248
Pro Val Thr Leu Asn Leu Pro Met Ala Tyr Ser Phe Pro Val Asn Arg
                405                 410                 415

aga gcc cgc cgt tat gcc cag att ggg aaa gtt tgc gtg gag aac tgc 1296
Arg Ala Arg Arg Tyr Ala Gln Ile Gly Lys Val Cys Val Glu Asn Cys
            420                 425                 430

cag gag ttc agc ggt gtc tcc atc cag tac aag ctg cag ccc tcc agc 1344
Gln Glu Phe Ser Gly Val Ser Ile Gln Tyr Lys Leu Gln Pro Ser Ser

435                 440                 445

acc aac tgc agt gcc cta ggt gtg gtc acc tca aca gaa gac acc tca     1392
Thr Asn Cys Ser Ala Leu Gly Val Val Thr Ser Thr Glu Asp Thr Ser
    450                 455                 460

ggg acc cta tat gta aat gac acg gag gcg ctg cgg cga cct gag tgt     1440
Gly Thr Leu Tyr Val Asn Asp Thr Glu Ala Leu Arg Arg Pro Glu Cys
465                 470                 475                 480

acc gag ctt cag tac aca gtg gta gcc act gac cgg cag acc cgn agg     1488
Thr Glu Leu Gln Tyr Thr Val Val Ala Thr Asp Arg Gln Thr Arg Arg
                485                 490                 495

cag acc caa gct tcg tta gtc gtc aca gtg gag ggg aca tac att gca     1536
Gln Thr Gln Ala Ser Leu Val Val Thr Val Glu Gly Thr Tyr Ile Ala
            500                 505                 510

gaa gaa gtg ggc tgc ccc aag tcc tgt gca gta aac aag agg cgc cct     1584
Glu Glu Val Gly Cys Pro Lys Ser Cys Ala Val Asn Lys Arg Arg Pro
        515                 520                 525

gag tgt gag gag tgt ggt ggc ctg ggt tct cca act ggc aga tgt gag     1632
Glu Cys Glu Glu Cys Gly Gly Leu Gly Ser Pro Thr Gly Arg Cys Glu
    530                 535                 540

tgg cgt cag gga gat ggt aaa ggg acc acc agg aac ttc tcc acc tgt     1680
Trp Arg Gln Gly Asp Gly Lys Gly Thr Thr Arg Asn Phe Ser Thr Cys
545                 550                 555                 560

tct cct agc acc agg acc tgt cct gat ggc cac tgt gat gct ctg gag     1728
Ser Pro Ser Thr Arg Thr Cys Pro Asp Gly His Cys Asp Ala Leu Glu
                565                 570                 575

agc cgg gat atc aac att tgc ccc cag gac tgt ctc cgt ggc ccc att     1776
Ser Arg Asp Ile Asn Ile Cys Pro Gln Asp Cys Leu Arg Gly Pro Ile
            580                 585                 590

gtt ggc ggg cat gag cga ggg gag cgc cag gga att aaa gcc ggc tat     1824
Val Gly Gly His Glu Arg Gly Glu Arg Gln Gly Ile Lys Ala Gly Tyr
        595                 600                 605

ggc atc tgc aac tgt ttc cct gat gag aag aag tgc ttc tgc gag cca     1872
Gly Ile Cys Asn Cys Phe Pro Asp Glu Lys Lys Cys Phe Cys Glu Pro
    610                 615                 620

gag gac agc cag gcc cca ttg tgc gat gag ctg tgc cgt acg gtc atc     1920
Glu Asp Ser Gln Ala Pro Leu Cys Asp Glu Leu Cys Arg Thr Val Ile
625                 630                 635                 640

aca gcc gct gtc ctc ttc tcc ttc ata atc tct gtc ctg ctg tcc acc     1968

Thr Ala Ala Val Leu Phe Ser Phe Ile Ile Ser Val Leu Leu Ser Thr
                645                 650                 655

ttc tgc atc cac cgc tac cac aag cat gng cnn aag cca ccc atc gng 2016
Phe Cys Ile His Arg Tyr His Lys His Xaa Xaa Lys Pro Pro Ile Xaa
            660                 665                 670

tca gcn gaa atg acc ttc tgc cgg ccn gcc cag ggn ttc cca atc agc 2064
Ser Ala Glu Met Thr Phe Cys Arg Pro Ala Gln Gly Phe Pro Ile Ser
        675                 680                 685

tat tct tcc tcg ggc ncc cgc cgg ccc tca ctg gac tcc atg gag aac 2112
Tyr Ser Ser Ser Gly Xaa Arg Arg Pro Ser Leu Asp Ser Met Glu Asn
    690                 695                 700

cag gtc tct gtg gac tct ttc aag atc ccg gag gat ccg aag tgg gaa 2160
Gln Val Ser Val Asp Ser Phe Lys Ile Pro Glu Asp Pro Lys Trp Glu
705                 710                 715                 720

ttt cct cgg aag aac tta gtt ctt ggg aaa acc ctg gga gaa ggc gag 2208
Phe Pro Arg Lys Asn Leu Val Leu Gly Lys Thr Leu Gly Glu Gly Glu
                725                 730                 735

ttt gga aaa gta gtc aag gcc aca gcc ttc cgt ctg aaa gac cgg gca 2256
Phe Gly Lys Val Val Lys Ala Thr Ala Phe Arg Leu Lys Asp Arg Ala
            740                 745                 750

gga tac acc aca gtg gct gtg aaa atg ctg aaa gaa aac gcc tcc cag 2304
Gly Tyr Thr Thr Val Ala Val Lys Met Leu Lys Glu Asn Ala Ser Gln
        755                 760                 765

agt gaa cta cga gac ctg ctc tct gag ttc aac ctt ctg aaa caa gtc 2352
Ser Glu Leu Arg Asp Leu Leu Ser Glu Phe Asn Leu Leu Lys Gln Val
    770                 775                 780

aac cat cca cat gtc atc aag ttg tac ggg gct tgc agc cag gat ggg 2400
Asn His Pro His Val Ile Lys Leu Tyr Gly Ala Cys Ser Gln Asp Gly
785                 790                 795                 800

cca ctt ctt ctc att gtg gag tat gca aag tat gga tcc ctg cgg ggg 2448
Pro Leu Leu Leu Ile Val Glu Tyr Ala Lys Tyr Gly Ser Leu Arg Gly
                805                 810                 815

ttc ctg cgg gac agc cgc aag atc ggg cct gcc tat gtg agc agt gga 2496
Phe Leu Arg Asp Ser Arg Lys Ile Gly Pro Ala Tyr Val Ser Ser Gly
            820                 825                 830

ggc agc cgc aat tcc agc tcc ctg gac cac cca gac gaa agg gtg ctg 2544
Gly Ser Arg Asn Ser Ser Ser Leu Asp His Pro Asp Glu Arg Val Leu
        835                 840                 845

acc atg ggc gac ctc atc tcc ttc gcc tgg cag atc tcg agg ggt atg 2592
Thr Met Gly Asp Leu Ile Ser Phe Ala Trp Gln Ile Ser Arg Gly Met
    850                 855                 860

cag tac ttg gct gaa atg aag ctc gta cat cga gac tta gct gcc aga 2640
Gln Tyr Leu Ala Glu Met Lys Leu Val His Arg Asp Leu Ala Ala Arg
865                 870                 875                 880

aac atc ttg gtg gca gag gga cgg aag atg aag atc tct gac ttt ggg 2688
Asn Ile Leu Val Ala Glu Gly Arg Lys Met Lys Ile Ser Asp Phe Gly
                885                 890                 895

ctg tcc cga gat gtt tat gaa gaa gat tcc tat gtg aag aaa agc ang 2736
Leu Ser Arg Asp Val Tyr Glu Glu Asp Ser Tyr Val Lys Lys Ser Xaa
            900                 905                 910

ggc cgg att ccc gtc aat tgg atg gca atc aat tct ctc tcc gat cac 2784
Gly Arg Ile Pro Val Asn Trp Met Ala Ile Asn Ser Leu Ser Asp His
        915                 920                 925

ttc tat acc act caa agt gat gtg tgg tcc ttt gga gtg ctg cta tgg 2832
Phe Tyr Thr Thr Gln Ser Asp Val Trp Ser Phe Gly Val Leu Leu Trp
    930                 935                 940

gaa att gtg acc ctg gga ggc aac ccc tac cct gga att cct cct gaa 2880
Glu Ile Val Thr Leu Gly Gly Asn Pro Tyr Pro Gly Ile Pro Pro Glu
945                 950                 955                 960

cga ctc ttc aac ctt ctg aag aca ggc cac agg atg gag agg cca aac 2928
Arg Leu Phe Asn Leu Leu Lys Thr Gly His Arg Met Glu Arg Pro Asn
                965                 970                 975

aac tgc agc gag gaa atg tac cgc ctg atg ctg cag tgc tgg aag cag 2976
Asn Cys Ser Glu Glu Met Tyr Arg Leu Met Leu Gln Cys Trp Lys Gln
            980                 985                 990

gag ccg gac aag agg cca gta ttt gct gac atc agc aag gat ctg gag 3024
Glu Pro Asp Lys Arg Pro Val Phe Ala Asp Ile Ser Lys Asp Leu Glu
        995                 1000                1005

aag atg atg gtc aaa agc aga gac tac ttg gac ctg gct gca tcc 3069
Lys Met Met Val Lys Ser Arg Asp Tyr Leu Asp Leu Ala Ala Ser
    1010                1015                1020

acc cct tcg gac tca ctg ctc tat gac gat ggg ctc tcg gaa gag 3114
Thr Pro Ser Asp Ser Leu Leu Tyr Asp Asp Gly Leu Ser Glu Glu
    1025                1030                1035

gag acg ccc ctg gtg gac tgt aac agt gct ccc ctc ccg cgc tcc 3159
Glu Thr Pro Leu Val Asp Cys Asn Ser Ala Pro Leu Pro Arg Ser
    1040                1045                1050

ctc cct tcc aca tgg att gaa aac aaa ctc tat ggt atg tca gac 3204
Leu Pro Ser Thr Trp Ile Glu Asn Lys Leu Tyr Gly Met Ser Asp
    1055                1060                1065

ccg aac tgg cct gga gag agt cct gta cca ctc acg aga gcc gat 3249
Pro Asn Trp Pro Gly Glu Ser Pro Val Pro Leu Thr Arg Ala Asp
    1070                1075                1080

ggc act agc act ggg ttc cca aga tat gca aat gat agt gta tat 3294
Gly Thr Ser Thr Gly Phe Pro Arg Tyr Ala Asn Asp Ser Val Tyr
    1085                1090                1095

gct aac tgg atg gtt tca ccc tca gcg gca aaa tta atg gac aca 3339
Ala Asn Trp Met Val Ser Pro Ser Ala Ala Lys Leu Met Asp Thr
    1100                1105                1110

ttt gat agc taa 3351
Phe Asp Ser
    1115

<210> 10
<211> 1116
<212> PRT
<213> Rattus norvegicus

<220>
<221> misc_feature
<222> (666)..(666)
<223> The 'Xaa' at location 666 stands for Glu, Gly, Ala, or Val.

<220>
<221> misc_feature
<222> (667)..(667)
<223> The 'Xaa' at location 667 stands for Gln, His, Arg, Pro, or Leu.

<220>
<221> misc_feature
<222> (672)..(672)
<223> The 'Xaa' at location 672 stands for Glu, Gly, Ala, or Val.

<220>
<221> misc_feature
<222> (694)..(694)
<223> The 'Xaa' at location 694 stands for Thr, Ala, Pro, or Ser.

<220>
<221> misc_feature
<222> (912)..(912)
<223> The 'Xaa' at location 912 stands for Lys, Arg, Thr, or Met.

<400> 10

Met Ala Lys Ala Arg Ser Gly Ala Ala Gly Leu Gly Leu Lys Leu Phe
1               5                   10                  15

Leu Leu Leu Pro Leu Leu Gly Glu Ala Pro Leu Gly Leu Cys Phe Ser
            20                  25                  30

Arg Asp Ala Tyr Trp Glu Arg Leu Tyr Val Asp Gln Pro Ala Gly Thr
        35                  40                  45

Pro Leu Leu Tyr Val His Ala Leu Arg Asp Ala Pro Gly Glu Val Pro
    50                  55                  60

Ser Phe Arg Leu Gly Gln Tyr Leu Tyr Gly Val Tyr Arg Thr Arg Leu
65                  70                  75                  80

His Glu Asn Asp Trp Ile His Ile Asp Ala Gly Thr Gly Leu Leu Tyr
                85                  90                  95

Leu Asn Gln Ser Leu Asp His Ser Ser Trp Glu Gln Leu Ser Ile Arg
            100                 105                 110

Asn Gly Gly Phe Pro Leu Leu Thr Val Phe Leu Gln Val Phe Leu Gly
        115                 120                 125

Ser Thr Ala Gln Arg Glu Gly Glu Cys His Trp Pro Gly Cys Ala Arg
    130                 135                 140

Val Tyr Phe Ser Phe Ile Asn Asp Thr Phe Pro Asn Cys Ser Ser Phe
145                 150                 155                 160

Lys Ala Arg Asp Leu Cys Thr Pro Glu Thr Gly Val Ser Phe Arg Ile
                165                 170                 175

Arg Glu Asn Arg Pro Pro Gly Thr Phe Tyr Gln Phe Arg Met Leu Pro
            180                 185                 190

Val Gln Phe Leu Cys Pro Asn Ile Ser Val Lys Tyr Lys Leu Leu Glu
        195                 200                 205

Gly Asp Gly Leu Pro Phe Arg Cys Asp Pro Asp Cys Leu Glu Val Ser
    210                 215                 220

Thr Arg Trp Ala Leu Asp Arg Glu Leu Gln Glu Lys Tyr Val Leu Glu
225                 230                 235                 240

Ala Glu Cys Ala Val Ala Gly Pro Gly Ala Asn Lys Glu Lys Val Ala
                245                 250                 255

Val Ser Phe Pro Val Thr Val Tyr Asp Asp Glu Asp Asp Ser Pro Pro
            260                 265                 270

Thr Phe Ser Gly Gly Val Gly Thr Ala Ser Ala Val Val Glu Phe Lys
        275                 280                 285

Arg Lys Glu Gly Thr Val Val Ala Thr Leu Gln Val Phe Asp Ala Asp
    290                 295                 300

Val Val Pro Ala Ser Gly Glu Leu Val Arg Arg Tyr Thr Ser Thr Leu
305                 310                 315                 320

Leu Ser Gly Asp Ser Trp Ala Gln Gln Thr Phe Arg Val Glu His Thr
                325                 330                 335

Pro Asn Glu Thr Leu Val Gln Ser Asn Asn Asn Ser Val Arg Ala Thr
            340                 345                 350

Met His Asn Tyr Arg Leu Val Leu Asn Arg Ser Leu Ser Ile Ser Glu
        355                 360                 365

Ser Arg Val Leu Gln Leu Val Val Leu Val Asn Asp Ser Asp Phe Gln
    370                 375                 380

Gly Pro Gly Ser Gly Phe Leu Phe Leu His Phe Asn Val Ser Val Leu
385                 390                 395                 400

Pro Val Thr Leu Asn Leu Pro Met Ala Tyr Ser Phe Pro Val Asn Arg
                405                 410                 415

Arg Ala Arg Arg Tyr Ala Gln Ile Gly Lys Val Cys Val Glu Asn Cys
            420                 425                 430

Gln Glu Phe Ser Gly Val Ser Ile Gln Tyr Lys Leu Gln Pro Ser Ser
        435                 440                 445

Thr Asn Cys Ser Ala Leu Gly Val Val Thr Ser Thr Glu Asp Thr Ser
    450                 455                 460

Gly Thr Leu Tyr Val Asn Asp Thr Glu Ala Leu Arg Arg Pro Glu Cys
465                 470                 475                 480

Thr Glu Leu Gln Tyr Thr Val Val Ala Thr Asp Arg Gln Thr Arg Arg
                485                 490                 495

Gln Thr Gln Ala Ser Leu Val Val Thr Val Glu Gly Thr Tyr Ile Ala
            500                 505                 510

Glu Glu Val Gly Cys Pro Lys Ser Cys Ala Val Asn Lys Arg Arg Pro
        515                 520                 525

Glu Cys Glu Glu Cys Gly Gly Leu Gly Ser Pro Thr Gly Arg Cys Glu
    530                 535                 540

Trp Arg Gln Gly Asp Gly Lys Gly Thr Thr Arg Asn Phe Ser Thr Cys
545                 550                 555                 560

Ser Pro Ser Thr Arg Thr Cys Pro Asp Gly His Cys Asp Ala Leu Glu
                565                 570                 575

Ser Arg Asp Ile Asn Ile Cys Pro Gln Asp Cys Leu Arg Gly Pro Ile
            580                 585                 590

Val Gly Gly His Glu Arg Gly Glu Arg Gln Gly Ile Lys Ala Gly Tyr
        595                 600                 605

Gly Ile Cys Asn Cys Phe Pro Asp Glu Lys Lys Cys Phe Cys Glu Pro
    610                 615                 620

Glu Asp Ser Gln Ala Pro Leu Cys Asp Glu Leu Cys Arg Thr Val Ile
625                 630                 635                 640

Thr Ala Ala Val Leu Phe Ser Phe Ile Ile Ser Val Leu Leu Ser Thr
                645                 650                 655

Phe Cys Ile His Arg Tyr His Lys His Xaa Xaa Lys Pro Pro Ile Xaa
            660                 665                 670

Ser Ala Glu Met Thr Phe Cys Arg Pro Ala Gln Gly Phe Pro Ile Ser
        675                 680                 685

Tyr Ser Ser Ser Gly Xaa Arg Arg Pro Ser Leu Asp Ser Met Glu Asn
    690                 695                 700

Gln Val Ser Val Asp Ser Phe Lys Ile Pro Glu Asp Pro Lys Trp Glu
705                 710                 715                 720

Phe Pro Arg Lys Asn Leu Val Leu Gly Lys Thr Leu Gly Glu Gly Glu
                725                 730                 735

Phe Gly Lys Val Val Lys Ala Thr Ala Phe Arg Leu Lys Asp Arg Ala
            740                 745                 750

Gly Tyr Thr Thr Val Ala Val Lys Met Leu Lys Glu Asn Ala Ser Gln
        755                 760                 765

Ser Glu Leu Arg Asp Leu Leu Ser Glu Phe Asn Leu Leu Lys Gln Val
    770                 775                 780

Asn His Pro His Val Ile Lys Leu Tyr Gly Ala Cys Ser Gln Asp Gly
785                 790                 795                 800

Pro Leu Leu Leu Ile Val Glu Tyr Ala Lys Tyr Gly Ser Leu Arg Gly

                805                 810                 815

Phe Leu Arg Asp Ser Arg Lys Ile Gly Pro Ala Tyr Val Ser Ser Gly
            820                 825                 830

Gly Ser Arg Asn Ser Ser Ser Leu Asp His Pro Asp Glu Arg Val Leu
        835                 840                 845

Thr Met Gly Asp Leu Ile Ser Phe Ala Trp Gln Ile Ser Arg Gly Met
    850                 855                 860

Gln Tyr Leu Ala Glu Met Lys Leu Val His Arg Asp Leu Ala Ala Arg
865                 870                 875                 880

Asn Ile Leu Val Ala Glu Gly Arg Lys Met Lys Ile Ser Asp Phe Gly
                885                 890                 895

Leu Ser Arg Asp Val Tyr Glu Glu Asp Ser Tyr Val Lys Lys Ser Xaa
            900                 905                 910

Gly Arg Ile Pro Val Asn Trp Met Ala Ile Asn Ser Leu Ser Asp His
        915                 920                 925

Phe Tyr Thr Thr Gln Ser Asp Val Trp Ser Phe Gly Val Leu Leu Trp
    930                 935                 940

Glu Ile Val Thr Leu Gly Gly Asn Pro Tyr Pro Gly Ile Pro Pro Glu
945                 950                 955                 960

Arg Leu Phe Asn Leu Leu Lys Thr Gly His Arg Met Glu Arg Pro Asn
                965                 970                 975

Asn Cys Ser Glu Glu Met Tyr Arg Leu Met Leu Gln Cys Trp Lys Gln
            980                 985                 990

Glu Pro Asp Lys Arg Pro Val Phe Ala Asp Ile Ser Lys Asp Leu Glu
        995                 1000                1005

Lys Met Met Val Lys Ser Arg Asp Tyr Leu Asp Leu Ala Ala Ser
    1010                1015                1020

Thr Pro Ser Asp Ser Leu Leu Tyr Asp Asp Gly Leu Ser Glu Glu
    1025                1030                1035

Glu Thr Pro Leu Val Asp Cys Asn Ser Ala Pro Leu Pro Arg Ser
    1040                1045                1050

Leu Pro Ser Thr Trp Ile Glu Asn Lys Leu Tyr Gly Met Ser Asp
    1055                1060                1065

Pro Asn Trp Pro Gly Glu Ser Pro Val Pro Leu Thr Arg Ala Asp
    1070                1075                1080

Gly Thr Ser Thr Gly Phe Pro Arg Tyr Ala Asn Asp Ser Val Tyr
    1085                1090                1095

Ala Asn Trp Met Val Ser Pro Ser Ala Ala Lys Leu Met Asp Thr
    1100                1105                1110

Phe Asp Ser
    1115

<210>  11
<211>  3225
<212>  DNA
<213>  Rattus norvegicus

<220>
<221>  CDS
<222>  (1)..(3222)

<220>
<221>  misc_feature
<222>  (1485)..(1485)
<223>  n is a, c, g, or t

<220>
<221>  misc_feature
<222>  (1997)..(1997)
<223>  n is a, c, g, or t

<220>
<221> misc_feature
<222> (2000)..(2001)
<223> n is a, c, g, or t

<220>
<221> misc_feature
<222> (2015)..(2015)
<223> n is a, c, g, or t

<220>
<221> misc_feature
<222> (2022)..(2022)
<223> n is a, c, g, or t

<220>
<221> misc_feature
<222> (2043)..(2043)
<223> n is a, c, g, or t

<220>
<221> misc_feature
<222> (2052)..(2052)
<223> n is a, c, g, or t

<220>
<221> misc_feature
<222> (2080)..(2080)
<223> n is a, c, g, or t

<220>
<221> misc_feature
<222> (2735)..(2735)
<223> n is a, c, g, or t

<400> 11
atg gcg aaa gcg agg tcc ggc gcc gca ggg ctg ggg ctg aag ctg ttt 48
Met Ala Lys Ala Arg Ser Gly Ala Ala Gly Leu Gly Leu Lys Leu Phe
1               5                   10                  15

ttg ctg ctg ccg cta ctg gga gaa gcc ccg ctg ggt ctc tgc ttc tca 96
Leu Leu Leu Pro Leu Leu Gly Glu Ala Pro Leu Gly Leu Cys Phe Ser
            20                  25                  30

agg gat gct tac tgg gag agg ctg tat gtg gac cag cca gct ggc aca 144
Arg Asp Ala Tyr Trp Glu Arg Leu Tyr Val Asp Gln Pro Ala Gly Thr
        35                  40                  45

cct ctg ctc tat gtc cat gcc cta cgg gat gcc cct gga gaa gtg ccc 192
Pro Leu Leu Tyr Val His Ala Leu Arg Asp Ala Pro Gly Glu Val Pro

50                  55                  60

agc ttc cgc ctg ggc cag tat ctc tat ggc gtc tac cgc acg cgt ctg        240
Ser Phe Arg Leu Gly Gln Tyr Leu Tyr Gly Val Tyr Arg Thr Arg Leu
65                  70                  75                  80

cat gag aat gac tgg atc cac atc gat gcg ggc act ggc ctc ctc tac        288
His Glu Asn Asp Trp Ile His Ile Asp Ala Gly Thr Gly Leu Leu Tyr
                85                  90                  95

ctc aat cag agc ctg gac cat agt tcc tgg gag cag ctc agc atc cga        336
Leu Asn Gln Ser Leu Asp His Ser Ser Trp Glu Gln Leu Ser Ile Arg
            100                 105                 110

aat ggc ggc ttc ccc ttg ctc acc gtc ttc ctc cag gtc ttc ctg ggg        384
Asn Gly Gly Phe Pro Leu Leu Thr Val Phe Leu Gln Val Phe Leu Gly
        115                 120                 125

tcc aca gcc cag aga gag gga gag tgt cat tgg cca ggc tgt gcc cgt        432
Ser Thr Ala Gln Arg Glu Gly Glu Cys His Trp Pro Gly Cys Ala Arg
    130                 135                 140

gtc tac ttc tcc ttc atc aac gac acc ttc cca aat tgt agc tcc ttc        480
Val Tyr Phe Ser Phe Ile Asn Asp Thr Phe Pro Asn Cys Ser Ser Phe
145                 150                 155                 160

aaa gcc cgg gat ctc tgc acc cca gag acg ggt gtg tcc ttc cgc atc        528
Lys Ala Arg Asp Leu Cys Thr Pro Glu Thr Gly Val Ser Phe Arg Ile
                165                 170                 175

agg gag aac agg ccc cct ggc acc ttc tac cag ttc cgc atg cta cct        576
Arg Glu Asn Arg Pro Pro Gly Thr Phe Tyr Gln Phe Arg Met Leu Pro
            180                 185                 190

gtg cag ttc ctt tgt cct aac atc agt gtg aag tac aaa ctc tta gaa        624
Val Gln Phe Leu Cys Pro Asn Ile Ser Val Lys Tyr Lys Leu Leu Glu
        195                 200                 205

ggg gac ggt ctg ccc ttc cgt tgt gac ccc gac tgt ctg gag gtg agc        672
Gly Asp Gly Leu Pro Phe Arg Cys Asp Pro Asp Cys Leu Glu Val Ser
    210                 215                 220

act cgg tgg gca ctg gat cgt gag ctt cag gag aag tat gtg ctg gag        720
Thr Arg Trp Ala Leu Asp Arg Glu Leu Gln Glu Lys Tyr Val Leu Glu
225                 230                 235                 240

gct gag tgc gca gtg gca ggc cct gga gcc aac aag gag aag gtg gcc        768
Ala Glu Cys Ala Val Ala Gly Pro Gly Ala Asn Lys Glu Lys Val Ala
                245                 250                 255

gtg tcc ttc ccg gtg acg gtg tat gat gat gaa gac gac tcc ccg ccc        816

Val Ser Phe Pro Val Thr Val Tyr Asp Asp Glu Asp Asp Ser Pro Pro
            260                 265                 270

acc ttc tcc gga ggt gtg ggc acc gcc agt gct gtg gtg gag ttt aag 864
Thr Phe Ser Gly Gly Val Gly Thr Ala Ser Ala Val Val Glu Phe Lys
        275                 280                 285

cgg aag gag ggc act gtg gta gcc act ctg cag gtg ttt gat gca gat 912
Arg Lys Glu Gly Thr Val Val Ala Thr Leu Gln Val Phe Asp Ala Asp
    290                 295                 300

gtg gtg cca gca tct ggg gag ctg gtg agg cgg tac aca agc aca cta 960
Val Val Pro Ala Ser Gly Glu Leu Val Arg Arg Tyr Thr Ser Thr Leu
305                 310                 315                 320

ctc tca ggg gat tcc tgg gcc cag cag acc ttc cgg gtg gag cac aca 1008
Leu Ser Gly Asp Ser Trp Ala Gln Gln Thr Phe Arg Val Glu His Thr
                325                 330                 335

ccc aac gag acc ttg gtc cag tcc aac aac aac tcc gtg cgg gca acc 1056
Pro Asn Glu Thr Leu Val Gln Ser Asn Asn Asn Ser Val Arg Ala Thr
            340                 345                 350

atg cac aat tac agg ctg gtt ctc aac agg agc ctg tcg atc tca gag 1104
Met His Asn Tyr Arg Leu Val Leu Asn Arg Ser Leu Ser Ile Ser Glu
        355                 360                 365

agc cga gtc ctg cag cta gta gtc ctg gtc aat gac tca gac ttc cag 1152
Ser Arg Val Leu Gln Leu Val Val Leu Val Asn Asp Ser Asp Phe Gln
    370                 375                 380

ggg cct ggg tca ggt ttt ctc ttc ctc cat ttc aac gtg tct gtg ctg 1200
Gly Pro Gly Ser Gly Phe Leu Phe Leu His Phe Asn Val Ser Val Leu
385                 390                 395                 400

cct gtc acc ctg aac cta ccc atg gcc tac tcc ttc cca gtg aat agg 1248
Pro Val Thr Leu Asn Leu Pro Met Ala Tyr Ser Phe Pro Val Asn Arg
                405                 410                 415

aga gcc cgc cgt tat gcc cag att ggg aaa gtt tgc gtg gag aac tgc 1296
Arg Ala Arg Arg Tyr Ala Gln Ile Gly Lys Val Cys Val Glu Asn Cys
            420                 425                 430

cag gag ttc agc ggt gtc tcc atc cag tac aag ctg cag ccc tcc agc 1344
Gln Glu Phe Ser Gly Val Ser Ile Gln Tyr Lys Leu Gln Pro Ser Ser
        435                 440                 445

acc aac tgc agt gcc cta ggt gtg gtc acc tca aca gaa gac acc tca 1392
Thr Asn Cys Ser Ala Leu Gly Val Val Thr Ser Thr Glu Asp Thr Ser
    450                 455                 460

ggg acc cta tat gta aat gac acg gag gcg ctg cgg cga cct gag tgt 1440
Gly Thr Leu Tyr Val Asn Asp Thr Glu Ala Leu Arg Arg Pro Glu Cys
465                 470                 475                 480

acc gag ctt cag tac aca gtg gta gcc act gac cgg cag acc cgn agg 1488
Thr Glu Leu Gln Tyr Thr Val Val Ala Thr Asp Arg Gln Thr Arg Arg
                485                 490                 495

cag acc caa gct tcg tta gtc gtc aca gtg gag ggg aca tac att gca 1536
Gln Thr Gln Ala Ser Leu Val Val Thr Val Glu Gly Thr Tyr Ile Ala
            500                 505                 510

gaa gaa gtg ggc tgc ccc aag tcc tgt gca gta aac aag agg cgc cct 1584
Glu Glu Val Gly Cys Pro Lys Ser Cys Ala Val Asn Lys Arg Arg Pro
        515                 520                 525

gag tgt gag gag tgt ggt ggc ctg ggt tct cca act ggc aga tgt gag 1632
Glu Cys Glu Glu Cys Gly Gly Leu Gly Ser Pro Thr Gly Arg Cys Glu
    530                 535                 540

tgg cgt cag gga gat ggt aaa ggg acc acc agg aac ttc tcc acc tgt 1680
Trp Arg Gln Gly Asp Gly Lys Gly Thr Thr Arg Asn Phe Ser Thr Cys
545                 550                 555                 560

tct cct agc acc agg acc tgt cct gat ggc cac tgt gat gct ctg gag 1728
Ser Pro Ser Thr Arg Thr Cys Pro Asp Gly His Cys Asp Ala Leu Glu
                565                 570                 575

agc cgg gat atc aac att tgc ccc cag gac tgt ctc cgt ggc ccc att 1776
Ser Arg Asp Ile Asn Ile Cys Pro Gln Asp Cys Leu Arg Gly Pro Ile
            580                 585                 590

gtt ggc ggg cat gag cga ggg gag cgc cag gga att aaa gcc ggc tat 1824
Val Gly Gly His Glu Arg Gly Glu Arg Gln Gly Ile Lys Ala Gly Tyr
        595                 600                 605

ggc atc tgc aac tgt ttc cct gat gag aag aag tgc ttc tgc gag cca 1872
Gly Ile Cys Asn Cys Phe Pro Asp Glu Lys Lys Cys Phe Cys Glu Pro
    610                 615                 620

gag gac agc cag gcc cca ttg tgc gat gag ctg tgc cgt acg gtc atc 1920
Glu Asp Ser Gln Ala Pro Leu Cys Asp Glu Leu Cys Arg Thr Val Ile
625                 630                 635                 640

aca gcc gct gtc ctc ttc tcc ttc ata atc tct gtc ctg ctg tcc acc 1968
Thr Ala Ala Val Leu Phe Ser Phe Ile Ile Ser Val Leu Leu Ser Thr
                645                 650                 655

ttc tgc atc cac cgc tac cac aag cat gng cnn aag cca ccc atc gng 2016
Phe Cys Ile His Arg Tyr His Lys His Xaa Xaa Lys Pro Pro Ile Xaa
            660                 665                 670

tca gcn gaa atg acc ttc tgc cgg ccn gcc cag ggn ttc cca atc agc 2064
Ser Ala Glu Met Thr Phe Cys Arg Pro Ala Gln Gly Phe Pro Ile Ser
        675                 680                 685

tat tct tcc tcg ggc ncc cgc cgg ccc tca ctg gac tcc atg gag aac 2112
Tyr Ser Ser Ser Gly Xaa Arg Arg Pro Ser Leu Asp Ser Met Glu Asn
    690                 695                 700

cag gtc tct gtg gac tct ttc aag atc ccg gag gat ccg aag tgg gaa 2160
Gln Val Ser Val Asp Ser Phe Lys Ile Pro Glu Asp Pro Lys Trp Glu
705                 710                 715                 720

ttt cct cgg aag aac tta gtt ctt ggg aaa acc ctg gga gaa ggc gag 2208
Phe Pro Arg Lys Asn Leu Val Leu Gly Lys Thr Leu Gly Glu Gly Glu
                725                 730                 735

ttt gga aaa gta gtc aag gcc aca gcc ttc cgt ctg aaa gac cgg gca 2256
Phe Gly Lys Val Val Lys Ala Thr Ala Phe Arg Leu Lys Asp Arg Ala
            740                 745                 750

gga tac acc aca gtg gct gtg aaa atg ctg aaa gaa aac gcc tcc cag 2304
Gly Tyr Thr Thr Val Ala Val Lys Met Leu Lys Glu Asn Ala Ser Gln
        755                 760                 765

agt gaa cta cga gac ctg ctc tct gag ttc aac ctt ctg aaa caa gtc 2352
Ser Glu Leu Arg Asp Leu Leu Ser Glu Phe Asn Leu Leu Lys Gln Val
    770                 775                 780

aac cat cca cat gtc atc aag ttg tac ggg gct tgc agc cag gat ggg 2400
Asn His Pro His Val Ile Lys Leu Tyr Gly Ala Cys Ser Gln Asp Gly
785                 790                 795                 800

cca ctt ctt ctc att gtg gag tat gca aag tat gga tcc ctg cgg ggg 2448
Pro Leu Leu Leu Ile Val Glu Tyr Ala Lys Tyr Gly Ser Leu Arg Gly
                805                 810                 815

ttc ctg cgg gac agc cgc aag atc ggg cct gcc tat gtg agc agt gga 2496
Phe Leu Arg Asp Ser Arg Lys Ile Gly Pro Ala Tyr Val Ser Ser Gly
            820                 825                 830

ggc agc cgc aat tcc agc tcc ctg gac cac cca gac gaa agg gtg ctg 2544
Gly Ser Arg Asn Ser Ser Ser Leu Asp His Pro Asp Glu Arg Val Leu
        835                 840                 845

acc atg ggc gac ctc atc tcc ttc gcc tgg cag atc tcg agg ggt atg 2592
Thr Met Gly Asp Leu Ile Ser Phe Ala Trp Gln Ile Ser Arg Gly Met
    850                 855                 860

cag tac ttg gct gaa atg aag ctc gta cat cga gac tta gct gcc aga 2640
Gln Tyr Leu Ala Glu Met Lys Leu Val His Arg Asp Leu Ala Ala Arg

865                 870                 875                 880

aac atc ttg gtg gca gag gga cgg aag atg aag atc tct gac ttt ggg     2688
Asn Ile Leu Val Ala Glu Gly Arg Lys Met Lys Ile Ser Asp Phe Gly
                885                 890                 895

ctg tcc cga gat gtt tat gaa gaa gat tcc tat gtg aag aaa agc ang     2736
Leu Ser Arg Asp Val Tyr Glu Glu Asp Ser Tyr Val Lys Lys Ser Xaa
            900                 905                 910

ggc cgg att ccc gtc aat tgg atg gca atc aat tct ctc tcc gat cac     2784
Gly Arg Ile Pro Val Asn Trp Met Ala Ile Asn Ser Leu Ser Asp His
        915                 920                 925

ttc tat acc act caa agt gat gtg tgg tcc ttt gga gtg ctg cta tgg     2832
Phe Tyr Thr Thr Gln Ser Asp Val Trp Ser Phe Gly Val Leu Leu Trp
    930                 935                 940

gaa att gtg acc ctg gga ggc aac ccc tac cct gga att cct cct gaa     2880
Glu Ile Val Thr Leu Gly Gly Asn Pro Tyr Pro Gly Ile Pro Pro Glu
945                 950                 955                 960

cga ctc ttc aac ctt ctg aag aca ggc cac agg atg gag agg cca aac     2928
Arg Leu Phe Asn Leu Leu Lys Thr Gly His Arg Met Glu Arg Pro Asn
                965                 970                 975

aac tgc agc gag gaa atg tac cgc ctg atg ctg cag tgc tgg aag cag     2976
Asn Cys Ser Glu Glu Met Tyr Arg Leu Met Leu Gln Cys Trp Lys Gln
            980                 985                 990

gag ccg gac aag agg cca gta ttt  gct gac atc agc aag  gat ctg gag     3024
Glu Pro Asp Lys Arg Pro Val Phe  Ala Asp Ile Ser Lys  Asp Leu Glu
        995                 1000                1005

aag atg  atg gtc aaa agc aga  gac tac ttg gac ctg  gct gca tcc     3069
Lys Met  Met Val Lys Ser Arg  Asp Tyr Leu Asp Leu  Ala Ala Ser
    1010                1015                1020

acc cct  tcg gac tca ctg ctc  tat gac gat ggg ctc  tcg gaa gag     3114
Thr Pro  Ser Asp Ser Leu Leu  Tyr Asp Asp Gly Leu  Ser Glu Glu
    1025                1030                1035

gag acg  ccc ctg gtg gac tgt  aac agt gct ccc ctc  ccg cgc tcc     3159
Glu Thr  Pro Leu Val Asp Cys  Asn Ser Ala Pro Leu  Pro Arg Ser
    1040                1045                1050

ctc cct  tcc aca tgg att gaa  aac aaa ctc tat ggt  aga att tca     3204
Leu Pro  Ser Thr Trp Ile Glu  Asn Lys Leu Tyr Gly  Arg Ile Ser
    1055                1060                1065

cat gca  ttt act aga ttc tag                                       3225

His Ala  Phe Thr Arg Phe
    1070

<210>  12
<211>  1074
<212>  PRT
<213>  Rattus norvegicus

<220>
<221>  misc_feature
<222>  (666)..(666)
<223>  The 'Xaa' at location 666 stands for Glu, Gly, Ala, or Val.

<220>
<221>  misc_feature
<222>  (667)..(667)
<223>  The 'Xaa' at location 667 stands for Gln, His, Arg, Pro, or Leu.

<220>
<221>  misc_feature
<222>  (672)..(672)
<223>  The 'Xaa' at location 672 stands for Glu, Gly, Ala, or Val.

<220>
<221>  misc_feature
<222>  (694)..(694)
<223>  The 'Xaa' at location 694 stands for Thr, Ala, Pro, or Ser.

<220>
<221>  misc_feature
<222>  (912)..(912)
<223>  The 'Xaa' at location 912 stands for Lys, Arg, Thr, or Met.

<400>  12

Met Ala Lys Ala Arg Ser Gly Ala Ala Gly Leu Gly Leu Lys Leu Phe
1               5                   10                  15

Leu Leu Leu Pro Leu Leu Gly Glu Ala Pro Leu Gly Leu Cys Phe Ser
            20                  25                  30

Arg Asp Ala Tyr Trp Glu Arg Leu Tyr Val Asp Gln Pro Ala Gly Thr
        35                  40                  45

Pro Leu Leu Tyr Val His Ala Leu Arg Asp Ala Pro Gly Glu Val Pro
    50                  55                  60

Ser Phe Arg Leu Gly Gln Tyr Leu Tyr Gly Val Tyr Arg Thr Arg Leu
65                  70                  75                  80

His Glu Asn Asp Trp Ile His Ile Asp Ala Gly Thr Gly Leu Leu Tyr
                85                  90                  95

Leu Asn Gln Ser Leu Asp His Ser Ser Trp Glu Gln Leu Ser Ile Arg
            100                 105                 110

Asn Gly Gly Phe Pro Leu Leu Thr Val Phe Leu Gln Val Phe Leu Gly
        115                 120                 125

Ser Thr Ala Gln Arg Glu Gly Glu Cys His Trp Pro Gly Cys Ala Arg
    130                 135                 140

Val Tyr Phe Ser Phe Ile Asn Asp Thr Phe Pro Asn Cys Ser Ser Phe
145                 150                 155                 160

Lys Ala Arg Asp Leu Cys Thr Pro Glu Thr Gly Val Ser Phe Arg Ile
                165                 170                 175

Arg Glu Asn Arg Pro Pro Gly Thr Phe Tyr Gln Phe Arg Met Leu Pro
            180                 185                 190

Val Gln Phe Leu Cys Pro Asn Ile Ser Val Lys Tyr Lys Leu Leu Glu
        195                 200                 205

Gly Asp Gly Leu Pro Phe Arg Cys Asp Pro Asp Cys Leu Glu Val Ser
    210                 215                 220

Thr Arg Trp Ala Leu Asp Arg Glu Leu Gln Glu Lys Tyr Val Leu Glu
225                 230                 235                 240

Ala Glu Cys Ala Val Ala Gly Pro Gly Ala Asn Lys Glu Lys Val Ala
                245                 250                 255

Val Ser Phe Pro Val Thr Val Tyr Asp Asp Glu Asp Asp Ser Pro Pro
            260                 265                 270

Thr Phe Ser Gly Gly Val Gly Thr Ala Ser Ala Val Val Glu Phe Lys
        275                 280                 285

Arg Lys Glu Gly Thr Val Val Ala Thr Leu Gln Val Phe Asp Ala Asp
    290                 295                 300

Val Val Pro Ala Ser Gly Glu Leu Val Arg Arg Tyr Thr Ser Thr Leu
305                 310                 315                 320

Leu Ser Gly Asp Ser Trp Ala Gln Gln Thr Phe Arg Val Glu His Thr
                325                 330                 335

Pro Asn Glu Thr Leu Val Gln Ser Asn Asn Asn Ser Val Arg Ala Thr
            340                 345                 350

Met His Asn Tyr Arg Leu Val Leu Asn Arg Ser Leu Ser Ile Ser Glu
        355                 360                 365

Ser Arg Val Leu Gln Leu Val Val Leu Val Asn Asp Ser Asp Phe Gln
    370                 375                 380

Gly Pro Gly Ser Gly Phe Leu Phe Leu His Phe Asn Val Ser Val Leu
385                 390                 395                 400

Pro Val Thr Leu Asn Leu Pro Met Ala Tyr Ser Phe Pro Val Asn Arg
                405                 410                 415

Arg Ala Arg Arg Tyr Ala Gln Ile Gly Lys Val Cys Val Glu Asn Cys
            420                 425                 430

Gln Glu Phe Ser Gly Val Ser Ile Gln Tyr Lys Leu Gln Pro Ser Ser
        435                 440                 445

Thr Asn Cys Ser Ala Leu Gly Val Val Thr Ser Thr Glu Asp Thr Ser
    450                 455                 460

Gly Thr Leu Tyr Val Asn Asp Thr Glu Ala Leu Arg Arg Pro Glu Cys

465                 470                 475                 480

Thr Glu Leu Gln Tyr Thr Val Val Ala Thr Asp Arg Gln Thr Arg Arg
                485                 490                 495

Gln Thr Gln Ala Ser Leu Val Val Thr Val Glu Gly Thr Tyr Ile Ala
            500                 505                 510

Glu Glu Val Gly Cys Pro Lys Ser Cys Ala Val Asn Lys Arg Arg Pro
        515                 520                 525

Glu Cys Glu Glu Cys Gly Gly Leu Gly Ser Pro Thr Gly Arg Cys Glu
    530                 535                 540

Trp Arg Gln Gly Asp Gly Lys Gly Thr Thr Arg Asn Phe Ser Thr Cys
545                 550                 555                 560

Ser Pro Ser Thr Arg Thr Cys Pro Asp Gly His Cys Asp Ala Leu Glu
                565                 570                 575

Ser Arg Asp Ile Asn Ile Cys Pro Gln Asp Cys Leu Arg Gly Pro Ile
            580                 585                 590

Val Gly Gly His Glu Arg Gly Glu Arg Gln Gly Ile Lys Ala Gly Tyr
        595                 600                 605

Gly Ile Cys Asn Cys Phe Pro Asp Glu Lys Lys Cys Phe Cys Glu Pro
    610                 615                 620

Glu Asp Ser Gln Ala Pro Leu Cys Asp Glu Leu Cys Arg Thr Val Ile
625                 630                 635                 640

Thr Ala Ala Val Leu Phe Ser Phe Ile Ile Ser Val Leu Leu Ser Thr
                645                 650                 655

Phe Cys Ile His Arg Tyr His Lys His Xaa Xaa Lys Pro Pro Ile Xaa
            660                 665                 670

Ser Ala Glu Met Thr Phe Cys Arg Pro Ala Gln Gly Phe Pro Ile Ser
        675                 680                 685

Tyr Ser Ser Ser Gly Xaa Arg Arg Pro Ser Leu Asp Ser Met Glu Asn
    690                 695                 700

Gln Val Ser Val Asp Ser Phe Lys Ile Pro Glu Asp Pro Lys Trp Glu
705                 710                 715                 720

Phe Pro Arg Lys Asn Leu Val Leu Gly Lys Thr Leu Gly Glu Gly Glu
                725                 730                 735

Phe Gly Lys Val Val Lys Ala Thr Ala Phe Arg Leu Lys Asp Arg Ala
            740                 745                 750

Gly Tyr Thr Thr Val Ala Val Lys Met Leu Lys Glu Asn Ala Ser Gln
        755                 760                 765

Ser Glu Leu Arg Asp Leu Leu Ser Glu Phe Asn Leu Leu Lys Gln Val
    770                 775                 780

Asn His Pro His Val Ile Lys Leu Tyr Gly Ala Cys Ser Gln Asp Gly
785                 790                 795                 800

Pro Leu Leu Leu Ile Val Glu Tyr Ala Lys Tyr Gly Ser Leu Arg Gly
                805                 810                 815

Phe Leu Arg Asp Ser Arg Lys Ile Gly Pro Ala Tyr Val Ser Ser Gly
            820                 825                 830

Gly Ser Arg Asn Ser Ser Ser Leu Asp His Pro Asp Glu Arg Val Leu
        835                 840                 845

Thr Met Gly Asp Leu Ile Ser Phe Ala Trp Gln Ile Ser Arg Gly Met
    850                 855                 860

Gln Tyr Leu Ala Glu Met Lys Leu Val His Arg Asp Leu Ala Ala Arg
865                 870                 875                 880

Asn Ile Leu Val Ala Glu Gly Arg Lys Met Lys Ile Ser Asp Phe Gly
                885                 890                 895

Leu Ser Arg Asp Val Tyr Glu Glu Asp Ser Tyr Val Lys Lys Ser Xaa
            900                 905                 910

Gly Arg Ile Pro Val Asn Trp Met Ala Ile Asn Ser Leu Ser Asp His
        915                 920                 925

Phe Tyr Thr Thr Gln Ser Asp Val Trp Ser Phe Gly Val Leu Leu Trp
    930                 935                 940

Glu Ile Val Thr Leu Gly Gly Asn Pro Tyr Pro Gly Ile Pro Pro Glu
945                 950                 955                 960

Arg Leu Phe Asn Leu Leu Lys Thr Gly His Arg Met Glu Arg Pro Asn
                965                 970                 975

Asn Cys Ser Glu Glu Met Tyr Arg Leu Met Leu Gln Cys Trp Lys Gln
            980                 985                 990

Glu Pro Asp Lys Arg Pro Val Phe  Ala Asp Ile Ser Lys  Asp Leu Glu
        995                 1000                1005

Lys Met  Met Val Lys Ser Arg  Asp Tyr Leu Asp Leu  Ala Ala Ser
    1010                1015                1020

Thr Pro  Ser Asp Ser Leu Leu  Tyr Asp Asp Gly Leu  Ser Glu Glu
    1025                1030                1035

Glu Thr  Pro Leu Val Asp Cys  Asn Ser Ala Pro Leu  Pro Arg Ser
    1040                1045                1050

Leu Pro  Ser Thr Trp Ile Glu  Asn Lys Leu Tyr Gly  Arg Ile Ser
    1055                1060                1065

His Ala  Phe Thr Arg Phe
    1070

<210> 13
<211> 3387
<212> DNA
<213> Rattus norvegicus

<220>
<221> CDS
<222> (1)..(3384)

<400> 13
atg gcg aaa gcg agg tcc ggc gcc gca ggg ctg ggg ctg aag ctg ttt 48
Met Ala Lys Ala Arg Ser Gly Ala Ala Gly Leu Gly Leu Lys Leu Phe
1               5                   10                  15

ttg ctg ctg ccg cta ctg gga gaa gcc ccg ctg ggt ctc tac ttc tca 96
Leu Leu Leu Pro Leu Leu Gly Glu Ala Pro Leu Gly Leu Tyr Phe Ser
            20                  25                  30

agg gat gct tac tgg gag agg ctg tat gtg gac cag cca gct ggc aca 144
Arg Asp Ala Tyr Trp Glu Arg Leu Tyr Val Asp Gln Pro Ala Gly Thr
        35                  40                  45

cct ctg ctc tat gtc cat gcc cta cgg gat gcc cct gga gaa gtg ccc 192
Pro Leu Leu Tyr Val His Ala Leu Arg Asp Ala Pro Gly Glu Val Pro
    50                  55                  60

agc ttc cgc ctg ggc cag tat ctc tat ggc gtc tac cgc acg cgt ctg 240
Ser Phe Arg Leu Gly Gln Tyr Leu Tyr Gly Val Tyr Arg Thr Arg Leu
65                  70                  75                  80

cat gag aat gac tgg atc cac atc gat gca ggc act ggc ctc ctc tac 288
His Glu Asn Asp Trp Ile His Ile Asp Ala Gly Thr Gly Leu Leu Tyr
                85                  90                  95

ctc aat cag agc ctg gac cat agt tcc tgg gag cag ctc agc atc cga 336
Leu Asn Gln Ser Leu Asp His Ser Ser Trp Glu Gln Leu Ser Ile Arg
            100                 105                 110

aat ggc ggc ttc ccc ttg ctc acc gtc ttc ctc cag gtc ttc ctg ggg 384
Asn Gly Gly Phe Pro Leu Leu Thr Val Phe Leu Gln Val Phe Leu Gly
        115                 120                 125

tcc aca gcc cag aga gag gga gag tgt cat tgg cca ggc tgt gcc cgt 432
Ser Thr Ala Gln Arg Glu Gly Glu Cys His Trp Pro Gly Cys Ala Arg
    130                 135                 140

gtg tac ttc tcc ttc atc aac gac acc ttc cca aat tgt agc tcc ttc 480
Val Tyr Phe Ser Phe Ile Asn Asp Thr Phe Pro Asn Cys Ser Ser Phe

145                 150                 155                 160

aaa gcc cgg gat ctc tgc acc ccg gag acg ggt gtg tcc ttc cgc atc       528
Lys Ala Arg Asp Leu Cys Thr Pro Glu Thr Gly Val Ser Phe Arg Ile
                165                 170                 175

agg gag aac agg ccc cct ggc acc ttc tac cag ttc cgc atg cta cct       576
Arg Glu Asn Arg Pro Pro Gly Thr Phe Tyr Gln Phe Arg Met Leu Pro
            180                 185                 190

gtg cag ttc ctt tgt cct aac atc agt gtg aag tac aaa ctc tta gaa       624
Val Gln Phe Leu Cys Pro Asn Ile Ser Val Lys Tyr Lys Leu Leu Glu
        195                 200                 205

ggg gac ggt ctg ccc ttc cgt tgt gac ccc gac tgt ctg gag gtg agc       672
Gly Asp Gly Leu Pro Phe Arg Cys Asp Pro Asp Cys Leu Glu Val Ser
    210                 215                 220

acg cgg tgg gcg ctg gat cgg gag ctt cag gag aag tat gtg ctg gag       720
Thr Arg Trp Ala Leu Asp Arg Glu Leu Gln Glu Lys Tyr Val Leu Glu
225                 230                 235                 240

gct gag tgc gca gtg gca ggc cct gga gcc aac aag gag aag gtg gcc       768
Ala Glu Cys Ala Val Ala Gly Pro Gly Ala Asn Lys Glu Lys Val Ala
                245                 250                 255

gtg tcc ttc ccg gtg acg gtg tat gat gaa gac gac tcc ccg ccc acc       816
Val Ser Phe Pro Val Thr Val Tyr Asp Glu Asp Asp Ser Pro Pro Thr
            260                 265                 270

ttc tcc gga ggt gtg ggc acc gcc agt gct gtg gtg gag ttt aag cgg       864
Phe Ser Gly Gly Val Gly Thr Ala Ser Ala Val Val Glu Phe Lys Arg
        275                 280                 285

aag gag ggc act gtg gta gcc act ctg cag gtg ttt gat gca gat gtg       912
Lys Glu Gly Thr Val Val Ala Thr Leu Gln Val Phe Asp Ala Asp Val
    290                 295                 300

gtg cca gca tct ggg gag ctg gtg agg cgg tac aca agc aca cta ctc       960
Val Pro Ala Ser Gly Glu Leu Val Arg Arg Tyr Thr Ser Thr Leu Leu
305                 310                 315                 320

tca ggg gat tcc tgg gcc cag cag acc ttc cgg gtg gag cac aca ccc      1008
Ser Gly Asp Ser Trp Ala Gln Gln Thr Phe Arg Val Glu His Thr Pro
                325                 330                 335

aac gag acc ttg gtc cag tcc aac aac aac tcc gtg cgg gca acc atg      1056
Asn Glu Thr Leu Val Gln Ser Asn Asn Asn Ser Val Arg Ala Thr Met
            340                 345                 350

cac aat tac aag ctg gtt ctc aac agg agc ctg tcc atc tca gag agc      1104

His Asn Tyr Lys Leu Val Leu Asn Arg Ser Leu Ser Ile Ser Glu Ser
        355                 360                 365

cga gtc ctg cag cta gta gtc ctg gtc aat gac tca gac ttc cag ggg 1152
Arg Val Leu Gln Leu Val Val Leu Val Asn Asp Ser Asp Phe Gln Gly
    370                 375                 380

cct ggg tca ggt gtt ctc ttc ctc cat ttc aac gtg tct gtg ctg cct 1200
Pro Gly Ser Gly Val Leu Phe Leu His Phe Asn Val Ser Val Leu Pro
385                 390                 395                 400

gtc acc ctg aac cta ccc atg gcc tac tcc ttc cca gtg aat agg aga 1248
Val Thr Leu Asn Leu Pro Met Ala Tyr Ser Phe Pro Val Asn Arg Arg
                405                 410                 415

gcc cgc cgt tat gcc cag att ggg aaa gtt tgc gtg gag aac tgc cag 1296
Ala Arg Arg Tyr Ala Gln Ile Gly Lys Val Cys Val Glu Asn Cys Gln
            420                 425                 430

gag ttc agc ggt gtc tcc atc cag tac aag ctg cag ccc tcc agc acc 1344
Glu Phe Ser Gly Val Ser Ile Gln Tyr Lys Leu Gln Pro Ser Ser Thr
        435                 440                 445

aac tgc agt gcc cta ggt gtg gtc acc tca aca gaa gac acc tca ggg 1392
Asn Cys Ser Ala Leu Gly Val Val Thr Ser Thr Glu Asp Thr Ser Gly
    450                 455                 460

acc cta tat gta aat gac acg gag gcc ctg cgg cga cct gag tgt acc 1440
Thr Leu Tyr Val Asn Asp Thr Glu Ala Leu Arg Arg Pro Glu Cys Thr
465                 470                 475                 480

gag ctt cag tac aca gtg gta gcc act gac cgg cag acc cgc agg cag 1488
Glu Leu Gln Tyr Thr Val Val Ala Thr Asp Arg Gln Thr Arg Arg Gln
                485                 490                 495

acc cag gct tcg tta gtc gtc aca gtg gag ggg aca tac att gca gaa 1536
Thr Gln Ala Ser Leu Val Val Thr Val Glu Gly Thr Tyr Ile Ala Glu
            500                 505                 510

gaa gtg ggc tgc ccc aag tcc tgt gca gta aac aag agg cga cct gag 1584
Glu Val Gly Cys Pro Lys Ser Cys Ala Val Asn Lys Arg Arg Pro Glu
        515                 520                 525

tgt gag gag tgt ggt ggc ctg ggt tct cca act ggc aga tgt gag tgg 1632
Cys Glu Glu Cys Gly Gly Leu Gly Ser Pro Thr Gly Arg Cys Glu Trp
    530                 535                 540

cgt cag gga gat ggt aaa ggg atc acc agg aac ttc tcc acc tgt tct 1680
Arg Gln Gly Asp Gly Lys Gly Ile Thr Arg Asn Phe Ser Thr Cys Ser
545                 550                 555                 560

cct agc acc agg acc tgt cct gat ggc cac tgt gat gct ctg gag agc 1728
Pro Ser Thr Arg Thr Cys Pro Asp Gly His Cys Asp Ala Leu Glu Ser
                565                 570                 575

cgg gat atc aac att tgc ccc cag gac tgt ctc cgt ggc ccc att gtt 1776
Arg Asp Ile Asn Ile Cys Pro Gln Asp Cys Leu Arg Gly Pro Ile Val
            580                 585                 590

ggc ggg cat gag cga ggg gag cgc cag ggg att aaa gcc ggc tat ggc 1824
Gly Gly His Glu Arg Gly Glu Arg Gln Gly Ile Lys Ala Gly Tyr Gly
        595                 600                 605

atc tgc aac tgt ttc cct gat gag aag aag tgc ttc tgc gag cca gag 1872
Ile Cys Asn Cys Phe Pro Asp Glu Lys Lys Cys Phe Cys Glu Pro Glu
    610                 615                 620

gac agc cag ggc cca ttg tgc gat gag ctg tgc cgt acg gtc atc aca 1920
Asp Ser Gln Gly Pro Leu Cys Asp Glu Leu Cys Arg Thr Val Ile Thr
625                 630                 635                 640

gcc gct gtc ctc ttc tcc ttc ata atc tct gtc ctg ctg tcc acc ttc 1968
Ala Ala Val Leu Phe Ser Phe Ile Ile Ser Val Leu Leu Ser Thr Phe
                645                 650                 655

tgc atc cac cgc tac cac aag cat gcg cac aag cca ccc atc gcg tca 2016
Cys Ile His Arg Tyr His Lys His Ala His Lys Pro Pro Ile Ala Ser
            660                 665                 670

gcc gaa atg acc ttc tgc cgg ccg gcc cag ggc ttc cca atc agc tat 2064
Ala Glu Met Thr Phe Cys Arg Pro Ala Gln Gly Phe Pro Ile Ser Tyr
        675                 680                 685

tct tcc tcg ggc acc cgc cgg ccc tca ctg gac tcc atg gag aac cag 2112
Ser Ser Ser Gly Thr Arg Arg Pro Ser Leu Asp Ser Met Glu Asn Gln
    690                 695                 700

gtc tct gtg gac tct ttc aag atc ccg gag gat ccg aag tgg gaa ttt 2160
Val Ser Val Asp Ser Phe Lys Ile Pro Glu Asp Pro Lys Trp Glu Phe
705                 710                 715                 720

cct cgg aag aac tta gtt ctt ggg aaa acc ctg gga gaa ggc gag ttt 2208
Pro Arg Lys Asn Leu Val Leu Gly Lys Thr Leu Gly Glu Gly Glu Phe
                725                 730                 735

gga aaa gta gtc aag gcc aca gcc ttc cgt ctg aaa ggc cgg gca gga 2256
Gly Lys Val Val Lys Ala Thr Ala Phe Arg Leu Lys Gly Arg Ala Gly
            740                 745                 750

tac acc aca gtg gct gtg aaa atg ctg aaa gaa aac gcc tcc cag agt 2304
Tyr Thr Thr Val Ala Val Lys Met Leu Lys Glu Asn Ala Ser Gln Ser
        755                 760                 765

gaa cta cga gac ctg ctc tct gag ttc aac ctt ctg aaa caa gtc aac 2352
Glu Leu Arg Asp Leu Leu Ser Glu Phe Asn Leu Leu Lys Gln Val Asn
    770                 775                 780

cat cca cat gtc atc aag ttg tac ggg gct tgc agc cag gat ggg cca 2400
His Pro His Val Ile Lys Leu Tyr Gly Ala Cys Ser Gln Asp Gly Pro
785                 790                 795                 800

ctt ctt ctc att gtg gag tat gca aag tat gga tcc ctg cgg ggg ttc 2448
Leu Leu Leu Ile Val Glu Tyr Ala Lys Tyr Gly Ser Leu Arg Gly Phe
                805                 810                 815

ctg cgg gac agc cgc aag atc ggg cct gcc tat gtg agc agt gga ggc 2496
Leu Arg Asp Ser Arg Lys Ile Gly Pro Ala Tyr Val Ser Ser Gly Gly
            820                 825                 830

agc cgc aat tcc agc tcc ctg gac cac cca gac gaa agg gtg ctg acc 2544
Ser Arg Asn Ser Ser Ser Leu Asp His Pro Asp Glu Arg Val Leu Thr
        835                 840                 845

atg ggc gac ctc atc tcc ttc gcc tgg cag atc tcg agg ggt atg cag 2592
Met Gly Asp Leu Ile Ser Phe Ala Trp Gln Ile Ser Arg Gly Met Gln
    850                 855                 860

tac ttg gct gaa atg aag ctc gta cat cga gac tta gct gcc aga aac 2640
Tyr Leu Ala Glu Met Lys Leu Val His Arg Asp Leu Ala Ala Arg Asn
865                 870                 875                 880

atc ttg gtg gca gag gga cgg aag atg aag atc tct gac ttt ggg ctg 2688
Ile Leu Val Ala Glu Gly Arg Lys Met Lys Ile Ser Asp Phe Gly Leu
                885                 890                 895

tcc cga gat gtt tat gaa gaa gat tcc tat gtg aag aaa agc aag ggc 2736
Ser Arg Asp Val Tyr Glu Glu Asp Ser Tyr Val Lys Lys Ser Lys Gly
            900                 905                 910

cgg att ccc gtc aaa tgg atg gca atc gag tct ctc ttc gat cac atc 2784
Arg Ile Pro Val Lys Trp Met Ala Ile Glu Ser Leu Phe Asp His Ile
        915                 920                 925

tat acc act caa agt gat gtg tgg tcc ttt gga gtg ctg cta tgg gag 2832
Tyr Thr Thr Gln Ser Asp Val Trp Ser Phe Gly Val Leu Leu Trp Glu
    930                 935                 940

att gtg acc ctg gga ggc aac ccc tac cct gga att cct cct gaa cga 2880
Ile Val Thr Leu Gly Gly Asn Pro Tyr Pro Gly Ile Pro Pro Glu Arg
945                 950                 955                 960

ctc ttc aac ctt ctg aag aca ggc cac agg atg gag agg cca gac aac 2928
Leu Phe Asn Leu Leu Lys Thr Gly His Arg Met Glu Arg Pro Asp Asn

965                 970                 975

tgc agc gag gaa atg tac cgc ctg atg ctg cag tgc tgg aag cag gag     2976
Cys Ser Glu Glu Met Tyr Arg Leu Met Leu Gln Cys Trp Lys Gln Glu
            980                 985                 990

ccg gac aag agg cca gta ttt gct gac atc agc aag gat ctg gag aag     3024
Pro Asp Lys Arg Pro Val Phe Ala Asp Ile Ser Lys Asp Leu Glu Lys
        995                 1000                1005

atg atg gtc aaa agc aga gac tac ttg gac ctg gct gca tcc acc     3069
Met Met Val Lys Ser Arg Asp Tyr Leu Asp Leu Ala Ala Ser Thr
    1010                1015                1020

cct tcg gac tca ctg ctc tat gac gat ggg ctc tcg gaa gag gag     3114
Pro Ser Asp Ser Leu Leu Tyr Asp Asp Gly Leu Ser Glu Glu Glu
    1025                1030                1035

acg ccc ctg gtg gac tgt aac agt gct ccc ctc ccg cgc tcc ctc     3159
Thr Pro Leu Val Asp Cys Asn Ser Ala Pro Leu Pro Arg Ser Leu
    1040                1045                1050

cct tcc aca tgg att gaa aac aaa ctc tat ggc atg tca gac ccg     3204
Pro Ser Thr Trp Ile Glu Asn Lys Leu Tyr Gly Met Ser Asp Pro
    1055                1060                1065

aac tgg cct gga gag agt cct gta cca ctc acg aga gcc gat ggc     3249
Asn Trp Pro Gly Glu Ser Pro Val Pro Leu Thr Arg Ala Asp Gly
    1070                1075                1080

act agc act ggg ttc cca aga tat gca aat gat agt gta tat gct     3294
Thr Ser Thr Gly Phe Pro Arg Tyr Ala Asn Asp Ser Val Tyr Ala
    1085                1090                1095

aac tgg atg gtt tca ccc tca gcg gca aaa tta atg gac aca ttt     3339
Asn Trp Met Val Ser Pro Ser Ala Ala Lys Leu Met Asp Thr Phe
    1100                1105                1110

gat agc gcg gcc gca gac tac cca tac gac gtc cca gac tac gct     3384
Asp Ser Ala Ala Ala Asp Tyr Pro Tyr Asp Val Pro Asp Tyr Ala
    1115                1120                1125

tag                                                             3387

<210>  14
<211>  1128
<212>  PRT
<213>  Rattus norvegicus

<400>  14

Met Ala Lys Ala Arg Ser Gly Ala Ala Gly Leu Gly Leu Lys Leu Phe
1               5                   10                  15

Leu Leu Leu Pro Leu Leu Gly Glu Ala Pro Leu Gly Leu Tyr Phe Ser
            20                  25                  30

Arg Asp Ala Tyr Trp Glu Arg Leu Tyr Val Asp Gln Pro Ala Gly Thr
        35                  40                  45

Pro Leu Leu Tyr Val His Ala Leu Arg Asp Ala Pro Gly Glu Val Pro
    50                  55                  60

Ser Phe Arg Leu Gly Gln Tyr Leu Tyr Gly Val Tyr Arg Thr Arg Leu
65                  70                  75                  80

His Glu Asn Asp Trp Ile His Ile Asp Ala Gly Thr Gly Leu Leu Tyr
                85                  90                  95

Leu Asn Gln Ser Leu Asp His Ser Ser Trp Glu Gln Leu Ser Ile Arg
            100                 105                 110

Asn Gly Gly Phe Pro Leu Leu Thr Val Phe Leu Gln Val Phe Leu Gly
        115                 120                 125

Ser Thr Ala Gln Arg Glu Gly Glu Cys His Trp Pro Gly Cys Ala Arg
    130                 135                 140

Val Tyr Phe Ser Phe Ile Asn Asp Thr Phe Pro Asn Cys Ser Ser Phe
145                 150                 155                 160

Lys Ala Arg Asp Leu Cys Thr Pro Glu Thr Gly Val Ser Phe Arg Ile
                165                 170                 175

Arg Glu Asn Arg Pro Pro Gly Thr Phe Tyr Gln Phe Arg Met Leu Pro
            180                 185                 190

Val Gln Phe Leu Cys Pro Asn Ile Ser Val Lys Tyr Lys Leu Leu Glu
        195                 200                 205

Gly Asp Gly Leu Pro Phe Arg Cys Asp Pro Asp Cys Leu Glu Val Ser
    210                 215                 220

Thr Arg Trp Ala Leu Asp Arg Glu Leu Gln Glu Lys Tyr Val Leu Glu
225                 230                 235                 240

Ala Glu Cys Ala Val Ala Gly Pro Gly Ala Asn Lys Glu Lys Val Ala
                245                 250                 255

Val Ser Phe Pro Val Thr Val Tyr Asp Glu Asp Asp Ser Pro Pro Thr
            260                 265                 270

Phe Ser Gly Gly Val Gly Thr Ala Ser Ala Val Val Glu Phe Lys Arg
        275                 280                 285

Lys Glu Gly Thr Val Val Ala Thr Leu Gln Val Phe Asp Ala Asp Val
    290                 295                 300

Val Pro Ala Ser Gly Glu Leu Val Arg Arg Tyr Thr Ser Thr Leu Leu
305                 310                 315                 320

Ser Gly Asp Ser Trp Ala Gln Gln Thr Phe Arg Val Glu His Thr Pro
                325                 330                 335

Asn Glu Thr Leu Val Gln Ser Asn Asn Asn Ser Val Arg Ala Thr Met
            340                 345                 350

His Asn Tyr Lys Leu Val Leu Asn Arg Ser Leu Ser Ile Ser Glu Ser
        355                 360                 365

Arg Val Leu Gln Leu Val Val Leu Val Asn Asp Ser Asp Phe Gln Gly
    370                 375                 380

Pro Gly Ser Gly Val Leu Phe Leu His Phe Asn Val Ser Val Leu Pro
385                 390                 395                 400

Val Thr Leu Asn Leu Pro Met Ala Tyr Ser Phe Pro Val Asn Arg Arg

                405                 410                 415

Ala Arg Arg Tyr Ala Gln Ile Gly Lys Val Cys Val Glu Asn Cys Gln
            420                 425                 430

Glu Phe Ser Gly Val Ser Ile Gln Tyr Lys Leu Gln Pro Ser Ser Thr
        435                 440                 445

Asn Cys Ser Ala Leu Gly Val Val Thr Ser Thr Glu Asp Thr Ser Gly
    450                 455                 460

Thr Leu Tyr Val Asn Asp Thr Glu Ala Leu Arg Arg Pro Glu Cys Thr
465                 470                 475                 480

Glu Leu Gln Tyr Thr Val Val Ala Thr Asp Arg Gln Thr Arg Arg Gln
                485                 490                 495

Thr Gln Ala Ser Leu Val Val Thr Val Glu Gly Thr Tyr Ile Ala Glu
            500                 505                 510

Glu Val Gly Cys Pro Lys Ser Cys Ala Val Asn Lys Arg Arg Pro Glu
        515                 520                 525

Cys Glu Glu Cys Gly Gly Leu Gly Ser Pro Thr Gly Arg Cys Glu Trp
    530                 535                 540

Arg Gln Gly Asp Gly Lys Gly Ile Thr Arg Asn Phe Ser Thr Cys Ser
545                 550                 555                 560

Pro Ser Thr Arg Thr Cys Pro Asp Gly His Cys Asp Ala Leu Glu Ser
                565                 570                 575

Arg Asp Ile Asn Ile Cys Pro Gln Asp Cys Leu Arg Gly Pro Ile Val
            580                 585                 590

Gly Gly His Glu Arg Gly Glu Arg Gln Gly Ile Lys Ala Gly Tyr Gly
        595                 600                 605

Ile Cys Asn Cys Phe Pro Asp Glu Lys Lys Cys Phe Cys Glu Pro Glu
    610                 615                 620

Asp Ser Gln Gly Pro Leu Cys Asp Glu Leu Cys Arg Thr Val Ile Thr
625                 630                 635                 640

Ala Ala Val Leu Phe Ser Phe Ile Ile Ser Val Leu Leu Ser Thr Phe
                645                 650                 655

Cys Ile His Arg Tyr His Lys His Ala His Lys Pro Pro Ile Ala Ser
            660                 665                 670

Ala Glu Met Thr Phe Cys Arg Pro Ala Gln Gly Phe Pro Ile Ser Tyr
        675                 680                 685

Ser Ser Ser Gly Thr Arg Arg Pro Ser Leu Asp Ser Met Glu Asn Gln
    690                 695                 700

Val Ser Val Asp Ser Phe Lys Ile Pro Glu Asp Pro Lys Trp Glu Phe
705                 710                 715                 720

Pro Arg Lys Asn Leu Val Leu Gly Lys Thr Leu Gly Glu Gly Glu Phe
                725                 730                 735

Gly Lys Val Val Lys Ala Thr Ala Phe Arg Leu Lys Gly Arg Ala Gly
            740                 745                 750

Tyr Thr Thr Val Ala Val Lys Met Leu Lys Glu Asn Ala Ser Gln Ser
        755                 760                 765

Glu Leu Arg Asp Leu Leu Ser Glu Phe Asn Leu Leu Lys Gln Val Asn
    770                 775                 780

His Pro His Val Ile Lys Leu Tyr Gly Ala Cys Ser Gln Asp Gly Pro
785                 790                 795                 800

Leu Leu Leu Ile Val Glu Tyr Ala Lys Tyr Gly Ser Leu Arg Gly Phe
                805                 810                 815

Leu Arg Asp Ser Arg Lys Ile Gly Pro Ala Tyr Val Ser Ser Gly Gly
            820                 825                 830

Ser Arg Asn Ser Ser Ser Leu Asp His Pro Asp Glu Arg Val Leu Thr
        835                 840                 845

Met Gly Asp Leu Ile Ser Phe Ala Trp Gln Ile Ser Arg Gly Met Gln
    850                 855                 860

Tyr Leu Ala Glu Met Lys Leu Val His Arg Asp Leu Ala Ala Arg Asn
865                 870                 875                 880

Ile Leu Val Ala Glu Gly Arg Lys Met Lys Ile Ser Asp Phe Gly Leu
                885                 890                 895

Ser Arg Asp Val Tyr Glu Glu Asp Ser Tyr Val Lys Lys Ser Lys Gly
            900                 905                 910

Arg Ile Pro Val Lys Trp Met Ala Ile Glu Ser Leu Phe Asp His Ile
        915                 920                 925

Tyr Thr Thr Gln Ser Asp Val Trp Ser Phe Gly Val Leu Leu Trp Glu
    930                 935                 940

Ile Val Thr Leu Gly Gly Asn Pro Tyr Pro Gly Ile Pro Pro Glu Arg
945                 950                 955                 960

Leu Phe Asn Leu Leu Lys Thr Gly His Arg Met Glu Arg Pro Asp Asn
                965                 970                 975

Cys Ser Glu Glu Met Tyr Arg Leu Met Leu Gln Cys Trp Lys Gln Glu
            980                 985                 990

Pro Asp Lys Arg Pro Val Phe Ala Asp Ile Ser Lys Asp Leu Glu Lys
        995                 1000                1005

Met Met Val Lys Ser Arg Asp Tyr Leu Asp Leu Ala Ala Ser Thr
    1010                1015                1020

Pro Ser Asp Ser Leu Leu Tyr Asp Asp Gly Leu Ser Glu Glu Glu
    1025                1030                1035

Thr Pro Leu Val Asp Cys Asn Ser Ala Pro Leu Pro Arg Ser Leu
    1040                1045                1050

Pro Ser Thr Trp Ile Glu Asn Lys Leu Tyr Gly Met Ser Asp Pro
    1055                1060                1065

Asn Trp Pro Gly Glu Ser Pro Val Pro Leu Thr Arg Ala Asp Gly
    1070                1075                1080

Thr Ser Thr Gly Phe Pro Arg Tyr Ala Asn Asp Ser Val Tyr Ala
    1085                1090                1095

Asn Trp Met Val Ser Pro Ser Ala Ala Lys Leu Met Asp Thr Phe
    1100                1105                1110

Asp Ser Ala Ala Ala Asp Tyr Pro Tyr Asp Val Pro Asp Tyr Ala
    1115                1120                1125

<210> 15
<211> 3348
<212> DNA
<213> Mus musculus

<220>
<221> CDS
<222> (1)..(3345)

<400> 15
atg gcg aaa gcg acg tcc ggc gcc gca ggg ctg ggg ctg aag ctg att      48
Met Ala Lys Ala Thr Ser Gly Ala Ala Gly Leu Gly Leu Lys Leu Ile
1               5                   10                  15

ttg ctc ctg ccg ctg cta gga gaa gcc cca ctg ggc ctc tat ttc tca      96
Leu Leu Leu Pro Leu Leu Gly Glu Ala Pro Leu Gly Leu Tyr Phe Ser
            20                  25                  30

agg gat gct tac tgg gag agg ctg tat gta gac cag cca gct ggc aca      144
Arg Asp Ala Tyr Trp Glu Arg Leu Tyr Val Asp Gln Pro Ala Gly Thr

35                  40                  45

cct ctg ctc tat gtc cat gcc cta cgg gat gcc cct gga gaa gtg ccg        192
Pro Leu Leu Tyr Val His Ala Leu Arg Asp Ala Pro Gly Glu Val Pro
    50                  55                  60

agc ttc cgc ctg ggc cag cat ctc tat ggc gtc tac cgt aca cgg ctg        240
Ser Phe Arg Leu Gly Gln His Leu Tyr Gly Val Tyr Arg Thr Arg Leu
65                  70                  75                  80

cat gag aat gac tgg atc cgc atc aat gag act act ggc ctt ctc tac        288
His Glu Asn Asp Trp Ile Arg Ile Asn Glu Thr Thr Gly Leu Leu Tyr
                85                  90                  95

ctc aat cag agc ctg gac cac agt tcc tgg gaa cag ctc agc atc cgc        336
Leu Asn Gln Ser Leu Asp His Ser Ser Trp Glu Gln Leu Ser Ile Arg
            100                 105                 110

aat ggt ggt ttc ccc ctg ctc acc atc ttc ctc cag gtc ttt ctg ggg        384
Asn Gly Gly Phe Pro Leu Leu Thr Ile Phe Leu Gln Val Phe Leu Gly
        115                 120                 125

tcc aca gcc cag aga gag gga gaa tgc cat tgg cca ggc tgt acc cgt        432
Ser Thr Ala Gln Arg Glu Gly Glu Cys His Trp Pro Gly Cys Thr Arg
    130                 135                 140

gtg tac ttc tcc ttc atc aac gac acc ttc cca aat tgt agc tcc ttc        480
Val Tyr Phe Ser Phe Ile Asn Asp Thr Phe Pro Asn Cys Ser Ser Phe
145                 150                 155                 160

aaa gcc cag gat ctc tgc atc cca gag aca gcc gtg tcc ttc cga gtc        528
Lys Ala Gln Asp Leu Cys Ile Pro Glu Thr Ala Val Ser Phe Arg Val
                165                 170                 175

agg gag aac agg cct cct ggc acc ttc tac cac ttc cac atg tta ccc        576
Arg Glu Asn Arg Pro Pro Gly Thr Phe Tyr His Phe His Met Leu Pro
            180                 185                 190

gtg cag ttc ctt tgt ccc aac atc agt gtg aag tac agt ctc tta gga        624
Val Gln Phe Leu Cys Pro Asn Ile Ser Val Lys Tyr Ser Leu Leu Gly
        195                 200                 205

ggg gat agt ctg ccc ttc cgt tgt gac cca gac tgc ctg gag gtg agc        672
Gly Asp Ser Leu Pro Phe Arg Cys Asp Pro Asp Cys Leu Glu Val Ser
    210                 215                 220

act cgc tgg gcc ctg gat cga gag ctc cgg gag aag tat gtg ctg gag        720
Thr Arg Trp Ala Leu Asp Arg Glu Leu Arg Glu Lys Tyr Val Leu Glu
225                 230                 235                 240

gct ttg tgc ata gtg gca ggc cct ggt gcc aac aaa gag acg gtg act        768

Ala Leu Cys Ile Val Ala Gly Pro Gly Ala Asn Lys Glu Thr Val Thr
                245                 250                 255

ctg tcc ttc cca gtg aca gtg tat gat gag gac gac tcg gcg ccc acc 816
Leu Ser Phe Pro Val Thr Val Tyr Asp Glu Asp Asp Ser Ala Pro Thr
            260                 265                 270

ttc tct gga ggt gtg ggc act gcc agc gcg gtg gtg gag ttt aag cgg 864
Phe Ser Gly Gly Val Gly Thr Ala Ser Ala Val Val Glu Phe Lys Arg
        275                 280                 285

aag gag ggc act gtg gtg gcc acc ctg cag gtg ttc gat gca gat gtg 912
Lys Glu Gly Thr Val Val Ala Thr Leu Gln Val Phe Asp Ala Asp Val
    290                 295                 300

gtg cca gcg tct ggg gag ctg gtg aga cgg tac aca aac aca ctc ctc 960
Val Pro Ala Ser Gly Glu Leu Val Arg Arg Tyr Thr Asn Thr Leu Leu
305                 310                 315                 320

tca ggg gac tcc tgg gcc cag cag acc ttc cgg gtg gag cat tcg ccc 1008
Ser Gly Asp Ser Trp Ala Gln Gln Thr Phe Arg Val Glu His Ser Pro
                325                 330                 335

atc gag acc ttg gtc cag gtc aac aac aac tcc gtt cgg gca acc atg 1056
Ile Glu Thr Leu Val Gln Val Asn Asn Asn Ser Val Arg Ala Thr Met
            340                 345                 350

cac aat tac aag ctg att ctc aac agg agc ctg tct atc tca gag agc 1104
His Asn Tyr Lys Leu Ile Leu Asn Arg Ser Leu Ser Ile Ser Glu Ser
        355                 360                 365

cga gtc ctg cag ctc gcg gtc ctg gtc aac gac tca gac ttc cag ggg 1152
Arg Val Leu Gln Leu Ala Val Leu Val Asn Asp Ser Asp Phe Gln Gly
    370                 375                 380

cct ggg gca ggt ggt atc ctc gtc ctc cat ttc aac gtg tct gta ctg 1200
Pro Gly Ala Gly Gly Ile Leu Val Leu His Phe Asn Val Ser Val Leu
385                 390                 395                 400

ccc gtc acc ctg aac cta ccc agg gcc tac tcc ttc cca gtg aat aag 1248
Pro Val Thr Leu Asn Leu Pro Arg Ala Tyr Ser Phe Pro Val Asn Lys
                405                 410                 415

agg gcc cgc cgc tat gcc cag atc ggg aaa gtc tgt gtg gaa aac tgc 1296
Arg Ala Arg Arg Tyr Ala Gln Ile Gly Lys Val Cys Val Glu Asn Cys
            420                 425                 430

cag gag ttc agc ggt gtc tcc atc cag tac aag ctg cag cct tcc agc 1344
Gln Glu Phe Ser Gly Val Ser Ile Gln Tyr Lys Leu Gln Pro Ser Ser
        435                 440                 445

atc aac tgc act gcc cta ggt gtg gtc acc tca ccc gag gac acc tcg     1392
Ile Asn Cys Thr Ala Leu Gly Val Val Thr Ser Pro Glu Asp Thr Ser
    450                 455                 460

ggg acc cta ttt gta aat gac aca gag gcc ctg cgg cga cct gag tgc     1440
Gly Thr Leu Phe Val Asn Asp Thr Glu Ala Leu Arg Arg Pro Glu Cys
465                 470                 475                 480

acc aag ctt cag tac acg gtg gta gcc act gac cgg cag acc cgc aga     1488
Thr Lys Leu Gln Tyr Thr Val Val Ala Thr Asp Arg Gln Thr Arg Arg
                485                 490                 495

cag acc cag gct tcg cta gtg gtc act gtg gag ggg aca tcc att act     1536
Gln Thr Gln Ala Ser Leu Val Val Thr Val Glu Gly Thr Ser Ile Thr
            500                 505                 510

gaa gaa gta ggc tgc ccc aag tcc tgt gca gta aac aag agg cgc ccc     1584
Glu Glu Val Gly Cys Pro Lys Ser Cys Ala Val Asn Lys Arg Arg Pro
        515                 520                 525

gag tgt gag gaa tgt ggt ggc ctg ggt tct cca act ggc agg tgc gag     1632
Glu Cys Glu Glu Cys Gly Gly Leu Gly Ser Pro Thr Gly Arg Cys Glu
    530                 535                 540

tgg cgc cag gga gat ggt aaa ggg atc acc agg aac ttc tcc acc tgc     1680
Trp Arg Gln Gly Asp Gly Lys Gly Ile Thr Arg Asn Phe Ser Thr Cys
545                 550                 555                 560

tcc ccc agt acc agg acc tgc ccc gac ggc cac tgt gat gct gtg gag     1728
Ser Pro Ser Thr Arg Thr Cys Pro Asp Gly His Cys Asp Ala Val Glu
                565                 570                 575

agc cgg gat gcc aac att tgc ccc cag gac tgt ctc cgt gcc gac att     1776
Ser Arg Asp Ala Asn Ile Cys Pro Gln Asp Cys Leu Arg Ala Asp Ile
            580                 585                 590

gtt gga gga cac gag cga ggg gag cgc cag ggc att aaa gca ggc tac     1824
Val Gly Gly His Glu Arg Gly Glu Arg Gln Gly Ile Lys Ala Gly Tyr
        595                 600                 605

ggc atc tgc aac tgt ttc cct gat gag aag aaa tgc ttc tgc gag cca     1872
Gly Ile Cys Asn Cys Phe Pro Asp Glu Lys Lys Cys Phe Cys Glu Pro
    610                 615                 620

gag gac agc cag ggc cca ctg tgt gat gcg ctg tgc cgc acg atc atc     1920
Glu Asp Ser Gln Gly Pro Leu Cys Asp Ala Leu Cys Arg Thr Ile Ile
625                 630                 635                 640

aca gct gcc ctc ttc tcc ctt atc atc tcc atc ctg ctg tcc atc ttc     1968
Thr Ala Ala Leu Phe Ser Leu Ile Ile Ser Ile Leu Leu Ser Ile Phe
                645                 650                 655

tgt gtc tgc cac cac cac aag cat ggg cac aag ccg ccc att gca tca 2016
Cys Val Cys His His His Lys His Gly His Lys Pro Pro Ile Ala Ser
            660                 665                 670

gcg gaa atg acc ttc tgc cgg ccg gcc cag ggc ttc cca atc agt tat 2064
Ala Glu Met Thr Phe Cys Arg Pro Ala Gln Gly Phe Pro Ile Ser Tyr
        675                 680                 685

tcc tcc tca ggc acc cgc cgg ccc tca ctg gat tcc acg gag aac cag 2112
Ser Ser Ser Gly Thr Arg Arg Pro Ser Leu Asp Ser Thr Glu Asn Gln
    690                 695                 700

gtt cct gtg gac tct ttc aag atc ccg gag gat cca aag tgg gaa ttt 2160
Val Pro Val Asp Ser Phe Lys Ile Pro Glu Asp Pro Lys Trp Glu Phe
705                 710                 715                 720

cct cgg aag aac tta gtt ctt ggg aaa act ctg gga gaa ggc gag ttt 2208
Pro Arg Lys Asn Leu Val Leu Gly Lys Thr Leu Gly Glu Gly Glu Phe
                725                 730                 735

gga aaa gtt gtc aag gcc aca gcc ttc cgt ctg aaa ggc cgg gca gga 2256
Gly Lys Val Val Lys Ala Thr Ala Phe Arg Leu Lys Gly Arg Ala Gly
            740                 745                 750

tac acc aca gtg gct gtg aaa atg ctg aaa gaa aac gcc tcc cag agt 2304
Tyr Thr Thr Val Ala Val Lys Met Leu Lys Glu Asn Ala Ser Gln Ser
        755                 760                 765

gag tta cga gac ctg ctg tct gag ttc aac ctt ctg aaa caa gtc aac 2352
Glu Leu Arg Asp Leu Leu Ser Glu Phe Asn Leu Leu Lys Gln Val Asn
    770                 775                 780

cat cca cat gtc atc aag ttg tat ggg gcc tgc agc cag gat ggg cca 2400
His Pro His Val Ile Lys Leu Tyr Gly Ala Cys Ser Gln Asp Gly Pro
785                 790                 795                 800

ctt ctt ctc att gtg gag tat gcc aag tat ggc tcc ctg cgg gga ttc 2448
Leu Leu Leu Ile Val Glu Tyr Ala Lys Tyr Gly Ser Leu Arg Gly Phe
                805                 810                 815

ctc cgt gac agc cgc aag att ggg cct gcc tat gtg agc ggt gga ggc 2496
Leu Arg Asp Ser Arg Lys Ile Gly Pro Ala Tyr Val Ser Gly Gly Gly
            820                 825                 830

agc cgc aac tcc agc tcg ctg gac cac cca gat gaa agg gta ctg acc 2544
Ser Arg Asn Ser Ser Ser Leu Asp His Pro Asp Glu Arg Val Leu Thr
        835                 840                 845

atg ggt gac ctc atc tcc ttc gcc tgg cag atc tcg agg ggt atg cag 2592
Met Gly Asp Leu Ile Ser Phe Ala Trp Gln Ile Ser Arg Gly Met Gln

850                 855                 860

tac ttg gca gaa atg aag ctt gta cat cgg gac tta gct gcc agg aac     2640
Tyr Leu Ala Glu Met Lys Leu Val His Arg Asp Leu Ala Ala Arg Asn
865                 870                 875                 880

atc ttg gtg gct gag gga cgg aag atg aag att tcc gac ttt ggg ctg     2688
Ile Leu Val Ala Glu Gly Arg Lys Met Lys Ile Ser Asp Phe Gly Leu
                885                 890                 895

tcc cga gat gtt tat gag gaa gat tcc tat gtg aag aaa agc aag ggc     2736
Ser Arg Asp Val Tyr Glu Glu Asp Ser Tyr Val Lys Lys Ser Lys Gly
            900                 905                 910

cgg att ccc gtc aag tgg atg gca att gag tcc ctt ttc gat cac atc     2784
Arg Ile Pro Val Lys Trp Met Ala Ile Glu Ser Leu Phe Asp His Ile
        915                 920                 925

tat act act caa agt gat gtg tgg tcc ttt gga gtg ctg ctc tgg gag     2832
Tyr Thr Thr Gln Ser Asp Val Trp Ser Phe Gly Val Leu Leu Trp Glu
    930                 935                 940

att gtg acc ctg gga ggc aac ccc tac cct gga att cct cct gaa cga     2880
Ile Val Thr Leu Gly Gly Asn Pro Tyr Pro Gly Ile Pro Pro Glu Arg
945                 950                 955                 960

ctc ttc aac ctt ctg aag aca ggc cac agg atg gag agg cca gac aac     2928
Leu Phe Asn Leu Leu Lys Thr Gly His Arg Met Glu Arg Pro Asp Asn
                965                 970                 975

tgc agc gag gaa atg tac cgt ctg atg ctg cag tgc tgg aag cag gag     2976
Cys Ser Glu Glu Met Tyr Arg Leu Met Leu Gln Cys Trp Lys Gln Glu
            980                 985                 990

cca gac aag agg cca gtg ttt gct  gac atc agc aag gat  ctg gag aag     3024
Pro Asp Lys Arg Pro Val Phe Ala  Asp Ile Ser Lys Asp  Leu Glu Lys
        995                 1000                1005

atg atg  gtc aag agc aga gac  tac ttg gac ctg gct  gca tcc aca     3069
Met Met  Val Lys Ser Arg Asp  Tyr Leu Asp Leu Ala  Ala Ser Thr
    1010                1015                1020

cct tcg  gac tca ctg ctg tat  gac gat ggg ctc tca  gaa gag gag     3114
Pro Ser  Asp Ser Leu Leu Tyr  Asp Asp Gly Leu Ser  Glu Glu Glu
    1025                1030                1035

aca ccc  ctg gtg gac tgt aac  aat gct ccc ctc ccg  cgc tcc ctc     3159
Thr Pro  Leu Val Asp Cys Asn  Asn Ala Pro Leu Pro  Arg Ser Leu
    1040                1045                1050

cct tcc  aca tgg att gaa aac  aaa ctc tat ggc atg  tca gac ccg     3204

Pro Ser Thr Trp Ile Glu Asn Lys Leu Tyr Gly Met Ser Asp Pro
    1055                1060                1065

aac tgg cct gga gag agt cct gta cca ctc acg aga gcc gat ggc        3249
Asn Trp Pro Gly Glu Ser Pro Val Pro Leu Thr Arg Ala Asp Gly
    1070                1075                1080

act agc act ggg ttc cca aga tat gca aat gat agt gta tat gct        3294
Thr Ser Thr Gly Phe Pro Arg Tyr Ala Asn Asp Ser Val Tyr Ala
    1085                1090                1095

aac tgg atg gtt tca ccc tca gcg gca aaa tta atg gac aca ttt        3339
Asn Trp Met Val Ser Pro Ser Ala Ala Lys Leu Met Asp Thr Phe
    1100                1105                1110

gat agc taa                                                        3348
Asp Ser
    1115

<210> 16
<211> 1115
<212> PRT
<213> Mus musculus

<400> 16

Met Ala Lys Ala Thr Ser Gly Ala Ala Gly Leu Gly Leu Lys Leu Ile
1               5                   10                  15

Leu Leu Leu Pro Leu Leu Gly Glu Ala Pro Leu Gly Leu Tyr Phe Ser
            20                  25                  30

Arg Asp Ala Tyr Trp Glu Arg Leu Tyr Val Asp Gln Pro Ala Gly Thr
        35                  40                  45

Pro Leu Leu Tyr Val His Ala Leu Arg Asp Ala Pro Gly Glu Val Pro
    50                  55                  60

Ser Phe Arg Leu Gly Gln His Leu Tyr Gly Val Tyr Arg Thr Arg Leu
65                  70                  75                  80

His Glu Asn Asp Trp Ile Arg Ile Asn Glu Thr Thr Gly Leu Leu Tyr
                85                  90                  95

Leu Asn Gln Ser Leu Asp His Ser Ser Trp Glu Gln Leu Ser Ile Arg
            100                 105                 110

Asn Gly Gly Phe Pro Leu Leu Thr Ile Phe Leu Gln Val Phe Leu Gly
        115                 120                 125

Ser Thr Ala Gln Arg Glu Gly Glu Cys His Trp Pro Gly Cys Thr Arg
    130                 135                 140

Val Tyr Phe Ser Phe Ile Asn Asp Thr Phe Pro Asn Cys Ser Ser Phe
145                 150                 155                 160

Lys Ala Gln Asp Leu Cys Ile Pro Glu Thr Ala Val Ser Phe Arg Val
                165                 170                 175

Arg Glu Asn Arg Pro Pro Gly Thr Phe Tyr His Phe His Met Leu Pro
            180                 185                 190

Val Gln Phe Leu Cys Pro Asn Ile Ser Val Lys Tyr Ser Leu Leu Gly
        195                 200                 205

Gly Asp Ser Leu Pro Phe Arg Cys Asp Pro Asp Cys Leu Glu Val Ser
    210                 215                 220

Thr Arg Trp Ala Leu Asp Arg Glu Leu Arg Glu Lys Tyr Val Leu Glu
225                 230                 235                 240

Ala Leu Cys Ile Val Ala Gly Pro Gly Ala Asn Lys Glu Thr Val Thr
                245                 250                 255

Leu Ser Phe Pro Val Thr Val Tyr Asp Glu Asp Asp Ser Ala Pro Thr
            260                 265                 270

Phe Ser Gly Gly Val Gly Thr Ala Ser Ala Val Val Glu Phe Lys Arg
        275                 280                 285

Lys Glu Gly Thr Val Val Ala Thr Leu Gln Val Phe Asp Ala Asp Val
    290                 295                 300

Val Pro Ala Ser Gly Glu Leu Val Arg Arg Tyr Thr Asn Thr Leu Leu
305                 310                 315                 320

Ser Gly Asp Ser Trp Ala Gln Gln Thr Phe Arg Val Glu His Ser Pro
                325                 330                 335

Ile Glu Thr Leu Val Gln Val Asn Asn Asn Ser Val Arg Ala Thr Met
            340                 345                 350

His Asn Tyr Lys Leu Ile Leu Asn Arg Ser Leu Ser Ile Ser Glu Ser
        355                 360                 365

Arg Val Leu Gln Leu Ala Val Leu Val Asn Asp Ser Asp Phe Gln Gly
    370                 375                 380

Pro Gly Ala Gly Gly Ile Leu Val Leu His Phe Asn Val Ser Val Leu
385                 390                 395                 400

Pro Val Thr Leu Asn Leu Pro Arg Ala Tyr Ser Phe Pro Val Asn Lys
                405                 410                 415

Arg Ala Arg Arg Tyr Ala Gln Ile Gly Lys Val Cys Val Glu Asn Cys
            420                 425                 430

Gln Glu Phe Ser Gly Val Ser Ile Gln Tyr Lys Leu Gln Pro Ser Ser
        435                 440                 445

Ile Asn Cys Thr Ala Leu Gly Val Val Thr Ser Pro Glu Asp Thr Ser
    450                 455                 460

Gly Thr Leu Phe Val Asn Asp Thr Glu Ala Leu Arg Arg Pro Glu Cys
465                 470                 475                 480

Thr Lys Leu Gln Tyr Thr Val Val Ala Thr Asp Arg Gln Thr Arg Arg
                485                 490                 495

Gln Thr Gln Ala Ser Leu Val Val Thr Val Glu Gly Thr Ser Ile Thr
            500                 505                 510

Glu Glu Val Gly Cys Pro Lys Ser Cys Ala Val Asn Lys Arg Arg Pro
        515                 520                 525

Glu Cys Glu Glu Cys Gly Gly Leu Gly Ser Pro Thr Gly Arg Cys Glu
    530                 535                 540

Trp Arg Gln Gly Asp Gly Lys Gly Ile Thr Arg Asn Phe Ser Thr Cys
545                 550                 555                 560

Ser Pro Ser Thr Arg Thr Cys Pro Asp Gly His Cys Asp Ala Val Glu
                565                 570                 575

Ser Arg Asp Ala Asn Ile Cys Pro Gln Asp Cys Leu Arg Ala Asp Ile
            580                 585                 590

Val Gly Gly His Glu Arg Gly Glu Arg Gln Gly Ile Lys Ala Gly Tyr
        595                 600                 605

Gly Ile Cys Asn Cys Phe Pro Asp Glu Lys Lys Cys Phe Cys Glu Pro
    610                 615                 620

Glu Asp Ser Gln Gly Pro Leu Cys Asp Ala Leu Cys Arg Thr Ile Ile
625                 630                 635                 640

Thr Ala Ala Leu Phe Ser Leu Ile Ile Ser Ile Leu Leu Ser Ile Phe
                645                 650                 655

Cys Val Cys His His His Lys His Gly His Lys Pro Pro Ile Ala Ser
            660                 665                 670

Ala Glu Met Thr Phe Cys Arg Pro Ala Gln Gly Phe Pro Ile Ser Tyr
        675                 680                 685

Ser Ser Ser Gly Thr Arg Arg Pro Ser Leu Asp Ser Thr Glu Asn Gln
    690                 695                 700

Val Pro Val Asp Ser Phe Lys Ile Pro Glu Asp Pro Lys Trp Glu Phe

705                 710                 715                 720

Pro Arg Lys Asn Leu Val Leu Gly Lys Thr Leu Gly Glu Gly Glu Phe
                725                 730                 735

Gly Lys Val Val Lys Ala Thr Ala Phe Arg Leu Lys Gly Arg Ala Gly
            740                 745                 750

Tyr Thr Thr Val Ala Val Lys Met Leu Lys Glu Asn Ala Ser Gln Ser
        755                 760                 765

Glu Leu Arg Asp Leu Leu Ser Glu Phe Asn Leu Leu Lys Gln Val Asn
    770                 775                 780

His Pro His Val Ile Lys Leu Tyr Gly Ala Cys Ser Gln Asp Gly Pro
785                 790                 795                 800

Leu Leu Leu Ile Val Glu Tyr Ala Lys Tyr Gly Ser Leu Arg Gly Phe
                805                 810                 815

Leu Arg Asp Ser Arg Lys Ile Gly Pro Ala Tyr Val Ser Gly Gly Gly
            820                 825                 830

Ser Arg Asn Ser Ser Ser Leu Asp His Pro Asp Glu Arg Val Leu Thr
        835                 840                 845

Met Gly Asp Leu Ile Ser Phe Ala Trp Gln Ile Ser Arg Gly Met Gln
    850                 855                 860

Tyr Leu Ala Glu Met Lys Leu Val His Arg Asp Leu Ala Ala Arg Asn
865                 870                 875                 880

Ile Leu Val Ala Glu Gly Arg Lys Met Lys Ile Ser Asp Phe Gly Leu
                885                 890                 895

Ser Arg Asp Val Tyr Glu Glu Asp Ser Tyr Val Lys Lys Ser Lys Gly
            900                 905                 910

Arg Ile Pro Val Lys Trp Met Ala Ile Glu Ser Leu Phe Asp His Ile
        915                 920                 925

Tyr Thr Thr Gln Ser Asp Val Trp Ser Phe Gly Val Leu Leu Trp Glu
    930                 935                 940

Ile Val Thr Leu Gly Gly Asn Pro Tyr Pro Gly Ile Pro Pro Glu Arg
945                 950                 955                 960

Leu Phe Asn Leu Leu Lys Thr Gly His Arg Met Glu Arg Pro Asp Asn
                965                 970                 975

Cys Ser Glu Glu Met Tyr Arg Leu Met Leu Gln Cys Trp Lys Gln Glu
            980                 985                 990

Pro Asp Lys Arg Pro Val Phe Ala Asp Ile Ser Lys Asp Leu Glu Lys
        995                 1000                1005

Met Met Val Lys Ser Arg Asp Tyr Leu Asp Leu Ala Ala Ser Thr
    1010                1015                1020

Pro Ser Asp Ser Leu Leu Tyr Asp Asp Gly Leu Ser Glu Glu Glu
    1025                1030                1035

Thr Pro Leu Val Asp Cys Asn Asn Ala Pro Leu Pro Arg Ser Leu
    1040                1045                1050

Pro Ser Thr Trp Ile Glu Asn Lys Leu Tyr Gly Met Ser Asp Pro
    1055                1060                1065

Asn Trp Pro Gly Glu Ser Pro Val Pro Leu Thr Arg Ala Asp Gly
    1070                1075                1080

Thr Ser Thr Gly Phe Pro Arg Tyr Ala Asn Asp Ser Val Tyr Ala
    1085                1090                1095

Asn Trp Met Val Ser Pro Ser Ala Ala Lys Leu Met Asp Thr Phe
    1100                1105                1110

Asp Ser
    1115

<210> 17
<211> 3348
<212> DNA
<213> Mus musculus

<220>
<221> CDS
<222> (1)..(3345)

<400> 17
atg gcg aaa gcg acg tcc ggc gcc gca ggg ctg ggg ctg aag ctg att 48
Met Ala Lys Ala Thr Ser Gly Ala Ala Gly Leu Gly Leu Lys Leu Ile
1               5                   10                  15

ttg ctc ctg ccg ctg cta gga gaa gcc cca ctg ggc ctc tat ttc tca 96
Leu Leu Leu Pro Leu Leu Gly Glu Ala Pro Leu Gly Leu Tyr Phe Ser
            20                  25                  30

agg gat gct tac tgg gag agg ctg tat gta gac cag cca gct ggc aca 144
Arg Asp Ala Tyr Trp Glu Arg Leu Tyr Val Asp Gln Pro Ala Gly Thr
        35                  40                  45

cct ctg ctc tat gtc cat gcc cta cgg gat gcc cct gga gaa gtg ccg 192
Pro Leu Leu Tyr Val His Ala Leu Arg Asp Ala Pro Gly Glu Val Pro
    50                  55                  60

agc ttc cgc ctg ggc cag cat ctc tat ggc gtc tac cgt aca cgg ctg 240
Ser Phe Arg Leu Gly Gln His Leu Tyr Gly Val Tyr Arg Thr Arg Leu
65                  70                  75                  80

cat gag aat gac tgg atc cgc atc aat gag act act ggc ctt ctc tac 288
His Glu Asn Asp Trp Ile Arg Ile Asn Glu Thr Thr Gly Leu Leu Tyr
                85                  90                  95

ctc aat cag agc ctg gac cac agt tcc tgg gaa cag ctc agc atc cgc 336
Leu Asn Gln Ser Leu Asp His Ser Ser Trp Glu Gln Leu Ser Ile Arg
            100                 105                 110

aat ggt ggt ttc ccc ctg ctc acc atc ttc ctc cag gtc ttt ctg ggg 384
Asn Gly Gly Phe Pro Leu Leu Thr Ile Phe Leu Gln Val Phe Leu Gly
        115                 120                 125

tcc aca gcc cag aga gag gga gaa tgc cat tgg cca ggc tgt acc cgt 432
Ser Thr Ala Gln Arg Glu Gly Glu Cys His Trp Pro Gly Cys Thr Arg
    130                 135                 140

gtg tac ttc tcc ttc atc aac gac acc ttc cca aat tgt agc tcc ttc 480
Val Tyr Phe Ser Phe Ile Asn Asp Thr Phe Pro Asn Cys Ser Ser Phe
145                 150                 155                 160

aaa gcc cag gat ctc tgc atc cca gag aca gcc gtg tcc tcc cga gtc 528
Lys Ala Gln Asp Leu Cys Ile Pro Glu Thr Ala Val Ser Ser Arg Val
                165                 170                 175

agg gag aac ags cct cct ggc acc ttc tac cac ttc cac atg tta ccc 576
Arg Glu Asn Xaa Pro Pro Gly Thr Phe Tyr His Phe His Met Leu Pro
            180                 185                 190

gtg cag ttc ctt tgt ccc aac atc agt gtg aag tac agt ctc tta gga 624
Val Gln Phe Leu Cys Pro Asn Ile Ser Val Lys Tyr Ser Leu Leu Gly
        195                 200                 205

ggg gat agt ctg ccc ttc cgt tgt gac cca gac tgc ctg gag gtg agc 672
Gly Asp Ser Leu Pro Phe Arg Cys Asp Pro Asp Cys Leu Glu Val Ser
    210                 215                 220

act cgc tgg gcc ctg gat cga gag ctc cgg gag aag tat gtg ctg gag 720
Thr Arg Trp Ala Leu Asp Arg Glu Leu Arg Glu Lys Tyr Val Leu Glu
225                 230                 235                 240

gct ttg tgc ata gtg gca ggc cct ggt gcc aac aaa gag acg gtg act 768
Ala Leu Cys Ile Val Ala Gly Pro Gly Ala Asn Lys Glu Thr Val Thr
                245                 250                 255

ctg tcc ttc cca gtg aca gtg tat gat gag gac gac tcg gcg ccc acc 816
Leu Ser Phe Pro Val Thr Val Tyr Asp Glu Asp Asp Ser Ala Pro Thr
            260                 265                 270

ttc tct gga ggt gtg ggc act gcc agc gcg gtg gtg gag ttt aag cgg 864
Phe Ser Gly Gly Val Gly Thr Ala Ser Ala Val Val Glu Phe Lys Arg
        275                 280                 285

aag gag ggc act gtg gtg gcc acc ctg cag gtg ttc gat gca gat gtg 912
Lys Glu Gly Thr Val Val Ala Thr Leu Gln Val Phe Asp Ala Asp Val
    290                 295                 300

gtg cca gcg tct ggg gag ctg gtg aga cgg tac aca aac aca ctc ctc 960
Val Pro Ala Ser Gly Glu Leu Val Arg Arg Tyr Thr Asn Thr Leu Leu
305                 310                 315                 320

tca ggg gac tcc tgg gcc cag cag acc ttc cgg gtg gag cat tcg ccc 1008
Ser Gly Asp Ser Trp Ala Gln Gln Thr Phe Arg Val Glu His Ser Pro
                325                 330                 335

atc gag acc ttg gtc cag gtc aac aac aac tcc gtt cgg gca acc atg 1056
Ile Glu Thr Leu Val Gln Val Asn Asn Asn Ser Val Arg Ala Thr Met

340                 345                 350

cac aat tac aag ctg att ctc aac agg agc ctg tct atc tca gag agc      1104
His Asn Tyr Lys Leu Ile Leu Asn Arg Ser Leu Ser Ile Ser Glu Ser
        355                 360                 365

cga gtc ctg cag ctc gcg gtc ctg gtc aac gac tca gac ttc cag ggg      1152
Arg Val Leu Gln Leu Ala Val Leu Val Asn Asp Ser Asp Phe Gln Gly
    370                 375                 380

cct ggg gca ggt ggt atc ctc gtc ctc cat ttc aac gtg tct gta ctg      1200
Pro Gly Ala Gly Gly Ile Leu Val Leu His Phe Asn Val Ser Val Leu
385                 390                 395                 400

ccc gtc acc ctg aac cta ccc agg gcc tac tcc ttc cca gtg aat aag      1248
Pro Val Thr Leu Asn Leu Pro Arg Ala Tyr Ser Phe Pro Val Asn Lys
                405                 410                 415

agg gcc cgc cgc tat gcc cag atc ggg aaa gtc tgt gtg gaa aac tgc      1296
Arg Ala Arg Arg Tyr Ala Gln Ile Gly Lys Val Cys Val Glu Asn Cys
            420                 425                 430

cag gag ttc agc ggt gtc tcc atc cag tac aag ctg cag cct tcc agc      1344
Gln Glu Phe Ser Gly Val Ser Ile Gln Tyr Lys Leu Gln Pro Ser Ser
        435                 440                 445

atc aac tgc act gcc cta ggt gtg gtc acc tca ccc gag gac acc tcg      1392
Ile Asn Cys Thr Ala Leu Gly Val Val Thr Ser Pro Glu Asp Thr Ser
    450                 455                 460

ggg acc cta ttt gta aat gac aca gag gcc ctg cgg cga cct gag tgc      1440
Gly Thr Leu Phe Val Asn Asp Thr Glu Ala Leu Arg Arg Pro Glu Cys
465                 470                 475                 480

acc aag ctt cag tac acg gtg gta gcc act gac cgg cag acc cgc aga      1488
Thr Lys Leu Gln Tyr Thr Val Val Ala Thr Asp Arg Gln Thr Arg Arg
                485                 490                 495

cag acc cag gct tcg cta gtg gtc act gtg gag ggg aca tcc att act      1536
Gln Thr Gln Ala Ser Leu Val Val Thr Val Glu Gly Thr Ser Ile Thr
            500                 505                 510

gaa gaa gta ggc tgc ccc aag tcc tgt gca gta aac aag agg cgc ccc      1584
Glu Glu Val Gly Cys Pro Lys Ser Cys Ala Val Asn Lys Arg Arg Pro
        515                 520                 525

gag tgt gag gaa tgt ggt ggc ctg ggt tct cca act ggc agg tgc gag      1632
Glu Cys Glu Glu Cys Gly Gly Leu Gly Ser Pro Thr Gly Arg Cys Glu
    530                 535                 540

tgg cgc cag gga gat ggt aaa ggg atc acc agg aac ttc tcc acc tgc      1680

Trp Arg Gln Gly Asp Gly Lys Gly Ile Thr Arg Asn Phe Ser Thr Cys
545                 550                 555                 560

tcc ccc agt acc agg acc tgc ccc gac ggc cac tgt gat gct gtg gag      1728
Ser Pro Ser Thr Arg Thr Cys Pro Asp Gly His Cys Asp Ala Val Glu
                565                 570                 575

agc cgg gat gcc aac att tgc ccc cag gac tgt ctc cgt gcc gac att      1776
Ser Arg Asp Ala Asn Ile Cys Pro Gln Asp Cys Leu Arg Ala Asp Ile
            580                 585                 590

gtt gga gga cac gag cga ggg gag cgc cag ggc att aaa gca ggc tac      1824
Val Gly Gly His Glu Arg Gly Glu Arg Gln Gly Ile Lys Ala Gly Tyr
        595                 600                 605

ggc atc tgc aac tgt ttc cct gat gag aag aaa tgc ttc tgc gag cca      1872
Gly Ile Cys Asn Cys Phe Pro Asp Glu Lys Lys Cys Phe Cys Glu Pro
    610                 615                 620

gag gac agc cag ggc cca ctg tgt gat gcg ctg tgc cgc acg atc atc      1920
Glu Asp Ser Gln Gly Pro Leu Cys Asp Ala Leu Cys Arg Thr Ile Ile
625                 630                 635                 640

aca gct gcc ctc ttc tcc ctt atc atc tcc atc ctg ctg tcc atc ttc      1968
Thr Ala Ala Leu Phe Ser Leu Ile Ile Ser Ile Leu Leu Ser Ile Phe
                645                 650                 655

tgt gtc tgc cac cac cac aag cat ggg cac aag ccg ccc att gca tca      2016
Cys Val Cys His His His Lys His Gly His Lys Pro Pro Ile Ala Ser
            660                 665                 670

gcg gaa atg acc ttc tgc cgg ccg gcc cag ggc ttc cca atc agt tat      2064
Ala Glu Met Thr Phe Cys Arg Pro Ala Gln Gly Phe Pro Ile Ser Tyr
        675                 680                 685

tcc tcc tca ggc acc cgc cgg ccc tca ctg gat tcc acg gag aac cag      2112
Ser Ser Ser Gly Thr Arg Arg Pro Ser Leu Asp Ser Thr Glu Asn Gln
    690                 695                 700

gtt cct gtg gac tct ttc aag atc ccg gag gat cca aag tgg gaa ttt      2160
Val Pro Val Asp Ser Phe Lys Ile Pro Glu Asp Pro Lys Trp Glu Phe
705                 710                 715                 720

cct cgg aag aac tta gtt ctt ggg aaa act ctg gga gaa ggc gag ttt      2208
Pro Arg Lys Asn Leu Val Leu Gly Lys Thr Leu Gly Glu Gly Glu Phe
                725                 730                 735

gga aaa gtt gtc vag gcc aca gcc ttc cgt ctg aaa ggc cgg gca gga      2256
Gly Lys Val Val Xaa Ala Thr Ala Phe Arg Leu Lys Gly Arg Ala Gly
            740                 745                 750

tac acc aca gtg gct gtg aaa ats ctg aaa gaa aac gcc tcc cag agt 2304
Tyr Thr Thr Val Ala Val Lys Xaa Leu Lys Glu Asn Ala Ser Gln Ser
        755                 760                 765

gag tta cga gac ctg ctg tct gag ttc aac ctt ctg aaa caa gtc aac 2352
Glu Leu Arg Asp Leu Leu Ser Glu Phe Asn Leu Leu Lys Gln Val Asn
    770                 775                 780

cat cca cat gtc atc aag ttg tat ggg gcc tgc agc cag gat ggg cca 2400
His Pro His Val Ile Lys Leu Tyr Gly Ala Cys Ser Gln Asp Gly Pro
785                 790                 795                 800

ctt ctt ctc att gtg gag tat gcc aag tat ggc tct ctg cgg gga ttc 2448
Leu Leu Leu Ile Val Glu Tyr Ala Lys Tyr Gly Ser Leu Arg Gly Phe
                805                 810                 815

ctc cgt gac agc cgc aag att ggg cct gcc tat gtg agc ggt gga ggc 2496
Leu Arg Asp Ser Arg Lys Ile Gly Pro Ala Tyr Val Ser Gly Gly Gly
            820                 825                 830

agc cgc aac tcc agc tcg ctg gac cac cca gat gaa agg gta ctg acc 2544
Ser Arg Asn Ser Ser Ser Leu Asp His Pro Asp Glu Arg Val Leu Thr
        835                 840                 845

atg ggt gac ctc atc tcc ttc gcc tgg cag atc tcg agg ggt atg cag 2592
Met Gly Asp Leu Ile Ser Phe Ala Trp Gln Ile Ser Arg Gly Met Gln
    850                 855                 860

tac ttg gca gaa atg aag ctt gta cat cgg gac tta gct gcc agg aac 2640
Tyr Leu Ala Glu Met Lys Leu Val His Arg Asp Leu Ala Ala Arg Asn
865                 870                 875                 880

atc ttg gtg gct gag gga cgg aag atg aag att tcc gac ttt ggg ctg 2688
Ile Leu Val Ala Glu Gly Arg Lys Met Lys Ile Ser Asp Phe Gly Leu
                885                 890                 895

tcc cga gat gtt tat gag gaa gat tcc tat gtg aag aaa agc aag ggc 2736
Ser Arg Asp Val Tyr Glu Glu Asp Ser Tyr Val Lys Lys Ser Lys Gly
            900                 905                 910

cgg att ccc gtc aag tgg atg gca att gag tcc ctt ttc gat cac atc 2784
Arg Ile Pro Val Lys Trp Met Ala Ile Glu Ser Leu Phe Asp His Ile
        915                 920                 925

tat act act caa agt gat gtg tgg tcc ttt gga gtg ctg ctc tgg gag 2832
Tyr Thr Thr Gln Ser Asp Val Trp Ser Phe Gly Val Leu Leu Trp Glu
    930                 935                 940

att gtg acc ctg gga ggc aac ccc tac cct gga att cct cct gaa cga 2880
Ile Val Thr Leu Gly Gly Asn Pro Tyr Pro Gly Ile Pro Pro Glu Arg
945                 950                 955                 960

ctc ttc aac ctt ctg aag aca ggc cac agg atg gag agg cca gac aac     2928
Leu Phe Asn Leu Leu Lys Thr Gly His Arg Met Glu Arg Pro Asp Asn
                965                 970                 975

tgc agc gag gaa atg tac cgt ctg atg ctg cag tgc tgg aag cag gag     2976
Cys Ser Glu Glu Met Tyr Arg Leu Met Leu Gln Cys Trp Lys Gln Glu
            980                 985                 990

cca gac aag agg cca gtg ttt gct  gac atc agc aag gat  ctg gag aag     3024
Pro Asp Lys Arg Pro Val Phe Ala  Asp Ile Ser Lys Asp  Leu Glu Lys
        995                 1000                 1005

atg atg  gtc aag agc aga gac  tac ttg gac ctg gct  gca tcc aca     3069
Met Met  Val Lys Ser Arg Asp  Tyr Leu Asp Leu Ala  Ala Ser Thr
    1010                 1015                 1020

cct tcg  gac tca ctg ctg tat  gac gat ggg ctc tca  gaa gag gag     3114
Pro Ser  Asp Ser Leu Leu Tyr  Asp Asp Gly Leu Ser  Glu Glu Glu
    1025                 1030                 1035

aca ccc  ctg gtg gac tgt aac  aat gct ccc ctc ccg  cgc tcc ctc     3159
Thr Pro  Leu Val Asp Cys Asn  Asn Ala Pro Leu Pro  Arg Ser Leu
    1040                 1045                 1050

cct tcc  aca tgg att gaa aac  aaa ctc tat ggc atg  tca gac ccg     3204
Pro Ser  Thr Trp Ile Glu Asn  Lys Leu Tyr Gly Met  Ser Asp Pro
    1055                 1060                 1065

aac tgg  cct gga gag agt cct  gta cca ctc acg aga  gcc gat ggc     3249
Asn Trp  Pro Gly Glu Ser Pro  Val Pro Leu Thr Arg  Ala Asp Gly
    1070                 1075                 1080

act agc  act ggg ttc cca aga  tat gca aat gat agt  gta tat gct     3294
Thr Ser  Thr Gly Phe Pro Arg  Tyr Ala Asn Asp Ser  Val Tyr Ala
    1085                 1090                 1095

aac tgg  atg gtt tca ccc tca  gcg gca aaa tta atg  gac aca ttt     3339
Asn Trp  Met Val Ser Pro Ser  Ala Ala Lys Leu Met  Asp Thr Phe
    1100                 1105                 1110

gat agc  taa                                                        3348
Asp Ser
    1115


<210>  18
<211>  1115
<212>  PRT
<213>  Mus musculus

<220>
<221> misc_feature
<222> (180)..(180)
<223> The 'Xaa' at location 180 stands for Arg, or Ser.

<220>
<221> misc_feature
<222> (741)..(741)
<223> The 'Xaa' at location 741 stands for Lys, Glu, or Gln.

<220>
<221> misc_feature
<222> (760)..(760)
<223> The 'Xaa' at location 760 stands for Met, or Ile.

<400> 18

Met Ala Lys Ala Thr Ser Gly Ala Ala Gly Leu Gly Leu Lys Leu Ile
1               5                   10                  15

Leu Leu Leu Pro Leu Leu Gly Glu Ala Pro Leu Gly Leu Tyr Phe Ser
            20                  25                  30

Arg Asp Ala Tyr Trp Glu Arg Leu Tyr Val Asp Gln Pro Ala Gly Thr
        35                  40                  45

Pro Leu Leu Tyr Val His Ala Leu Arg Asp Ala Pro Gly Glu Val Pro
    50                  55                  60

Ser Phe Arg Leu Gly Gln His Leu Tyr Gly Val Tyr Arg Thr Arg Leu
65                  70                  75                  80

His Glu Asn Asp Trp Ile Arg Ile Asn Glu Thr Thr Gly Leu Leu Tyr
                85                  90                  95

Leu Asn Gln Ser Leu Asp His Ser Ser Trp Glu Gln Leu Ser Ile Arg
            100                 105                 110

Asn Gly Gly Phe Pro Leu Leu Thr Ile Phe Leu Gln Val Phe Leu Gly
        115                 120                 125

Ser Thr Ala Gln Arg Glu Gly Glu Cys His Trp Pro Gly Cys Thr Arg
    130                 135                 140

Val Tyr Phe Ser Phe Ile Asn Asp Thr Phe Pro Asn Cys Ser Ser Phe
145                 150                 155                 160

Lys Ala Gln Asp Leu Cys Ile Pro Glu Thr Ala Val Ser Ser Arg Val
                165                 170                 175

Arg Glu Asn Xaa Pro Pro Gly Thr Phe Tyr His Phe His Met Leu Pro
            180                 185                 190

Val Gln Phe Leu Cys Pro Asn Ile Ser Val Lys Tyr Ser Leu Leu Gly
        195                 200                 205

Gly Asp Ser Leu Pro Phe Arg Cys Asp Pro Asp Cys Leu Glu Val Ser
    210                 215                 220

Thr Arg Trp Ala Leu Asp Arg Glu Leu Arg Glu Lys Tyr Val Leu Glu
225                 230                 235                 240

Ala Leu Cys Ile Val Ala Gly Pro Gly Ala Asn Lys Glu Thr Val Thr
                245                 250                 255

Leu Ser Phe Pro Val Thr Val Tyr Asp Glu Asp Asp Ser Ala Pro Thr
            260                 265                 270

Phe Ser Gly Gly Val Gly Thr Ala Ser Ala Val Val Glu Phe Lys Arg
        275                 280                 285

Lys Glu Gly Thr Val Val Ala Thr Leu Gln Val Phe Asp Ala Asp Val
    290                 295                 300

Val Pro Ala Ser Gly Glu Leu Val Arg Arg Tyr Thr Asn Thr Leu Leu
305                 310                 315                 320

Ser Gly Asp Ser Trp Ala Gln Gln Thr Phe Arg Val Glu His Ser Pro
                325                 330                 335

Ile Glu Thr Leu Val Gln Val Asn Asn Asn Ser Val Arg Ala Thr Met

            340                 345                 350

His Asn Tyr Lys Leu Ile Leu Asn Arg Ser Leu Ser Ile Ser Glu Ser
        355                 360                 365

Arg Val Leu Gln Leu Ala Val Leu Val Asn Asp Ser Asp Phe Gln Gly
    370                 375                 380

Pro Gly Ala Gly Gly Ile Leu Val Leu His Phe Asn Val Ser Val Leu
385                 390                 395                 400

Pro Val Thr Leu Asn Leu Pro Arg Ala Tyr Ser Phe Pro Val Asn Lys
                405                 410                 415

Arg Ala Arg Arg Tyr Ala Gln Ile Gly Lys Val Cys Val Glu Asn Cys
            420                 425                 430

Gln Glu Phe Ser Gly Val Ser Ile Gln Tyr Lys Leu Gln Pro Ser Ser
        435                 440                 445

Ile Asn Cys Thr Ala Leu Gly Val Val Thr Ser Pro Glu Asp Thr Ser
    450                 455                 460

Gly Thr Leu Phe Val Asn Asp Thr Glu Ala Leu Arg Arg Pro Glu Cys
465                 470                 475                 480

Thr Lys Leu Gln Tyr Thr Val Val Ala Thr Asp Arg Gln Thr Arg Arg
                485                 490                 495

Gln Thr Gln Ala Ser Leu Val Val Thr Val Glu Gly Thr Ser Ile Thr
            500                 505                 510

Glu Glu Val Gly Cys Pro Lys Ser Cys Ala Val Asn Lys Arg Arg Pro
        515                 520                 525

Glu Cys Glu Glu Cys Gly Gly Leu Gly Ser Pro Thr Gly Arg Cys Glu
    530                 535                 540

Trp Arg Gln Gly Asp Gly Lys Gly Ile Thr Arg Asn Phe Ser Thr Cys
545                 550                 555                 560

Ser Pro Ser Thr Arg Thr Cys Pro Asp Gly His Cys Asp Ala Val Glu
                565                 570                 575

Ser Arg Asp Ala Asn Ile Cys Pro Gln Asp Cys Leu Arg Ala Asp Ile
            580                 585                 590

Val Gly Gly His Glu Arg Gly Glu Arg Gln Gly Ile Lys Ala Gly Tyr
        595                 600                 605

Gly Ile Cys Asn Cys Phe Pro Asp Glu Lys Lys Cys Phe Cys Glu Pro
    610                 615                 620

Glu Asp Ser Gln Gly Pro Leu Cys Asp Ala Leu Cys Arg Thr Ile Ile
625                 630                 635                 640

Thr Ala Ala Leu Phe Ser Leu Ile Ile Ser Ile Leu Leu Ser Ile Phe
                645                 650                 655

Cys Val Cys His His His Lys His Gly His Lys Pro Pro Ile Ala Ser
            660                 665                 670

Ala Glu Met Thr Phe Cys Arg Pro Ala Gln Gly Phe Pro Ile Ser Tyr
        675                 680                 685

Ser Ser Ser Gly Thr Arg Arg Pro Ser Leu Asp Ser Thr Glu Asn Gln
    690                 695                 700

Val Pro Val Asp Ser Phe Lys Ile Pro Glu Asp Pro Lys Trp Glu Phe
705                 710                 715                 720

Pro Arg Lys Asn Leu Val Leu Gly Lys Thr Leu Gly Glu Gly Glu Phe
                725                 730                 735

Gly Lys Val Val Xaa Ala Thr Ala Phe Arg Leu Lys Gly Arg Ala Gly
            740                 745                 750

Tyr Thr Thr Val Ala Val Lys Xaa Leu Lys Glu Asn Ala Ser Gln Ser
        755                 760                 765

Glu Leu Arg Asp Leu Leu Ser Glu Phe Asn Leu Leu Lys Gln Val Asn
    770                 775                 780

His Pro His Val Ile Lys Leu Tyr Gly Ala Cys Ser Gln Asp Gly Pro
785                 790                 795                 800

Leu Leu Leu Ile Val Glu Tyr Ala Lys Tyr Gly Ser Leu Arg Gly Phe
                805                 810                 815

Leu Arg Asp Ser Arg Lys Ile Gly Pro Ala Tyr Val Ser Gly Gly Gly
            820                 825                 830

Ser Arg Asn Ser Ser Ser Leu Asp His Pro Asp Glu Arg Val Leu Thr
        835                 840                 845

Met Gly Asp Leu Ile Ser Phe Ala Trp Gln Ile Ser Arg Gly Met Gln
    850                 855                 860

Tyr Leu Ala Glu Met Lys Leu Val His Arg Asp Leu Ala Ala Arg Asn
865                 870                 875                 880

Ile Leu Val Ala Glu Gly Arg Lys Met Lys Ile Ser Asp Phe Gly Leu
                885                 890                 895

Ser Arg Asp Val Tyr Glu Glu Asp Ser Tyr Val Lys Lys Ser Lys Gly
            900                 905                 910

Arg Ile Pro Val Lys Trp Met Ala Ile Glu Ser Leu Phe Asp His Ile
        915                 920                 925

Tyr Thr Thr Gln Ser Asp Val Trp Ser Phe Gly Val Leu Leu Trp Glu
    930                 935                 940

Ile Val Thr Leu Gly Gly Asn Pro Tyr Pro Gly Ile Pro Pro Glu Arg
945                 950                 955                 960

Leu Phe Asn Leu Leu Lys Thr Gly His Arg Met Glu Arg Pro Asp Asn
                965                 970                 975

Cys Ser Glu Glu Met Tyr Arg Leu Met Leu Gln Cys Trp Lys Gln Glu
            980                 985                 990

Pro Asp Lys Arg Pro Val Phe Ala  Asp Ile Ser Lys Asp  Leu Glu Lys
        995                 1000                 1005

Met Met  Val Lys Ser Arg Asp  Tyr Leu Asp Leu Ala  Ala Ser Thr
    1010                 1015                 1020

Pro Ser  Asp Ser Leu Leu Tyr  Asp Asp Gly Leu Ser  Glu Glu Glu
    1025                 1030                 1035

Thr Pro  Leu Val Asp Cys Asn  Asn Ala Pro Leu Pro  Arg Ser Leu
    1040                 1045                 1050

Pro Ser  Thr Trp Ile Glu Asn  Lys Leu Tyr Gly Met  Ser Asp Pro
    1055                 1060                 1065

Asn Trp  Pro Gly Glu Ser Pro  Val Pro Leu Thr Arg  Ala Asp Gly
    1070                 1075                 1080

Thr Ser  Thr Gly Phe Pro Arg  Tyr Ala Asn Asp Ser  Val Tyr Ala
    1085                 1090                 1095

Asn Trp  Met Val Ser Pro Ser  Ala Ala Lys Leu Met  Asp Thr Phe
    1100                 1105                 1110

Asp Ser
    1115

<210>  19
<211>  3222
<212>  DNA
<213>  Mus musculus

<220>
<221> CDS
<222> (1)..(3219)

<400> 19
atg gcg aaa gcg acg tcc ggc gcc gca ggg ctg ggg ctg aag ctg att 48
Met Ala Lys Ala Thr Ser Gly Ala Ala Gly Leu Gly Leu Lys Leu Ile
1               5                   10                  15

ttg ctc ctg ccg ctg cta gga gaa gcc cca ctg ggc ctc tat ttc tca 96
Leu Leu Leu Pro Leu Leu Gly Glu Ala Pro Leu Gly Leu Tyr Phe Ser
            20                  25                  30

agg gat gct tac tgg gag agg ctg tat gta gac cag cca gct ggc aca 144
Arg Asp Ala Tyr Trp Glu Arg Leu Tyr Val Asp Gln Pro Ala Gly Thr
        35                  40                  45

cct ctg ctc tat gtc cat gcc cta cgg gat gcc cct gga gaa gtg ccg 192
Pro Leu Leu Tyr Val His Ala Leu Arg Asp Ala Pro Gly Glu Val Pro
    50                  55                  60

agc ttc cgc ctg ggc cag cat ctc tat ggc gtc tac cgt aca cgg ctg 240
Ser Phe Arg Leu Gly Gln His Leu Tyr Gly Val Tyr Arg Thr Arg Leu
65                  70                  75                  80

cat gag aat gac tgg atc cgc atc aat gag act act ggc ctt ctc tac 288
His Glu Asn Asp Trp Ile Arg Ile Asn Glu Thr Thr Gly Leu Leu Tyr
                85                  90                  95

ctc aat cag agc ctg gac cac agt tcc tgg gaa cag ctc agc atc cgc 336
Leu Asn Gln Ser Leu Asp His Ser Ser Trp Glu Gln Leu Ser Ile Arg
            100                 105                 110

aat ggt ggt ttc ccc ctg ctc acc atc ttc ctc cag gtc ttt ctg ggg 384
Asn Gly Gly Phe Pro Leu Leu Thr Ile Phe Leu Gln Val Phe Leu Gly
        115                 120                 125

tcc aca gcc cag aga gag gga gaa tgc cat tgg cca ggc tgt acc cgt 432
Ser Thr Ala Gln Arg Glu Gly Glu Cys His Trp Pro Gly Cys Thr Arg
    130                 135                 140

gtg tac ttc tcc ttc atc aac gac acc ttc cca aat tgt agc tcc ttc 480
Val Tyr Phe Ser Phe Ile Asn Asp Thr Phe Pro Asn Cys Ser Ser Phe
145                 150                 155                 160

aaa gcc cag gat ctc tgc atc cca gag aca gcc gtg tcc ttc cga gtc 528
Lys Ala Gln Asp Leu Cys Ile Pro Glu Thr Ala Val Ser Phe Arg Val
                165                 170                 175

agg gag aac agg cct cct ggc acc ttc tac cac ttc cac atg tta ccc 576

Arg Glu Asn Arg Pro Pro Gly Thr Phe Tyr His Phe His Met Leu Pro
            180                 185                 190

gtg cag ttc ctt tgt ccc aac atc agt gtg aag tac agt ctc tta gga      624
Val Gln Phe Leu Cys Pro Asn Ile Ser Val Lys Tyr Ser Leu Leu Gly
        195                 200                 205

ggg gat agt ctg ccc ttc cgt tgt gac cca gac tgc ctg gag gtg agc      672
Gly Asp Ser Leu Pro Phe Arg Cys Asp Pro Asp Cys Leu Glu Val Ser
    210                 215                 220

act cgc tgg gcc ctg gat cga gag ctc cgg gag aag tat gtg ctg gag      720
Thr Arg Trp Ala Leu Asp Arg Glu Leu Arg Glu Lys Tyr Val Leu Glu
225                 230                 235                 240

gct ttg tgc ata gtg gca ggc cct ggt gcc aac aaa gag acg gtg act      768
Ala Leu Cys Ile Val Ala Gly Pro Gly Ala Asn Lys Glu Thr Val Thr
                245                 250                 255

ctg tcc ttc cca gtg aca gtg tat gat gag gac gac tcg gcg ccc acc      816
Leu Ser Phe Pro Val Thr Val Tyr Asp Glu Asp Asp Ser Ala Pro Thr
            260                 265                 270

ttc tct gga ggt gtg ggc act gcc agc gcg gtg gtg gag ttt aag cgg      864
Phe Ser Gly Gly Val Gly Thr Ala Ser Ala Val Val Glu Phe Lys Arg
        275                 280                 285

aag gag ggc act gtg gtg gcc acc ctg cag gtg ttc gat gca gat gtg      912
Lys Glu Gly Thr Val Val Ala Thr Leu Gln Val Phe Asp Ala Asp Val
    290                 295                 300

gtg cca gcg tct ggg gag ctg gtg aga cgg tac aca aac aca ctc ctc      960
Val Pro Ala Ser Gly Glu Leu Val Arg Arg Tyr Thr Asn Thr Leu Leu
305                 310                 315                 320

tca ggg gac tcc tgg gcc cag cag acc ttc cgg gtg gag cat tcg ccc     1008
Ser Gly Asp Ser Trp Ala Gln Gln Thr Phe Arg Val Glu His Ser Pro
                325                 330                 335

atc gag acc ttg gtc cag gtc aac aac aac tcc gtt cgg gca acc atg     1056
Ile Glu Thr Leu Val Gln Val Asn Asn Asn Ser Val Arg Ala Thr Met
            340                 345                 350

cac aat tac aag ctg att ctc aac agg agc ctg tct atc tca gag agc     1104
His Asn Tyr Lys Leu Ile Leu Asn Arg Ser Leu Ser Ile Ser Glu Ser
        355                 360                 365

cga gtc ctg cag ctc gcg gtc ctg gtc aac gac tca gac ttc cag ggg     1152
Arg Val Leu Gln Leu Ala Val Leu Val Asn Asp Ser Asp Phe Gln Gly
    370                 375                 380

cct ggg gca ggt ggt atc ctc gtc ctc cat ttc aac gtg tct gta ctg 1200
Pro Gly Ala Gly Gly Ile Leu Val Leu His Phe Asn Val Ser Val Leu
385                 390                 395                 400

ccc gtc acc ctg aac cta ccc agg gcc tac tcc ttc cca gtg aat aag 1248
Pro Val Thr Leu Asn Leu Pro Arg Ala Tyr Ser Phe Pro Val Asn Lys
                405                 410                 415

agg gcc cgc cgc tat gcc cag atc ggg aaa gtc tgt gtg gaa aac tgc 1296
Arg Ala Arg Arg Tyr Ala Gln Ile Gly Lys Val Cys Val Glu Asn Cys
            420                 425                 430

cag gag ttc agc ggt gtc tcc atc cag tac aag ctg cag cct tcc agc 1344
Gln Glu Phe Ser Gly Val Ser Ile Gln Tyr Lys Leu Gln Pro Ser Ser
        435                 440                 445

atc aac tgc act gcc cta ggt gtg gtc acc tca ccc gag gac acc tcg 1392
Ile Asn Cys Thr Ala Leu Gly Val Val Thr Ser Pro Glu Asp Thr Ser
    450                 455                 460

ggg acc cta ttt gta aat gac aca gag gcc ctg cgg cga cct gag tgc 1440
Gly Thr Leu Phe Val Asn Asp Thr Glu Ala Leu Arg Arg Pro Glu Cys
465                 470                 475                 480

acc aag ctt cag tac acg gtg gta gcc act gac cgg cag acc cgc aga 1488
Thr Lys Leu Gln Tyr Thr Val Val Ala Thr Asp Arg Gln Thr Arg Arg
                485                 490                 495

cag acc cag gct tcg cta gtg gtc act gtg gag ggg aca tcc att act 1536
Gln Thr Gln Ala Ser Leu Val Val Thr Val Glu Gly Thr Ser Ile Thr
            500                 505                 510

gaa gaa gta ggc tgc ccc aag tcc tgt gca gta aac aag agg cgc ccc 1584
Glu Glu Val Gly Cys Pro Lys Ser Cys Ala Val Asn Lys Arg Arg Pro
        515                 520                 525

gag tgt gag gaa tgt ggt ggc ctg ggt tct cca act ggc agg tgc gag 1632
Glu Cys Glu Glu Cys Gly Gly Leu Gly Ser Pro Thr Gly Arg Cys Glu
    530                 535                 540

tgg cgc cag gga gat ggt aaa ggg atc acc agg aac ttc tcc acc tgc 1680
Trp Arg Gln Gly Asp Gly Lys Gly Ile Thr Arg Asn Phe Ser Thr Cys
545                 550                 555                 560

tcc ccc agt acc agg acc tgc ccc gac ggc cac tgt gat gct gtg gag 1728
Ser Pro Ser Thr Arg Thr Cys Pro Asp Gly His Cys Asp Ala Val Glu
                565                 570                 575

agc cgg gat gcc aac att tgc ccc cag gac tgt ctc cgt gcc gac att 1776
Ser Arg Asp Ala Asn Ile Cys Pro Gln Asp Cys Leu Arg Ala Asp Ile
            580                 585                 590

gtt gga gga cac gag cga ggg gag cgc cag ggc att aaa gca ggc tac 1824
Val Gly Gly His Glu Arg Gly Glu Arg Gln Gly Ile Lys Ala Gly Tyr
        595                 600                 605

ggc atc tgc aac tgt ttc cct gat gag aag aaa tgc ttc tgc gag cca 1872
Gly Ile Cys Asn Cys Phe Pro Asp Glu Lys Lys Cys Phe Cys Glu Pro
    610                 615                 620

gag gac agc cag ggc cca ctg tgt gat gcg ctg tgc cgc acg atc atc 1920
Glu Asp Ser Gln Gly Pro Leu Cys Asp Ala Leu Cys Arg Thr Ile Ile
625                 630                 635                 640

aca gct gcc ctc ttc tcc ctt atc atc tcc atc ctg ctg tcc atc ttc 1968
Thr Ala Ala Leu Phe Ser Leu Ile Ile Ser Ile Leu Leu Ser Ile Phe
                645                 650                 655

tgt gtc tgc cac cac cac aag cat ggg cac aag ccg ccc att gca tca 2016
Cys Val Cys His His His Lys His Gly His Lys Pro Pro Ile Ala Ser
            660                 665                 670

gcg gaa atg acc ttc tgc cgg ccg gcc cag ggc ttc cca atc agt tat 2064
Ala Glu Met Thr Phe Cys Arg Pro Ala Gln Gly Phe Pro Ile Ser Tyr
        675                 680                 685

tcc tcc tca ggc acc cgc cgg ccc tca ctg gat tcc acg gag aac cag 2112
Ser Ser Ser Gly Thr Arg Arg Pro Ser Leu Asp Ser Thr Glu Asn Gln
    690                 695                 700

gtt cct gtg gac tct ttc aag atc ccg gag gat cca aag tgg gaa ttt 2160
Val Pro Val Asp Ser Phe Lys Ile Pro Glu Asp Pro Lys Trp Glu Phe
705                 710                 715                 720

cct cgg aag aac tta gtt ctt ggg aaa act ctg gga gaa ggc gag ttt 2208
Pro Arg Lys Asn Leu Val Leu Gly Lys Thr Leu Gly Glu Gly Glu Phe
                725                 730                 735

gga aaa gtt gtc aag gcc aca gcc ttc cgt ctg aaa ggc cgg gca gga 2256
Gly Lys Val Val Lys Ala Thr Ala Phe Arg Leu Lys Gly Arg Ala Gly
            740                 745                 750

tac acc aca gtg gct gtg aaa atg ctg aaa gaa aac gcc tcc cag agt 2304
Tyr Thr Thr Val Ala Val Lys Met Leu Lys Glu Asn Ala Ser Gln Ser
        755                 760                 765

gag tta cga gac ctg ctg tct gag ttc aac ctt ctg aaa caa gtc aac 2352
Glu Leu Arg Asp Leu Leu Ser Glu Phe Asn Leu Leu Lys Gln Val Asn
    770                 775                 780

cat cca cat gtc atc aag ttg tat ggg gcc tgc agc cag gat ggg cca 2400
His Pro His Val Ile Lys Leu Tyr Gly Ala Cys Ser Gln Asp Gly Pro

785                 790                 795                 800

ctt ctt ctc att gtg gag tat gcc aag tat ggc tcc ctg cgg gga ttc     2448
Leu Leu Leu Ile Val Glu Tyr Ala Lys Tyr Gly Ser Leu Arg Gly Phe
                805                 810                 815

ctc cgt gac agc cgc aag att ggg cct gcc tat gtg agc ggt gga ggc     2496
Leu Arg Asp Ser Arg Lys Ile Gly Pro Ala Tyr Val Ser Gly Gly Gly
            820                 825                 830

agc cgc aac tcc agc tcg ctg gac cac cca gat gaa agg gta ctg acc     2544
Ser Arg Asn Ser Ser Ser Leu Asp His Pro Asp Glu Arg Val Leu Thr
        835                 840                 845

atg ggt gac ctc atc tcc ttc gcc tgg cag atc tcg agg ggt atg cag     2592
Met Gly Asp Leu Ile Ser Phe Ala Trp Gln Ile Ser Arg Gly Met Gln
    850                 855                 860

tac ttg gca gaa atg aag ctt gta cat cgg gac tta gct gcc agg aac     2640
Tyr Leu Ala Glu Met Lys Leu Val His Arg Asp Leu Ala Ala Arg Asn
865                 870                 875                 880

atc ttg gtg gct gag gga cgg aag atg aag att tcc gac ttt ggg ctg     2688
Ile Leu Val Ala Glu Gly Arg Lys Met Lys Ile Ser Asp Phe Gly Leu
                885                 890                 895

tcc cga gat gtt tat gag gaa gat tcc tat gtg aag aaa agc aag ggc     2736
Ser Arg Asp Val Tyr Glu Glu Asp Ser Tyr Val Lys Lys Ser Lys Gly
            900                 905                 910

cgg att ccc gtc aag tgg atg gca att gag tcc ctt ttc gat cac atc     2784
Arg Ile Pro Val Lys Trp Met Ala Ile Glu Ser Leu Phe Asp His Ile
        915                 920                 925

tat act act caa agt gat gtg tgg tcc ttt gga gtg ctg ctc tgg gag     2832
Tyr Thr Thr Gln Ser Asp Val Trp Ser Phe Gly Val Leu Leu Trp Glu
    930                 935                 940

att gtg acc ctg gga ggc aac ccc tac cct gga att cct cct gaa cga     2880
Ile Val Thr Leu Gly Gly Asn Pro Tyr Pro Gly Ile Pro Pro Glu Arg
945                 950                 955                 960

ctc ttc aac ctt ctg aag aca ggc cac agg atg gag agg cca gac aac     2928
Leu Phe Asn Leu Leu Lys Thr Gly His Arg Met Glu Arg Pro Asp Asn
                965                 970                 975

tgc agc gag gaa atg tac cgt ctg atg ctg cag tgc tgg aag cag gag     2976
Cys Ser Glu Glu Met Tyr Arg Leu Met Leu Gln Cys Trp Lys Gln Glu
            980                 985                 990

cca gac aag agg cca gtg ttt gct  gac atc agc aag gat  ctg gag aag     3024

Pro Asp Lys Arg Pro Val Phe Ala Asp Ile Ser Lys Asp Leu Glu Lys
        995                 1000                1005

atg atg gtc aag agc aga gac tac ttg gac ctg gct gca tcc aca 3069
Met Met Val Lys Ser Arg Asp Tyr Leu Asp Leu Ala Ala Ser Thr
    1010                1015                1020

cct tcg gac tca ctg ctg tat gac gat ggg ctc tca gaa gag gag 3114
Pro Ser Asp Ser Leu Leu Tyr Asp Asp Gly Leu Ser Glu Glu Glu
    1025                1030                1035

aca ccc ctg gtg gac tgt aac aat gct ccc ctc ccg cgc tcc ctc 3159
Thr Pro Leu Val Asp Cys Asn Asn Ala Pro Leu Pro Arg Ser Leu
    1040                1045                1050

cct tcc aca tgg att gaa aac aaa ctc tat ggt aga att tca cat 3204
Pro Ser Thr Trp Ile Glu Asn Lys Leu Tyr Gly Arg Ile Ser His
    1055                1060                1065

gca ttt act aga ttc tag 3222
Ala Phe Thr Arg Phe
    1070

<210> 20
<211> 1073
<212> PRT
<213> Mus musculus

<400> 20

Met Ala Lys Ala Thr Ser Gly Ala Ala Gly Leu Gly Leu Lys Leu Ile
1               5                   10                  15

Leu Leu Leu Pro Leu Leu Gly Glu Ala Pro Leu Gly Leu Tyr Phe Ser
            20                  25                  30

Arg Asp Ala Tyr Trp Glu Arg Leu Tyr Val Asp Gln Pro Ala Gly Thr
        35                  40                  45

Pro Leu Leu Tyr Val His Ala Leu Arg Asp Ala Pro Gly Glu Val Pro
    50                  55                  60

Ser Phe Arg Leu Gly Gln His Leu Tyr Gly Val Tyr Arg Thr Arg Leu
65                  70                  75                  80

His Glu Asn Asp Trp Ile Arg Ile Asn Glu Thr Thr Gly Leu Leu Tyr
                85                  90                  95

Leu Asn Gln Ser Leu Asp His Ser Ser Trp Glu Gln Leu Ser Ile Arg
            100                 105                 110

Asn Gly Gly Phe Pro Leu Leu Thr Ile Phe Leu Gln Val Phe Leu Gly
        115                 120                 125

Ser Thr Ala Gln Arg Glu Gly Glu Cys His Trp Pro Gly Cys Thr Arg
    130                 135                 140

Val Tyr Phe Ser Phe Ile Asn Asp Thr Phe Pro Asn Cys Ser Ser Phe
145                 150                 155                 160

Lys Ala Gln Asp Leu Cys Ile Pro Glu Thr Ala Val Ser Phe Arg Val
                165                 170                 175

Arg Glu Asn Arg Pro Pro Gly Thr Phe Tyr His Phe His Met Leu Pro
            180                 185                 190

Val Gln Phe Leu Cys Pro Asn Ile Ser Val Lys Tyr Ser Leu Leu Gly
        195                 200                 205

Gly Asp Ser Leu Pro Phe Arg Cys Asp Pro Asp Cys Leu Glu Val Ser
    210                 215                 220

Thr Arg Trp Ala Leu Asp Arg Glu Leu Arg Glu Lys Tyr Val Leu Glu
225                 230                 235                 240

Ala Leu Cys Ile Val Ala Gly Pro Gly Ala Asn Lys Glu Thr Val Thr
                245                 250                 255

Leu Ser Phe Pro Val Thr Val Tyr Asp Glu Asp Asp Ser Ala Pro Thr
            260                 265                 270

Phe Ser Gly Gly Val Gly Thr Ala Ser Ala Val Val Glu Phe Lys Arg
        275                 280                 285

Lys Glu Gly Thr Val Val Ala Thr Leu Gln Val Phe Asp Ala Asp Val
    290                 295                 300

Val Pro Ala Ser Gly Glu Leu Val Arg Arg Tyr Thr Asn Thr Leu Leu
305                 310                 315                 320

Ser Gly Asp Ser Trp Ala Gln Gln Thr Phe Arg Val Glu His Ser Pro
                325                 330                 335

Ile Glu Thr Leu Val Gln Val Asn Asn Asn Ser Val Arg Ala Thr Met
            340                 345                 350

His Asn Tyr Lys Leu Ile Leu Asn Arg Ser Leu Ser Ile Ser Glu Ser
        355                 360                 365

Arg Val Leu Gln Leu Ala Val Leu Val Asn Asp Ser Asp Phe Gln Gly
    370                 375                 380

Pro Gly Ala Gly Gly Ile Leu Val Leu His Phe Asn Val Ser Val Leu
385                 390                 395                 400

Pro Val Thr Leu Asn Leu Pro Arg Ala Tyr Ser Phe Pro Val Asn Lys
                405                 410                 415

Arg Ala Arg Arg Tyr Ala Gln Ile Gly Lys Val Cys Val Glu Asn Cys
            420                 425                 430

Gln Glu Phe Ser Gly Val Ser Ile Gln Tyr Lys Leu Gln Pro Ser Ser
        435                 440                 445

Ile Asn Cys Thr Ala Leu Gly Val Val Thr Ser Pro Glu Asp Thr Ser
    450                 455                 460

Gly Thr Leu Phe Val Asn Asp Thr Glu Ala Leu Arg Arg Pro Glu Cys
465                 470                 475                 480

Thr Lys Leu Gln Tyr Thr Val Val Ala Thr Asp Arg Gln Thr Arg Arg
                485                 490                 495

Gln Thr Gln Ala Ser Leu Val Val Thr Val Glu Gly Thr Ser Ile Thr
            500                 505                 510

Glu Glu Val Gly Cys Pro Lys Ser Cys Ala Val Asn Lys Arg Arg Pro
        515                 520                 525

Glu Cys Glu Glu Cys Gly Gly Leu Gly Ser Pro Thr Gly Arg Cys Glu
    530                 535                 540

Trp Arg Gln Gly Asp Gly Lys Gly Ile Thr Arg Asn Phe Ser Thr Cys
545                 550                 555                 560

Ser Pro Ser Thr Arg Thr Cys Pro Asp Gly His Cys Asp Ala Val Glu
                565                 570                 575

Ser Arg Asp Ala Asn Ile Cys Pro Gln Asp Cys Leu Arg Ala Asp Ile
            580                 585                 590

Val Gly Gly His Glu Arg Gly Glu Arg Gln Gly Ile Lys Ala Gly Tyr
        595                 600                 605

Gly Ile Cys Asn Cys Phe Pro Asp Glu Lys Lys Cys Phe Cys Glu Pro
    610                 615                 620

Glu Asp Ser Gln Gly Pro Leu Cys Asp Ala Leu Cys Arg Thr Ile Ile
625                 630                 635                 640

Thr Ala Ala Leu Phe Ser Leu Ile Ile Ser Ile Leu Leu Ser Ile Phe
                645                 650                 655

Cys Val Cys His His His Lys His Gly His Lys Pro Pro Ile Ala Ser
            660                 665                 670

Ala Glu Met Thr Phe Cys Arg Pro Ala Gln Gly Phe Pro Ile Ser Tyr
        675                 680                 685

Ser Ser Ser Gly Thr Arg Arg Pro Ser Leu Asp Ser Thr Glu Asn Gln

690                 695                 700

Val Pro Val Asp Ser Phe Lys Ile Pro Glu Asp Pro Lys Trp Glu Phe
705                 710                 715                 720

Pro Arg Lys Asn Leu Val Leu Gly Lys Thr Leu Gly Glu Gly Glu Phe
                725                 730                 735

Gly Lys Val Val Lys Ala Thr Ala Phe Arg Leu Lys Gly Arg Ala Gly
            740                 745                 750

Tyr Thr Thr Val Ala Val Lys Met Leu Lys Glu Asn Ala Ser Gln Ser
        755                 760                 765

Glu Leu Arg Asp Leu Leu Ser Glu Phe Asn Leu Leu Lys Gln Val Asn
    770                 775                 780

His Pro His Val Ile Lys Leu Tyr Gly Ala Cys Ser Gln Asp Gly Pro
785                 790                 795                 800

Leu Leu Leu Ile Val Glu Tyr Ala Lys Tyr Gly Ser Leu Arg Gly Phe
                805                 810                 815

Leu Arg Asp Ser Arg Lys Ile Gly Pro Ala Tyr Val Ser Gly Gly Gly
            820                 825                 830

Ser Arg Asn Ser Ser Ser Leu Asp His Pro Asp Glu Arg Val Leu Thr
        835                 840                 845

Met Gly Asp Leu Ile Ser Phe Ala Trp Gln Ile Ser Arg Gly Met Gln
    850                 855                 860

Tyr Leu Ala Glu Met Lys Leu Val His Arg Asp Leu Ala Ala Arg Asn
865                 870                 875                 880

Ile Leu Val Ala Glu Gly Arg Lys Met Lys Ile Ser Asp Phe Gly Leu
                885                 890                 895

Ser Arg Asp Val Tyr Glu Glu Asp Ser Tyr Val Lys Lys Ser Lys Gly
            900                 905                 910

Arg Ile Pro Val Lys Trp Met Ala Ile Glu Ser Leu Phe Asp His Ile
        915                 920                 925

Tyr Thr Thr Gln Ser Asp Val Trp Ser Phe Gly Val Leu Leu Trp Glu
    930                 935                 940

Ile Val Thr Leu Gly Gly Asn Pro Tyr Pro Gly Ile Pro Pro Glu Arg
945                 950                 955                 960

Leu Phe Asn Leu Leu Lys Thr Gly His Arg Met Glu Arg Pro Asp Asn
                965                 970                 975

Cys Ser Glu Glu Met Tyr Arg Leu Met Leu Gln Cys Trp Lys Gln Glu
            980                 985                 990

Pro Asp Lys Arg Pro Val Phe Ala  Asp Ile Ser Lys Asp  Leu Glu Lys
        995                 1000                1005

Met Met  Val Lys Ser Arg Asp  Tyr Leu Asp Leu Ala  Ala Ser Thr
    1010                1015                1020

Pro Ser  Asp Ser Leu Leu Tyr  Asp Asp Gly Leu Ser  Glu Glu Glu
    1025                1030                1035

Thr Pro  Leu Val Asp Cys Asn  Asn Ala Pro Leu Pro  Arg Ser Leu
    1040                1045                1050

Pro Ser  Thr Trp Ile Glu Asn  Lys Leu Tyr Gly Arg  Ile Ser His
    1055                1060                1065

Ala Phe  Thr Arg Phe
    1070

<210>  21
<211>  2388
<212>  DNA

<213>  Mus musculus

<220>
<221>  CDS
<222>  (1)..(2385)

<400>  21
atg tcc atg ccc tac ggg atg ccc ctg gag aag tgc cga gct tcc gcc      48
Met Ser Met Pro Tyr Gly Met Pro Leu Glu Lys Cys Arg Ala Ser Ala
1               5                   10                  15

tgg gcc agc atc tct atg gcg tct acc gta cac ggc tgc atg aga atg      96
Trp Ala Ser Ile Ser Met Ala Ser Thr Val His Gly Cys Met Arg Met
            20                  25                  30

act gga tcc gca tca atg aga cta ctg gcc ttc tct acc tca atc aga      144
Thr Gly Ser Ala Ser Met Arg Leu Leu Ala Phe Ser Thr Ser Ile Arg
        35                  40                  45

gcc tgg acc aca gtt cct ggg aac agc tca gca tcc gca atg ggt ggt      192
Ala Trp Thr Thr Val Pro Gly Asn Ser Ser Ala Ser Ala Met Gly Gly
    50                  55                  60

ttc ccc ctg ctc acc atc ttc ctc cag gtc ttt ctg ggg tcc aca gcc      240
Phe Pro Leu Leu Thr Ile Phe Leu Gln Val Phe Leu Gly Ser Thr Ala
65                  70                  75                  80

cag aga gag gga gaa tgc cat tgg cca ggc tgt acc cgt gtg tac ttc      288
Gln Arg Glu Gly Glu Cys His Trp Pro Gly Cys Thr Arg Val Tyr Phe
                85                  90                  95

tcc ttc atc aac gac acc ttc cca aat tgt agc tcc ttc aaa gcc cag      336
Ser Phe Ile Asn Asp Thr Phe Pro Asn Cys Ser Ser Phe Lys Ala Gln
            100                 105                 110

gat ctc tgc atc cca gag aca gcc gtg tcc ttc cga gtc agg gag aac      384
Asp Leu Cys Ile Pro Glu Thr Ala Val Ser Phe Arg Val Arg Glu Asn
        115                 120                 125

agg cct cct ggc acc ttc tac cac ttc cac atg tta ccc gtg cag ttc      432
Arg Pro Pro Gly Thr Phe Tyr His Phe His Met Leu Pro Val Gln Phe
    130                 135                 140

ctt tgt ccc aac atc agt gtg aag tac agt ctc tta gga ggg gat agt      480
Leu Cys Pro Asn Ile Ser Val Lys Tyr Ser Leu Leu Gly Gly Asp Ser
145                 150                 155                 160

ctg ccc ttc cgt tgt gac cca gac tgc ctg gag gtg agc act cgc tgg      528
Leu Pro Phe Arg Cys Asp Pro Asp Cys Leu Glu Val Ser Thr Arg Trp
                165                 170                 175

gcc ctg gat cga gag ctc cgg gag aag tat gtg ctg gag gct ttg tgc 576
Ala Leu Asp Arg Glu Leu Arg Glu Lys Tyr Val Leu Glu Ala Leu Cys
            180                 185                 190

ata gtg gca ggc cct ggt gcc aac aaa gag acg gtg act ctg tcc ttc 624
Ile Val Ala Gly Pro Gly Ala Asn Lys Glu Thr Val Thr Leu Ser Phe
        195                 200                 205

ccg gtg aca gtg tat gat gag gac gac tcg gcg ccc acc ttc tct gga 672
Pro Val Thr Val Tyr Asp Glu Asp Asp Ser Ala Pro Thr Phe Ser Gly
    210                 215                 220

ggt gtg ggc act gcc agc gcg gtg gtg gag ttt aag cgg aag gag ggc 720
Gly Val Gly Thr Ala Ser Ala Val Val Glu Phe Lys Arg Lys Glu Gly
225                 230                 235                 240

act gtg gtg gcc acc ctg cag gtg ttc gat gca gat gtg gtg cca gcg 768
Thr Val Val Ala Thr Leu Gln Val Phe Asp Ala Asp Val Val Pro Ala
                245                 250                 255

tct ggg gag ctg gtg aga cgg tac aca aac aca ctc ctc tca ggg gac 816
Ser Gly Glu Leu Val Arg Arg Tyr Thr Asn Thr Leu Leu Ser Gly Asp
            260                 265                 270

tcc tgg gcc cag cag acc ttc cgg gtg gag cat tcg ccc atc gag acc 864
Ser Trp Ala Gln Gln Thr Phe Arg Val Glu His Ser Pro Ile Glu Thr
        275                 280                 285

ttg gtc cag gtc aac aac aac tcc gtt cgg gca acc atg cac aat tac 912
Leu Val Gln Val Asn Asn Asn Ser Val Arg Ala Thr Met His Asn Tyr
    290                 295                 300

aag ctg att ctc aac agg agc ctg tct atc tca gag agc cga gtc ctg 960
Lys Leu Ile Leu Asn Arg Ser Leu Ser Ile Ser Glu Ser Arg Val Leu
305                 310                 315                 320

cag ctc gcg gtc ctg gtc aac gac tca gac ttc cag ggg cct ggg gca 1008
Gln Leu Ala Val Leu Val Asn Asp Ser Asp Phe Gln Gly Pro Gly Ala
                325                 330                 335

ggt ggt atc ctc gtc ctc cat ttc aac gtg tct gta ctg ccc gtc acc 1056
Gly Gly Ile Leu Val Leu His Phe Asn Val Ser Val Leu Pro Val Thr
            340                 345                 350

ctg aac cta ccc agg gcc tac tcc ttc cca gtg aat aag agg gcc cgc 1104
Leu Asn Leu Pro Arg Ala Tyr Ser Phe Pro Val Asn Lys Arg Ala Arg
        355                 360                 365

cgc tat gcc cag atc ggg aaa gtc tgt gtg gaa aac tgc cag gag ttc 1152
Arg Tyr Ala Gln Ile Gly Lys Val Cys Val Glu Asn Cys Gln Glu Phe

370                 375                 380

agc ggt gtc tcc atc cag tac aag ctg cag cct tcc agc atc aac tgc      1200
Ser Gly Val Ser Ile Gln Tyr Lys Leu Gln Pro Ser Ser Ile Asn Cys
385                 390                 395                 400

act gcc cta ggt gtg gtc acc tca ccc gag gac acc tcg ggg acc cta      1248
Thr Ala Leu Gly Val Val Thr Ser Pro Glu Asp Thr Ser Gly Thr Leu
                405                 410                 415

ttt gta aat gac aca gag gcc ctg cgg cga cct gag tgc acc aag ctt      1296
Phe Val Asn Asp Thr Glu Ala Leu Arg Arg Pro Glu Cys Thr Lys Leu
            420                 425                 430

cag tac acg gtg gta gcc act gac cgg cag acc cgc aga cag acc cag      1344
Gln Tyr Thr Val Val Ala Thr Asp Arg Gln Thr Arg Arg Gln Thr Gln
        435                 440                 445

gct tcg cta gtg gtc act gtg gag ggg aca tcc att act gaa gaa gta      1392
Ala Ser Leu Val Val Thr Val Glu Gly Thr Ser Ile Thr Glu Glu Val
    450                 455                 460

ggc tgc ccc aag tcc tgt gca gta aac aag agg cgc ccc gag tgt gag      1440
Gly Cys Pro Lys Ser Cys Ala Val Asn Lys Arg Arg Pro Glu Cys Glu
465                 470                 475                 480

gaa tgt ggt ggc ctg ggt tct cca act ggc agg tgc gag tgg cgc cag      1488
Glu Cys Gly Gly Leu Gly Ser Pro Thr Gly Arg Cys Glu Trp Arg Gln
                485                 490                 495

gga gat ggt aaa ggg atc acc agg aac ttc tcc acc tgc tcc ccc agt      1536
Gly Asp Gly Lys Gly Ile Thr Arg Asn Phe Ser Thr Cys Ser Pro Ser
            500                 505                 510

acc agg acc tgc ccc gac ggc cac tgt gat gct gtg gag agc cgg gat      1584
Thr Arg Thr Cys Pro Asp Gly His Cys Asp Ala Val Glu Ser Arg Asp
        515                 520                 525

gcc aac att tgc ccc cag gac tgt ctc cgt gcc gac att gtt gga gga      1632
Ala Asn Ile Cys Pro Gln Asp Cys Leu Arg Ala Asp Ile Val Gly Gly
    530                 535                 540

cac gag cga ggg gag cgc cag ggc att aaa gca ggc tac ggc atc tgc      1680
His Glu Arg Gly Glu Arg Gln Gly Ile Lys Ala Gly Tyr Gly Ile Cys
545                 550                 555                 560

aac tgt ttc cct gat gag aag aaa tgc ttc tgc gag cca gag gac agc      1728
Asn Cys Phe Pro Asp Glu Lys Lys Cys Phe Cys Glu Pro Glu Asp Ser
                565                 570                 575

cag ggc cca ctg tgt gat gcg ctg tgc cgc acg atc atc aca gct gcc      1776

Gln Gly Pro Leu Cys Asp Ala Leu Cys Arg Thr Ile Ile Thr Ala Ala
            580                 585                 590

ctc ttc tcc ctt atc atc tcc atc ctg ctg tcc atc ttc tgt gtc tgc 1824
Leu Phe Ser Leu Ile Ile Ser Ile Leu Leu Ser Ile Phe Cys Val Cys
        595                 600                 605

cac cac cac aag cat ggg cac aag ccg ccc att gca tca gcg gaa atg 1872
His His His Lys His Gly His Lys Pro Pro Ile Ala Ser Ala Glu Met
    610                 615                 620

acc ttc tgc cgg ccg gcc cag ggc ttc cca atc agt tat tcc tcc tca 1920
Thr Phe Cys Arg Pro Ala Gln Gly Phe Pro Ile Ser Tyr Ser Ser Ser
625                 630                 635                 640

ggc acc cgc cgg ccc tca ctg gat tcc acg gag aac cag gtt cct gtg 1968
Gly Thr Arg Arg Pro Ser Leu Asp Ser Thr Glu Asn Gln Val Pro Val
                645                 650                 655

gac tct ttc aag atc ccg gag gat cca aag tgg gaa ttt cct cgg aag 2016
Asp Ser Phe Lys Ile Pro Glu Asp Pro Lys Trp Glu Phe Pro Arg Lys
            660                 665                 670

aac tta gtt ctt ggg aaa act ctg gga gaa ggc gag ttt gga aaa gtt 2064
Asn Leu Val Leu Gly Lys Thr Leu Gly Glu Gly Glu Phe Gly Lys Val
        675                 680                 685

gtc aag gcc aca gcc ttc cgt ctg aaa ggc cgg gca gga tac acc aca 2112
Val Lys Ala Thr Ala Phe Arg Leu Lys Gly Arg Ala Gly Tyr Thr Thr
    690                 695                 700

gtg gct gtg aaa atg ctg aaa gaa aac gcc tcc cag agt gag tta cga 2160
Val Ala Val Lys Met Leu Lys Glu Asn Ala Ser Gln Ser Glu Leu Arg
705                 710                 715                 720

gac ctg ctg tct gag ttc aac ctt ctg aaa caa gtc aac cat cca cat 2208
Asp Leu Leu Ser Glu Phe Asn Leu Leu Lys Gln Val Asn His Pro His
                725                 730                 735

gtc atc aag ttg tat ggg gcc tgc agc cag gat ggg cca ctt ctt ctc 2256
Val Ile Lys Leu Tyr Gly Ala Cys Ser Gln Asp Gly Pro Leu Leu Leu
            740                 745                 750

att gtg gag tat gcc aag tat ggc tcc ctg cgg gga ttc ctc cgt gac 2304
Ile Val Glu Tyr Ala Lys Tyr Gly Ser Leu Arg Gly Phe Leu Arg Asp
        755                 760                 765

agc cgc aag att ggg cct gcc tat gtg agc ggt gga ggc agc cgc aac 2352
Ser Arg Lys Ile Gly Pro Ala Tyr Val Ser Gly Gly Gly Ser Arg Asn
    770                 775                 780

tcc agc tcg ctg gac cac cca gat gaa ggg tac tga                    2388
Ser Ser Ser Leu Asp His Pro Asp Glu Gly Tyr
785                 790                 795

<210>  22
<211>  795
<212>  PRT
<213>  Mus musculus

<400>  22

Met Ser Met Pro Tyr Gly Met Pro Leu Glu Lys Cys Arg Ala Ser Ala
1               5                   10                  15

Trp Ala Ser Ile Ser Met Ala Ser Thr Val His Gly Cys Met Arg Met
            20                  25                  30

Thr Gly Ser Ala Ser Met Arg Leu Leu Ala Phe Ser Thr Ser Ile Arg
        35                  40                  45

Ala Trp Thr Thr Val Pro Gly Asn Ser Ser Ala Ser Ala Met Gly Gly
    50                  55                  60

Phe Pro Leu Leu Thr Ile Phe Leu Gln Val Phe Leu Gly Ser Thr Ala
65                  70                  75                  80

Gln Arg Glu Gly Glu Cys His Trp Pro Gly Cys Thr Arg Val Tyr Phe
                85                  90                  95

Ser Phe Ile Asn Asp Thr Phe Pro Asn Cys Ser Ser Phe Lys Ala Gln
            100                 105                 110

Asp Leu Cys Ile Pro Glu Thr Ala Val Ser Phe Arg Val Arg Glu Asn
        115                 120                 125

Arg Pro Pro Gly Thr Phe Tyr His Phe His Met Leu Pro Val Gln Phe
    130                 135                 140

Leu Cys Pro Asn Ile Ser Val Lys Tyr Ser Leu Leu Gly Gly Asp Ser
145                 150                 155                 160

Leu Pro Phe Arg Cys Asp Pro Asp Cys Leu Glu Val Ser Thr Arg Trp
                165                 170                 175

Ala Leu Asp Arg Glu Leu Arg Glu Lys Tyr Val Leu Glu Ala Leu Cys
            180                 185                 190

Ile Val Ala Gly Pro Gly Ala Asn Lys Glu Thr Val Thr Leu Ser Phe
        195                 200                 205

Pro Val Thr Val Tyr Asp Glu Asp Asp Ser Ala Pro Thr Phe Ser Gly
    210                 215                 220

Gly Val Gly Thr Ala Ser Ala Val Val Glu Phe Lys Arg Lys Glu Gly
225                 230                 235                 240

Thr Val Val Ala Thr Leu Gln Val Phe Asp Ala Asp Val Val Pro Ala
                245                 250                 255

Ser Gly Glu Leu Val Arg Arg Tyr Thr Asn Thr Leu Leu Ser Gly Asp
            260                 265                 270

Ser Trp Ala Gln Gln Thr Phe Arg Val Glu His Ser Pro Ile Glu Thr
        275                 280                 285

Leu Val Gln Val Asn Asn Asn Ser Val Arg Ala Thr Met His Asn Tyr
    290                 295                 300

Lys Leu Ile Leu Asn Arg Ser Leu Ser Ile Ser Glu Ser Arg Val Leu
305                 310                 315                 320

Gln Leu Ala Val Leu Val Asn Asp Ser Asp Phe Gln Gly Pro Gly Ala
                325                 330                 335

Gly Gly Ile Leu Val Leu His Phe Asn Val Ser Val Leu Pro Val Thr
            340                 345                 350

Leu Asn Leu Pro Arg Ala Tyr Ser Phe Pro Val Asn Lys Arg Ala Arg
        355                 360                 365

Arg Tyr Ala Gln Ile Gly Lys Val Cys Val Glu Asn Cys Gln Glu Phe
    370                 375                 380

Ser Gly Val Ser Ile Gln Tyr Lys Leu Gln Pro Ser Ser Ile Asn Cys
385                 390                 395                 400

Thr Ala Leu Gly Val Val Thr Ser Pro Glu Asp Thr Ser Gly Thr Leu
                405                 410                 415

Phe Val Asn Asp Thr Glu Ala Leu Arg Arg Pro Glu Cys Thr Lys Leu
            420                 425                 430

Gln Tyr Thr Val Val Ala Thr Asp Arg Gln Thr Arg Arg Gln Thr Gln
        435                 440                 445

Ala Ser Leu Val Val Thr Val Glu Gly Thr Ser Ile Thr Glu Glu Val
    450                 455                 460

Gly Cys Pro Lys Ser Cys Ala Val Asn Lys Arg Arg Pro Glu Cys Glu
465                 470                 475                 480

Glu Cys Gly Gly Leu Gly Ser Pro Thr Gly Arg Cys Glu Trp Arg Gln
                485                 490                 495

Gly Asp Gly Lys Gly Ile Thr Arg Asn Phe Ser Thr Cys Ser Pro Ser
            500                 505                 510

Thr Arg Thr Cys Pro Asp Gly His Cys Asp Ala Val Glu Ser Arg Asp
        515                 520                 525

Ala Asn Ile Cys Pro Gln Asp Cys Leu Arg Ala Asp Ile Val Gly Gly
    530                 535                 540

His Glu Arg Gly Glu Arg Gln Gly Ile Lys Ala Gly Tyr Gly Ile Cys
545                 550                 555                 560

Asn Cys Phe Pro Asp Glu Lys Lys Cys Phe Cys Glu Pro Glu Asp Ser

                565                 570                 575

Gln Gly Pro Leu Cys Asp Ala Leu Cys Arg Thr Ile Ile Thr Ala Ala
            580                 585                 590

Leu Phe Ser Leu Ile Ile Ser Ile Leu Leu Ser Ile Phe Cys Val Cys
        595                 600                 605

His His His Lys His Gly His Lys Pro Pro Ile Ala Ser Ala Glu Met
    610                 615                 620

Thr Phe Cys Arg Pro Ala Gln Gly Phe Pro Ile Ser Tyr Ser Ser Ser
625                 630                 635                 640

Gly Thr Arg Arg Pro Ser Leu Asp Ser Thr Glu Asn Gln Val Pro Val
                645                 650                 655

Asp Ser Phe Lys Ile Pro Glu Asp Pro Lys Trp Glu Phe Pro Arg Lys
            660                 665                 670

Asn Leu Val Leu Gly Lys Thr Leu Gly Glu Gly Glu Phe Gly Lys Val
        675                 680                 685

Val Lys Ala Thr Ala Phe Arg Leu Lys Gly Arg Ala Gly Tyr Thr Thr
    690                 695                 700

Val Ala Val Lys Met Leu Lys Glu Asn Ala Ser Gln Ser Glu Leu Arg
705                 710                 715                 720

Asp Leu Leu Ser Glu Phe Asn Leu Leu Lys Gln Val Asn His Pro His
                725                 730                 735

Val Ile Lys Leu Tyr Gly Ala Cys Ser Gln Asp Gly Pro Leu Leu Leu
            740                 745                 750

Ile Val Glu Tyr Ala Lys Tyr Gly Ser Leu Arg Gly Phe Leu Arg Asp
        755                 760                 765

Ser Arg Lys Ile Gly Pro Ala Tyr Val Ser Gly Gly Gly Ser Arg Asn
    770                 775                 780

Ser Ser Ser Leu Asp His Pro Asp Glu Gly Tyr
785                 790                 795

<210>  23
<211>  3327
<212>  DNA
<213>  Canis lupus familiaris

<220>
<221>  CDS
<222>  (1)..(3324)

<400>  23
atg gca gca tct cgg atc att aag ggt tgg ttc gtg ccc ttc tgc cag        48
Met Ala Ala Ser Arg Ile Ile Lys Gly Trp Phe Val Pro Phe Cys Gln
1               5                   10                  15

gct cca ctg gga ctc tac ttc tcc agg gat gct tat tgg gag aag ctg        96
Ala Pro Leu Gly Leu Tyr Phe Ser Arg Asp Ala Tyr Trp Glu Lys Leu
            20                  25                  30

tat gtg gac cag cca tct ggc atg ccc ctg ctc tat gtc cat gcc ctg       144
Tyr Val Asp Gln Pro Ser Gly Met Pro Leu Leu Tyr Val His Ala Leu
        35                  40                  45

cgg gac atc ccc gag gag gtg cct agc ttc cgc cta ggc cag cac ctt       192
Arg Asp Ile Pro Glu Glu Val Pro Ser Phe Arg Leu Gly Gln His Leu
    50                  55                  60

tac ggc atc gcc tac cgt gca agg ctg cat gag aac gac tgg atc cgc       240
Tyr Gly Ile Ala Tyr Arg Ala Arg Leu His Glu Asn Asp Trp Ile Arg
65                  70                  75                  80

atc gag gag gac aca ggc ctt ctc tac ctt aac cgg agc ctg gat cac       288
Ile Glu Glu Asp Thr Gly Leu Leu Tyr Leu Asn Arg Ser Leu Asp His
                85                  90                  95

agc acc tgg gag aag ctc agc atc cgc aat ggc ggc ttc cct gtg ctc       336
Ser Thr Trp Glu Lys Leu Ser Ile Arg Asn Gly Gly Phe Pro Val Leu
            100                 105                 110

acc atc tac gtc cag gtc ttc ctg tca tct gca tcc ctg cgt gag ggc       384
Thr Ile Tyr Val Gln Val Phe Leu Ser Ser Ala Ser Leu Arg Glu Gly
        115                 120                 125

gag tgc cag tgg cca ggc tgt gcc cgt gtg tac ttc tcc ttc att aac 432
Glu Cys Gln Trp Pro Gly Cys Ala Arg Val Tyr Phe Ser Phe Ile Asn
    130                 135                 140

acc tcc ttc cca gct tgt ggt tcc ctc aaa ccc cgg gag ctc tgc ttc 480
Thr Ser Phe Pro Ala Cys Gly Ser Leu Lys Pro Arg Glu Leu Cys Phe
145                 150                 155                 160

cct gag act ggt gtc tcc ttc cgc att cga gag aac agg cct ccc ggc 528
Pro Glu Thr Gly Val Ser Phe Arg Ile Arg Glu Asn Arg Pro Pro Gly
                165                 170                 175

acc ttc cac caa ttc cgt ctg ctg ccc atg cag ttc ctc tgc ccc aac 576
Thr Phe His Gln Phe Arg Leu Leu Pro Met Gln Phe Leu Cys Pro Asn
            180                 185                 190

atc agc gtg tcc tac aag ctc cta gaa ggt gag aat ctg ccc ttc cgt 624
Ile Ser Val Ser Tyr Lys Leu Leu Glu Gly Glu Asn Leu Pro Phe Arg
        195                 200                 205

tgc gcc ccg gac agc ctg gag gtg agc aca cgc tgg gcc ctg gac cgt 672
Cys Ala Pro Asp Ser Leu Glu Val Ser Thr Arg Trp Ala Leu Asp Arg
    210                 215                 220

gag ctg cgg gag aag tat gag ctg gtg gcc gcg tgc acc gtg cgc gtc 720
Glu Leu Arg Glu Lys Tyr Glu Leu Val Ala Ala Cys Thr Val Arg Val
225                 230                 235                 240

ggt gcg cgt aaa gag gag gtg gtg atg gtg ccc ttc ccc gtg acc gtg 768
Gly Ala Arg Lys Glu Glu Val Val Met Val Pro Phe Pro Val Thr Val
                245                 250                 255

tat gac gag gac gac tcg gcg ccc acc ttc ctc ggg ggc ttc gac act 816
Tyr Asp Glu Asp Asp Ser Ala Pro Thr Phe Leu Gly Gly Phe Asp Thr
            260                 265                 270

gcc agc gcc gtg gtg gag ttc aag agg aag gag ggc act gtg gtg gcc 864
Ala Ser Ala Val Val Glu Phe Lys Arg Lys Glu Gly Thr Val Val Ala
        275                 280                 285

aca ata cat gtc ttt gac gcg gac gtg gta cca gca tct ggg gag ctc 912
Thr Ile His Val Phe Asp Ala Asp Val Val Pro Ala Ser Gly Glu Leu
    290                 295                 300

ctg agg cga tac aca agt aca cta ctc cct ggg gat gcc tgg gcc ctc 960
Leu Arg Arg Tyr Thr Ser Thr Leu Leu Pro Gly Asp Ala Trp Ala Leu
305                 310                 315                 320

cag aca ttt cgt gtg gaa cac tca ccc aac gag acc ttg gtc cag gcc 1008
Gln Thr Phe Arg Val Glu His Ser Pro Asn Glu Thr Leu Val Gln Ala
                325                 330                 335

aac ggc agc ttt gtg cgg gca act gtg cat gac tac agg ctg gtt ctc 1056
Asn Gly Ser Phe Val Arg Ala Thr Val His Asp Tyr Arg Leu Val Leu
            340                 345                 350

aac cgg agc ctc ccc atc tcg gag agc cgc tcc ctg cag ctg gcc gtt 1104
Asn Arg Ser Leu Pro Ile Ser Glu Ser Arg Ser Leu Gln Leu Ala Val
        355                 360                 365

ctg gtc aat gac tcg gac ttc cag ggt ccc ggg gag ggc atc ctc ttg 1152
Leu Val Asn Asp Ser Asp Phe Gln Gly Pro Gly Glu Gly Ile Leu Leu
    370                 375                 380

ctc cac ttc aac gtg acg gtg ctg cct gtc agc ctg cac tta ccc agc 1200
Leu His Phe Asn Val Thr Val Leu Pro Val Ser Leu His Leu Pro Ser
385                 390                 395                 400

gcc tac acc ttc act gtg agc aga cgg gcc cgc cgc ttt gcc cag att 1248
Ala Tyr Thr Phe Thr Val Ser Arg Arg Ala Arg Arg Phe Ala Gln Ile
                405                 410                 415

ggg aaa gtc tgt gtg gaa aac tgc cag gag ttc agt ggc atc cac gtg 1296
Gly Lys Val Cys Val Glu Asn Cys Gln Glu Phe Ser Gly Ile His Val
            420                 425                 430

cag tac aag ctg cag ctc tcc agc acc aac tgt agc gtc ctg ggg gtg 1344
Gln Tyr Lys Leu Gln Leu Ser Ser Thr Asn Cys Ser Val Leu Gly Val
        435                 440                 445

gtc acg tca gcc gag gac acc aca ggg acc ctg tac gtg aat gac acg 1392
Val Thr Ser Ala Glu Asp Thr Thr Gly Thr Leu Tyr Val Asn Asp Thr
    450                 455                 460

gaa gcc ctg cag cgg ctc gat tgt tct caa ctc cag tac aca gtg gtg 1440
Glu Ala Leu Gln Arg Leu Asp Cys Ser Gln Leu Gln Tyr Thr Val Val
465                 470                 475                 480

gct gcc gac cgg cca acc cgc agg cag acc cag gcc ccg ctg gtc gtc 1488
Ala Ala Asp Arg Pro Thr Arg Arg Gln Thr Gln Ala Pro Leu Val Val
                485                 490                 495

acc gtg gag ggg aca tat gtg gct gag gag cca ggc tgc ccc ctg tcc 1536
Thr Val Glu Gly Thr Tyr Val Ala Glu Glu Pro Gly Cys Pro Leu Ser
            500                 505                 510

tgt gca gtc agc aag aga cgg cct gag tgt gaa gag tgc ggc ggc ctg 1584
Cys Ala Val Ser Lys Arg Arg Pro Glu Cys Glu Glu Cys Gly Gly Leu
        515                 520                 525

ggc tct ctg acg ggc aag tgc gag tgg aga cag gga gat ggc aaa ggg 1632
Gly Ser Leu Thr Gly Lys Cys Glu Trp Arg Gln Gly Asp Gly Lys Gly

530                 535                 540

atc acc aga aac ttc tcc acc tgc tcc ccc aat gtc aag acc tgc cct      1680
Ile Thr Arg Asn Phe Ser Thr Cys Ser Pro Asn Val Lys Thr Cys Pro
545                 550                 555                 560

gat ggc cac tgt gat gct gta gag agc agg aat gtc aac atc tgc ccc      1728
Asp Gly His Cys Asp Ala Val Glu Ser Arg Asn Val Asn Ile Cys Pro
                565                 570                 575

cag gac tgc ctc cgt ggc agc atc atc ggt ggg cat gag cca ggg gac      1776
Gln Asp Cys Leu Arg Gly Ser Ile Ile Gly Gly His Glu Pro Gly Asp
            580                 585                 590

cgc tgg ggg ata aaa gct ggc ttc gga atc tgc aac tgt ttt cct gaa      1824
Arg Trp Gly Ile Lys Ala Gly Phe Gly Ile Cys Asn Cys Phe Pro Glu
        595                 600                 605

gag aag aag tgc ttc tgc gag cct gaa gac agc cag gac cca ctg tgt      1872
Glu Lys Lys Cys Phe Cys Glu Pro Glu Asp Ser Gln Asp Pro Leu Cys
    610                 615                 620

gat gag ctc tgc cgc acg gtg att gca gcg gct gtg ctc ttc tcc ttc      1920
Asp Glu Leu Cys Arg Thr Val Ile Ala Ala Ala Val Leu Phe Ser Phe
625                 630                 635                 640

atc gtc tcc atg ctg ctc tcc acc ttc tgc atc cac cgc tac cac aag      1968
Ile Val Ser Met Leu Leu Ser Thr Phe Cys Ile His Arg Tyr His Lys
                645                 650                 655

aat gcc cac aag ccg ccc atc gcc tct gcc gaa atg acc ttc cgc cgg      2016
Asn Ala His Lys Pro Pro Ile Ala Ser Ala Glu Met Thr Phe Arg Arg
            660                 665                 670

ccc gcc cag gcc ttc cca gtc agc tac tcc tca tca ggc gcc cgc cgg      2064
Pro Ala Gln Ala Phe Pro Val Ser Tyr Ser Ser Ser Gly Ala Arg Arg
        675                 680                 685

ccc tca ctg gac tcc atg gag aac cag gtc tct gtg gat gcc ttc aaa      2112
Pro Ser Leu Asp Ser Met Glu Asn Gln Val Ser Val Asp Ala Phe Lys
    690                 695                 700

atc ccg gag gat cct aag tgg gaa ttc cct cgg aag aac tta gtt ctt      2160
Ile Pro Glu Asp Pro Lys Trp Glu Phe Pro Arg Lys Asn Leu Val Leu
705                 710                 715                 720

gga aaa act ctg gga gaa ggt gaa ttt gga aaa gtg gtt aag gca aca      2208
Gly Lys Thr Leu Gly Glu Gly Glu Phe Gly Lys Val Val Lys Ala Thr
                725                 730                 735

gct ttc cgg cta aaa ggc aaa gca gga tac acg acc gtt gcc gtg aag      2256

Ala Phe Arg Leu Lys Gly Lys Ala Gly Tyr Thr Thr Val Ala Val Lys
            740                 745                 750

atg ctg aaa gag aat gct tcc cca agc gag ctg cgg gat ctg cta tca      2304
Met Leu Lys Glu Asn Ala Ser Pro Ser Glu Leu Arg Asp Leu Leu Ser
        755                 760                 765

gaa ttc aat ctc ctg aag cag gtc aac cac ccg cat gtc atc aag ctg      2352
Glu Phe Asn Leu Leu Lys Gln Val Asn His Pro His Val Ile Lys Leu
    770                 775                 780

tac gga gcc tgc agc cag gat ggg cca ctc ttc ctc att gtg gag tat      2400
Tyr Gly Ala Cys Ser Gln Asp Gly Pro Leu Phe Leu Ile Val Glu Tyr
785                 790                 795                 800

gcc aag tat ggc tca ctg cgg ggc ttc ctc cga gaa agc cgc aag gcg      2448
Ala Lys Tyr Gly Ser Leu Arg Gly Phe Leu Arg Glu Ser Arg Lys Ala
                805                 810                 815

ggt cca ggc tac gtg ggc agt gga ggc agc cga agc tcc agc tac ctg      2496
Gly Pro Gly Tyr Val Gly Ser Gly Gly Ser Arg Ser Ser Ser Tyr Leu
            820                 825                 830

gac aac ccc gag gag cgg gcg ctg acc atg ggc gac ctc atc tcc ttc      2544
Asp Asn Pro Glu Glu Arg Ala Leu Thr Met Gly Asp Leu Ile Ser Phe
        835                 840                 845

gcc tgg cag atc tct cgg ggg atg cgg tac ctg gca gag atg aag ctt      2592
Ala Trp Gln Ile Ser Arg Gly Met Arg Tyr Leu Ala Glu Met Lys Leu
    850                 855                 860

gtc cat cgg gac ttg gcc gcc aga aac gtc ctg gtg gct gag ggg cgg      2640
Val His Arg Asp Leu Ala Ala Arg Asn Val Leu Val Ala Glu Gly Arg
865                 870                 875                 880

aag atg aag att tca gat ttt ggc ctc tcc cga gat gtt tat gag gag      2688
Lys Met Lys Ile Ser Asp Phe Gly Leu Ser Arg Asp Val Tyr Glu Glu
                885                 890                 895

gat tcc tac gtg aag agg agc aag ggt cgg att cca gtc aag tgg atg      2736
Asp Ser Tyr Val Lys Arg Ser Lys Gly Arg Ile Pro Val Lys Trp Met
            900                 905                 910

gca att gaa tct ctt ttc gat cat atc tat acc acc caa agt gat gtg      2784
Ala Ile Glu Ser Leu Phe Asp His Ile Tyr Thr Thr Gln Ser Asp Val
        915                 920                 925

tgg tcc ttc ggt gtc ctg ctg tgg gag att gtg act ctg ggg ggc aac      2832
Trp Ser Phe Gly Val Leu Leu Trp Glu Ile Val Thr Leu Gly Gly Asn
    930                 935                 940

ccc tac cct ggg att cct cca gag cgg ctc ttc aac ctt ctg aag aca     2880
Pro Tyr Pro Gly Ile Pro Pro Glu Arg Leu Phe Asn Leu Leu Lys Thr
945                 950                 955                 960

ggc tac cgg atg gag agg cct gac aac tgc agc gag gag atg tat ggt     2928
Gly Tyr Arg Met Glu Arg Pro Asp Asn Cys Ser Glu Glu Met Tyr Gly
                965                 970                 975

cta atg ctg cag tgt tgg aag cag gaa cca gac aag agg cca gtg ttt     2976
Leu Met Leu Gln Cys Trp Lys Gln Glu Pro Asp Lys Arg Pro Val Phe
            980                 985                 990

gct gac atc agc aaa gac cta gag  aag atg atg gtt aag  aac aga gac     3024
Ala Asp Ile Ser Lys Asp Leu Glu  Lys Met Met Val Lys  Asn Arg Asp
        995                 1000                1005

tac ttg  gac ctg gcg gcg tcc  acc cca tct gac tcc  ctg ctt tat     3069
Tyr Leu  Asp Leu Ala Ala Ser  Thr Pro Ser Asp Ser  Leu Leu Tyr
    1010                1015                1020

gac gat  ggc ctc tcg gag gag  gag aca cct ctg gtg  gac tgt aat     3114
Asp Asp  Gly Leu Ser Glu Glu  Glu Thr Pro Leu Val  Asp Cys Asn
    1025                1030                1035

aat gct  ccc ctc cct cga acc  ctt ccc tcc acg tgg  att gaa aac     3159
Asn Ala  Pro Leu Pro Arg Thr  Leu Pro Ser Thr Trp  Ile Glu Asn
    1040                1045                1050

aaa ctc  tat ggc atg tca gac  ccg aac tgg cct gaa  gag agt cct     3204
Lys Leu  Tyr Gly Met Ser Asp  Pro Asn Trp Pro Glu  Glu Ser Pro
    1055                1060                1065

gta cca  ctc acg aga gct gat  ggc act aac tct gtg  tgt cca aga     3249
Val Pro  Leu Thr Arg Ala Asp  Gly Thr Asn Ser Val  Cys Pro Arg
    1070                1075                1080

tat gca  aat gat agt gta tat  gct aac tgg atg gtt  tca ccc tca     3294
Tyr Ala  Asn Asp Ser Val Tyr  Ala Asn Trp Met Val  Ser Pro Ser
    1085                1090                1095

gcg gca  caa tta atg gac gca  ttt gat agt taa                     3327
Ala Ala  Gln Leu Met Asp Ala  Phe Asp Ser
    1100                1105

<210>  24
<211>  1108
<212>  PRT
<213>  Canis lupus familiaris

<400>  24

Met Ala Ala Ser Arg Ile Ile Lys Gly Trp Phe Val Pro Phe Cys Gln
1               5                   10                  15

Ala Pro Leu Gly Leu Tyr Phe Ser Arg Asp Ala Tyr Trp Glu Lys Leu
            20                  25                  30

Tyr Val Asp Gln Pro Ser Gly Met Pro Leu Leu Tyr Val His Ala Leu
        35                  40                  45

Arg Asp Ile Pro Glu Glu Val Pro Ser Phe Arg Leu Gly Gln His Leu
    50                  55                  60

Tyr Gly Ile Ala Tyr Arg Ala Arg Leu His Glu Asn Asp Trp Ile Arg
65                  70                  75                  80

Ile Glu Glu Asp Thr Gly Leu Leu Tyr Leu Asn Arg Ser Leu Asp His
                85                  90                  95

Ser Thr Trp Glu Lys Leu Ser Ile Arg Asn Gly Gly Phe Pro Val Leu
            100                 105                 110

Thr Ile Tyr Val Gln Val Phe Leu Ser Ser Ala Ser Leu Arg Glu Gly
        115                 120                 125

Glu Cys Gln Trp Pro Gly Cys Ala Arg Val Tyr Phe Ser Phe Ile Asn
    130                 135                 140

Thr Ser Phe Pro Ala Cys Gly Ser Leu Lys Pro Arg Glu Leu Cys Phe
145                 150                 155                 160

Pro Glu Thr Gly Val Ser Phe Arg Ile Arg Glu Asn Arg Pro Pro Gly
                165                 170                 175

Thr Phe His Gln Phe Arg Leu Leu Pro Met Gln Phe Leu Cys Pro Asn
            180                 185                 190

Ile Ser Val Ser Tyr Lys Leu Leu Glu Gly Glu Asn Leu Pro Phe Arg
        195                 200                 205

Cys Ala Pro Asp Ser Leu Glu Val Ser Thr Arg Trp Ala Leu Asp Arg
    210                 215                 220

Glu Leu Arg Glu Lys Tyr Glu Leu Val Ala Ala Cys Thr Val Arg Val
225                 230                 235                 240

Gly Ala Arg Lys Glu Glu Val Val Met Val Pro Phe Pro Val Thr Val
                245                 250                 255

Tyr Asp Glu Asp Asp Ser Ala Pro Thr Phe Leu Gly Gly Phe Asp Thr
            260                 265                 270

Ala Ser Ala Val Val Glu Phe Lys Arg Lys Glu Gly Thr Val Val Ala
        275                 280                 285

Thr Ile His Val Phe Asp Ala Asp Val Val Pro Ala Ser Gly Glu Leu
    290                 295                 300

Leu Arg Arg Tyr Thr Ser Thr Leu Leu Pro Gly Asp Ala Trp Ala Leu
305                 310                 315                 320

Gln Thr Phe Arg Val Glu His Ser Pro Asn Glu Thr Leu Val Gln Ala
                325                 330                 335

Asn Gly Ser Phe Val Arg Ala Thr Val His Asp Tyr Arg Leu Val Leu
            340                 345                 350

Asn Arg Ser Leu Pro Ile Ser Glu Ser Arg Ser Leu Gln Leu Ala Val
        355                 360                 365

Leu Val Asn Asp Ser Asp Phe Gln Gly Pro Gly Glu Gly Ile Leu Leu
    370                 375                 380

Leu His Phe Asn Val Thr Val Leu Pro Val Ser Leu His Leu Pro Ser
385                 390                 395                 400

Ala Tyr Thr Phe Thr Val Ser Arg Arg Ala Arg Arg Phe Ala Gln Ile

                405                 410                 415

Gly Lys Val Cys Val Glu Asn Cys Gln Glu Phe Ser Gly Ile His Val
            420                 425                 430

Gln Tyr Lys Leu Gln Leu Ser Ser Thr Asn Cys Ser Val Leu Gly Val
        435                 440                 445

Val Thr Ser Ala Glu Asp Thr Thr Gly Thr Leu Tyr Val Asn Asp Thr
    450                 455                 460

Glu Ala Leu Gln Arg Leu Asp Cys Ser Gln Leu Gln Tyr Thr Val Val
465                 470                 475                 480

Ala Ala Asp Arg Pro Thr Arg Arg Gln Thr Gln Ala Pro Leu Val Val
                485                 490                 495

Thr Val Glu Gly Thr Tyr Val Ala Glu Glu Pro Gly Cys Pro Leu Ser
            500                 505                 510

Cys Ala Val Ser Lys Arg Arg Pro Glu Cys Glu Glu Cys Gly Gly Leu
        515                 520                 525

Gly Ser Leu Thr Gly Lys Cys Glu Trp Arg Gln Gly Asp Gly Lys Gly
    530                 535                 540

Ile Thr Arg Asn Phe Ser Thr Cys Ser Pro Asn Val Lys Thr Cys Pro
545                 550                 555                 560

Asp Gly His Cys Asp Ala Val Glu Ser Arg Asn Val Asn Ile Cys Pro
                565                 570                 575

Gln Asp Cys Leu Arg Gly Ser Ile Ile Gly Gly His Glu Pro Gly Asp
            580                 585                 590

Arg Trp Gly Ile Lys Ala Gly Phe Gly Ile Cys Asn Cys Phe Pro Glu
        595                 600                 605

Glu Lys Lys Cys Phe Cys Glu Pro Glu Asp Ser Gln Asp Pro Leu Cys
    610                 615                 620

Asp Glu Leu Cys Arg Thr Val Ile Ala Ala Ala Val Leu Phe Ser Phe
625                 630                 635                 640

Ile Val Ser Met Leu Leu Ser Thr Phe Cys Ile His Arg Tyr His Lys
                645                 650                 655

Asn Ala His Lys Pro Pro Ile Ala Ser Ala Glu Met Thr Phe Arg Arg
            660                 665                 670

Pro Ala Gln Ala Phe Pro Val Ser Tyr Ser Ser Ser Gly Ala Arg Arg
        675                 680                 685

Pro Ser Leu Asp Ser Met Glu Asn Gln Val Ser Val Asp Ala Phe Lys
    690                 695                 700

Ile Pro Glu Asp Pro Lys Trp Glu Phe Pro Arg Lys Asn Leu Val Leu
705                 710                 715                 720

Gly Lys Thr Leu Gly Glu Gly Glu Phe Gly Lys Val Val Lys Ala Thr
                725                 730                 735

Ala Phe Arg Leu Lys Gly Lys Ala Gly Tyr Thr Thr Val Ala Val Lys
            740                 745                 750

Met Leu Lys Glu Asn Ala Ser Pro Ser Glu Leu Arg Asp Leu Leu Ser
        755                 760                 765

Glu Phe Asn Leu Leu Lys Gln Val Asn His Pro His Val Ile Lys Leu
    770                 775                 780

Tyr Gly Ala Cys Ser Gln Asp Gly Pro Leu Phe Leu Ile Val Glu Tyr
785                 790                 795                 800

Ala Lys Tyr Gly Ser Leu Arg Gly Phe Leu Arg Glu Ser Arg Lys Ala
                805                 810                 815

Gly Pro Gly Tyr Val Gly Ser Gly Gly Ser Arg Ser Ser Ser Tyr Leu
            820                 825                 830

Asp Asn Pro Glu Glu Arg Ala Leu Thr Met Gly Asp Leu Ile Ser Phe
        835                 840                 845

Ala Trp Gln Ile Ser Arg Gly Met Arg Tyr Leu Ala Glu Met Lys Leu
    850                 855                 860

Val His Arg Asp Leu Ala Ala Arg Asn Val Leu Val Ala Glu Gly Arg
865                 870                 875                 880

Lys Met Lys Ile Ser Asp Phe Gly Leu Ser Arg Asp Val Tyr Glu Glu
                885                 890                 895

Asp Ser Tyr Val Lys Arg Ser Lys Gly Arg Ile Pro Val Lys Trp Met
            900                 905                 910

Ala Ile Glu Ser Leu Phe Asp His Ile Tyr Thr Thr Gln Ser Asp Val
        915                 920                 925

Trp Ser Phe Gly Val Leu Leu Trp Glu Ile Val Thr Leu Gly Gly Asn
    930                 935                 940

Pro Tyr Pro Gly Ile Pro Pro Glu Arg Leu Phe Asn Leu Leu Lys Thr
945                 950                 955                 960

Gly Tyr Arg Met Glu Arg Pro Asp Asn Cys Ser Glu Glu Met Tyr Gly
                965                 970                 975

Leu Met Leu Gln Cys Trp Lys Gln Glu Pro Asp Lys Arg Pro Val Phe
            980                 985                 990

Ala Asp Ile Ser Lys Asp Leu Glu Lys Met Met Val Lys Asn Arg Asp
        995                 1000                1005

Tyr Leu Asp Leu Ala Ala Ser Thr Pro Ser Asp Ser Leu Leu Tyr
    1010                1015                1020

Asp Asp Gly Leu Ser Glu Glu Glu Thr Pro Leu Val Asp Cys Asn
    1025                1030                1035

Asn Ala Pro Leu Pro Arg Thr Leu Pro Ser Thr Trp Ile Glu Asn
    1040                1045                1050

Lys Leu Tyr Gly Met Ser Asp Pro Asn Trp Pro Glu Glu Ser Pro
    1055                1060                1065

Val Pro Leu Thr Arg Ala Asp Gly Thr Asn Ser Val Cys Pro Arg
    1070                1075                1080

Tyr Ala Asn Asp Ser Val Tyr Ala Asn Trp Met Val Ser Pro Ser
    1085                1090                1095

Ala Ala Gln Leu Met Asp Ala Phe Asp Ser
    1100                1105

<210> 25
<211> 3195
<212> DNA
<213> Gallus gallus

<220>
<221> CDS
<222> (1)..(3192)

<400> 25
atg cgc tcg gtg cgg ggg gcg gcc ggc ctc ctg ctg ctg tgc ggg gct 48
Met Arg Ser Val Arg Gly Ala Ala Gly Leu Leu Leu Leu Cys Gly Ala
1               5                   10                  15

tcc ttt ggt ctg tac ttc ccc aga aag gag tac tca gag aac gtc tac 96
Ser Phe Gly Leu Tyr Phe Pro Arg Lys Glu Tyr Ser Glu Asn Val Tyr
            20                  25                  30

att gac cag cca gca ggt gcg ccg ctc cta cgc atc cac gcc ttg agg 144
Ile Asp Gln Pro Ala Gly Ala Pro Leu Leu Arg Ile His Ala Leu Arg
        35                  40                  45

gat tca cat ggg aaa cag ccc act ttc atc tgt gcc aga agt ctc atc 192
Asp Ser His Gly Lys Gln Pro Thr Phe Ile Cys Ala Arg Ser Leu Ile

50                  55                  60

att tct cga gca aga tcc cat gaa aat cac tgg ttt caa atc aga gaa        240
Ile Ser Arg Ala Arg Ser His Glu Asn His Trp Phe Gln Ile Arg Glu
65                  70                  75                  80

aaa atg gga ctt ctc tac ctc agc aag agc cta gat aga gaa gac ttt        288
Lys Met Gly Leu Leu Tyr Leu Ser Lys Ser Leu Asp Arg Glu Asp Phe
                85                  90                  95

aac atg ctg tct gta gga aac tgg atg cca tta tca aag gtg atg ctg        336
Asn Met Leu Ser Val Gly Asn Trp Met Pro Leu Ser Lys Val Met Leu
            100                 105                 110

tat gtc ttc ctc tca tct cac cct ttc caa gag aag gaa tgt gac tct        384
Tyr Val Phe Leu Ser Ser His Pro Phe Gln Glu Lys Glu Cys Asp Ser
        115                 120                 125

gct act cgt acc aca gtc gtc ctc tct ttg atc aat gct act gca cca        432
Ala Thr Arg Thr Thr Val Val Leu Ser Leu Ile Asn Ala Thr Ala Pro
    130                 135                 140

gct tgc agt tca ctg tca gca agg cag ctt tgc ttc aca gaa atg gat        480
Ala Cys Ser Ser Leu Ser Ala Arg Gln Leu Cys Phe Thr Glu Met Asp
145                 150                 155                 160

ctc tcc ttt cac atc aag gag aat aaa ccc cct ggt aca ttt cat cag        528
Leu Ser Phe His Ile Lys Glu Asn Lys Pro Pro Gly Thr Phe His Gln
                165                 170                 175

ctc cag tta ccc tca gtt cat cat ctg tgt cag aat ctc agc att acc        576
Leu Gln Leu Pro Ser Val His His Leu Cys Gln Asn Leu Ser Ile Thr
            180                 185                 190

tac aaa ctg ttg gca gcc gaa ggc ctg cct ttt cgg tac aat gag aac        624
Tyr Lys Leu Leu Ala Ala Glu Gly Leu Pro Phe Arg Tyr Asn Glu Asn
        195                 200                 205

acc act ggt gtg agt gta aca cag cgc cta gat cga gag gag aga gag        672
Thr Thr Gly Val Ser Val Thr Gln Arg Leu Asp Arg Glu Glu Arg Glu
    210                 215                 220

aga tat gag ctg atc gcc aaa tgc acc gtg aga gaa ggc ttc agg gaa        720
Arg Tyr Glu Leu Ile Ala Lys Cys Thr Val Arg Glu Gly Phe Arg Glu
225                 230                 235                 240

atg gag gtt gag gtg ccc ttc ctc gtc aac gtg tta gat gaa gat gac        768
Met Glu Val Glu Val Pro Phe Leu Val Asn Val Leu Asp Glu Asp Asp
                245                 250                 255

tct cct ccc ttc ctt ccc aac ggc act gac act gca gat gct gtc gtg        816

Ser Pro Pro Phe Leu Pro Asn Gly Thr Asp Thr Ala Asp Ala Val Val
            260                 265                 270

gag ttc aat aga aaa gag gga act gtc ctt gca acg cta act gtg tat 864
Glu Phe Asn Arg Lys Glu Gly Thr Val Leu Ala Thr Leu Thr Val Tyr
        275                 280                 285

gat gca gac acc aca cct att tat ccc ctt gaa agc agc aga aag aaa 912
Asp Ala Asp Thr Thr Pro Ile Tyr Pro Leu Glu Ser Ser Arg Lys Lys
    290                 295                 300

tac aca gga aca ata att act gac gat cca tgg ata aat gaa aca ttt 960
Tyr Thr Gly Thr Ile Ile Thr Asp Asp Pro Trp Ile Asn Glu Thr Phe
305                 310                 315                 320

cgg gtg gag cac att ttc gat gaa atc cac ttc agc cca aat ggc agc 1008
Arg Val Glu His Ile Phe Asp Glu Ile His Phe Ser Pro Asn Gly Ser
                325                 330                 335

caa gag aga gga act caa cac gaa tac aaa ctg gta ctg aat aaa agt 1056
Gln Glu Arg Gly Thr Gln His Glu Tyr Lys Leu Val Leu Asn Lys Ser
            340                 345                 350

att tca gtc aca gag cat cgt tcc ttc cag ttg gat gtt ttg gtg aac 1104
Ile Ser Val Thr Glu His Arg Ser Phe Gln Leu Asp Val Leu Val Asn
        355                 360                 365

gac aca gaa ttt aat ggc cca gaa aaa tca gtg atg ctt cat ttc aat 1152
Asp Thr Glu Phe Asn Gly Pro Glu Lys Ser Val Met Leu His Phe Asn
    370                 375                 380

gtc tcc atc ctt cct gtc agc att cag ttt cca aac aca act tat cga 1200
Val Ser Ile Leu Pro Val Ser Ile Gln Phe Pro Asn Thr Thr Tyr Arg
385                 390                 395                 400

ttc aca gta aac aga aat gct caa cgt ttt gca caa ata gga aaa atc 1248
Phe Thr Val Asn Arg Asn Ala Gln Arg Phe Ala Gln Ile Gly Lys Ile
                405                 410                 415

tgc atc gaa aac tgt atg aag ttc cgt ggt gtg aac atc acc tac aag 1296
Cys Ile Glu Asn Cys Met Lys Phe Arg Gly Val Asn Ile Thr Tyr Lys
            420                 425                 430

tta ctg tcc ccc aat gtg agc tgc tat gct gtt ggc ata ctt caa ggc 1344
Leu Leu Ser Pro Asn Val Ser Cys Tyr Ala Val Gly Ile Leu Gln Gly
        435                 440                 445

cga gaa gac aaa tat gga agc ctt tat gtg aat gat tcc tct gtg cta 1392
Arg Glu Asp Lys Tyr Gly Ser Leu Tyr Val Asn Asp Ser Ser Val Leu
    450                 455                 460

ctt aga cct gaa tgc aaa gaa ata caa tac acc gtt caa gct aca gac 1440
Leu Arg Pro Glu Cys Lys Glu Ile Gln Tyr Thr Val Gln Ala Thr Asp
465                 470                 475                 480

aag cag agc agg aaa cat acc aaa acc ctt ttc act gtt gta tta gaa 1488
Lys Gln Ser Arg Lys His Thr Lys Thr Leu Phe Thr Val Val Leu Glu
                485                 490                 495

gga act ctt ttt aaa aaa gaa gag gac tgc cct gac tct tgt gct atg 1536
Gly Thr Leu Phe Lys Lys Glu Glu Asp Cys Pro Asp Ser Cys Ala Met
            500                 505                 510

agt aag cac ctt gct gaa tgt gaa gag tgt ggt ggc ctg gga gtg cta 1584
Ser Lys His Leu Ala Glu Cys Glu Glu Cys Gly Gly Leu Gly Val Leu
        515                 520                 525

aca gga aga tgc cag tgg aga cag ggg agt gga aaa gga atc acc aca 1632
Thr Gly Arg Cys Gln Trp Arg Gln Gly Ser Gly Lys Gly Ile Thr Thr
    530                 535                 540

aac tat tcc aca tgc acc cca agt atc aag aac ttg ctc cag atg gcc 1680
Asn Tyr Ser Thr Cys Thr Pro Ser Ile Lys Asn Leu Leu Gln Met Ala
545                 550                 555                 560

acc tgt gat atc gtg gag agc aaa gat cca gct att tgc ccc cag gac 1728
Thr Cys Asp Ile Val Glu Ser Lys Asp Pro Ala Ile Cys Pro Gln Asp
                565                 570                 575

tgc aca aat gga aat att att ggt ggt cat ggg aaa ggc act gga aac 1776
Cys Thr Asn Gly Asn Ile Ile Gly Gly His Gly Lys Gly Thr Gly Asn
            580                 585                 590

ctt gga att aag tca gga cat ggg att tgt tac tgc ttc cca aga cag 1824
Leu Gly Ile Lys Ser Gly His Gly Ile Cys Tyr Cys Phe Pro Arg Gln
        595                 600                 605

aac tgt tat tgt gag aaa gat gat atc aag gag caa ttt tgt gat gaa 1872
Asn Cys Tyr Cys Glu Lys Asp Asp Ile Lys Glu Gln Phe Cys Asp Glu
    610                 615                 620

gtg tgc aag act gtg ata gca ggg gcg gtg ctg ttg tcc ttc atc atc 1920
Val Cys Lys Thr Val Ile Ala Gly Ala Val Leu Leu Ser Phe Ile Ile
625                 630                 635                 640

tct gtg ctg ctt tct tcc tat ttc atc cat cgc tac cac aag aat tct 1968
Ser Val Leu Leu Ser Ser Tyr Phe Ile His Arg Tyr His Lys Asn Ser
                645                 650                 655

cca aag cca cct att gcc tct gca gaa atg aca ttc cgg cgc cca gcc 2016
Pro Lys Pro Pro Ile Ala Ser Ala Glu Met Thr Phe Arg Arg Pro Ala
            660                 665                 670

cag tca tat ccc atc agt tat tct tct act aat gtc cgt cga cct tct 2064
Gln Ser Tyr Pro Ile Ser Tyr Ser Ser Thr Asn Val Arg Arg Pro Ser
        675                 680                 685

tta gac tcc atg gag aac cag gtg tct gta gac acc ttt aag ata cca 2112
Leu Asp Ser Met Glu Asn Gln Val Ser Val Asp Thr Phe Lys Ile Pro
    690                 695                 700

gaa gat cca aag tgg gaa ttc cct cgg aag aac ctg gtt cta ggc aag 2160
Glu Asp Pro Lys Trp Glu Phe Pro Arg Lys Asn Leu Val Leu Gly Lys
705                 710                 715                 720

act ctt gga gaa gga gaa ttt ggg aag gtt gtc aag gca aca gct ttc 2208
Thr Leu Gly Glu Gly Glu Phe Gly Lys Val Val Lys Ala Thr Ala Phe
                725                 730                 735

aga ctc aag gga aga gct ggc tat act act gtg gct gtg aaa atg cta 2256
Arg Leu Lys Gly Arg Ala Gly Tyr Thr Thr Val Ala Val Lys Met Leu
            740                 745                 750

aaa gaa aat gct tcc cag agc gag ctg cga gat ctg ctg tca gag ttc 2304
Lys Glu Asn Ala Ser Gln Ser Glu Leu Arg Asp Leu Leu Ser Glu Phe
        755                 760                 765

aac ctt ttg aaa cag gtg aac cat cct cac gtc atc aaa ctt tac gga 2352
Asn Leu Leu Lys Gln Val Asn His Pro His Val Ile Lys Leu Tyr Gly
    770                 775                 780

gcc tgc agt caa gat ggc ccg ctc tat tta att gtg gaa tat gct aaa 2400
Ala Cys Ser Gln Asp Gly Pro Leu Tyr Leu Ile Val Glu Tyr Ala Lys
785                 790                 795                 800

tat ggc tcc ttg cgc agt ttt ctc agg gaa agc cga aaa gtg gga cca 2448
Tyr Gly Ser Leu Arg Ser Phe Leu Arg Glu Ser Arg Lys Val Gly Pro
                805                 810                 815

agc tac gtg ggt agt gat ggc aac aga aat tcc agt tac ttg gat aac 2496
Ser Tyr Val Gly Ser Asp Gly Asn Arg Asn Ser Ser Tyr Leu Asp Asn
            820                 825                 830

ccc gat gag aga gcc tta aca atg gga gat ctg ata tcg ttt gca tgg 2544
Pro Asp Glu Arg Ala Leu Thr Met Gly Asp Leu Ile Ser Phe Ala Trp
        835                 840                 845

cag ata tca cga ggg atg cag tac ctg gca gaa atg aag ctc gtc cat 2592
Gln Ile Ser Arg Gly Met Gln Tyr Leu Ala Glu Met Lys Leu Val His
    850                 855                 860

cgt gac tta gca gcc agg aat gtg ctg gtg gca gaa ggg cgc aaa atg 2640
Arg Asp Leu Ala Ala Arg Asn Val Leu Val Ala Glu Gly Arg Lys Met

865                 870                 875                 880

aag att tct gat ttt ggc cta tcc cgt gat gtg tat gaa gaa gat tcc      2688
Lys Ile Ser Asp Phe Gly Leu Ser Arg Asp Val Tyr Glu Glu Asp Ser
                885                 890                 895

tat gtc aag agg agc aag ggt cgg ata cct gtt aaa tgg atg gcc ata      2736
Tyr Val Lys Arg Ser Lys Gly Arg Ile Pro Val Lys Trp Met Ala Ile
            900                 905                 910

gaa tcc ctg ttt gat cat atc tat aca aca caa agt gat gtg tgg tcg      2784
Glu Ser Leu Phe Asp His Ile Tyr Thr Thr Gln Ser Asp Val Trp Ser
        915                 920                 925

ttt gga gtt ctg ctc tgg gag att gta act ctg gga ggc aat ccc tac      2832
Phe Gly Val Leu Leu Trp Glu Ile Val Thr Leu Gly Gly Asn Pro Tyr
    930                 935                 940

cca ggt att gct cct gaa aga ctc ttt aac ctc cta aaa aca ggc tac      2880
Pro Gly Ile Ala Pro Glu Arg Leu Phe Asn Leu Leu Lys Thr Gly Tyr
945                 950                 955                 960

aga atg gag aga cca gaa aac tgt agt gaa gaa atg tac aac ctg atg      2928
Arg Met Glu Arg Pro Glu Asn Cys Ser Glu Glu Met Tyr Asn Leu Met
                965                 970                 975

ttg cgc tgc tgg aag caa gaa cca gat aaa agg cca aca ttt gct gag      2976
Leu Arg Cys Trp Lys Gln Glu Pro Asp Lys Arg Pro Thr Phe Ala Glu
            980                 985                 990

atc agc aag gaa ctg gag aaa atg  atg gtg aag agt agg  gac tac tta      3024
Ile Ser Lys Glu Leu Glu Lys Met  Met Val Lys Ser Arg  Asp Tyr Leu
        995                 1000                1005

gat ctt  gcg gct tcc acc cct  tct gat tcc tta cta  tat gat gat      3069
Asp Leu  Ala Ala Ser Thr Pro  Ser Asp Ser Leu Leu  Tyr Asp Asp
    1010                1015                1020

ggg ctc  tca gaa gag gag aca  cct ctc gtg gac tgt  aat aac gct      3114
Gly Leu  Ser Glu Glu Glu Thr  Pro Leu Val Asp Cys  Asn Asn Ala
    1025                1030                1035

ccc ctc  cct cga acc ctt cct  tcc aca tgg att gaa  aac aaa ctc      3159
Pro Leu  Pro Arg Thr Leu Pro  Ser Thr Trp Ile Glu  Asn Lys Leu
    1040                1045                1050

tat ggt  aga att tca cat gca  ttt act aga ttc tag                  3195
Tyr Gly  Arg Ile Ser His Ala  Phe Thr Arg Phe
    1055                1060

<210> 26
<211> 1064
<212> PRT
<213> Gallus gallus

<400> 26

Met Arg Ser Val Arg Gly Ala Ala Gly Leu Leu Leu Leu Cys Gly Ala
1               5                   10                  15

Ser Phe Gly Leu Tyr Phe Pro Arg Lys Glu Tyr Ser Glu Asn Val Tyr
            20                  25                  30

Ile Asp Gln Pro Ala Gly Ala Pro Leu Leu Arg Ile His Ala Leu Arg
        35                  40                  45

Asp Ser His Gly Lys Gln Pro Thr Phe Ile Cys Ala Arg Ser Leu Ile
    50                  55                  60

Ile Ser Arg Ala Arg Ser His Glu Asn His Trp Phe Gln Ile Arg Glu
65                  70                  75                  80

Lys Met Gly Leu Leu Tyr Leu Ser Lys Ser Leu Asp Arg Glu Asp Phe
                85                  90                  95

Asn Met Leu Ser Val Gly Asn Trp Met Pro Leu Ser Lys Val Met Leu
            100                 105                 110

Tyr Val Phe Leu Ser Ser His Pro Phe Gln Glu Lys Glu Cys Asp Ser
        115                 120                 125

Ala Thr Arg Thr Thr Val Val Leu Ser Leu Ile Asn Ala Thr Ala Pro
    130                 135                 140

Ala Cys Ser Ser Leu Ser Ala Arg Gln Leu Cys Phe Thr Glu Met Asp
145                 150                 155                 160

Leu Ser Phe His Ile Lys Glu Asn Lys Pro Pro Gly Thr Phe His Gln
                165                 170                 175

Leu Gln Leu Pro Ser Val His His Leu Cys Gln Asn Leu Ser Ile Thr
            180                 185                 190

Tyr Lys Leu Leu Ala Ala Glu Gly Leu Pro Phe Arg Tyr Asn Glu Asn
        195                 200                 205

Thr Thr Gly Val Ser Val Thr Gln Arg Leu Asp Arg Glu Glu Arg Glu
    210                 215                 220

Arg Tyr Glu Leu Ile Ala Lys Cys Thr Val Arg Glu Gly Phe Arg Glu
225                 230                 235                 240

Met Glu Val Glu Val Pro Phe Leu Val Asn Val Leu Asp Glu Asp Asp
                245                 250                 255

Ser Pro Pro Phe Leu Pro Asn Gly Thr Asp Thr Ala Asp Ala Val Val
            260                 265                 270

Glu Phe Asn Arg Lys Glu Gly Thr Val Leu Ala Thr Leu Thr Val Tyr
        275                 280                 285

Asp Ala Asp Thr Thr Pro Ile Tyr Pro Leu Glu Ser Ser Arg Lys Lys
    290                 295                 300

Tyr Thr Gly Thr Ile Ile Thr Asp Asp Pro Trp Ile Asn Glu Thr Phe
305                 310                 315                 320

Arg Val Glu His Ile Phe Asp Glu Ile His Phe Ser Pro Asn Gly Ser
                325                 330                 335

Gln Glu Arg Gly Thr Gln His Glu Tyr Lys Leu Val Leu Asn Lys Ser
            340                 345                 350

Ile Ser Val Thr Glu His Arg Ser Phe Gln Leu Asp Val Leu Val Asn
        355                 360                 365

Asp Thr Glu Phe Asn Gly Pro Glu Lys Ser Val Met Leu His Phe Asn
    370                 375                 380

Val Ser Ile Leu Pro Val Ser Ile Gln Phe Pro Asn Thr Thr Tyr Arg
385                 390                 395                 400

Phe Thr Val Asn Arg Asn Ala Gln Arg Phe Ala Gln Ile Gly Lys Ile
                405                 410                 415

Cys Ile Glu Asn Cys Met Lys Phe Arg Gly Val Asn Ile Thr Tyr Lys
            420                 425                 430

Leu Leu Ser Pro Asn Val Ser Cys Tyr Ala Val Gly Ile Leu Gln Gly
        435                 440                 445

Arg Glu Asp Lys Tyr Gly Ser Leu Tyr Val Asn Asp Ser Ser Val Leu
    450                 455                 460

Leu Arg Pro Glu Cys Lys Glu Ile Gln Tyr Thr Val Gln Ala Thr Asp
465                 470                 475                 480

Lys Gln Ser Arg Lys His Thr Lys Thr Leu Phe Thr Val Val Leu Glu
                485                 490                 495

Gly Thr Leu Phe Lys Lys Glu Glu Asp Cys Pro Asp Ser Cys Ala Met
            500                 505                 510

Ser Lys His Leu Ala Glu Cys Glu Glu Cys Gly Gly Leu Gly Val Leu
        515                 520                 525

Thr Gly Arg Cys Gln Trp Arg Gln Gly Ser Gly Lys Gly Ile Thr Thr
    530                 535                 540

Asn Tyr Ser Thr Cys Thr Pro Ser Ile Lys Asn Leu Leu Gln Met Ala
545                 550                 555                 560

Thr Cys Asp Ile Val Glu Ser Lys Asp Pro Ala Ile Cys Pro Gln Asp
                565                 570                 575

Cys Thr Asn Gly Asn Ile Ile Gly Gly His Gly Lys Gly Thr Gly Asn
            580                 585                 590

Leu Gly Ile Lys Ser Gly His Gly Ile Cys Tyr Cys Phe Pro Arg Gln
        595                 600                 605

Asn Cys Tyr Cys Glu Lys Asp Asp Ile Lys Glu Gln Phe Cys Asp Glu
    610                 615                 620

Val Cys Lys Thr Val Ile Ala Gly Ala Val Leu Leu Ser Phe Ile Ile
625                 630                 635                 640

Ser Val Leu Leu Ser Ser Tyr Phe Ile His Arg Tyr His Lys Asn Ser
                645                 650                 655

Pro Lys Pro Pro Ile Ala Ser Ala Glu Met Thr Phe Arg Arg Pro Ala
            660                 665                 670

Gln Ser Tyr Pro Ile Ser Tyr Ser Ser Thr Asn Val Arg Arg Pro Ser
        675                 680                 685

Leu Asp Ser Met Glu Asn Gln Val Ser Val Asp Thr Phe Lys Ile Pro
    690                 695                 700

Glu Asp Pro Lys Trp Glu Phe Pro Arg Lys Asn Leu Val Leu Gly Lys
705                 710                 715                 720

Thr Leu Gly Glu Gly Glu Phe Gly Lys Val Val Lys Ala Thr Ala Phe
                725                 730                 735

Arg Leu Lys Gly Arg Ala Gly Tyr Thr Thr Val Ala Val Lys Met Leu
            740                 745                 750

Lys Glu Asn Ala Ser Gln Ser Glu Leu Arg Asp Leu Leu Ser Glu Phe
        755                 760                 765

Asn Leu Leu Lys Gln Val Asn His Pro His Val Ile Lys Leu Tyr Gly
    770                 775                 780

Ala Cys Ser Gln Asp Gly Pro Leu Tyr Leu Ile Val Glu Tyr Ala Lys

785                 790                 795                 800

Tyr Gly Ser Leu Arg Ser Phe Leu Arg Glu Ser Arg Lys Val Gly Pro
                805                 810                 815

Ser Tyr Val Gly Ser Asp Gly Asn Arg Asn Ser Ser Tyr Leu Asp Asn
            820                 825                 830

Pro Asp Glu Arg Ala Leu Thr Met Gly Asp Leu Ile Ser Phe Ala Trp
        835                 840                 845

Gln Ile Ser Arg Gly Met Gln Tyr Leu Ala Glu Met Lys Leu Val His
    850                 855                 860

Arg Asp Leu Ala Ala Arg Asn Val Leu Val Ala Glu Gly Arg Lys Met
865                 870                 875                 880

Lys Ile Ser Asp Phe Gly Leu Ser Arg Asp Val Tyr Glu Glu Asp Ser
                885                 890                 895

Tyr Val Lys Arg Ser Lys Gly Arg Ile Pro Val Lys Trp Met Ala Ile
            900                 905                 910

Glu Ser Leu Phe Asp His Ile Tyr Thr Thr Gln Ser Asp Val Trp Ser
        915                 920                 925

Phe Gly Val Leu Leu Trp Glu Ile Val Thr Leu Gly Gly Asn Pro Tyr
    930                 935                 940

Pro Gly Ile Ala Pro Glu Arg Leu Phe Asn Leu Leu Lys Thr Gly Tyr
945                 950                 955                 960

Arg Met Glu Arg Pro Glu Asn Cys Ser Glu Glu Met Tyr Asn Leu Met
                965                 970                 975

Leu Arg Cys Trp Lys Gln Glu Pro Asp Lys Arg Pro Thr Phe Ala Glu
            980                 985                 990

Ile Ser Lys Glu Leu Glu Lys Met  Met Val Lys Ser Arg  Asp Tyr Leu
      995                    1000                    1005

Asp Leu  Ala Ala Ser Thr Pro  Ser Asp Ser Leu Leu  Tyr Asp Asp
    1010                 1015                 1020

Gly Leu  Ser Glu Glu Glu Thr  Pro Leu Val Asp Cys  Asn Asn Ala
    1025                 1030                 1035

Pro Leu  Pro Arg Thr Leu Pro  Ser Thr Trp Ile Glu  Asn Lys Leu
    1040                 1045                 1050

Tyr Gly  Arg Ile Ser His Ala  Phe Thr Arg Phe
    1055                 1060

<210>  27
<211>  1998
<212>  DNA
<213>  Ornithorhynchus anatinus

<220>
<221>  CDS
<222>  (1)..(1995)

<400>  27
atg acg gtc atg aag ttt gtg aaa ttt gtg aaa tgt gca gaa ctt aca       48
Met Thr Val Met Lys Phe Val Lys Phe Val Lys Cys Ala Glu Leu Thr
1               5                   10                  15

tat tgc tcc agt gtc acc agt tgt cct ctt cac ggc ctg cat tgc cca       96
Tyr Cys Ser Ser Val Thr Ser Cys Pro Leu His Gly Leu His Cys Pro
            20                  25                  30

aga gcc cat ccg ttc ccc ctc aga gct tca gaa act ggt gag cgg cag      144
Arg Ala His Pro Phe Pro Leu Arg Ala Ser Glu Thr Gly Glu Arg Gln
        35                  40                  45

agg agc gga gcg tgc ggc gtg ggc ctg caa cta cta tta tta cta atg      192
Arg Ser Gly Ala Cys Gly Val Gly Leu Gln Leu Leu Leu Leu Leu Met
    50                  55                  60

ttt cca ttt tgt gga agc tct gcc ctg ggc ctc tat ttc ccc cgg gat      240
Phe Pro Phe Cys Gly Ser Ser Ala Leu Gly Leu Tyr Phe Pro Arg Asp
65                  70                  75                  80

acg tac tca gag aag ctg tac gtt gac cag ccg gcc ggc acg ccc ctg 288
Thr Tyr Ser Glu Lys Leu Tyr Val Asp Gln Pro Ala Gly Thr Pro Leu
                85                  90                  95

ctc tac gtc cag gcc ttg cgg gat tct cag cag gag gtt cca cgc ttc 336
Leu Tyr Val Gln Ala Leu Arg Asp Ser Gln Gln Glu Val Pro Arg Phe
            100                 105                 110

cag ctg ggg cag cac ctg tac ggc agt tac cgt tcc cag ctc cac gag 384
Gln Leu Gly Gln His Leu Tyr Gly Ser Tyr Arg Ser Gln Leu His Glu
        115                 120                 125

aat ctc tgg ttc cag atc cat gaa gac aca gga ctt ctc tac gtc aac 432
Asn Leu Trp Phe Gln Ile His Glu Asp Thr Gly Leu Leu Tyr Val Asn
    130                 135                 140

agg agt ctg ggc tgg aag gac ttg aag agc cta cgc gcc ctt aac gga 480
Arg Ser Leu Gly Trp Lys Asp Leu Lys Ser Leu Arg Ala Leu Asn Gly
145                 150                 155                 160

gga tat cct tta cta aac atc cac ctc cat gtt ttc ctt tcc ccc aaa 528
Gly Tyr Pro Leu Leu Asn Ile His Leu His Val Phe Leu Ser Pro Lys
                165                 170                 175

cct ttc tgg gag aac gag tgc ctc tgg cct agc tgc gcc aaa gtg tct 576
Pro Phe Trp Glu Asn Glu Cys Leu Trp Pro Ser Cys Ala Lys Val Ser
            180                 185                 190

ctc tcc atc atc aac acc acc gcc cca acc tgc agc tct ctg aag gcg 624
Leu Ser Ile Ile Asn Thr Thr Ala Pro Thr Cys Ser Ser Leu Lys Ala
        195                 200                 205

agg gag ttc tgc ttc ctg gat acg ggc ctc tct ttc cgc atc aag gag 672
Arg Glu Phe Cys Phe Leu Asp Thr Gly Leu Ser Phe Arg Ile Lys Glu
    210                 215                 220

aac aag cct cct ggc aca ttc cac cag ttc cgt ctc ctc cct gtg cag 720
Asn Lys Pro Pro Gly Thr Phe His Gln Phe Arg Leu Leu Pro Val Gln
225                 230                 235                 240

cag ctc tgt ccc aac atc agc atc tcc tac cag ctg ata gca gtc tta 768
Gln Leu Cys Pro Asn Ile Ser Ile Ser Tyr Gln Leu Ile Ala Val Leu
                245                 250                 255

atc ccc gtt tta cag ttg aag gaa gat cca aaa tgg gag ttt cct cgg 816
Ile Pro Val Leu Gln Leu Lys Glu Asp Pro Lys Trp Glu Phe Pro Arg
            260                 265                 270

aag aac ctg gtt ctt gga aaa act ctt ggg gaa ggc gag ttt ggg aaa 864
Lys Asn Leu Val Leu Gly Lys Thr Leu Gly Glu Gly Glu Phe Gly Lys
        275                 280                 285

gtt gtc aaa gct aca gcc ttc cga ctc aag gga aaa gct ggc tac acc     912
Val Val Lys Ala Thr Ala Phe Arg Leu Lys Gly Lys Ala Gly Tyr Thr
    290                 295                 300

acc gtg gct gtg aaa atg ctg aaa gaa aat gca tcc cag agt gaa ctc     960
Thr Val Ala Val Lys Met Leu Lys Glu Asn Ala Ser Gln Ser Glu Leu
305                 310                 315                 320

cgt gat ctg ctg tca gag ttc aac ctc ctg aaa cag gtc aac cat cca     1008
Arg Asp Leu Leu Ser Glu Phe Asn Leu Leu Lys Gln Val Asn His Pro
                325                 330                 335

cac gtc att aaa ctc tac gga gct tgc agt cag gat ggg cct ctc tac     1056
His Val Ile Lys Leu Tyr Gly Ala Cys Ser Gln Asp Gly Pro Leu Tyr
            340                 345                 350

ctg att gtg gag tat gcc aaa tac ggc tcc ctt cgg agc ttt ctc agg     1104
Leu Ile Val Glu Tyr Ala Lys Tyr Gly Ser Leu Arg Ser Phe Leu Arg
        355                 360                 365

gaa agc cgg aag gtg ggg ccc agc tac gtg ggc agt gat ggc aat cgg     1152
Glu Ser Arg Lys Val Gly Pro Ser Tyr Val Gly Ser Asp Gly Asn Arg
    370                 375                 380

aat tcg agc tac ttg gac aac cct gat gag agg gca ctg aca atg gga     1200
Asn Ser Ser Tyr Leu Asp Asn Pro Asp Glu Arg Ala Leu Thr Met Gly
385                 390                 395                 400

gac ttg atc tca ttc gcc tgg cag atc tct cga gga atg caa tat ctt     1248
Asp Leu Ile Ser Phe Ala Trp Gln Ile Ser Arg Gly Met Gln Tyr Leu
                405                 410                 415

gca gaa atg aag ctg gtt cat cgt gat ctc gct gct aga aac gtg ctg     1296
Ala Glu Met Lys Leu Val His Arg Asp Leu Ala Ala Arg Asn Val Leu
            420                 425                 430

gtg gca gaa gga cgt aaa atg aag att tca gat ttt ggc ttg tct agg     1344
Val Ala Glu Gly Arg Lys Met Lys Ile Ser Asp Phe Gly Leu Ser Arg
        435                 440                 445

gat gtg tac gaa gag gac tcc tat gtc aag agg agc aag ggt cga att     1392
Asp Val Tyr Glu Glu Asp Ser Tyr Val Lys Arg Ser Lys Gly Arg Ile
    450                 455                 460

cct gtc aag tgg atg gcg att gag tct ctt ttt gat cat atc tac act     1440
Pro Val Lys Trp Met Ala Ile Glu Ser Leu Phe Asp His Ile Tyr Thr
465                 470                 475                 480

acc caa agt gat gtg tgg tca ttt ggt gtt cta ctc tgg gag atc gtg     1488
Thr Gln Ser Asp Val Trp Ser Phe Gly Val Leu Leu Trp Glu Ile Val

485                 490                 495

acc ctc ggc gga aac cct tat cct ggg att gct cca gaa cga ctc ttt      1536
Thr Leu Gly Gly Asn Pro Tyr Pro Gly Ile Ala Pro Glu Arg Leu Phe
            500                 505                 510

aac ctc ctg aaa act ggt tac aga atg gaa agg cct gaa aac tgc agt      1584
Asn Leu Leu Lys Thr Gly Tyr Arg Met Glu Arg Pro Glu Asn Cys Ser
        515                 520                 525

gag gaa atg tac aat ttg atg ttg cgg tgc tgg aag caa gaa cca gat      1632
Glu Glu Met Tyr Asn Leu Met Leu Arg Cys Trp Lys Gln Glu Pro Asp
    530                 535                 540

aag aga cca acc ttt gca gag att agc aaa gaa cta gag aaa atg atg      1680
Lys Arg Pro Thr Phe Ala Glu Ile Ser Lys Glu Leu Glu Lys Met Met
545                 550                 555                 560

gtg aaa agt agg gac tac ttg gat ctt gct gct tcc acc cca tcc gac      1728
Val Lys Ser Arg Asp Tyr Leu Asp Leu Ala Ala Ser Thr Pro Ser Asp
                565                 570                 575

tcc tta ctc tat gat gac ggc ctc tcc gaa gag gag aca ccc ctc gtg      1776
Ser Leu Leu Tyr Asp Asp Gly Leu Ser Glu Glu Glu Thr Pro Leu Val
            580                 585                 590

gac tgt aat aat gct ccc ctc cct cga acc ctt cct tcc aca tgg att      1824
Asp Cys Asn Asn Ala Pro Leu Pro Arg Thr Leu Pro Ser Thr Trp Ile
        595                 600                 605

gaa aac aaa ctc tat ggc atg tca tac ccg aac tgg cct gag gag agt      1872
Glu Asn Lys Leu Tyr Gly Met Ser Tyr Pro Asn Trp Pro Glu Glu Ser
    610                 615                 620

cct gta cca ctc aca aga ttc gat ggc act aac tct gtg ttt tca aga      1920
Pro Val Pro Leu Thr Arg Phe Asp Gly Thr Asn Ser Val Phe Ser Arg
625                 630                 635                 640

tat gca aat gat agt gta tat gct aac tgg atg gtt tca ccc tca gcg      1968
Tyr Ala Asn Asp Ser Val Tyr Ala Asn Trp Met Val Ser Pro Ser Ala
                645                 650                 655

gca aaa ttt atg gac aag ttt gat agt taa                              1998
Ala Lys Phe Met Asp Lys Phe Asp Ser
            660                 665

<210>  28
<211>  665
<212>  PRT
<213>  Ornithorhynchus anatinus

<400> 28

Met Thr Val Met Lys Phe Val Lys Phe Val Lys Cys Ala Glu Leu Thr
1               5                   10                  15

Tyr Cys Ser Ser Val Thr Ser Cys Pro Leu His Gly Leu His Cys Pro
            20                  25                  30

Arg Ala His Pro Phe Pro Leu Arg Ala Ser Glu Thr Gly Glu Arg Gln
        35                  40                  45

Arg Ser Gly Ala Cys Gly Val Gly Leu Gln Leu Leu Leu Leu Leu Met
    50                  55                  60

Phe Pro Phe Cys Gly Ser Ser Ala Leu Gly Leu Tyr Phe Pro Arg Asp
65                  70                  75                  80

Thr Tyr Ser Glu Lys Leu Tyr Val Asp Gln Pro Ala Gly Thr Pro Leu
                85                  90                  95

Leu Tyr Val Gln Ala Leu Arg Asp Ser Gln Gln Glu Val Pro Arg Phe
            100                 105                 110

Gln Leu Gly Gln His Leu Tyr Gly Ser Tyr Arg Ser Gln Leu His Glu
        115                 120                 125

Asn Leu Trp Phe Gln Ile His Glu Asp Thr Gly Leu Leu Tyr Val Asn
    130                 135                 140

Arg Ser Leu Gly Trp Lys Asp Leu Lys Ser Leu Arg Ala Leu Asn Gly
145                 150                 155                 160

Gly Tyr Pro Leu Leu Asn Ile His Leu His Val Phe Leu Ser Pro Lys
                165                 170                 175

Pro Phe Trp Glu Asn Glu Cys Leu Trp Pro Ser Cys Ala Lys Val Ser
            180                 185                 190

Leu Ser Ile Ile Asn Thr Thr Ala Pro Thr Cys Ser Ser Leu Lys Ala
        195                 200                 205

Arg Glu Phe Cys Phe Leu Asp Thr Gly Leu Ser Phe Arg Ile Lys Glu
    210                 215                 220

Asn Lys Pro Pro Gly Thr Phe His Gln Phe Arg Leu Leu Pro Val Gln
225                 230                 235                 240

Gln Leu Cys Pro Asn Ile Ser Ile Ser Tyr Gln Leu Ile Ala Val Leu
                245                 250                 255

Ile Pro Val Leu Gln Leu Lys Glu Asp Pro Lys Trp Glu Phe Pro Arg
            260                 265                 270

Lys Asn Leu Val Leu Gly Lys Thr Leu Gly Glu Gly Glu Phe Gly Lys
        275                 280                 285

Val Val Lys Ala Thr Ala Phe Arg Leu Lys Gly Lys Ala Gly Tyr Thr
    290                 295                 300

Thr Val Ala Val Lys Met Leu Lys Glu Asn Ala Ser Gln Ser Glu Leu
305                 310                 315                 320

Arg Asp Leu Leu Ser Glu Phe Asn Leu Leu Lys Gln Val Asn His Pro
                325                 330                 335

His Val Ile Lys Leu Tyr Gly Ala Cys Ser Gln Asp Gly Pro Leu Tyr
            340                 345                 350

Leu Ile Val Glu Tyr Ala Lys Tyr Gly Ser Leu Arg Ser Phe Leu Arg
        355                 360                 365

Glu Ser Arg Lys Val Gly Pro Ser Tyr Val Gly Ser Asp Gly Asn Arg
    370                 375                 380

Asn Ser Ser Tyr Leu Asp Asn Pro Asp Glu Arg Ala Leu Thr Met Gly
385                 390                 395                 400

Asp Leu Ile Ser Phe Ala Trp Gln Ile Ser Arg Gly Met Gln Tyr Leu
                405                 410                 415

Ala Glu Met Lys Leu Val His Arg Asp Leu Ala Ala Arg Asn Val Leu
            420                 425                 430

Val Ala Glu Gly Arg Lys Met Lys Ile Ser Asp Phe Gly Leu Ser Arg
        435                 440                 445

Asp Val Tyr Glu Glu Asp Ser Tyr Val Lys Arg Ser Lys Gly Arg Ile
    450                 455                 460

Pro Val Lys Trp Met Ala Ile Glu Ser Leu Phe Asp His Ile Tyr Thr
465                 470                 475                 480

Thr Gln Ser Asp Val Trp Ser Phe Gly Val Leu Leu Trp Glu Ile Val
                485                 490                 495

Thr Leu Gly Gly Asn Pro Tyr Pro Gly Ile Ala Pro Glu Arg Leu Phe
            500                 505                 510

Asn Leu Leu Lys Thr Gly Tyr Arg Met Glu Arg Pro Glu Asn Cys Ser
        515                 520                 525

Glu Glu Met Tyr Asn Leu Met Leu Arg Cys Trp Lys Gln Glu Pro Asp
    530                 535                 540

Lys Arg Pro Thr Phe Ala Glu Ile Ser Lys Glu Leu Glu Lys Met Met
545                 550                 555                 560

Val Lys Ser Arg Asp Tyr Leu Asp Leu Ala Ala Ser Thr Pro Ser Asp
                565                 570                 575

Ser Leu Leu Tyr Asp Asp Gly Leu Ser Glu Glu Glu Thr Pro Leu Val
            580                 585                 590

Asp Cys Asn Asn Ala Pro Leu Pro Arg Thr Leu Pro Ser Thr Trp Ile
        595                 600                 605

Glu Asn Lys Leu Tyr Gly Met Ser Tyr Pro Asn Trp Pro Glu Glu Ser
    610                 615                 620

Pro Val Pro Leu Thr Arg Phe Asp Gly Thr Asn Ser Val Phe Ser Arg
625                 630                 635                 640

Tyr Ala Asn Asp Ser Val Tyr Ala Asn Trp Met Val Ser Pro Ser Ala
                645                 650                 655

Ala Lys Phe Met Asp Lys Phe Asp Ser
            660                 665

<210>  29
<211>  3180
<212>  DNA
<213>  Xenopus laevis

<220>
<221>  CDS
<222>  (1)..(3177)

<400>  29
atg gca cct gca gag tgg gca ctc tgt ttt ctc tgc ctc ttt caa gtg        48
Met Ala Pro Ala Glu Trp Ala Leu Cys Phe Leu Cys Leu Phe Gln Val
1               5                   10                  15

acc tct gga ctt tac ttt cta aga aag gac tat tat gaa aat gta tat        96
Thr Ser Gly Leu Tyr Phe Leu Arg Lys Asp Tyr Tyr Glu Asn Val Tyr
            20                  25                  30

gtg gat caa ccg gcg ggc aca cta ctt cta cag gtc cat gtg atg aag       144
Val Asp Gln Pro Ala Gly Thr Leu Leu Leu Gln Val His Val Met Lys
        35                  40                  45

gac tca cca tcg gag aaa cct cac ttt aga ctc tgc cca aca att aac       192
Asp Ser Pro Ser Glu Lys Pro His Phe Arg Leu Cys Pro Thr Ile Asn
    50                  55                  60

ttc agt aga aac tct ttt tac cat tgg ttt cgg ata gat gag cat aca       240
Phe Ser Arg Asn Ser Phe Tyr His Trp Phe Arg Ile Asp Glu His Thr
65                  70                  75                  80

ggg aac ctc tac ctc aac cgg agt ctt gac agg agc gac tat gaa gta       288
Gly Asn Leu Tyr Leu Asn Arg Ser Leu Asp Arg Ser Asp Tyr Glu Val

                85                  90                  95

ttc tcc caa gga agt caa tca gct att cag aag ata acc ctg aag gtg 336
Phe Ser Gln Gly Ser Gln Ser Ala Ile Gln Lys Ile Thr Leu Lys Val
            100                 105                 110

tca aca aag cca cat cca ttt ttg gat aag gac tgt aat aat gca ccc 384
Ser Thr Lys Pro His Pro Phe Leu Asp Lys Asp Cys Asn Asn Ala Pro
        115                 120                 125

tta act tgg gtt cat ctt aac ttt ata aac ctt aca tct tcc atc tgt 432
Leu Thr Trp Val His Leu Asn Phe Ile Asn Leu Thr Ser Ser Ile Cys
    130                 135                 140

ttc tca ctt aaa cca aag gat ctg tgc ttc aat gac aaa gat ctc tca 480
Phe Ser Leu Lys Pro Lys Asp Leu Cys Phe Asn Asp Lys Asp Leu Ser
145                 150                 155                 160

ttc cac ata aaa gaa aac aaa cct cct gga aaa ttt tac aaa ttt cac 528
Phe His Ile Lys Glu Asn Lys Pro Pro Gly Lys Phe Tyr Lys Phe His
                165                 170                 175

ccc cgg cct att gtt cag tcc tgt cca aat gtc agt gtg agt tac atg 576
Pro Arg Pro Ile Val Gln Ser Cys Pro Asn Val Ser Val Ser Tyr Met
            180                 185                 190

cta att tca gac cac agc tac cca ttc agg ttc aat cct aat acc tct 624
Leu Ile Ser Asp His Ser Tyr Pro Phe Arg Phe Asn Pro Asn Thr Ser
        195                 200                 205

gaa gtg agc ctt acg cac tct gtg gac cgc gag gag agg gag aat tat 672
Glu Val Ser Leu Thr His Ser Val Asp Arg Glu Glu Arg Glu Asn Tyr
    210                 215                 220

gat ctt gtg gca aaa tgt ttg ctg agg gat gca tcc tca gag gtg gag 720
Asp Leu Val Ala Lys Cys Leu Leu Arg Asp Ala Ser Ser Glu Val Glu
225                 230                 235                 240

gta gaa caa tct ttt cag atc aaa gtg gat gat gaa gat gac aca gcc 768
Val Glu Gln Ser Phe Gln Ile Lys Val Asp Asp Glu Asp Asp Thr Ala
                245                 250                 255

cca ttc ctc ccg aat gga aca gac act gct cat gtg gtg gtg gac ttc 816
Pro Phe Leu Pro Asn Gly Thr Asp Thr Ala His Val Val Val Asp Phe
            260                 265                 270

aaa aga aag aat ggg acc gtc ctt ggc atg ctg act gta tgg gat gct 864
Lys Arg Lys Asn Gly Thr Val Leu Gly Met Leu Thr Val Trp Asp Ala
        275                 280                 285

gac tcc aca cct gtg tat cct gca gat gtc agt cgt aaa aaa tac aca 912

Asp Ser Thr Pro Val Tyr Pro Ala Asp Val Ser Arg Lys Lys Tyr Thr
    290                 295                 300

aag acc att gtc agc aaa gac cgg ttc att ctg gaa aac ttc cgg gtg      960
Lys Thr Ile Val Ser Lys Asp Arg Phe Ile Leu Glu Asn Phe Arg Val
305                 310                 315                 320

gag cac agc ttt aag gag gtg aaa ttc cag cca aaa ggc aac tta atc      1008
Glu His Ser Phe Lys Glu Val Lys Phe Gln Pro Lys Gly Asn Leu Ile
                325                 330                 335

cgt gga aca ctt cat gaa tac agg tta gtt ctg aac aag agt att cct      1056
Arg Gly Thr Leu His Glu Tyr Arg Leu Val Leu Asn Lys Ser Ile Pro
            340                 345                 350

att atc aga aat gga tct ctg ctt gtt agt gtt ctg gtg aat gac act      1104
Ile Ile Arg Asn Gly Ser Leu Leu Val Ser Val Leu Val Asn Asp Thr
        355                 360                 365

gaa ttt cat gga cca aat gga gat gtt atg ttg tac ttc aat gtg aca      1152
Glu Phe His Gly Pro Asn Gly Asp Val Met Leu Tyr Phe Asn Val Thr
    370                 375                 380

atc tta tcg atg gaa att aga ttc cca aac atc tca tac cag tat act      1200
Ile Leu Ser Met Glu Ile Arg Phe Pro Asn Ile Ser Tyr Gln Tyr Thr
385                 390                 395                 400

gtc aac cga aat gca gac cga tat gct cag att gga aaa atc tgc ata      1248
Val Asn Arg Asn Ala Asp Arg Tyr Ala Gln Ile Gly Lys Ile Cys Ile
                405                 410                 415

gaa aat tgc aag ctt gtt gaa ggc gtc aac att aac tac aga ctg caa      1296
Glu Asn Cys Lys Leu Val Glu Gly Val Asn Ile Asn Tyr Arg Leu Gln
            420                 425                 430

aca gaa aat aac act cat gcc ttt gcc att att caa gga aga gag agc      1344
Thr Glu Asn Asn Thr His Ala Phe Ala Ile Ile Gln Gly Arg Glu Ser
        435                 440                 445

aca ttt gct act ctt att gtc aat gat tct gat gct ctg gcc acc cat      1392
Thr Phe Ala Thr Leu Ile Val Asn Asp Ser Asp Ala Leu Ala Thr His
    450                 455                 460

gag agt aag gaa ata gtc tgc act gta ttt gcc aca aac aag cat ctg      1440
Glu Ser Lys Glu Ile Val Cys Thr Val Phe Ala Thr Asn Lys His Leu
465                 470                 475                 480

aag gaa cca gtc agg aca cag atc att ata gtg tta gaa gga aca cct      1488
Lys Glu Pro Val Arg Thr Gln Ile Ile Ile Val Leu Glu Gly Thr Pro
                485                 490                 495

atg aaa gat gaa gat aat tgt ccc cat tca tgt tct gag agc agg caa     1536
Met Lys Asp Glu Asp Asn Cys Pro His Ser Cys Ser Glu Ser Arg Gln
            500                 505                 510

aag tct gaa tgt gag gag tgt ggg gga ctg ggc tcc ctt act gga agg     1584
Lys Ser Glu Cys Glu Glu Cys Gly Gly Leu Gly Ser Leu Thr Gly Arg
        515                 520                 525

tgc cag tgg aga cag ggt cat gga gaa gga atc aca aca aac tat tct     1632
Cys Gln Trp Arg Gln Gly His Gly Glu Gly Ile Thr Thr Asn Tyr Ser
    530                 535                 540

acc tgc acg cct agt ctt ctg aca tgc cct gat gga atc tgt gat gct     1680
Thr Cys Thr Pro Ser Leu Leu Thr Cys Pro Asp Gly Ile Cys Asp Ala
545                 550                 555                 560

gtg gag atg aat gat tca gca ata tgt cca cag gac tgc act gag ata     1728
Val Glu Met Asn Asp Ser Ala Ile Cys Pro Gln Asp Cys Thr Glu Ile
                565                 570                 575

act atc att ggt ggg cat gaa aaa gga cgt cta aaa ggg ata aaa gct     1776
Thr Ile Ile Gly Gly His Glu Lys Gly Arg Leu Lys Gly Ile Lys Ala
            580                 585                 590

gga tat ggc acc tgt ttt tgc ctt ctg cct gat aaa tgc ttc tgt gaa     1824
Gly Tyr Gly Thr Cys Phe Cys Leu Leu Pro Asp Lys Cys Phe Cys Glu
        595                 600                 605

aag aat att gtt gaa gga caa ctg tgt gat gac acc tgc aag act gtg     1872
Lys Asn Ile Val Glu Gly Gln Leu Cys Asp Asp Thr Cys Lys Thr Val
    610                 615                 620

att gca tgt ggg gtc ttt ctg tcc ttc ata atc tct gtt cta ctc tcc     1920
Ile Ala Cys Gly Val Phe Leu Ser Phe Ile Ile Ser Val Leu Leu Ser
625                 630                 635                 640

tca tac ttc att cat cga tac cat aaa aac tct cca aag ccc cca att     1968
Ser Tyr Phe Ile His Arg Tyr His Lys Asn Ser Pro Lys Pro Pro Ile
                645                 650                 655

gct tct gct gaa atg acc ttc cgc cgc cct gcc cag tct tat cca gtg     2016
Ala Ser Ala Glu Met Thr Phe Arg Arg Pro Ala Gln Ser Tyr Pro Val
            660                 665                 670

agt tac tct tca aac aat gcc aga cgc cca tct cta gac tcc atg gag     2064
Ser Tyr Ser Ser Asn Asn Ala Arg Arg Pro Ser Leu Asp Ser Met Glu
        675                 680                 685

aat cag atg tca gtg gat aca ttt aaa ata ccg gag gat cca aag tgg     2112
Asn Gln Met Ser Val Asp Thr Phe Lys Ile Pro Glu Asp Pro Lys Trp
    690                 695                 700

gag ttc cca cgt aaa aac ctg gtc ttg ggt aag acg ctt ggt gaa gga     2160
Glu Phe Pro Arg Lys Asn Leu Val Leu Gly Lys Thr Leu Gly Glu Gly
705                 710                 715                 720

gag ttt gga aaa gtt gta aag gca act gca ttt aga ctg aag ggt aaa     2208
Glu Phe Gly Lys Val Val Lys Ala Thr Ala Phe Arg Leu Lys Gly Lys
                725                 730                 735

gct ggc tac act aca gtg gca gta aaa atg ctg aaa gag agt gca tcc     2256
Ala Gly Tyr Thr Thr Val Ala Val Lys Met Leu Lys Glu Ser Ala Ser
            740                 745                 750

cag agt gag ctg cga gat ctt ctg tca gag ttc aat ctc ttg aaa cag     2304
Gln Ser Glu Leu Arg Asp Leu Leu Ser Glu Phe Asn Leu Leu Lys Gln
        755                 760                 765

gtc aat cat cct cat gtc atc aaa ctt tat ggg gct tgt aca cag gat     2352
Val Asn His Pro His Val Ile Lys Leu Tyr Gly Ala Cys Thr Gln Asp
    770                 775                 780

ggt cct ttg cat ctc att gta gaa tat tca aag tat gga tcc cta cgc     2400
Gly Pro Leu His Leu Ile Val Glu Tyr Ser Lys Tyr Gly Ser Leu Arg
785                 790                 795                 800

agt ttt cta agg gaa agt cgc aaa gta ggg cct agc act ttg gga gga     2448
Ser Phe Leu Arg Glu Ser Arg Lys Val Gly Pro Ser Thr Leu Gly Gly
                805                 810                 815

gat tca aat cgt aac tcc agc tac ctg gat aac cca gat gaa agg gca     2496
Asp Ser Asn Arg Asn Ser Ser Tyr Leu Asp Asn Pro Asp Glu Arg Ala
            820                 825                 830

ctt aca atg ggg gat cta att tca ttt gct tgg cag ata tcc agg gga     2544
Leu Thr Met Gly Asp Leu Ile Ser Phe Ala Trp Gln Ile Ser Arg Gly
        835                 840                 845

atg cag tat ctg gca gaa atg aag ctt gtc cat cga gac ttg gcg gcc     2592
Met Gln Tyr Leu Ala Glu Met Lys Leu Val His Arg Asp Leu Ala Ala
    850                 855                 860

cga aat gtg ttg gtc gca gaa gga cgc aaa atg aaa ata tca gat ttt     2640
Arg Asn Val Leu Val Ala Glu Gly Arg Lys Met Lys Ile Ser Asp Phe
865                 870                 875                 880

ggt ctt tcc aga gat gtt tat gaa gaa gat tcc tat gta aag aga agc     2688
Gly Leu Ser Arg Asp Val Tyr Glu Glu Asp Ser Tyr Val Lys Arg Ser
                885                 890                 895

aag ggt aga att cct gta aaa tgg atg gct ata gaa tcc ctg ttt gat     2736
Lys Gly Arg Ile Pro Val Lys Trp Met Ala Ile Glu Ser Leu Phe Asp

900                 905                 910

cac atc tat aca aca caa agt gac gtg tgg tcg ttt gga gtg ctc ctg     2784
His Ile Tyr Thr Thr Gln Ser Asp Val Trp Ser Phe Gly Val Leu Leu
        915                 920                 925

tgg gag att gtt acc ttg gga gga aat cca tat ccg ggt att gct ccg     2832
Trp Glu Ile Val Thr Leu Gly Gly Asn Pro Tyr Pro Gly Ile Ala Pro
    930                 935                 940

gag agg ctc ttt aat ctc cta aaa act gga tat aga atg gaa aaa cca     2880
Glu Arg Leu Phe Asn Leu Leu Lys Thr Gly Tyr Arg Met Glu Lys Pro
945                 950                 955                 960

gag aac tgc agt gat gaa atg tac aat ctt atg ctt aag tgc tgg aaa     2928
Glu Asn Cys Ser Asp Glu Met Tyr Asn Leu Met Leu Lys Cys Trp Lys
                965                 970                 975

caa gag cca gaa aag aga caa aca ttt ggc gaa atc agt aag gaa ctg     2976
Gln Glu Pro Glu Lys Arg Gln Thr Phe Gly Glu Ile Ser Lys Glu Leu
            980                 985                 990

gag aag atg atg gtg aaa agc agg  gat tat ttg gac ttg  gct gca tcc     3024
Glu Lys Met Met Val Lys Ser Arg  Asp Tyr Leu Asp Leu  Ala Ala Ser
        995                 1000                1005

act cct  gct gac tcc tta ctg  tat gac gat ggt ctt  tct gaa gag     3069
Thr Pro  Ala Asp Ser Leu Leu  Tyr Asp Asp Gly Leu  Ser Glu Glu
    1010                1015                1020

gag acc  cct ctg gtg gac tgc  aat aat gcc cca ctt  cct cga act     3114
Glu Thr  Pro Leu Val Asp Cys  Asn Asn Ala Pro Leu  Pro Arg Thr
    1025                1030                1035

ctc cct  tct acc tgg ata gaa  aac aaa ctc tat ggt  aga atc tcc     3159
Leu Pro  Ser Thr Trp Ile Glu  Asn Lys Leu Tyr Gly  Arg Ile Ser
    1040                1045                1050

cat gcg  ttt act aga ttc tag                                     3180
His Ala  Phe Thr Arg Phe
    1055

<210>  30
<211>  1059
<212>  PRT
<213>  Xenopus laevis

<400>  30

Met Ala Pro Ala Glu Trp Ala Leu Cys Phe Leu Cys Leu Phe Gln Val

1                   5                   10                  15

Thr Ser Gly Leu Tyr Phe Leu Arg Lys Asp Tyr Tyr Glu Asn Val Tyr
            20                  25                  30

Val Asp Gln Pro Ala Gly Thr Leu Leu Leu Gln Val His Val Met Lys
        35                  40                  45

Asp Ser Pro Ser Glu Lys Pro His Phe Arg Leu Cys Pro Thr Ile Asn
    50                  55                  60

Phe Ser Arg Asn Ser Phe Tyr His Trp Phe Arg Ile Asp Glu His Thr
65                  70                  75                  80

Gly Asn Leu Tyr Leu Asn Arg Ser Leu Asp Arg Ser Asp Tyr Glu Val
                85                  90                  95

Phe Ser Gln Gly Ser Gln Ser Ala Ile Gln Lys Ile Thr Leu Lys Val
            100                 105                 110

Ser Thr Lys Pro His Pro Phe Leu Asp Lys Asp Cys Asn Asn Ala Pro
        115                 120                 125

Leu Thr Trp Val His Leu Asn Phe Ile Asn Leu Thr Ser Ser Ile Cys
    130                 135                 140

Phe Ser Leu Lys Pro Lys Asp Leu Cys Phe Asn Asp Lys Asp Leu Ser
145                 150                 155                 160

Phe His Ile Lys Glu Asn Lys Pro Pro Gly Lys Phe Tyr Lys Phe His
                165                 170                 175

Pro Arg Pro Ile Val Gln Ser Cys Pro Asn Val Ser Val Ser Tyr Met
            180                 185                 190

Leu Ile Ser Asp His Ser Tyr Pro Phe Arg Phe Asn Pro Asn Thr Ser
        195                 200                 205

Glu Val Ser Leu Thr His Ser Val Asp Arg Glu Glu Arg Glu Asn Tyr
    210                 215                 220

Asp Leu Val Ala Lys Cys Leu Leu Arg Asp Ala Ser Ser Glu Val Glu
225                 230                 235                 240

Val Glu Gln Ser Phe Gln Ile Lys Val Asp Asp Glu Asp Asp Thr Ala
                245                 250                 255

Pro Phe Leu Pro Asn Gly Thr Asp Thr Ala His Val Val Val Asp Phe
            260                 265                 270

Lys Arg Lys Asn Gly Thr Val Leu Gly Met Leu Thr Val Trp Asp Ala
        275                 280                 285

Asp Ser Thr Pro Val Tyr Pro Ala Asp Val Ser Arg Lys Lys Tyr Thr
    290                 295                 300

Lys Thr Ile Val Ser Lys Asp Arg Phe Ile Leu Glu Asn Phe Arg Val
305                 310                 315                 320

Glu His Ser Phe Lys Glu Val Lys Phe Gln Pro Lys Gly Asn Leu Ile
                325                 330                 335

Arg Gly Thr Leu His Glu Tyr Arg Leu Val Leu Asn Lys Ser Ile Pro
            340                 345                 350

Ile Ile Arg Asn Gly Ser Leu Leu Val Ser Val Leu Val Asn Asp Thr
        355                 360                 365

Glu Phe His Gly Pro Asn Gly Asp Val Met Leu Tyr Phe Asn Val Thr
    370                 375                 380

Ile Leu Ser Met Glu Ile Arg Phe Pro Asn Ile Ser Tyr Gln Tyr Thr
385                 390                 395                 400

Val Asn Arg Asn Ala Asp Arg Tyr Ala Gln Ile Gly Lys Ile Cys Ile
                405                 410                 415

Glu Asn Cys Lys Leu Val Glu Gly Val Asn Ile Asn Tyr Arg Leu Gln
            420                 425                 430

Thr Glu Asn Asn Thr His Ala Phe Ala Ile Ile Gln Gly Arg Glu Ser
        435                 440                 445

Thr Phe Ala Thr Leu Ile Val Asn Asp Ser Asp Ala Leu Ala Thr His
    450                 455                 460

Glu Ser Lys Glu Ile Val Cys Thr Val Phe Ala Thr Asn Lys His Leu
465                 470                 475                 480

Lys Glu Pro Val Arg Thr Gln Ile Ile Ile Val Leu Glu Gly Thr Pro
                485                 490                 495

Met Lys Asp Glu Asp Asn Cys Pro His Ser Cys Ser Glu Ser Arg Gln
            500                 505                 510

Lys Ser Glu Cys Glu Glu Cys Gly Gly Leu Gly Ser Leu Thr Gly Arg
        515                 520                 525

Cys Gln Trp Arg Gln Gly His Gly Glu Gly Ile Thr Thr Asn Tyr Ser
    530                 535                 540

Thr Cys Thr Pro Ser Leu Leu Thr Cys Pro Asp Gly Ile Cys Asp Ala
545                 550                 555                 560

Val Glu Met Asn Asp Ser Ala Ile Cys Pro Gln Asp Cys Thr Glu Ile
                565                 570                 575

Thr Ile Ile Gly Gly His Glu Lys Gly Arg Leu Lys Gly Ile Lys Ala
            580                 585                 590

Gly Tyr Gly Thr Cys Phe Cys Leu Leu Pro Asp Lys Cys Phe Cys Glu
        595                 600                 605

Lys Asn Ile Val Glu Gly Gln Leu Cys Asp Asp Thr Cys Lys Thr Val
    610                 615                 620

Ile Ala Cys Gly Val Phe Leu Ser Phe Ile Ile Ser Val Leu Leu Ser
625                 630                 635                 640

Ser Tyr Phe Ile His Arg Tyr His Lys Asn Ser Pro Lys Pro Pro Ile
                645                 650                 655

Ala Ser Ala Glu Met Thr Phe Arg Arg Pro Ala Gln Ser Tyr Pro Val
            660                 665                 670

Ser Tyr Ser Ser Asn Asn Ala Arg Arg Pro Ser Leu Asp Ser Met Glu
        675                 680                 685

Asn Gln Met Ser Val Asp Thr Phe Lys Ile Pro Glu Asp Pro Lys Trp
    690                 695                 700

Glu Phe Pro Arg Lys Asn Leu Val Leu Gly Lys Thr Leu Gly Glu Gly
705                 710                 715                 720

Glu Phe Gly Lys Val Val Lys Ala Thr Ala Phe Arg Leu Lys Gly Lys
                725                 730                 735

Ala Gly Tyr Thr Thr Val Ala Val Lys Met Leu Lys Glu Ser Ala Ser
            740                 745                 750

Gln Ser Glu Leu Arg Asp Leu Leu Ser Glu Phe Asn Leu Leu Lys Gln
        755                 760                 765

Val Asn His Pro His Val Ile Lys Leu Tyr Gly Ala Cys Thr Gln Asp
    770                 775                 780

Gly Pro Leu His Leu Ile Val Glu Tyr Ser Lys Tyr Gly Ser Leu Arg
785                 790                 795                 800

Ser Phe Leu Arg Glu Ser Arg Lys Val Gly Pro Ser Thr Leu Gly Gly
                805                 810                 815

Asp Ser Asn Arg Asn Ser Ser Tyr Leu Asp Asn Pro Asp Glu Arg Ala

            820                 825                 830

Leu Thr Met Gly Asp Leu Ile Ser Phe Ala Trp Gln Ile Ser Arg Gly
        835                 840                 845

Met Gln Tyr Leu Ala Glu Met Lys Leu Val His Arg Asp Leu Ala Ala
    850                 855                 860

Arg Asn Val Leu Val Ala Glu Gly Arg Lys Met Lys Ile Ser Asp Phe
865                 870                 875                 880

Gly Leu Ser Arg Asp Val Tyr Glu Glu Asp Ser Tyr Val Lys Arg Ser
                885                 890                 895

Lys Gly Arg Ile Pro Val Lys Trp Met Ala Ile Glu Ser Leu Phe Asp
            900                 905                 910

His Ile Tyr Thr Thr Gln Ser Asp Val Trp Ser Phe Gly Val Leu Leu
        915                 920                 925

Trp Glu Ile Val Thr Leu Gly Gly Asn Pro Tyr Pro Gly Ile Ala Pro
    930                 935                 940

Glu Arg Leu Phe Asn Leu Leu Lys Thr Gly Tyr Arg Met Glu Lys Pro
945                 950                 955                 960

Glu Asn Cys Ser Asp Glu Met Tyr Asn Leu Met Leu Lys Cys Trp Lys
                965                 970                 975

Gln Glu Pro Glu Lys Arg Gln Thr Phe Gly Glu Ile Ser Lys Glu Leu
            980                 985                 990

Glu Lys Met Met Val Lys Ser Arg Asp Tyr Leu Asp Leu Ala Ala Ser
        995                 1000                1005

Thr Pro Ala Asp Ser Leu Leu Tyr Asp Asp Gly Leu Ser Glu Glu
    1010                1015                1020

Glu Thr Pro Leu Val Asp Cys Asn Asn Ala Pro Leu Pro Arg Thr
    1025                1030                1035

Leu Pro Ser Thr Trp Ile Glu Asn Lys Leu Tyr Gly Arg Ile Ser
    1040                1045                1050

His Ala Phe Thr Arg Phe
    1055

<210> 31
<211> 38
<212> DNA
<213> Artificial

<220>
<223> Synthetic DNA

<400> 31
gaggtcgacg ccaccatggc gaaagcgagg tccggcgc 38

<210> 32
<211> 23
<212> DNA
<213> Artificial

<220>
<223> Synthetic DNA

<400> 32
acggagacag tcctgggggc aaa 23

<210> 33
<211> 23
<212> DNA
<213> Artificial

<220>
<223> Synthetic DNA

<400> 33
tctcctagca ccaggacctg tcc 23

<210> 34
<211> 35
<212> DNA

<213>  Artificial

<220>
<223>  Synthetic DNA

<400>  34
gaggcggccg cgctatcaaa tgtgtccatt aattt                                35

## Description

### TECHNICAL FIELD

The present invention relates to a screening method using c-Ret, which is a novel substrate for γ-secretase, and a kit for use in the method.

### BACKGROUND ART

γ-Secretase is a complex protein (aspartate protease) comprising presenilin, nicastrin, Aph-1 and Pen-2 as basic components. Presenilin is the catalytic domain; presenilin gene has been identified as a causative gene for familial Alzheimer's disease (AD). γ-Secretase acts on single-pass transmembrane proteins as its substrates. As most representative substrates thereof, amyloid precursor protein (APP) and Notch are known. As a substance which specifically inhibits γ-secretase (γ-secretase inhibitor), (2S)-2-{[(3,5-Difluorophenyl)acetyl]amino}-N-[(3S)-1-methyl-2-oxo-5-phenyl-2,3-dihydro-1H-1,4-benzodiazepin-3-yl]propanamide is known (Non-Patent Documents Nos. 1 and 2). It is also known that APP produces amyloid ß protein (Aß) when cleaved by ß-secretase at ß-site and by γ-secretase at γ-site (Non-Patent Document No. 3). The thus-produced Aß is classified into peptides with different lengths depending on the cleavage site in the amino acid sequence (C-terminal side). Of these peptides, Aß42 which is strongly hydrophobic and ready to aggregate (ready to take the ß-sheet structure) exhibits neurotoxicity. It has been considered that this phenomenon may be the major cause of Alzheimer's disease. Recently, however, a report has been made that presenilin 1 (PS1) and presenilin 2 (PS2) double-knockout mice capable of producing no Aß show AD-like phenotypes such as decrease of synapses and neuronal death; this suggests existence of a pathogenic mechanism of AD independent from APP (Non-Patent Document No. 4).

On the other hand, c-Ret is a member of the receptor tyrosine kinase family and is also known as one of GDNF (Glial-cell-line-derived neurotrophic factor) receptor subunits. It is known that GDNF signaling through c-Ret is regulating cell differentiation and growth of cells; in particular, it has been revealed that this signaling shows protective effect on neurons in the central nervous system (Non-Patent Document No. 5).

However, it has never been reported to date that c-Ret is a substrate for γ-secretase. [Non-Patent Document No. 1] Camilla A. Hansson, Susanne Frykman, Mark R. Farmery, Lars O. Tjernberg, Camilla Nilsberth, Sharon E. Pursglove, Akira Ito, Bengt Winblad, Richard F. Cowburn, Johan Thyberg, and Maria Ankarcrona Nicastrin, presenilin, APH-1, and PEN-2 form active γ-secretase complexes in mitochondria. J. Biol. Chem., Vol. 279, Issue 49, 51654-51660, December 3, 2004
[Non-Patent Document No. 2] Zhiying Zou, Brian Chung, Thao Nguyen, Sueann Mentone, Brent Thomson, and Daniel Biemesderfer Linking Receptor-mediated Endocytosis and Cell Signaling. J. Biol. Chem., Vol. 279, Issue 33, 34302-34310, August 13, 2004
[Non-Patent Document No. 3] Amtul Z., Lewis P.A., Piper S., Crook R., Baker M., Findlay K., Singleton A., Hogg M., Younkin L., Younkin S.G, Hardy J., Hutton M., Boeve B.F., Tang-Wai D., and Golde T.E., A Presenilin 1 Mutation Associated with Familial Frontotemporal Dementia Inhibits γ-Secretase Cleavage of APP and Notch Neurobiology of disease. 2002 Mar 9; 2:269-273
[Non-Patent Document No. 4] Saura CA, Choi SY, Beglopoulos V, Malkani S, Zhang D, Shankaranarayana Rao BS, Chattarji S, Kelleher RJ 3rd, Kandel ER, Duff K, Kirkwood A, and Shen J. Loss of presenilin function causes impairments of memory and synaptic plasticity followed by age-dependent neurodegeneration. Neuron. 2004 Apr 8;42(1):23-36
[Non-Patent Document No. 5] Sariola H, and Saarma M. Novel functions and signalling pathways for GDNF. J Cell Sci. 2003 Oct 1; 116:3855-62

### DISCLOSURE OF THE INVENTION

### PROBLEM FOR SOLUTION BY THE INVENTION

It is an object of the present invention to provide a screening method using c-Ret, a novel substrate for γ-secretase, in particular a method of screening for compounds which affect the processing of c-Ret by γ-secretase.

### MEANS TO SOLVE THE PROBLEM

The present inventors have proved for the first time that c-Ret is cleaved by γ-secretase in HEK293 cells by using a γ-secretase inhibitor (2S)-2-{[(3,5-difluorophenyl)acetyl]amino}-N-[(3S)-1-methyl-2-oxo-5-phenyl-2,3-dihydro-1H-1,4-benzodiazepin-3-yl]propanamide. The present inventors have also found for the first time that it is possible to detect the cleavage of c-Ret by using an antibody specific to a hemagglutinin tag added at the C-terminus of c-Ret.

Briefly, the present inventors have demonstrated that a screening method with c-Ret utilizing the c-Ret degradation activity of γ-secretase (in particular, cleavage accelerating activity or cleavage inhibiting activity) is effective by showing that the cleavage of c-Ret is inhibited by γ-secretase inhibitor.

An accelerator for the c-Ret degradation activity of γ-secretase obtainable by the screening method of the present invention is a compound which accelerates the processing of c-Ret through γ-secretase. An inhibitor for the c-Ret degradation activity of γ-secretase obtainable by the screening method of the present invention is a compound which reduces the processing of c-Ret through γ-secretase. According to the present invention, it has become possible to develop therapeutics for memory disorders of interest (preferably AD) by selecting those compounds which act on γ-secretase selectively.

The present invention provides the following embodiments.

In one embodiment, the present invention relates to a method of screening for compounds which affect the processing of c-Ret by γ-secretase. More specifically, the method comprises the following steps: (a) a step of detecting the cleavage of c-Ret by γ-secretase, wherein a first biological composition containing γ-secretase or a biologically active fragment thereof is contacted with a second biological composition containing c-Ret to thereby measure the cleavage of the c-Ret; and (b) a step of evaluating whether or not candidate compounds affect the cleavage of c-Ret by γ-secretase, wherein those candidate compounds which affect the cleavage of the c-Ret by γ-secretase are selected and the thus selected compounds are identified as compounds which affect the processing of c-Ret by γ-secretase.

In another embodiment, the present invention relates to a screening method further comprising, in addition to the above-described steps, a step of evaluating a candidate compound as a compound which inhibits the processing of c-Ret through γ-secretase or an inhibitor for the c-Ret degradation activity of γ-secretase, when c-Ret undegraded product in the presence of the candidate compound was increased relative to c-Ret undegraded product in the absence of the candidate compound.

In still another embodiment, the present invention relates to a screening method further comprising, in addition to the above-described steps, a step of evaluating a candidate compound as a compound which accelerates the processing of c-Ret through γ-secretase or an accelerator for the c-Ret degradation activity of γ-secretase, when c-Ret undegraded product in the presence of the candidate compound was decreased relative to c-Ret undegraded product in the absence of the candidate compound.

In still another embodiment, the present invention relates to a screening method further comprising, in addition to the above-described steps, a step of evaluating a candidate compound as a compound which accelerates the processing of c-Ret through γ-secretase or an accelerator for the c-Ret degradation activity of γ-secretase, when c-Ret cleavage product in the presence of the candidate compound was increased relative to c-Ret cleavage product in the absence of the candidate compound.

In still another embodiment, the present invention relates to a screening method further comprising, in addition to the above-described steps, a step of evaluating a candidate compound as a compound which inhibits the processing of c-Ret through γ-secretase or an inhibitor for the c-Ret degradation activity of γ-secretase, when c-Ret cleavage product in the presence of the candidate compound was decreased relative to c-Ret cleavage product in the absence of the candidate compound.

In still another embodiment, the present invention relates to a screening method further comprising, in addition to the above-described method, a step of measuring the cleavage of APP or a polypeptide comprising a γ-secretase cleavage site of APP (hereinafter, expressed as "polypeptide comprising an APP γ-secretase cleavage site").

In still another embodiment, the present invention relates to a screening method further comprising, in addition to the above-described method, a step of measuring the cleavage of Notch or a polypeptide comprising a γ-secretase cleavage site of Notch (hereinafter, expressed as "polypeptide comprising a Notch γ-secretase cleavage site").

In still another embodiment, the present invention provides a pharmaceutical composition comprising at least one compound identified by the screening method of the present invention and a pharmacologically acceptable carrier. Preferably, the above compound is (2S)-2-{[(3,5-difluorophenyl)acetyl]amino}-N-[(3S)-1-methyl-2-oxo-5-phenyl-2,3-dihydro-1H-1,4-benzodiazepin-3-yl]propanamide (hereinafter, sometimes referred to as "Compound E").

In still another embodiment, the present invention provides a method of treating a disease or condition characterized by abnormality in the processing of c-Ret by γ-secretase, comprising a step of administering to a patient in need of treatment (e.g., a patient with a memory disorder, especially a condition of dementia (preferably AD)) an effective dose of the compound of the present invention or a pharmaceutical composition comprising the same (preferably a therapeutic for dementia), wherein preferably the dose is effective for altering the c-Ret processing activity of γ-secretase in the cells of the patient.

In still another embodiment, the present invention provides a test kit for measuring the processing of c-Ret by γ-secretase, which is applicable to the method of the present invention, comprising c-Ret and preferably further comprising a substrate for γ-secretase other than c-Ret (preferably APP and/or Notch).

In still another embodiment, the present invention provides a test kit for measuring the processing of c-Ret by γ-secretase, comprising a first biological composition containing γ-secretase or a biologically active fragment thereof and a second biological composition containing c-Ret.

### EFFECT OF THE INVENTION

According to the present invention, there is provided a method for screening for compounds which affect the processing of c-Ret by γ-secretase. The compound screened by the present invention is useful as a therapeutic for a memory disorder of interest, especially dementia (preferably AD).

### BRIEF DESCRIPTION OF THE DRAWINGS

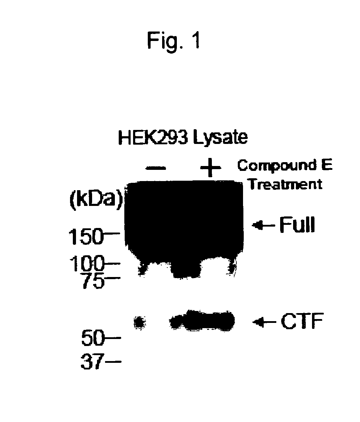

Fig. 1 is a diagram showing the results of analysis of c-Ret processing using *c-Ret*-transfected 293/EBNA-1 cell strain.

### BEST MODE FOR CARRYING OUT THE INVENTION

Hereinbelow, the present invention will be described in more detail. The present specification encompasses the contents disclosed in the specification and the drawings of US Provisional Patent Application No. 60/953,230 (filed on August 1, 2007) based on which the present patent application claims priority.

The term "patient" used herein refers to an animal, preferably a mammal.

The term "mammal" used herein means any animal classified as mammal, including human and non-human mammals (such as mouse, rat, hamster, guinea pig, rabbit, pig, dog, horse, cattle, monkey, etc.). Preferably, the mammal in the present specification is human. When the mammal is human, the term "patient" include adults and children, and also male and female. Children include newborn infants, infants and adolescents.

The term "γ-secretase" used herein means an enzyme or a complex composed of a plurality of molecules, each of which is in charge of the production of Aβ by cleaving (degrading) APP within its transmembrane domain. The plurality of molecules comprise at least one molecule selected from presenilin, nicastrin, Aph-1 and Pen-2. Examples of the γ-secretase of the present invention include mouse Presenilin 1(NM_008943), rat Presenilin1 (D82363), human Presenilin1 (NM_000021), mouse Presenilin2 (NM_011183), rat Presenilin2 (NM_031087), human Presenilin2 (NM_000447), mouse Nicastrin (NM_021607), rat Nicastrin (NM_174864), human Nicastrin (NM_015331), mouse Aph-1 (NM_146104), rat Aph-1 (NM_001014255), human Aph-1 (NM_016022), mouse Pen-2 (NM_025498), rat Pen-2 (NM_001008764) and human Pen-2 (NM_172341). Each component molecule of the γ-secretase of the present invention may be a full-length molecule or a part thereof, as long as that γ-secretase has an enzyme activity equivalent to that of γ-secretase functioning *in vivo.* Further, the γ-secretase of the present invention may be a mutant γ-secretase. The mutant γ-secretase is a polypeptide composed of full-length component molecules which may have deletion, substitution, insertion and/or addition of one or more (preferable one or several) amino acids, or a polypeptide having an amino acid sequence comprising a combination of such mutations, each of the above polypeptide having an enzyme activity equivalent to that of γ-secretase functioning *in vivo.*

The term "biologically active fragment of γ-secretase" means a fragment having an enzyme activity equivalent to that of γ-secretase functioning *in vivo.* Examples of such fragments include fragments capable of cleaving APP or c-Ret.

It should be noted that sometimes, the term "γ-secretase" is intended to include the "biologically active fragment of γ-secretase" in the present specification.

The term "c-Ret" used herein refers to a polypeptide which is a member of the receptor tyrosine kinase family and is also known as one of GDNF (Glial-cell-line-derived neurotrophic factor) receptor subunits (Sariola H, and Saarma M., Novel functions and signalling pathways for GDNF, J Cell Sci. 2003 Oct 1; 116:3855-62).

The c-Ret used in the present invention is preferably a c-Ret derived from a mammal. For example, human c-Ret (NM_020975, X12949, M57464, NM_020630, BC004257), rat c-Ret (NM_012643, AJ299017), mouse c-Ret (NM_009050, X67812, NM_001080780, AK044686), dog (Canis lupus familiaris) c-Ret (XM_543915), chicken (Gallus gallus) c-Ret (NM_205190), platypus (Ornithorhynchus anatinus) c-Ret (XM_001507665), African clawed frog (Xenopus laevis) c-Ret (NM_001085623) and the like are included in the c-Ret of the present invention.

Genes encoding the above-listed c-Rets and the corresponding c-Ret polypeptides are included in the c-Ret of the present invention. Table 1 below shows correspondence between various animal-derived c-Ret polypeptides and their nucleotide and amino acid sequences.

**Table 1**

| Source or Type | Accession No. | Nucleotide Sequence | Amino Acid Sequence |
|---|---|---|---|
| Human (Homo sapiens) | NM_020975 | SEQ ID NO: 1 | SEQ ID NO: 2 |
| | X12949 or M57464 | SEQ ID NO: 3 | SEQ ID NO: 4 |
| | NM_020630 | SEQ ID NO: 5 | SEQ ID NO: 6 |
| | BC004257 | SEQ ID NO: 7 | SEQ ID NO: 8 |
| Rat (Rattus norvegicus) | NM_012643 | SEQ ID NO: 9 | SEQ ID NO: 10 |
| | AJ299017 | SEQ ID NO: 11 | SEQ ID NO: 12 |
| Rat-HA (Rattus norvegicus) | | SEQ ID NO: 13 | SEQ ID NO: 14 |
| Mouse (Mus musculus) | NM_009050 | SEQ ID NO: 15 | SEQ ID NO: 16 |
| | X67812 | SEQ ID NO: 17 | SEQ ID NO: 18 |
| | NM_001080780 | SEQ ID NO: 19 | SEQ ID NO: 20 |
| | AK044686 | SEQ ID NO: 21 | SEQ ID NO: 22 |
| Dog (Canis lupus familiaris) | XM_543915 | SEQ ID NO: 23 | SEQ ID NO: 24 |
| Chicken (Gallus gallus) | NM_205190 | SEQ ID NO: 25 | SEQ ID NO: 26 |
| Platypus (Ornithorhynchus anatinus) | XM_001507665 | SEQ ID NO: 27 | SEQ ID NO: 28 |
| African clawed frog (Xenopus laevis) | NM_001085623 | SEQ ID NO: 29 | SEQ ID NO: 30 |

In the present invention, c-Ret structurally comprises a cadherin domain, a transmembrane domain and a kinase activity site (Sariola H et al., J Cell Sci. 2003 Oct 1; 116:3855-62). The ligand of c-Ret is GDNF (Sariola H et al., J Cell Sci. 2003 Oct 1; 116:3855-62). The c-Ret of the present invention may be either a full-length polypeptide or a partial sequence thereof, as long as it comprises the γ-secretase cleavage site of c-Ret.

Further, in the present invention, c-Ret may be a mutant c-Ret. The mutant c-Ret is a full-length c-Ret polypeptide which may have deletion, substitution, insertion and/or addition of one or more (preferable one or several) amino acids, or a c-Ret polypeptide having an amino acid sequence comprising a combination of such mutations, each of the above polypeptides being functionally substantially identical with c-Ret.

The "polypeptide which is functionally substantially identical with c-Ret" means a polypeptide having an activity of c-Ret, for example, a polypeptide which has a cleavage activity in a γ-secretase-dependent manner, and includes any and all c-Ret derivatives comprising at least the γ-secretase cleavage site of c-Ret. These polypeptides are especially useful in detecting and purifying c-Ret.

The term "substitution" used herein means preferably conservative substitution in which one or more (preferably one or several) amino acid residues are substituted with other chemically similar amino acid residues so that the activity of the peptide is not substantially modified. Examples of conservative substitution include substitution of a hydrophobic residue with other hydrophobic residue and substitution of a polar residue with other polar residue with the same electric charge. Functionally similar amino acids which allow such substitution are known to those skilled in the art for each amino acid. Specifically, examples of non-polar (hydrophobic) amino acids include alanine, valine, isoleucine, leucine, proline, tryptophan, phenylalanine and methionine; examples of polar (neutral) amino acids include glycine, serine, threonine, tyrosine, glutamine, asparagine and cystein. Examples of positively charged (basic) amino acids include arginine, histidine and lysine. Examples of negatively charged (acidic) amino acids include aspartic acid and glutamic acid.

The number of amino acids which may be deleted, substituted, inserted and/or added as described above is, for example, 1 to 30, preferably 1 to 20, more preferably 1 to 10, still more preferably 1 to 5, particularly preferably 1 to 2.

The above-described identify means that the amino acid sequence of a polypeptide may not be identical with the amino acid sequence of c-Ret as long as the polypeptide has substantially the same function as that of c-Ret. Therefore, when a polypeptide has an activity functionally substantially the same as that of c-Ret (e.g., γ-secretase-dependent degradation activity), the degree of homology of the amino acid sequence of the relevant polypeptide is disregarded. The animal species from which the polypeptide is derived is also disregarded.

In a preferred embodiment of the present invention, a polypeptide functionally substantially identical to c-Ret is an amino acid sequence which has preferably 80% or more,
more preferably 85% or more, still more preferably 90% or more, still more preferably 95% or more, particularly preferably 98% or more, most preferably 99% or more homology to the amino acid sequence of c-Ret (e.g., the amino acid sequence of a polypeptide consisting of SEQ ID NO: 10 (rat c-Ret)) and has substantially the same activity as that of c-Ret. When a polypeptide has these features, the polypeptide may be any of a polypeptide derived from the above-listed animals, a recombinant polypeptide or a synthetic polypeptide.

The identity described above may be values calculated by homology search programs known to those skilled in the art. For example, identity can be calculated by using default parameters in the homology algorithm BLAST (Basic local alignment search tool; http://www.ncbi.nlm.nih.gov/BLAST/) of The National Center for Biotechnology Information (NCBI).

The c-Ret may take any of the following forms: a fusion polypeptide fused to other polypeptide, a tagged or labeled polypeptide or a polypeptide otherwise modified. These polypeptides may be obtained by recombinant DNA techniques, site-directed mutagenesis, treatment with mutagenic agents (such as hydroxylamine), or automated peptide synthesis.

Examples of particularly useful systems as tagged c-Ret polypeptides include hemagglutinin (HA) system, glutathione-S-transferase (GST) system, maltose-binding protein system, 6x histidine system, 8x histidine system and the like.

Examples of modification incorporating the above-mentioned label or other detectable moieties include biotin label, radioactive label, fluorescence label, chemiluminescence label and the like. The c-Ret of the present invention may be labeled with one, two or more of these labels.

In a preferred embodiment of the present invention, c-Ret is a rat c-Ret, for example, a polypeptide comprising the amino acid sequence as shown in SEQ ID NO: 10 or SEQ ID NO: 12. In a still preferred embodiment of the present invention, c-Ret is a rat c-Ret to which an HA tag is added. For example, a rat c-Ret polypeptide with an HA tag added at its C-terminus (SEQ ID NO: 14) may be used (Example 1). It is for granted that polypeptides comprising the entire human c-Ret amino acid sequence or a part thereof (e.g., polypeptides comprising the amino acid sequence as shown in SEQ ID NO: 2, 4, 6 or 8) or c-Ret polypeptide sequences derived from other animals listed in Table 1 may also be used in the same manner as rat c-Ret.

The present invention further provides a polynucleotide comprising a nucleotide sequence encoding the above-described c-Ret. The polynucleotide encoding the c-Ret of the present invention is a polynucleotide encoding a polypeptide having substantially the same activity as that of c-Ret (e.g., γ-secretase-dependent degradation activity). In a preferred embodiment of the present invention, the polynucleotide encoding the c-Ret of the present invention is a polynucleotide encoding a rat c-Ret, e.g., the polynucleotide as shown in SEQ ID NO: 9 or SEQ ID NO: 11. Further, in a preferred embodiment of the present invention, the polynucleotide encoding the c-Ret of the present invention is a polynucleotide encoding a rat c-Ret polypeptide with an HA tag added. For example, a polynucleotide encoding a rat c-Ret polypeptide to which an HA tag is added at its C-terminus (SEQ ID NO: 13) may be given (Example 1). It is for granted that polynucleotides comprising the entire human c-Ret nucleotide sequence or a part thereof (e.g., the nucleotide sequence as shown in SEQ ID NO: 1, 3, 5 or 7) or nucleotide sequences derived from other animals listed in Table 1 may also be used in the same manner as rat c-Ret polynucleotides.

The polynucleotide encoding the c-Ret of the present invention may be a polynucleotide encoding the above-described c-Ret mutant or c-Ret derivative. For example, a polynucleotide which comprises a nucleotide sequence having 80% or more, preferably 85% or more, still more preferably 90% or more, yet still more preferably 95% or more, particularly preferably 98% or more, most preferably 99% or more homology (identity) with the nucleotide sequence as shown in SEQ ID NO: 9 or SEQ ID NO: 11 and encodes a polypeptide having substantially the same activity as that of c-Ret may be included.

Further, the polynucleotide encoding the c-Ret of the present invention includes a polynucleotide which hybridizes to a polynucleotide consisting of a nucleotide sequence complementary to the nucleotide sequence as shown in SEQ ID NO: SEQ ID NO: 9 or SEQ ID NO: 11 under stringent conditions and yet encodes a polypeptide having substantially the same activity as that of c-Ret. The stringent conditions refer to, for example, "2 x SSC, 0.1% SDS, 42°C" or "1 x SSC, 0.1% SDS, 37°C" as washing conditions after hybridization. As more stringent conditions, "1 x SSC, 0.1% SDS, 65°C" or "0.5 x SSC, 0.1% SDS, 50°C" may be given, for example. More specifically, a method using Rapid-hyb buffer (Amersham Life Science) may be employed in which pre-hybridization is performed at 68 °C for more than 30 minutes; then, with addition of a probe, a hybrid is formed while keeping the reaction solution at 68 °C for more than 1 hour, followed by washing in 2 x SSC, 0.1% SDS at room temperature for 20 minutes 3 times, washing in 1 x SSC, 0.1% SDS at 37°C for 20 minutes 3 times and finally washing in 1 x SSC, 0.1% SDS at 50°C for 20 minutes twice. Alternatively, other method may be employed in which pre-hybridization is performed in Expresshyb Hybridization Solution (CLONTECH) at 55 °C for more than 30 minutes; then, with addition of a labeled probe, the reaction solution is incubated at 37-55°C for more than 1 hour, followed by washing in 2 x SSC, 0.1% SDS at room temperature for 20 minutes 3 times, washing in 1 x SSC, 0.1% SDS at 37°C for 20 minutes once. In these methods, more stringent conditions may be achieved, for example, by raising the temperature of prehybridization, hybridization or the second washing. For example, the temperatures of prehybridization and hybridization may be raised to 60°C, respectively; for more stringent conditions, the temperatures may be raised to 68°C, respectively. Those skilled in the art could appropriately select conditions for obtaining c-Ret isoforms, allelic mutants, and corresponding genes derived from other organisms, by taking into account various conditions such as probe concentration, probe length, reaction time, etc. in addition to the above-described salt concentrations and reaction temperatures.

Such polynucleotides may be obtained by gene amplification techniques, hybridization techniques, recombinant DNA techniques, and the like.

The term "biological composition" used herein means a composition comprising γ-secretase or a biologically active fragment thereof, or c-Ret, and is not particularly limited. For example, the biological composition may be a cell-free reconstruction system, a mammal or a part thereof, or a transgenic non-human mammal so engineered to overexpress APP or a part of this transgenic mammal.

In the expressions "first biological composition containing γ-secretase or a biologically active fragment thereof" and "second biological composition containing c-Ret" used herein, the γ-secretase and/or c-Ret may be either endogenous or exogenous.

When the γ-secretase or c-Ret is endogenous, the composition may be any composition as long as it contains γ-secretase or c-Ret derived from the above-mentioned animal or a part thereof.

The term "a part of the above-mentioned animal" include tissues, cells, cell lysates, cell membrane fractions or purified membranes of the above-mentioned animal. As examples of the cells, cells in the central nervous system; neurons such as brain-derived neurons, cerebral cortex-derived neurons, cerebral cortex-derived primarily cultured neurons, or hippocampus-derived primarily cultured neurons; or glial cells may be enumerated. The γ-secretase or c-Ret may be in the state of being contained in a mammal or a part thereof. Alternatively, the γ-secretase or c-Ret may be a γ-secretase fraction or a c-Ret fraction of cell lysate prepared from a mammal. The cell lysate may be obtained by subjecting γ-secretase-or c-Ret-containing cells to lysis with a hypotonic solution or surfactant, or to sonication or other physical disruption. Optionally, the cell lysate may be purified with some purification means such as columns.

When the γ-secretase or c-Ret is exogenous, the biological composition may be γ-secretase expressing cells or c-Ret expressing cells prepared by allowing host cells to express the whole or a part of the sequences in expression vectors comprising a polynucleotide encoding the individual molecules constituting γ-secretase or a polynucleotide encoding c-Ret. Alternatively, the biological composition may be the γ-secretase fraction of a cell lysate derived from γ-secretase expressing cells, or the c-Ret fraction or cell membrane fraction of a cell lysate derived from c-Ret expressing cells. The cell lysate may be obtained by subjecting γ-secretase- or c-Ret-containing cells to lysis with a hypotonic solution or surfactant, or to sonication or physical disruption. Optionally, the cell lysate may be purified with some purification means such as columns. The expression vector may be a vector which is transformed or transfected into a host cell and temporarily expresses the gene of interest. Alternatively, the expression vector may be a vector which is integrated into the genome of a host cell and expresses the gene of interest stably.

The term "transformation" or "transfection" used herein means any and all methods which change DNA contents in eukaryotic cells or microorganisms. These methods include calcium phosphate transfection, protoplast fusion transfection, electroporation transfection, DEAE-dextran transfection, liposome transfection, polybrene transfection and direct microinjection transfection (Sambrook, et al., Molecular Cloning 3: 16.30-16.31 (1989)).

The host cell into which the above-described expression vector is to be transformed or transfected may be any cell (or cell line) or microorganism capable of expressing the gene of interest. Known cultured cells or microorganisms may be used as host cells. Examples of mammal cells or cell lines which may be used as host cells include HEK 293 cells, Chinese hamster ovary (CHO) cells, fibroblast cells, primary endothelial cells (HUVEC cells), human glioma cells, HeLa cells, COS cells, PC12 cells, lymphoblast cells, melanoma cells, hybridoma cells, oocytes and embryonic stem cells. Examples of known microorganisms which may be used as host cells include *Escherichia coli* and yeast. Further, insect cells such as BmN4 cells may also be used.

The expression vector used in the above-described transformation or transfection is not particularly limited, as long as the vector comprises a polynucleotide encoding the individual molecules constituting γ-secretase or a polynucleotide encoding c-Ret. Such an expression vector may be a plasmid obtainable by introducing the polynucleotide into a known expression vector selected appropriately depending on the host cell to be used.

Examples of the above-mentioned expression vector include pUC, pTV, pGEX, pKK or pTrcHis for *E. coli;* pEMBLY or pYES2 for yeast; pcDNA3, pMAMneo and pBabe Puro for CHO cells, HEK293 cells or COS cells; and a vector comprising the polyhedrin promoter of *Bombyx mori* nuclear polyhedrosis virus (BmNPV) (such as pBK283) for BmN4 cells.

In mammal cells, examples of promoters which give a strong transcription activity include CMV immediate early promoter, retrovirus promoters (e.g., LTR from MLV or MMTV), promoters of SV40, RSV LTR, HTv-1 LTR and HIV-2 LTR, adenovirus promoters (e.g., those from E1A region, E2A region or MLP region), and promoters of AAV LTR, cauliflower mosaic virus, HSV-TK and avian sarcoma virus.

The above-described transformed or transfected host cell is not particularly limited, as long as the host cell comprises a polynucleotide encoding the individual molecules constituting γ-secretase or a polynucleotide encoding c-Ret. For example, the transformed cell may be a transformant in which the polynucleotide has been integrated into the chromosome thereof. Alternatively, the transformed cell may be a transformant comprising the polynucleotide in the form of a plasmid. It is also possible that the transformed cell is a transformant which is not expressing γ-secretase or c-Ret. These transformants may be obtained by transforming a desired host cell with the above-mentioned plasmid or the above-described polynucleotide *per se.*

Cells containing the above-described γ-secretase and/or c-Ret are not particularly limited as long as the cell is capable of expressing γ-secretase and/or c-Ret on the surface of its cell membrane. As examples of such cells, a cell expressing endogenous γ-secretase and endogenous c-Ret, a cell expressing γ-secretase and c-Ret one of which is endogenous and the other is exogenous or a cell expressing exogenous γ-secretase and exogenous c-Ret may be given. Such cells may also be obtained by culturing under conditions which allow expression of γ-secretase and/or c-Ret. Alternatively, such cells may be obtained by injecting into an appropriate cell an RNA encoding the individual molecules constituting γ-secretase and/or an RNA encoding c-Ret and culturing the resultant cell under conditions which allow expression of γ-secretase and/or c-Ret.

The above-described cell membrane fraction may be obtained, for example, by disrupting cells expressing the γ-secretase or c-Ret of the present invention and isolating cell membrane-rich fractions. As methods for disrupting cells, homogenizing in a homogenizer; disrupting in a Waring blender or Polytron; disrupting by sonication; ejecting cells from a thin nozzle while applying pressure with a French press; and so on may be used.
As methods for fractionating cell membrane, fractionation methods with centrifugal force such as differential centrifugation or density gradient centrifugation may be used.

For purification, a known method for protein purification may be used. The method comprises a step of crudely fractionating cells into polypeptide fractions and non-polypeptide fractions. After the γ-secretase or c-Ret of the present invention has been isolated from other polypeptides with a column or the like, the desired γ-secretase or c-Ret is further purified by chromatography or electrophoresis to thereby achieve partial purification or complete purification (or homogeneity by purification). Examples of analysis methods particularly suitable for preparation/purification of pure peptides include precipitation using ammonium sulfate, PEG, antibodies, etc.; centrifugation after thermal denaturation; chromatography step; isoelectric focusing; gel electrophoresis; SDS (sodium dodecyl sulfate)-polyacrylamide electrophoresis (SDS-PAGE); and a combination of these methods and other method. Examples of chromatography step includes ion exchange chromatography, gel filtration chromatography, reversed-phase chromatography, hydroxyapatite chromatography, affinity chromatography, fast protein liquid chromatography (FPLC), high performance liquid chromatography (HPLC) and immobilized metal ion affinity chromatography (IMAC).

Alternatively, γ-secretase or c-Ret may be tagged in advance; then, a crude polypeptide may be applied to a purification column to which a protein that recognizes the tag has been bound; the desired γ-secretase or c-Ret adsorbed onto the column may be desorbed from the column by feeding an appropriate solvent thereinto.

Various purification steps may be performed in a different order, or some of the steps may be omitted. A preferable method for evaluating the purity of a fraction is a method in which the specific activity of the fraction is calculated and compared with the specific activity of the first extract, followed by calculation of the magnitude of purity for evaluation.

The term "cleavage of c-Ret" used herein refers to an event in which c-Ret, cut by γ-secretase, produces a fragment shorter than the initial c-Ret before cutting. The term "c-Ret undegraded product" used herein refers to a polypeptide which is produced as a result of non-cleavage of c-Ret; this polypeptide means the c-Ret polypeptide before degradation by γ-secretase.

For monitoring the cleavage of c-Ret by γ-secretase, any of the following antibodies may be used: an anti-c-Ret antibody; an antibody which recognizes c-Ret undegraded product produced as a result of non-cleavage of c-Ret; or an antibody which recognizes c-Ret cleaved product produced as a result of cleavage of c-Ret, preferably an antibody which recognizes the intracellular domain of c-Ret, more preferably an antibody which recognizes the C-terminal domain of c-Ret. When the undegraded product or cleaved product of a tagged c-Ret polypeptide is to be detected, an antibody which recognizes the selected tag may be used. For example, when an HA tag has been added to the C-terminus of c-Ret, it is possible to detect the undegradation or cleavage of c-Ret using an anti-HA tag antibody. In this case, the antibody is capable of clarifying the presence and concentration of a C-terminal fragment of c-Ret that is produced as a result of non-cleavage of c-Ret or the presence and concentration of a C-terminal fragment of c-Ret that is produced as a result of cleavage of c-Ret.

The term "APP" used herein β-amyloid precursor protein (βAPP) or a mutant thereof. APP is a single-pass transmembrane protein comprising an Aβ domain in its C-terminal region, expressed in a large variety of cells in many mammals. In human, APP is encoded by the gene *APP* located in the long arm of chromosome No. 21 and has three major isotypes (APP695, APP751 and APP770). APP695, APP751 and APP770 consist of 695, 751 and 770 amino acid residues, respectively. Examples of APP proteins include human APP695 (NM_201414, NP_958817 and P05067-4), human APP751 (NM_201413, NP_958816 and P05067-8), human APP770 (NM_000484, NP_000475, P05067-1 and P05067), mouse APP695 (NM_007471, NP_031497 and P12023-2), mouse APP751 (P12023-3), mouse APP770 (AY267348, AAP23169, P12023-1 and P12023), rat APP695 (P08592-2), rat APP751 (P08592-7) and rat APP770 (NM_019288, NP_062161, P08592-1 and P08592). Examples of APP mutants include Swedish FAD double mutant, London mutant, valine717 to phenylalanine mutant, valine717 to isoleucine mutant and valine717 to glycine mutant.

The term "Aβ" used herein means the term β-amyloid protein, amyloid β protein, β-amyloid peptide, amyloid β peptide or amyloid beta. For example, Aβ is a peptide consisting of about 33-44 amino acids residues in human APP695 amino acid isotype. Preferably, Aβ includes any peptide comprising a part or all of the amino acid residues from positions 597 to 640 in APP, and means every peptide produced from APP by its N-terminal protein degradation and subsequent C-terminal protein degradation. Aβ40 and Aβ42 are peptides comprising 40 amino acid residues and 42 amino acid residues, respectively.

The term "Notch" used herein refers to one of the competing substrates for γ-secretase belonging to the cell surface receptor Notch family. For example, human Notch 1 (AF308602.1), mouse Notch 1 (NM_008714.2) and rat Notch 1 (NM_001105721.1) may be enumerated. Since Notch 1 has an important function in hematopoiesis, inhibition of the processing of Notch 1 may potentially cause immunodeficiency and anemia.

The term "candidate compound" used herein means a compound which is tested in the compound screening method. Although every molecule may be used as a candidate compound, preferably a compound capable of changing the activity of γ-secretase (preferably the activity of γ-secretase of mammals) is used. The candidate compound is one or more compounds contained in expression products of gene libraries; natural or synthetic low molecular weight compound libraries; nucleic acids (oligo DNA or oligo RNA); natural or synthetic peptide libraries; antibodies; substances released from bacteria (including those released from bacteria as a result of metabolism); cell (from microorganisms, plant cells or animal cells) extracts; cell (microorganism, plant cell or animal cell) culture supernatants; purified or partially purified peptides; extracts from marine organisms, plants or animals; soils; or random phage peptide display libraries. The above-described compound may be either a novel compound or a known compound. Further, the above-described compound may be a compound modified by conventional chemical means, physical means and/or biochemical means. For example, the above-desribed compound may be a structural analogue which is obtained by subjecting the initial compound to direct chemical modification (such as acylation, alkylation, esterification or amidation) or random chemical modification. The candidate compound may also be a compound identified by pharmacophore search of the above-described compound or structure comparison programs with computer. The candidate compound may be in the form of a salt. Further, the candidate compound or a salt thereof may be in the form of a solvate (including hydrate).

Further, the candidate compound may be a known γ-secretase accelerator or γ-secretase inhibitor involved in the processing of APP and/or the processing of Notch, or a structural analogue of the above accelerator or inhibitor. A known compound which accelerates or inhibits the activity of γ-secretase and/or the processing of APP and/or the processing of Notch may be a compound that can be designed through rational drug design. For example, DAPT (N-[N-(3,5-difluorophenacetyl)-L-alanyl]-S-phenylglycine t-butyl ester), CM256 (N- [ (1, 1dimethylethoxy) carbonyl]-L-valyl- (4S, 5S) -4-amino-2, 2-difluoro-5-methyl-3-oxoheptanoyl-L-valyl-L-lsoleucine methyl ester) and (2S)-2-{[(3,5-difluorophenyl)acetyl]amino}-N-[(3S)-1-methyl-2-oxo-5-phenyl-2,3-dihydro-1H-1,4-benzodiazepin-3-yl]propanamide (Alexis Biochemicals) may be enumerated.

The expression "compound which affects the processing of c-Ret by γ-secretase" used herein means either a compound which inhibits the c-Ret cleavage activity by γ-secretase (γ-secretase inhibitor) or a compound which accelerates the c-Ret cleavage activity by γ-secretase (γ-secretase accelerator). It should be noted that γ-secretase inhibitor includes antagonist to γ-secretase and that γ-secretase accelerator includes agonist to γ-secretase. γ-Secretase inhibitor and γ-secretase accelerator also include those compounds which alter the cleavage site of c-Ret cleavage products by γ-secretase to thereby produce c-Ret cleavage products with different peptide lengths.

The term "salt" used herein refers to a pharmacologically acceptable salt, and is not particularly limited as long as it forms a pharmacologically acceptable salt with the above-described compound. Preferred examples thereof are hydrohalogenic acid salts (such as hydrofluoride, hydrochloride, hydrobromide or hydroiodide), inorganic acid salts (such as sulfate, nitrate, perchlorate, phosphate, carbonate or hydrogencarbonate), organic carboxylic acid salts (such as acetate, oxalate, maleate, tartrate, fumarate or citrate), organic sulfonic acid salts (such as methanesulfonate, trifluoromethanesulfonate, ethanesulfonate, benzenesulfonate, toluenesulfonate or camphorsulfonate), amino acid salts (such as aspartate or glutamate), quaternary amine salts, alkali metal salts (such as lithium salt, sodium salt or potassium salt) and alkaline earth metal salts (such as magnesium salt or calcium salt).

According to the present invention, there are provided (a) an assay method for examining the cleavage of c-Ret and (b) a method of evaluating whether or not a candidate compound is a compound which affects γ-secretase (screening method) utilizing the assay method. Briefly, the following method is provided as the method of the present invention.

The method of screening for compounds which affect the processing of c-Ret by γ-secretase (which is an embodiment of the present invention) comprises the following steps:
(i) contacting a first biological composition containing γ-secretase or a biologically active fragment thereof with a second biological composition containing c-Ret in the presence and absence of a candidate compound;
(ii) measuring the cleavage of the c-Ret in the presence and absence of the candidate compound;
(iii) selecting those candidate compounds which affect the cleavage of the c-Ret by γ-secretase; and
(iv) identifying the candidate compounds selected in step (iii) as compounds which affect the processing of c-Ret by γ-secretase.

The method of the present invention may be performed in an appropriate cell system containing γ-secretase and c-Ret or a cell-free system containing γ-secretase and c-Ret.

The cell system containing γ-secretase and c-Ret may be either a cell system expressing endogenous genes or a cell system containing an exogenous gene(s). It is possible to contact a first biological composition containing γ-secretase with a second biological composition containing c-Ret by culturing a cell containing γ-secretase and c-Ret in an appropriate medium in the presence and absence of a candidate compound and incubating the cell under reaction conditions which allow the cleavage of c-Ret by γ-secretase activity. If a cell system containing an exogenous gene(s) is used, the above-described contact may be performed under culture conditions which allow the expression of the exogenous gene. It is also possible to apply conditions that allow the cleavage of other γ-secretase substrate, e.g., reaction conditions known to those skilled in the art when the substrate is APP.

Examples of reaction conditions are enumerated below. For cell systems expressing endogenous genes, in the case of primary culture of neurons, culture conditions are in MEM (Invitrogen) medium supplemented with 5% FBS (Hyclone), 1X B27 Supplement (Invitrogen), 0.5 mM L-glutamine (Invitrogen), 25 µg/ml insulin (SIGMA) and 8 µM AraC (SIGMA); under 5% CO₂; and at 37°C. For cell systems containing an exogenous gene(s), in the case of HEK293 cell line, culture conditions are in 10% FBS (Hyclone)/DMEM (Invitrogen), under 5% CO₂and at 37°C.

In cell-free systems, a first biological composition containing γ-secretase or a biologically active fragment thereof (e.g., cell membrane fraction containing γ-secretase) and a second biological composition containing c-Ret (e.g., cell membrane fraction containing c-Ret) may be contacted with each other by incubating these compositions by mixing them in the presence and absence of a candidate compound. These compositions may be mixed under reaction conditions which allow the cleavage of c-Ret by γ-secretase activity, e.g., 10 mM HEPES, pH 7.4, 150 mM NaCl, 10% glycerol, 5 mM EDTA, 5 mM 1,10-phenanthroline, 10 µg/ml phosphoramidon, Complete protease inhibitor cocktail (Roche Biochemicals) (Tomita et al., Molecular Neurodegeneration 2006 1:2). Alternatively, these compositions may be mixed under conditions which allow the cleavage of other γ-secretase substrate, e.g., reaction conditions known to those skilled in the art when the substrate is APP. γ-Secretase or c-Ret may be a purified γ-secretase or c-Ret, a biologically active fragment of γ-secretase or c-Ret, an analogue of γ-secretase or c-Ret, or a mutant of γ-secretase or c-Ret.

The candidate compound may be added generally within a range from approx. 1 nM to 1 mM, usually within a range from approx. 10 µM to 1 mM.

By contacting a first biological composition containing γ-secretase with a second biological composition containing c-Ret as described above, c-Ret cleavage reaction by γ-secretase occurs.

In order to identify a compound which changes the cleavage of c-Ret by γ-secretase, the steps described above are performed in the presence and absence of a candidate compound. Then, the c-Ret cleavage activity of γ-secretase in the presence of the candidate compound is compared with that activity in the absence of the candidate compound (control) to evaluate the effect of the candidate compound upon cleavage activity. By these procedures, it is possible to identify compounds which change the cleavage of c-Ret by γ-secretase.

Even a slight change in the quantity or degree of c-Ret in the presence of a candidate compound indicates that the c-Ret cleavage activity of γ-secretase has been changed in the presence of the candidate compound. Therefore, the candidate compound can be identified as a compound which affects the processing of c-Ret by γ-secretase. For example, a compound which increases c-Ret cleavage product or decreases c-Ret undegraded product compared with control is evaluated as an accelerator for the c-Ret degradation activity of γ-secretase. On the other hand, a compound which decreases c-Ret cleavage product or increases c-Ret undegraded product compared with control is evaluated as an inhibitor for the c-Ret degradation activity of γ-secretase. The accelerator for the degradation activity of γ-secretase obtained by the method of the present invention is potentially useful for treatment of AD.

Analysis of c-Ret cleavage is performed by measuring an indicator of cleavage for one or both of the N-terminal fragment and C-terminal fragment of c-Ret.

As indicators of cleavage, the following antibodies may be used: anti-c-Ret antibodies; antibodies recognizing a c-Ret derivative (c-Ret undegraded product) before degradation by γ-secretase, produced as a result of non-cleavage of c-Ret; antibodies recognizing a c-Ret cleavage product produced as a result of cleavage of c-Ret; or antibodies recognizing the intracellular domain of c-Ret.

When c-Ret is tagged, a substance which binds to the tag (e.g., antibody) may be used to detect the indicator.

For example, when a tagged undegraded product of c-Ret polypeptide is to be detected, an hemagglutinin (HA) tag may be added to the C-terminus of c-Ret, followed by detection with an anti-HA tag antibody. In this case, it is possible to clarify the presence and concentration of a C-terminal fragment of c-Ret produced as c-Ret undegraded product, by detecting or quantitatively determining the HA tag. When c-Ret or tagged c-Ret is labeled, it is possible to detect its undegraded product by detecting or quantitatively determining the label.

On the other hand, when a cleavage product of c-Ret polypeptide is to be detected, the membrane fraction is purified from c-Ret-expressing cells; then, the purified membrane fraction is subjected to cleavage reaction by γ-secretase to thereby allow the c-Ret cleavage product to be released from the membrane fraction. When this reaction product is centrifuged, the c-Ret undegraded product is precipitated into the membrane fraction. Thus, the membrane fraction and other fractions can be separated. By detecting the released fragment, the c-Ret cleavage product can be detected. For this detection, an antibody recognizing the intracellular domain of c-Ret is used when endogenous c-Ret is used. When exogenous recombinant c-Ret is used, a cDNA encoding c-Ret with a tag sequence added to its C-terminus is used to express the recombinant c-Ret. Then, the c-Ret cleavage product is detected using an antibody that recognizes the tag. The tag is not particularly limited. For example, HA, Myc, FLAG or the like may be used.

The antibody to c-Ret is not particularly limited as long as the antibody recognizes c-Ret. Preferably, an antibody which recognizes the intracellular domain of c-Ret is used.

Those skilled in the art could prepare an antibody which recognizes c-Ret by immunizing an animal with an immunogen (antigen) and following the conventional, general procedures for preparing monoclonal antibodies. As the immunogen for example, c-Ret or a fragment thereof, or a fusion protein prepared by adding a tag or label to c-Ret or a fragment thereof may be used.

For example, a non-human mammal is immunized with the immunogen alone or, if necessary, together with Freund's adjuvant. Polyclonal antibodies may be obtained from the serum collected from the immunized animal. Monoclonal antibodies may be obtained by fusing antibody producing cells from the immunized animal with myeloma cells without autoantibody producing ability to prepare fusion cells (hybridomas), cloning the hybridomas, and selecting those clones which produce a monoclonal antibody showing specific affinity to the antigen used for immunizing the animal. The preparation of monoclonal antibodies from hybridomas may be performed *in vitro.* Alternatively, the preparation may be performed *in vivo* in a non-human mammal, preferably mouse or rat, more preferably in the abdominal dropsy in mouse. Monoclonal antibodies may be isolated from the resultant culture supernatant or the abdominal dropsy of the mammal. The isolation and purification of monoclonal antibodies may be performed by subjecting the above-mentioned culture supernatant or abdominal dropsy to methods such as saturated ammonium sulfate precipitation, euglobulin precipitation, caproic acid method, caprilic acid method, ion exchange chromatography (DEAE, DE52, etc.), or affinity column chromatography using anti-immunoglobulin column or protein A column. The monoclonal antibody include those monoclonal antibodies consisting of heavy chains and/or light chains having the amino acid sequences which have deletion, substitution or addition of one or several amino acids in the heavy chains and/or light chains constituting the initial antibody.

By the procedures described above, it is possible to incubate γ-secretase and c-Ret in the presence or absence of a candidate compound and to evaluate the cleavage of c-Ret by γ-secretase.

In another embodiment of the present invention, it is possible to evaluate whether or not a candidate compound affects the processing of APP and/or Notch, in parallel with, simultaneously with, or before or after the above-described method of the present invention.
For example, a step of measuring the cleavage of APP or a polypeptide containing an APP cleavage site by γ-secretase (i.e., polypeptide containing APP γ-secretase cleavage site) may be included in parallel with, simultaneously with, or before or after the above-described method of the present invention. Further, a step of measuring the cleavage of Notch or a polypeptide containing a Notch cleavage site by γ-secretase (i.e., polypeptide containing Notch γ-secretase cleavage site) may be included.

By including such steps, it is possible to evaluate whether or not a candidate compound selectively acts on the processing of c-Ret compared to the processing of APP and/or Notch. The expression "selectively act on" used herein means to have more inhibitory effect or accelerating effect upon the cleavage of substrate c-Ret by γ-secretase than upon the cleavage of other substrate(s). Specifically, according to this embodiment of the present invention, it is possible to identify a compound which selectively acts only on the processing of c-Ret; a compound which selectively acts only on the processing of APP; a compound which selectively acts only on the processing of Notch; or a compound which selectively acts on the processing of APP and c-Ret. This means that, with the method of the present invention, it is possible to obtain compounds which selectively inhibit or activate the γ-secretase's cleavage activity on c-Ret in addition to γ-secretase's known substrates such as APP or Notch. Therefore, it is possible to obtain compounds which selectively inhibit or activate the cleavage function of γ-secretase on a specific substrate; compounds with increased selectivity on the substrate of γ-secretase can be obtained.

As a candidate compound in drug development, a compound which acts in the same manner as metabolic activity in healthy animals *in vivo* or a compound which regulates the metabolic activity is preferable. A preferable example of such a candidate compound is a compound which inhibits the production of Aβ42 by inhibiting the APP cleavage activity of γ-secretase, does not inhibit the c-Ret cleavage activity of γ-secretase, and does not inhibit the Notch cleavage activity of γ-secretase. Another preferable example is a compound which inhibits the production of Aβ42 by accelerating the production of Aβ40 through acceleration of the APP cleavage activity of γ-secretase; accelerates the degradation of c-Ret by accelerating the c-Ret cleavage activity of γ-secretase; and does not inhibit the Notch cleavage activity of γ-secretase.

As methods for measuring the cleavage by γ-secretase of APP, Notch or a polypeptide containing an APP or Notch γ-secretase cleavage site, assay methods known to those skilled in the art may be applicable (Song et al. PNAS 1999 96 6959-6963; Moehlmann et al. PNAS 2002 99 8025-8030). For example, a first biological composition containing γ-secretase or a biologically active fragment thereof may be contacted with a biological composition containing APP or a polypeptide containing an APP γ-secretase cleavage site or a biological composition containing Notch or a polypeptide containing a Notch γ-secretase cleavage site in the presence and absence of a candidate compound, and then the cleavage of the APP or the polypeptide containing an APP γ-secretase cleavage site or the cleavage of the Notch or the polypeptide containing a Notch γ-secretase cleavage site.
This measurement may be performed by measuring the cleavage product from the APP or the polypeptide containing an APP γ-secretase cleavage site or the cleavage product from the Notch or the polypeptide containing a Notch γ-secretase cleavage site. As one example of the cleavage product from APP or a polypeptide containing an APP γ-secretase cleavage site, Aβ may be given. The quantity of Aβ may be measured, and changes in the quantity between the presence and absence of the candidate compound may be compared. Alternatively, the degree of cleavage and the quantity of cleavage product may be measured by using a known antibody which recognizes the cleavage product from APP or a polypeptide containing an APP γ-secretase cleavage site or the cleavage product from Notch or a polypeptide containing a Notch γ-secretase cleavage site. As the antibody which recognizes the cleavage product from APP or a polypeptide containing an APP γ-secretase cleavage site, commercial antibodies (Sigma or Chemicon) may be used. The measurement may be performed, for example, by Western blotting. As the antibody which recognizes the cleavage product from Notch or a polypeptide containing a Notch γ-secretase cleavage site, commercial antibodies (Santacruze) may be used. The measurement may be performed, for example, by Western blotting.

The method of the present invention also includes a high through put screening (HTS) known to those skilled in the art, which tests a large number of compounds simultaneously (US5876946; US5902732; Jayawickreme and Kost, Curr. Opin. Biotechnol., 8, pp. 629-634, 1997; Houston and Banks, Curr. Opin. Biotechnol., 8, pp. 734-740, 1997).

The method of the present invention also includes the use of known model animals.
It is possible to analyze the *in vivo* effect of a compound selected by the *in vitro* method of the present invention by using, for example, non-human models for APP processing and/or AD. As non-human models for AD, APP transgenic non-human animal models are well-known in the art. As a method of using a model animal, Tg2576 mouse (J. Neurosci. 21(2), 372-381, 2001; J. Clin. Invest., 112, 440-449, 2003) may be used, and the following evaluations may be made after administering to Tg2576 mouse a known γ-secretase inhibitor DAPT, (2S)-2-{[(3,5-difluorophenyl)acetyl]amino}-N-[(3S)-1-methyl-2-oxo-5-phenyl-2,3-dihydro-1H-1,4-benzodiazepin-3-yl]propanamide (Alexis Biochemicals) or a compound of the present invention: evaluation by a method of measuring the Aβ quantities in the brain, cerebrospinal fluid and serum of the mouse (J. Pharmacol. Exp. Ther. 305, 864-871, 2003); pathological examination of changes in the brain (e.g., changes in Aβ yield, the degree of cerebral atrophy, etc.) resulted from changes in γ-secretase activity; and evaluation of the survival ratio, momentum or food consumption of the mouse.

The pharmaceutical composition comprising the compound identified by the method of the present invention, preferably the AD therapeutic of the present invention, may be administered to patients in various forms through an oral or parenteral (e.g., intravenous injection, muscle injection, subcutaneous administration, rectal administration or transdermal administration) route. Therefore, the pharmaceutical composition comprising the compound of the present invention may be formulated into various preparations using a pharmacologically acceptable carrier by a conventional method depending on the administration route, though the pharmaceutical composition may be used alone.

Preferred dosage forms include oral preparations such as tablets, powders, subtle granules, granules, coated tablets, capsules, syrups and troches; and parenteral preparations such as inhalants, suppositories, injections (including drops), ointments, eye drops, ophthalmic ointments, nasal drops, ear drops, cataplasms, and lotions and liposomes.

Examples of carriers used in the formulation include conventionally used fillers, binders, disintegrants, lubricants, coloring agents and flavoring agents, as well as stabilizers, emulsifiers, absorbefacients, surfactants, pH adjusting agents, antiseptics, antioxidants, expanders, wetting agents, surface activators, dispersing agents, buffers, preservatives, dissolution aids and analgesic agents according to necessity. They can be formulated according to a conventional procedure using components commonly used as raw materials for pharmaceutical preparations. Examples of nontoxic these components which may be used in the present invention include animal and vegetable oils such as soybean oil, beef tallow and synthetic glycerides; hydrocarbons such as liquid paraffins, squalane and solid paraffins; ester oils such as octyldodecyl myristate and isopropyl myristate; higher alcohols such as cetostearyl alcohol and behenyl alcohol; silicone resins; silicone oils; surfactants such as polyoxyethylene fatty acid esters, sorbitan fatty acid esters, glycerin fatty acid esters, polyoxyethylene sorbitan fatty acid esters, polyoxyethylene hydrogenated castor oils and polyoxyethylene-polyoxypropylene block copolymers; water-soluble polymers such as hydroxyethyl cellulose, polyacrylic acids, carboxyvinyl polymers, polyethylene glycol, polyvinylpyrrolidone and methylcellulose; lower alcohols such as ethanol and isopropanol; polyhydric alcohols (polyols) such as glycerol, propylene glycol, dipropylene glycol, sorbitol and polyethylene glycol; sugars such as glucose and sucrose; inorganic powders such as silicic anhydride, magnesium aluminium silicate and aluminium silicate; inorganic salts such as sodium chloride and sodium phosphate; and purified water.

The fillers include, for example, lactose, fructose, corn starch, white sugar, glucose, mannitol, sorbitol, crystalline cellulose and silicon dioxide. The binders include, for example, polyvinyl alcohol, polyvinyl ether, methylcellulose, ethylcellulose, gum arabic, gum tragacanth, gelatin, shellac, hydroxypropyl methylcellulose, hydroxypropyl cellulose, polyvinylpyrrolidone, polypropylene glycol-polyoxyethylene block polymers and meglumine. The disintegrants include, for example, starch, agar, gelatin powder, crystalline cellulose, calcium carbonate, sodium hydrogencarbonate, calcium citrate, dextrin, pectin and carboxymethylcellulose calcium. The lubricants include, for example, magnesium stearate, talc, polyethylene glycol, silica and hardened vegetable oils. The coloring agents may be any coloring agents which are approved to be added to pharmaceutical preparations. The flavoring agents include, for example, cocoa powder, menthol, aromatic powder, peppermint oil, camphol and cinnamon powder. The above-listed components may be in the form of a salt or solvate thereof.

The oral preparation is produced by mixing the compound of the present invention with a filler, and if necessary, a binder, disintegrant, lubricant, coloring agent, flavoring agent, etc. and formulating the mixture according to conventional procedures into, for example, a powder, subtle granules, granules, tablet, coated tablet, capsules or the like. Resultant tablets and granules can be appropriately coated with, for example, sugar according to necessity. The syrups and injection preparations can be prepared according to conventional procedures by adding a pH adjusting agent, solubilizer, and isotonizing agent, and if necessary, a dissolution aid, stabilizer, etc. The external preparations can be produced according to conventional procedures not specifically limited. Base materials which may be used in the present invention include various raw materials conventionally used in pharmaceutical preparations, quasi drugs and cosmetics. Such raw materials include, for example, animal and vegetable oils, mineral oils, ester oils, waxes, higher alcohols, fatty acids, silicone oils, surfactants, phospholipids, alcohols, polyhydric alcohols, water-soluble polymers, clay minerals and purified water. If necessary, pH adjusting agents, antioxidants, chelating agents, antiseptics and antimolds, coloring agents, flavors, or the like can be added.
In addition, components such as blood-flow accelerators, bactericides, anti-inflammatory agents, cell activators, vitamins, amino acids, humectants, keratolytic agents or the like be added according to necessity. The ratio of the active ingredient to carriers may vary from 1 to 90% by weight. When the compounds used in the present invention, the peptides used in the present invention or the polynucleotides used in the present invention are used in the above-described treatment, it is preferable to use those compounds, peptides or polynucleotides purified to 90% or more, preferably 95% or more, more preferably 98% or more, still more preferably 99% or more.

The effective dose of the pharmaceutical composition comprising the compound of the present invention varies depending on the severity of symptom, the age, sex and body weight of the patient, administration mode, type of the salt, specific type of the disease and other factors. Generally, the pharmaceutical composition may be administered to an adult (body weight: 60 kg) in one to several divided doses at a daily dose of about 30 µg to about 10 g, preferably 100 µg to 5 g, and more preferably 100 µg to 100 mg for oral administration; or at a daily dose of about 30 µg to about 1 g, preferably 100 µg to 500 mg, and more preferably 100 µg to 30 mg for injection administration. Considering that efficacy varies depending on the administration route, the required dose is expected to vary widely. For example, it is expected that oral administration requires a higher dose than intravenous injection. When administered to children, the dose may be smaller than the dose for adults. These variations in the dose level can be adjusted by standard empirical optimization procedures which are well understood in the industry.

The term "treatment" is herein to generally mean obtaining a desired pharmacological and/or physiological effect. The effect may be prophylactic in terms of completely or partially preventing a disease and/or a symptom and may be therapeutic in terms of partially or completely curing a disease and/or an adverse effect attributed to the disease. The term "treatment" as used herein covers any treatment of a disease in a patient, preferably a human, and includes at least one treatment selected from the following (a) to (c):
(a) preventing a disease or a symptom from occurring in a patient who may be predisposed to the disease but has not yet been diagnosed as having it;
(b) inhibiting a disease symptom, i.e. preventing or delaying its progress; or
(c) relieving a disease symptom, i.e. causing regression or elimination of the disease or symptom, or causing reversal of the progress of the disease.

For example, as clinical symptoms of AD, progressive disorientation, memory loss and aphasia are enumerated. Finally, disablement, speech loss and akinesia occur. Pathological signs of AD include neurofibrillary tangle, senile plaques and amyloid angiopathy. To prevent the progress of AD is interpreted to mean to prevent the onset or further progress of the clinical symptoms and/or pathological signs of AD. For example, in patients who do not have the clinical symptoms or pathological signs of AD, it is possible to prevent the progress of clinical symptoms or pathological signs. In patients suffering from mild AD, it is possible to prevent the development of more severe AD forms. To delay the progress of AD is interpreted to mean to delay the point of onset of AD-related symptoms and/or pathological signs, or to reduce the speed of progress of AD that is determined by the speed of progress of clinical symptoms and pathological signs. To reverse the progress of AD is interpreted to mean to relieve the severity of AD symptoms, i.e., to change the severity of AD conditions of patients from severe to mild. At that time, the change to mild is indicated by decrease of clinical symptoms or pathological signs.

Diagnosis of AD in patients may be performed by various known methods. Typically, AD is diagnosed by combining clinical and pathological assessments. For example, the progress or severity of AD may be judged using Mini Mental State Examination (MMSE) (Mohs et al. (1996) Int Psychogeriatr 8: 195-203), Alzheimer's Disease Assessment Scale-Cognitive Subscale (ADAS-cog) (Galasko et al., (1997) Alzheimer Dis Assoc Disord, 11 suppl 2: S33-9), Alzheimer's Disease Cooperative Study-Activities of Daily Living (ADCS-ADL) (McKhann et al., (1984) Neurology 34: 939-944) and Criteria of National Institute of Neurologic Communicative Disorders and Stroke-Alzheimer's Disease and Related Disorders Association (NINCDS-ADRDA) (Folstein et al., (1975) J Psychiatr Res 12: 189-198; McKhann et al., (1984) Neurology 34: 939-944). Further, methods which evaluate various regions of the brain and enable the estimation of frequency of senile plaques or neurofibrillary tangle may be used (Braak et al., (1991) Acta Neuropathol 82: 239-259; Khachaturian (1985) Arch Neuro 42: 1097-1105; Mirra et al., (1991) Neurology 41: 479-486; and Mirra et al., (1993) Arch Pathol Lab Med 117: 132-144).

The present invention provides a test kit for measuring the processing of c-Ret by γ-secretase as a test kit for use in the method of the present invention.

The kit of the present invention comprises at least γ-secretase or a biological composition containing γ-secretase, and a biological composition containing c-Ret. The kit may further comprise a substrate for γ-secretase other than c-Ret (e.g., APP and/or Notch) or a biological composition containing such a substrate. Preferably, the kit comprises a plurality of substrates for γ-secretase. It is preferred that the kit comprises APP and/or Notch in addition to c-Ret.

Further, the test kit of the present invention may comprise tools, reagents, instructions, and so forth which are used in technologies for detecting the processing of c-Ret by γ-secretase. Examples of technologies for detecting the processing of c-Ret by γ-secretase include immunoblotting and Western blotting. Examples of tools used in these technologies include reaction vessels and blotting membranes; and examples of reagents used in these technologies include buffers, culture broths and anti-c-Ret antibodies.

With the kit of the present invention, it is possible to measure the processing of c-Ret by γ-secretase. As a result, it becomes possible to evaluate whether or not a candidate compound affects the processing of c-Ret by γ-secretase. Therefore, this kit can serve as a test kit for screening for compounds which affect the processing of c-Ret by γ-secretase. The test kit of the present invention includes a test kit for screening for compounds which affect the processing of c-Ret by γ-secretase, the kit having the same constitution as that of the test kit for measuring the processing of c-Ret by γ-secretase.

Further, the present invention includes the use of the above-described kit in measuring the processing of c-Ret, or in methods of screening or testing γ-secretase inhibitors.

### EXAMPLES

Hereinbelow, the present invention will be described in more detail with reference to the following Examples and Preparation Examples. However, the present invention is not limited to these Examples, which are provided only for the purpose of full disclosure of the present invention to those skilled in the art. It is not meant or even implied that the experiments described herein are all or only one experiment actually carried out. Although efforts have been made to guarantee the accuracy of the numerical values used herein (e.g., volume, temperature, concentration, etc.), experimental errors and deviations are considered to some extent. Thus, such values may be changed within a range which does not depart from the scope of the present invention.

### Example 1: Analysis of c-Ret Processing in c-Ret-Transfected 293/EBNA-1 Cell Strain

Whether or not c-Ret is a substrate for γ-secretase was evaluated using HEK293 cells expressing a gene encoding c-Ret with an HA tag added to its C-terminus, in the presence of a γ-secretase inhibitor. As the γ-secretase inhibitor, (2S)-2-{[(3,5-difluorophenyl)acetyl]amino}-N-[(3S)-1-methyl-2-oxo-5-phenyl-2,3-dihydro-1H-1,4-benzodiazepin-3-yl]propanamide (hereinafter, sometimes referred to as "Compound E") (Alexis Biochemicals) was used.

### 1. Experimental Conditions and Methods

### (1) Cloning of Rat c-Ret

RNA was purified from rat brain with Trisol (Invitrogen), followed by synthesis of 1st strand cDNA with RNA PCR Kit (TaKaRa). *c-Ret* was amplified in two separate fragments of 1-767 bp (positions from 1 to 767 in the nucleotide sequence as shown in SEQ ID NO: 9) and 1681 bp-stop (positions from 1681 to 3351 in the nucleotide sequence as shown in SEQ ID NO: 9). Specifically, *c-Ret* was amplified using 1 µl of the finally synthesized 1 st strand cDNA product, the following primers and Pfu (Stratagene).
Primer 1 - SalI site added: GAGGTCGACGCCACCATGGCGAAAGCGAGGTCCGGCGC (SEQ ID NO: 31)
Primer 2: ACGGAGACAGTCCTGGGGGCAAA (SEQ ID NO: 32)
Primer 3: TCTCCTAGCACCAGGACCTGTCC (SEQ ID NO: 33)
Primer 4 - NotI site added: GAGGCGGCCGCGCTATCAAATGTGTCCATTAATTT (SEQ ID NO: 34)

PCR conditions were as follows: first reaction at 95°C for 2 minutes; then 35 cycles of (at 95°C for 45 seconds →at 60°C for 45 seconds→at 72°C for 6 minutes); then final reaction at 72°C for 10 minutes. The PCR products were purified with Quiaquick PCR purification kit (QIAGEN). Then, the 1-767 bp fragment was treated with restriction enzymes SalI (TaKaRa) and EcoRV (TaKaRa), and the 1681 bp-stop fragment was treated with EcoRV (TaKaRa) and NotI (TaKaRa). Subsequently, they were cloned into pBluescript (Stratagene), separately. The resultant pBluescripts were subjected to sequence analysis with a DNA sequencer (Applied Biosystems; 3130x1). The resultant pBluescript 1-767 bp was treated with restriction enzymes EcoRV and NotI. Then, the 1681 bp-stop fragment was inserted thereto.

### (2) Construction of a Gene Encoding Rat c-Ret with HA Tag Added to Its C-Terminus and an Expression Vector

pcDNA (Invitrogen) was treated with restriction enzymes SalI (TaKaRa) and NotI (TaKaRa). c-Ret obtained by treating the pBluescript obtained in (1) above with restriction enzymes SalI (TaKaRa) and NotI (TaKaRa) was inserted into the resultant pcDNA to thereby prepare an expression vector. This expression vector was constructed so that an HA tag is added to the C-terminus of the incorporated c-Ret. The DNA sequence of the resultant c-Ret-HA was analyzed with a DNA sequencer (Applied Biosystems; 3130x1). The resultant DNA sequence is shown in SEQ ID NO: 13.

In the resultant DNA sequence, the nucleotide at position 89 was changed from g to a, compared with rat c-Ret (NM_012643; SEQ ID NO: 9). (Hereinafter, this mutation is expressed as "g→a". Other mutations will also be expressed in the same manner.) Besides, when compared with rat c-Ret (NM_012643), the resultant DNA sequence had g→a mutation at position 270, c→g mutation at position 435, a→g mutation at position 504, t→g mutation at position 675, a→g mutation at position 684, t→g mutation at position 693, g→a mutation at position 1070, g→c mutation at position 1095, t→g mutation at position 1168, g→c mutation at position 1422, a→g mutation at position 1497, c→a mutation at position 1581, c→t mutation at position 1658, a→g mutation at position 1809, c→g mutation at position 1886, a→g mutation at position 2249, t→a mutation at position 2754, a→g mutation at position 2767, t→g mutation at position 2769, c→t mutation at position 2777, t→a mutation at position 2785, a→g mutation at position 2835, a→g mutation at position 2926 and t→c mutation at position 3195, with a deletion of tga at positions from 798 to 800. With respect to the relation of nucleotide positions when the DNA sequences of c-Ret-HA (SEQ ID NO: 13) and rat c-Ret (NM_012643; SEQ ID NO: 9) are compared, the nucleotide positions of NM_012643 (SEQ ID NO: 9) are regarded as reference. Therefore, in the nucleotide sequence of SEQ ID NO: 13, nucleotides at position 798 and thereafter are shifted by three nucleotides compared with the nucleotide sequence of SEQ ID NO: 9 because of the above-described deletion. For example, the position corresponding to position 1070 in SEQ ID NO: 9 is 1067 in SEQ ID NO: 13. Further, the unidentified nucleotides in NM_012643 were identified as follows: position 1485 was c; position 1997 was c; position 2000 was a; position 2001 was c; position 2015 was c; position 2022 was c; position 2043 was g; position 2052 was c; position 2080 was a; and position 2735 was a. At the amino acid level, the sequence had C→Y mutation (mutation from C to Y) at position 30, R→K mutation at position 357, F→V mutation at position 390, T→I mutation at position 553, A→G mutation at position 629, N→K mutation at position 918, N→E mutation at position 923, S→F mutation at position 926, F→I mutation at position 929 and N→D mutation at position 976, with a deletion of D at position 266 because of the above-described tga deletion at positions 798-800. Further, the unidentified amino acid residues were identified as follows: position 495 was R; position 666 was A; position 667 was H; position 672 was A, position 674 was A; position 681 was P; position 684 was G; position 694 was T; and position 912 was K. The expression vector was prepared in large quantity with Endofree Plasmid Maxi Kit (QIAGEN). It should be noted here that when the amino acid sequence of c-Ret-HA (SEQ ID NO: 14) is compared with that of rat c-Ret (NM_012643; SEQ ID NO: 10), the amino acid sequence from position 266 and thereafter of SEQ ID NO: 14 is shifted by one amino acid relative to the amino acid sequence as shown in SEQ ID NO: 10.

The 3' terminal sequence (from g at 3355 to t at 3384) of the nucleotide sequence as shown in SEQ ID NO: 13 is a nucleotide sequence encoding the HA tag.

### (3) Cells Expressing the Gene Encoding Rat c-Ret with HA Tag Added to Its C-Terminus

293/EBNA-1 cell strain (Invitrogen) was cultured in 6-well dishes containing 10% FBS (Hyclone)/DMEM (Invitrogen) under 5% CO₂ at 37°C, followed by transfection thereinto of the gene encoding rat c-Ret with an HA tag added to its C-terminus using Lipofectamine 2000 (Invitrogen). After one day culture under the same conditions, 50 mM Compound E (Alexis Biochemicals) was added to the culture broth. Cells were cultured for another day under the same conditions. Then, the transfected HEK293 cells were collected with PBS (Sigma) and sonicated with a sonicator (Taitec VP-5S) to disrupt cells. Then, the quantity of protein was determined with Protein Assay Kit (BioRad). Samples (2 µg each) were taken from proteins obtained from Compound E-added cells and Compound E-not added cells, respectively, and subjected to SDS-PAGE, followed by Western blotting with an anti-HA antibody (Roche) (final concentration: 0.2 µg/ml).

### 2. Experimental Results

Fig. 1 shows the results.

In Fig. 1, the left lane represents the sample untreated with Compound E and the right lane represents the sample treated with Compound E. "Full" represents the full-length of c-Ret before degradation (about 150 kDa). CTF (C terminal fragment) represents a region spanning from the transmembrane domain of c-Ret to the C terminal domain. When Compound E (γ-secretase inhibitor) was added, a band around 50 kDa (CTF) was accumulated specifically as a c-Ret undegraded product. Since this band is equal in size to the region spanning from the transmembrane domain of c-Ret to its C-terminal domain, it has become clear that c-Ret is cleaved by γ-secretase in HEK293 cells.

The technical terms used herein are used only for the purpose of illustrating a specific embodiment and not intended to limit the embodiment.

Unless otherwise specifically defined, all technical terms and scientific terms used herein have the same meaning as generally understood by those skilled in the art. Although any methods and materials similar to or equivalent to those described herein may be used in the practice or test of the present invention, those skilled in the art can consult the description provided herein, for preferable methods and materials.

All publications cited herein are incorporated herein by reference in their entirety for the purpose of describing and disclosing, for example, the cell systems, constructs and methods described in the publications that are used in connection with the present invention, or incorporated herein as references with respect to the disclosure of the compound identification method, screening method and methodologies therefor, and composition of the present invention; such publications may be used for the practice of the present invention.

## Claims

1. A method of screening for compounds which affect the processing of c-Ret by γ-secretase, comprising the following steps:
(i) contacting a first biological composition containing γ-secretase or a biologically active fragment thereof with a second biological composition containing c-Ret in the presence and absence of a candidate compound;
(ii) measuring the cleavage of the c-Ret in the presence and absence of the candidate compound;
(iii) selecting those candidate compounds which affect the cleavage of the c-Ret by γ-secretase; and
(iv) identifying the candidate compounds selected in step (iii) as compounds which affect the processing of c-Ret by γ-secretase.

2. The method of claim 1, further comprising evaluating a candidate compound as an inhibitor for the c-Ret degradation activity of γ-secretase when c-Ret undegraded product in the presence of said candidate compound was increased relative to c-Ret undegraded product in the absence of said candidate compound in the cleavage of c-Ret measured in step (ii).

3. The method of claim 1, further comprising evaluating a candidate compound as an accelerator for the c-Ret degradation activity of γ-secretase when c-Ret undegraded product in the presence of said candidate compound was decreased relative to c-Ret undegraded product in the absence of said candidate compound in the cleavage of c-Ret measured in step (ii).

4. The method of claim 1, further comprising evaluating a candidate compound as an accelerator for the c-Ret degradation activity of γ-secretase when c-Ret cleavage product in the presence of said candidate compound was increased relative to c-Ret cleavage product in the absence of said candidate compound in the cleavage of c-Ret measured in step (ii).

5. The method of claim 1, further comprising evaluating a candidate compound as an inhibitor for the c-Ret degradation activity of γ-secretase when c-Ret cleavage product in the presence of said candidate compound was decreased relative to c-Ret cleavage product in the absence of said candidate compound in the cleavage of c-Ret measured in step (ii).

6. The method of any one of claims 1 to 5, further comprising measuring the cleavage of APP or a polypeptide comprising an APP γ-secretase cleavage site.

7. The method of any one of claims 1 to 5, further comprising measuring the cleavage of Notch or a polypeptide comprising a Notch γ-secretase cleavage site.

8. A test kit for measuring the processing of c-Ret by γ-secretase, comprising a first biological composition containing γ-secretase or a biologically active fragment thereof and a second biological composition containing c-Ret.
